(19) Europäisches Patentamt
European Patent Office
Office européen des brevets

(11) EP 0 486 948 B1

(12) EUROPEAN PATENT SPECIFICATION

(45) Date of publication and mention of the grant of the patent:
**04.10.2000 Bulletin 2000/40**

(21) Application number: **91119464.5**

(22) Date of filing: **04.11.1991**

(51) Int. Cl.$^7$: **C07D 213/26**, C07D 213/30, C07D 213/40, C07K 5/06, A61K 38/55, C07D 213/56, C07D 211/16, C07D 295/20, C07D 277/30, C07D 277/42, C07D 417/12, C07D 277/40, C07D 401/12, C07D 277/24, C07D 277/28, C07D 235/14, C07D 235/16

# (54) Retroviral protease inhibiting compounds

Inhibitoren retroviraler Proteasen

Inhibiteurs de protéases rétrovirales

(84) Designated Contracting States:
**AT BE DE DK FR GB GR LU NL SE**

(30) Priority: **20.11.1990 US 616170**
**15.08.1991 US 746020**
**23.10.1991 US 777626**

(43) Date of publication of application:
**27.05.1992 Bulletin 1992/22**

(60) Divisional application:
**00101297.0 / 0 997 459**

(73) Proprietor: **ABBOTT LABORATORIES**
**Abbott Park, Illinois 60064-3500 (US)**

(72) Inventors:
- **Kempf, Dale J.**
  **Libertyville, Illinois 60048 (US)**
- **Codacovi, Lynn M.**
  **Antioch, Illinois 60002 (US)**
- **Norbeck, Daniel W.**
  **Lindenhurst, Illinois 60046 (US)**
- **Plattner, Jacob J.**
  **Libertyville, Illinois 60048 (US)**
- **Sham, Hing L.**
  **Gurnee, Illinois 60031 (US)**
- **Wittenberger, Steven J.**
  **Mundelein, Illinois 60060 (US)**
- **Zhao, Chen**
  **Gurnee, Illinois 60031 (US)**

(74) Representative:
**Modiano, Guido, Dr.-Ing. et al**
**Modiano & Associati SpA**
**Via Meravigli, 16**
**20123 Milano (IT)**

(56) References cited:
**EP-A- 0 337 714**

- **JOURNAL OF THE CHEMICAL SOCIETY, CHEMICAL COMMUNICATIONS no. 2, 15 January 1991, LETCHWORTH GB pages 110 - 112 H.L. SHAM ET AL. 'Synthesis of (2S,5S,4R) 2,5-diamino-3,3-difluoro-1,6-diphenyl-4-hydroxyhexane: the core unit of a potent HIV proteinase inhibitor.'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 486 948 B1

## Description

### Technical Field

This invention was made with Government support under contract number AI27220 awarded by the National Institute of Allergy and Infectious Diseases. The Government has certain rights in this invention.

The present invention relates to novel compounds and a composition and method for inhibiting retroviral proteases and in particular for inhibiting human immunodeficiency virus (HIV) protease, a composition and method for treating a retroviral infection and in particular an HIV infection, processes for making such compounds and synthetic intermediates employed in these processes.

### Background Art

Retroviruses are those viruses which utilize a ribonucleic acid (RNA) intermediate and a RNA-dependent deoxyribonucleic acid (DNA) polymerase, reverse transcriptase, during their life cycle. Retroviruses include, but are not limited to, the RNA viruses of the Retroviridae family, and also the DNA viruses of the Hepadnavirus and Caulimovirus families. Retroviruses cause a variety of disease states in man, animals and plants. Some of the more important retroviruses from a pathological standpoint include human immunodeficiency viruses (HIV-1 and HIV-2), which cause acquired immune deficiency syndrome (AIDS) in man, hepatitis B virus, which causes hepatitis and hepatic carcinomas in man, human T-cell lymphotrophic viruses I, II, IV and V, which cause human acute cell leukemia, and bovine and feline leukemia viruses which cause leukemia in domestic animals.

Proteases are enzymes which cleave proteins at specific peptide bonds. Many biological functions are controlled or mediated by proteases and their complementary protease inhibitors. For example, the protease renin cleaves the peptide angiotensinogen to produce the peptide angiotensin I. Angiotensin I is further cleaved by the protease angiotensin converting enzyme (ACE) to form the hypotensive peptide angiotensin II. Inhibitors of renin and ACE are known to reduce high blood pressure in vivo. An inhibitor of a retroviral protease should provide a therapeutic agent for diseases caused by the retrovirus.

The genomes of retroviruses encode a protease that is responsible for the proteolytic processing of one or more polyprotein precursors such as the pol and gag gene products. See Wellink, Arch. Virol. 98 1 (1988). Retroviral proteases most commonly process the gag precursor into core proteins, and also process the pol precursor into reverse transciptase and retroviral protease. In addition, retroviral proteases are sequence specific. See Pearl, Nature 328 482 (1987).

The correct processing of the precursor polyproteins by the retroviral protease is necessary for the assembly of infectious virions. It has been shown that in vitro mutagenesis that produces protease-defective virus leads to the production of immature core forms which lack infectivity. See Crawford, J. Virol. 53 899 (1985); Katoh, et al., Virology 145 280 (1985). Therefore, retroviral protease inhibition provides an attractive target for antiviral therapy. See Mitsuya, Nature 325 775 (1987).

EP-A-0 337 714 discloses HIV protease inhibitors useful for the treatment of AIDS.

H. L. Sham et al., J. Chem. Soc. Chem. Commun., no. 2, 15 January 1991, pp. 110-112 disclose (2S, 5S, 4R)-2,5-Diamino-3,3-difluoro-1,6-diphenylhydroxyhexane as the core unit of a potent HIV proteinase inhibitor.

Current treatments for viral diseases usually involve administration of compounds that inhibit viral DNA synthesis. Current treatments for AIDS (Dagani, Chem. Eng. News, November 23, 1987 pp. 41-49) involve administration of compounds such as 2',3'-dideoxycytidine, 2',3'-dideoxyinosine, trisodium phosphonoformate, ammonium 21-tungsto-9-antimoniate, 1-beta-D-ribofuranosyl-1,2,4-triazole-3-carboxamide, 3'-azido-3'-deoxythymidine, and adriamycin that inhibit viral DNA synthesis; compounds such as AL-721 and polymannoacetate which may prevent HIV from penetrating the host cell; and compounds which treat the opportunistic infections caused by the immunosuppression resulting from HIV infection. None of the current AIDS treatments have proven to be totally effective in treating and/or reversing the disease. In addition, many of the compounds currently used to treat AIDS cause adverse side effects including low platelet count, renal toxicity and bone marrow cytopenia.

### Disclosure of the Invention

In accordance with the present invention, there are retroviral protease inhibiting compounds of the formula:

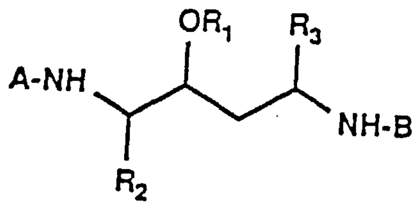

wherein $R_1$ is hydrogen and $R_2$ and $R_3$ are independently selected from arylalkyl, cycloalkylalkyl and (heterocyclic)alkyl;
A and B are independently selected from $R_6$-C(O)-(NH)-(CH($R_5$))-C(O)- and $R_6$-C(O)- wherein at each occurrence $R_5$ is independently selected from alkyl and at each occurrence $R_6$ is independently selected from $R_7$-NH-, $R_7$-N(alkyl)-, $R_7$-O-and $R_7$-S- wherein $R_7$ is heterocyclic or (heterocyclic)alkyl and $R_7$ on the heterocyclic ring may be optionally substituted with one or two substituents independently selected from hydroxy, halo, oxo, alkylimino, amino, (N-protected)amino, alkylamino, (N-protected)alkylamino, dialkylamino, alkoxy, benzyloxy, polyalkoxy, haloalkyl, cycloalkyl, aryl, arylalkyl, -COOH, -$SO_3$H and alkyl, and a nitrogen containing heterocyclic in $R_7$ may be N-protected; or a pharmaceutically acceptable salt, prodrug or ester thereof.

Preferred compounds of the invention are compounds of the formula:

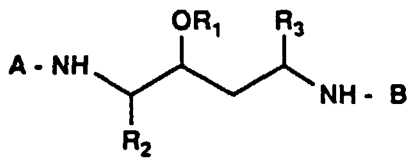

wherein $R_1$ is hydrogen,
$R_2$ and $R_3$ are independently selected from (aryl)alkyl,
A is $R_6$-C(O)-NH-CH($R_{5a}$)-C(O)- wherein $R_{5a}$ is alkyl and $R_6$ is $R_7$-$R_9$- wherein $R_7$ is heterocyclic or (heterocyclic)alkyl and $R_9$ is -N($R_{7a}$)-, S or O wherein $R_{7a}$ is hydrogen or alkyl and
B is -C(O)-$R_{6'}$ wherein $R_{6'}$ is $R_{7'}$-$R_{9'}$- wherein $R_{7'}$ is heterocyclic or (heterocyclic)alkyl and $R_{9'}$ is -N($R_{7a'}$)-, S or O wherein $R_{7a'}$ is hydrogen or alkyl;
or a pharmaceutically acceptable salt, prodrug or ester thereof.

Most preferred compounds of the invention also are compounds of the formula:

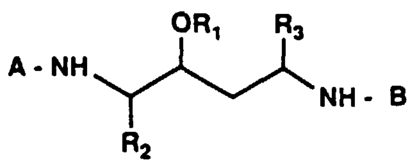

wherein $R_1$ is hydrogen,
$R_2$ and $R_3$ are independently selected from (aryl)alkyl,
A is -C(O)-$R_{6'}$ wherein $R_{6'}$ is $R_{7'}$-$R_{9'}$- wherein $R_{7'}$ is heterocyclic or (heterocyclic)alkyl and $R_{9'}$ is -N($R_{7a'}$)-, S or O wherein $R_{7a'}$ is hydrogen or alkyl and

B is $R_6$-C(O)-NH-CH($R_{5a}$)-C(O)- wherein $R_{5a}$ is alkyl and $R_6$ is $R_7$-$R_9$- wherein $R_7$ is heterocyclic or (heterocyclic)alkyl and $R_9$ is -N($R_{7a}$)-, S or O wherein $R_{7a}$ is hydrogen or alkyl;
or a pharmaceutically acceptable salt, prodrug or ester thereof.

Most preferred compounds of the invention also are compounds of the formula:

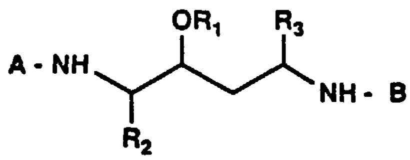

wherein $R_1$ is hydrogen,
$R_2$ and $R_3$ are independently selected from (aryl)alkyl,
and A and B are independently is -C(O)-$R_6$ wherein $R_6$ is $R_7$-$R_9$-wherein at each occurrence $R_7$ is independently selected from heterocyclic and (heterocyclic)alkyl and at each occurrence $R_9$ is independently selected from -N($R_{7a}$)-, S and O wherein $R_{7a}$ is hydrogen or alkyl;
or a pharmaceutically acceptable salt, prodrug or ester thereof.

The compounds of the invention comprise asymmetrically substituted centers. Such centers can be racemic or asymmetric. Racemic mixtures, mixtures of diastereomers, as well as single diastereomers of the compounds of the invention are included in the present invention. The terms "S" and "R" configuration are as defined by the IUPAC 1974 Recommendations for Section E, Fundamental Stereochemistry, Pure Appl. Chem. (1976) 45, 13 - 30.

The terms "Ile", "Val" and "Thr" as used herein refer to isoleucine, valine and threonine, respectively. In general, the amino acid abbreviations used herein follow the IUPAC-IUB Joint Commission on Biochemical Nomenclature for amino acids and peptides (Eur. J. Biochem. 1984, 158, 9-31).

The term "N-protecting group" or "N-protected" as used herein refers to those groups intended to protect the N-terminus of an amino acid or peptide or to protect an amino group against undersirable reactions during synthetic procedures. Commonly used N-protecting groups are disclosed in Greene, "Protective Groups In Organic Synthesis," (John Wiley & Sons, New York (1981)), which is hereby incorporated by reference. N-protecting groups comprise carbamates, amides, N-alkyl derivatives, amino acetal derivatives, N-benzyl derivatives, imine derivatives, enamine derivatives and N-heteroatom derivatives. Preferred N-protecting groups are formyl, acetyl, benzoyl, pivaloyl, t-butylacetyl, phenylsulfonyl, benzyl, t-butyloxycarbonyl (Boc), benzyloxycarbonyl (Cbz) and the like. N-protecting groups also refer to an L- or D-aminoacyl residue, which is derived from an L- or D- amino acid.

The term "O-protecting group" as used herein refers to a substituent which protects hydroxyl groups against undesirable reactions during synthetic procedures such as those O-protecting groups disclosed in Greene, "Protective Groups In Organic Synthesis," (John Wiley & Sons, New York (1981)). O-protecting groups comprise substituted methyl ethers, for example, methoxymethyl, benzyloxymethyl, 2-methoxyethoxymethyl, 2-(trimethylsilyl)ethoxymethyl, t-butyl, benzyl and triphenylmethyl; tetrahydropyranyl ethers; substituted ethyl ethers, for example, 2,2,2-trichloroethyl; silyl ethers, for example, trimethylsilyl, t-butyldimethylsilyl and t-butyldiphenylsilyl; and esters prepared by reacting the hydroxyl group with a carboxylic acid, for example, acetate, propionate, benzoate and the like.

The term "alkyl" as used herein refers to straight or branched chain alkyl radicals containing from 1 to 6 carbon atoms including, but not limited to, methyl, ethyl, n-propyl, iso-propyl, n-butyl, iso-butyl, sec-butyl, n-pentyl, 1-methylbutyl, 2,2-dimethylbutyl, 2-methylpentyl, 2,2-dimethylpropyl and n-hexyl.

The term "aryl" as used herein refers to a $C_6$ monocyclic aromatic ring system or a $C_9$ or $C_{10}$ bicyclic carbocyclic ring system having one or more aromatic rings including, but not limited to, phenyl, naphthyl, tetrahydronaphthyl, indanyl and indenyl.

The term "arylalkyl" as used herein refers to an aryl group appended to an alkyl radical including, but not limited to, benzyl and 1-naphthylmethyl.

The term "alkylamino" as used herein refers to an alkyl radical appended to an NH radical.

The term "cycloalkyl" as used herein refers to an aliphatic ring having 3 to 7 carbon atoms including, but not limited to, cyclopropyl, cyclopentyl, cyclohexyl and the like.

The term "cycloalkylalkyl" as used herein refers to a cycloalkyl group appended to an alkyl radical, including but not limited to cyclohexylmethyl.

The term "alkoxy" as used herein refers to $R_{29}O$- wherein $R_{29}$ is an alkyl group.

The term "dialkylamino" as used herein refers to $-NR_{36}R_{37}$ wherein $R_{36}$ and $R_{37}$ are independently selected from alkyl groups.

The term "polyalkoxy" as used herein refers to $-OR_{67}$ wherein $R_{67}$ is a straight or branched chain containing 1-5, $C_{n'}$-O-$C_{n''}$ linkages wherein n' and n" are independently selected from 1 to 3, including but not limited to methoxyethoxymethoxy and methoxymethoxy.

The term "halo" or "halogen" as used herein refers to -Cl, -Br, -I or -F.

The term "haloalkyl" as used herein refers to an alkyl radical in which one or more of the hydrogen atoms are replaced by halogen including, but not limited to, chloromethyl, trifluoromethyl and 1-chloro-2-fluoroethyl.

The term "aminoalkoxy" as used herein refers to an alkoxy radical to which is appended an amino ($-NH_2$) group.

The term "alkylaminoalkoxy" as used herein refers to an alkoxy radical to which is appended an alkylamino group.

The term "dialkylaminoalkoxy" as used herein refers to an alkoxy radical to which is appended a dialkylamino group.

The term "(alkoxyalkyl)aminoalkyl" refers to an alkyl radical to which is appended an (alkoxyalkyl)amino group.

The term "(alkoxyalkyl)aminoalkoxy" as used herein refers to an alkoxy radical to which is appended an (alkoxyalkyl)amino group.

The term "(alkoxyalkyl)(alkyl)aminoalkyl" refers to an alkyl radical to which is appended an (alkoxyalkyl)(alkyl)amino group.

The term "(alkoxyalkyl)(alkyl)aminoalkoxy" as used herein refers to an alkoxy radical to which is appended an (alkoxyalkyl)(alkyl)amino group.

The term "di-(alkoxyalkyl)aminoalkyl" refers to an alkyl radical to which is appended an di-(alkoxyalkyl)amino group.

The term "di-(alkoxyalkyl)aminoalkoxy" as used herein refers to an alkoxy radical to which is appended an di-(alkoxyalkyl)amino group.

The term "carboxyalkoxy" as used herein refers to an alkoxy radical to which is appended a carboxy (-COOH) group.

At each occurrence, the term "heterocyclic ring" or "heterocyclic" as used herein independently refers to a group selected from

pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, pyrimidinyl, pyridazinyl, oxazolyl, oxazolinyl, oxazolidinyl, isoxazolyl, isoxazolinyl, isoxazolidinyl, morpholinyl, thiazolyl, thiazolidinyl, isothiazolyl, isothiazolidinyl, indolyl, quinolinyl, tetrahydroquinolyl, isoquinolinyl, benzimidazolyl, benzothiazolyl, benzoxazolyl, benzofuranyl, furyl, dihydrofuranyl, tetrahydrofuranyl, pyranyl, dihydropyranyl, tetrahydropyranyl, dioxanyl, dioxolanyl, thienyl and benzothienyl.

Heterocyclics in the group $R_7$ can be unsubstituted or monosubstituted or disubstituted with substituents independently selected from halo, amino, (N-protected)amino, alkoxy, polyalkoxy and alkyl.

The term "(heterocyclic)alkyl" as used herein refers to a heterocyclic group appended to an alkyl radical, including but not limited to imidazolylmethyl and thiazolylmethyl.

When any variable occurs more than one time in any substituent or in a compound of formula I, its definition on each occurrence is independent of its definition at every other occurrence. Also, combinations of substituents and/or variables are permissible only if such combinations result in stable compounds.

Preferred compounds of the invention are selected from the group consisting of:

(2S, 3S, 5S)-2-(N-(N-((2-Pyridinyl)methoxycarbonyl)-valinyl)amino-5-(N-((3-pyridinyl)methoxycarbonyl)amino)-1,6-diphenyl-3-hydroxyhexane;
(2S, 3S, 5S)-5-(N-(N-((N-Methyl-N-((2-pyridinyl)-methyl)amino)carbonyl)valinyl)amino)-2-(N-((3-pyridinyl)-methoxycarbonyl)amino)-1,6-diphenyl-3-hydroxyhexane;
(2S,3S,5S)-2-(N-((3-Pyridinyl)-methoxycarbonyl)amino-5-(N-(N-((N-Methyl-N-((6-methyl-2-pyridinyl)methyl)-amino)carbonyl)valinyl)amino)-1,6-diphenyl-3-hydroxyhexane;
(2S,3S,5S)-2-(N-(N-((N-Methyl-N-((-pyridinyl)-methyl)amino)carbonyl)valinyl)amino)-5-(N-((3-pyridinyl)-methoxycarbonyl)amino)-1,6-diphenyl-3-hydroxyhexane;
(2S,3S,5S)-5-(N-(N-((2-Pyridinyl)methoxycarbonyl)-valinyl)amino)-2-(N-((3-pyridinyl)methoxycarbonyl)amino)-1,6-diphenyl-3-hydroxyhexane;
(2S,3S,5S)-5-(N-(N-((N-methyl-N-((2-pyridinyl)-methyl)amino)carbonyl)isoleucinyl)amino)-2-(N-((3-pyridinyl)methoxycarbonyl)amino)-1,6-diphenyl-3-hydroxyhexane.

(2S,3S,5S)-2,5-Di{N-(3-pyridylmethyl)oxy-carbonyl)amino}-3-hydroxy-1,6-diphenylhexane;

(2S,3S-5S)-2-(N-(N-((N-Methyl-N-((6-methyl-2-pyridinyl)methyl)amino)carbonyl)valinyl)amino)-5-(N-((3-pyridinyl)-methoxycarbonyl)amino)-1,6-diphenyl-3-hydroxyhexane; and

(2S,3S,5S)-2-(N-[(pyridin-3-yl)methoxycarbonyl]amino)-5-(N-[(6-methylpyridin-2-yl)methoxycarbonylvalyllamino)-1,6-diphenyl-3-hydroxyhexane;

or a pharmaceutically acceptable salt, ester or prodrug thereof.

Compounds useful as intermediates for the preparation of the compound of formula I include the compound of the formula:

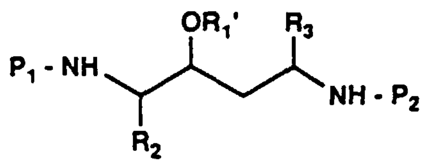

wherein $P_1$ and $P_2$ are independently selected from hydrogen and an N-protecting group; $R_1$' is hydrogen or an O-protecting group; and $R_2$ and $R_3$ are independently selected from arylalkyl, cycloalkylalkyl and (heterocyclic)alkyl; or a salt thereof.

The compounds of the invention can be prepared as shown in Schemes 1-2. The syntheses of carboxylic acids (A-OH and B-OH) and p-nitrophenyl esters (A-OPNP and B-OPNP) are described in the Examples. The process shown in Scheme 1 discloses the pinacol coupling of a protected aminoaldehyde (I) to give (II) and (III). Diols (II) and (III) are independently deoxygenated by initial reaction with α-acetoxyisobutyryl bromide and lithium bromide followed by reduction of the intermediate bromoacetate with tri-n-butyltin hydride to provide (IV) and (V), respectively. Basic hydrolysis of (IV) and (V) leads to (VI) and (VII), respectively.

The process described in Scheme 2 discloses the assemblage of HIV protease inhibitors from intermediate (XXVI), which represents structures (VI, (X'= $-CH(OH)CH_2-$)) and (VII, (X'= $-CH(OH)CH_2-$)).

The transformation of (XXVI) to (XXVII) can be achieved via an active ester such as a p-nitrophenyl ester of a carboxylic or sulfonic acid, or through direct coupling of the acid with (XXVI) in the presence of a coupling reagent. Alternately, protected α-aminoacids (W) can be coupled to (XXVI) to provide (XXVIII). Deprotect ion to give (XXIX), followed by coupling with Z-OH or activated derivatives thereof provides (XXVII). Alternatively, (XXVI) can be coupled with Z-W-OH or activated derivatives thereof to provide (XXVII).

Coupling reagents known in the art which can be used include, but are not limited to, dicyclohexylcarbodiimide (DCC), 3-ethyl-3'-(dimethylamino)propylcarbodiimide (EDC), bis(2-oxo-3-oxazolidinyl)-phosphinic chloride (BOP-Cl), diphenylphosphoryl azide (DPPA) and the like.

In addition to the use of the carboxylic acids or sulfonic acids for coupling with amines, acid halides and other activated esters are useful for coupling with amines. Acid halide derivatives include the acid chloride. Activated ester derivatives include activated esters commonly used by those skilled in the art for activating carboxylic acid groups for coupling with an amine to form an amide bond or for coupling with an alcohol for forming an ester bond including, but not limited to, formic and acetic acid derived anhydrides, anhydrides derived from alkoxycarbonyl halides such as isobutyloxycarbonylchloride and the like, N-hydroxysuccinimide derived seters, N-hydroxyphthalimide derived esters, N-hydroxybenzotriazole derived esters, N-hydroxy-5-norbornene-2,3-dicarboxamide derived esters, 2,4,5-trichlorophenol derived esters and the like.

Scheme 3 illustrates the preparation of a particular substituent A which is N-(N'-2-pyridylmethyl-N'-methyl-aminocarbonyl)-L-valine (XXXV). 2-Picolinaldehyde (XXXI) is converted to 2-(N-methyl)aminomethylpyridine (XXXII) by treatment with methylamine, followed by hydrogenation. Reaction of (XXXII) with the methyl or benzyl ester of N-phenoxycarbonyl-L-valine ((XXXIII) provides (XXXV). Hydrolysis (R=Me) or hydrogenation (R=benzyl) of (XXXIV) provides (XXXVI).

Scheme 4 illustrates the preparation of the compounds of the invention wherein A and B are not identical. Starting with diamine (XXXVII) as a representative substituent X, monoacylation of the diamine with the p-nitrophenyl ester or p-nitrophenyl carbonate of A-OH provides a mixture of (XXXVIII) and (XXXIX). This mixture can be separated by silica gel chromatography. Acylation of (XXXVIII) with the p-nitrophenyl ester or p-nitrophenyl carbonate of B-OH provides (XL). Similarly, acylation of (XXXIX) with the p-nitrophenyl ester or p-nitrophenyl carbonate of B-OH provides (XLI).

Scheme 1

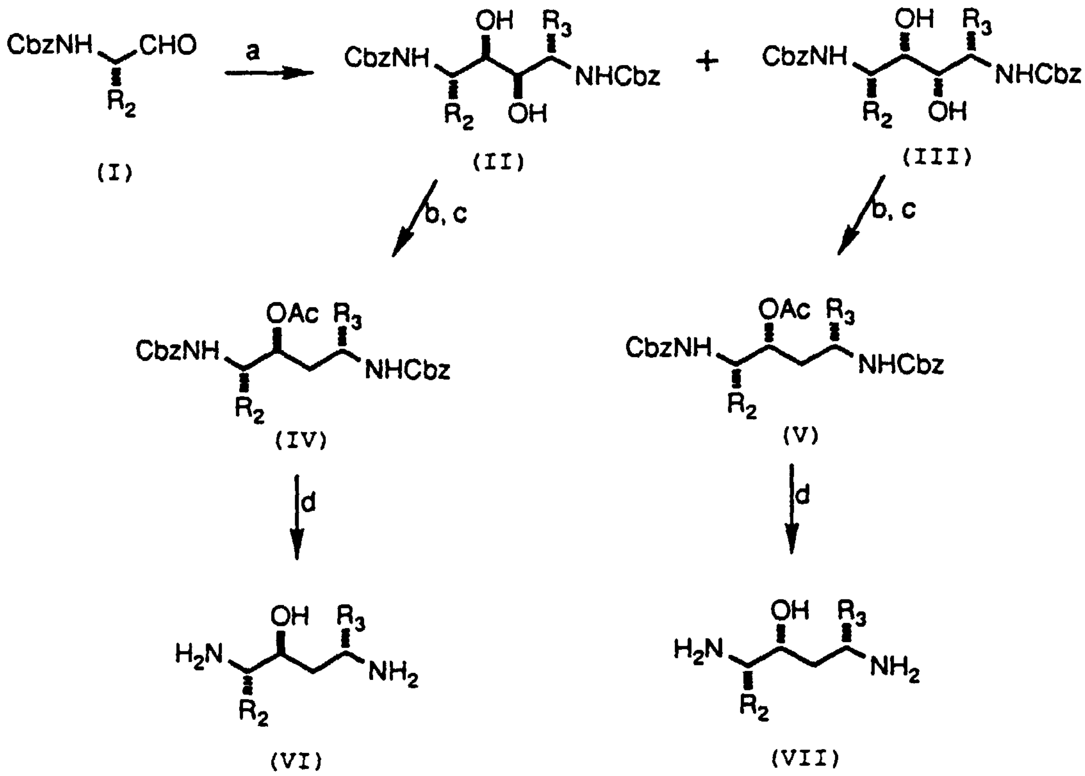


(I) (II) (III) (IV) (V) (VI) (VII)

a, $VCl_3 \cdot (THF)_3$, Zn, $CH_2Cl_2$; b, LiBr, α-acetoxyisobutyryl bromide, $CH_3CN$; c, $(n\text{-}Bu)_3SnH$, AIBN, THF; d, $Ba(OH)2 \cdot 8H_2O$, $H_2O$, dioxane.

Scheme 2

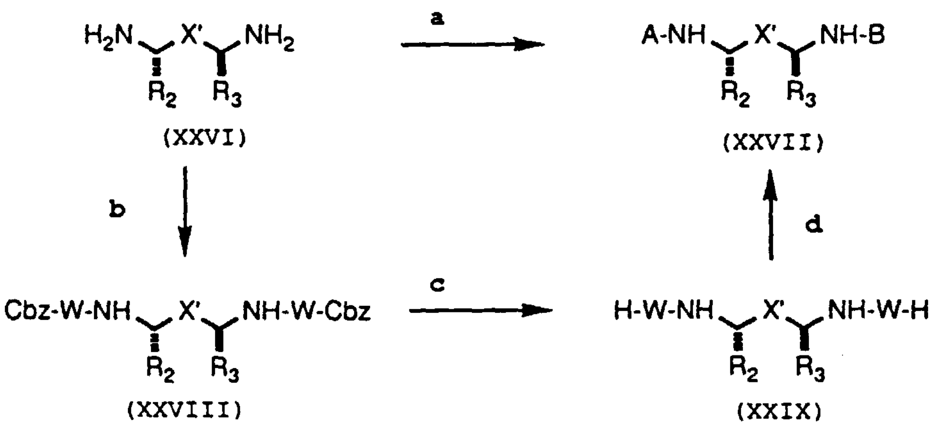


(XXVI) (XXVII) (XXVIII) (XXIX)

a, A-OPNP/B-OPNP or A-OH/B-OH + carbodiimide; b, Cbz-W-OPNP or Cbz-W-OH + carbodiimide; c, $H_2$, Pd/C,

$CH_3OH$;
d, Z-OH or Z-OPNP.

SCHEME 3

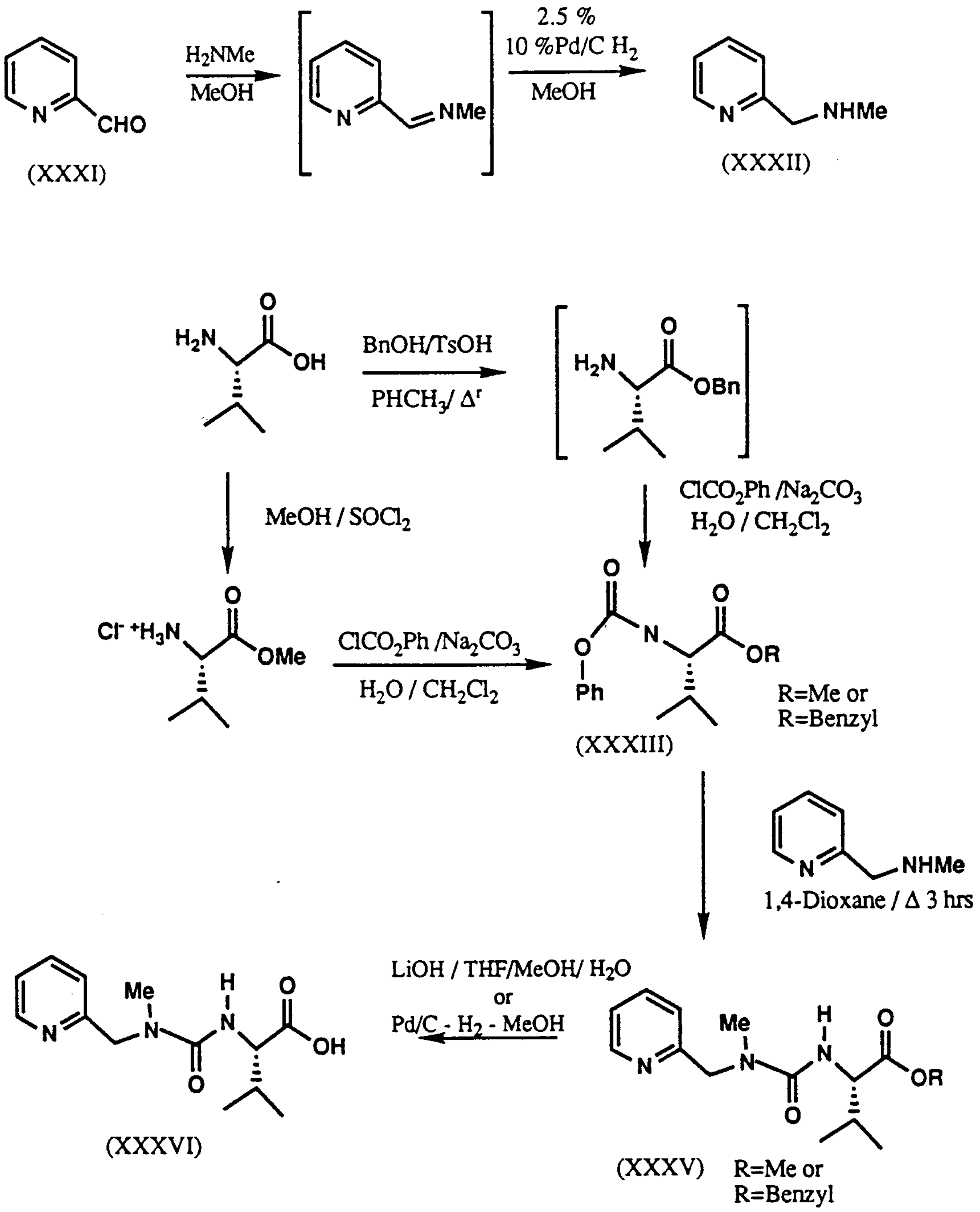


(XXXI) (XXXII) (XXXIII) (XXXV) (XXXVI)

SCHEME 4

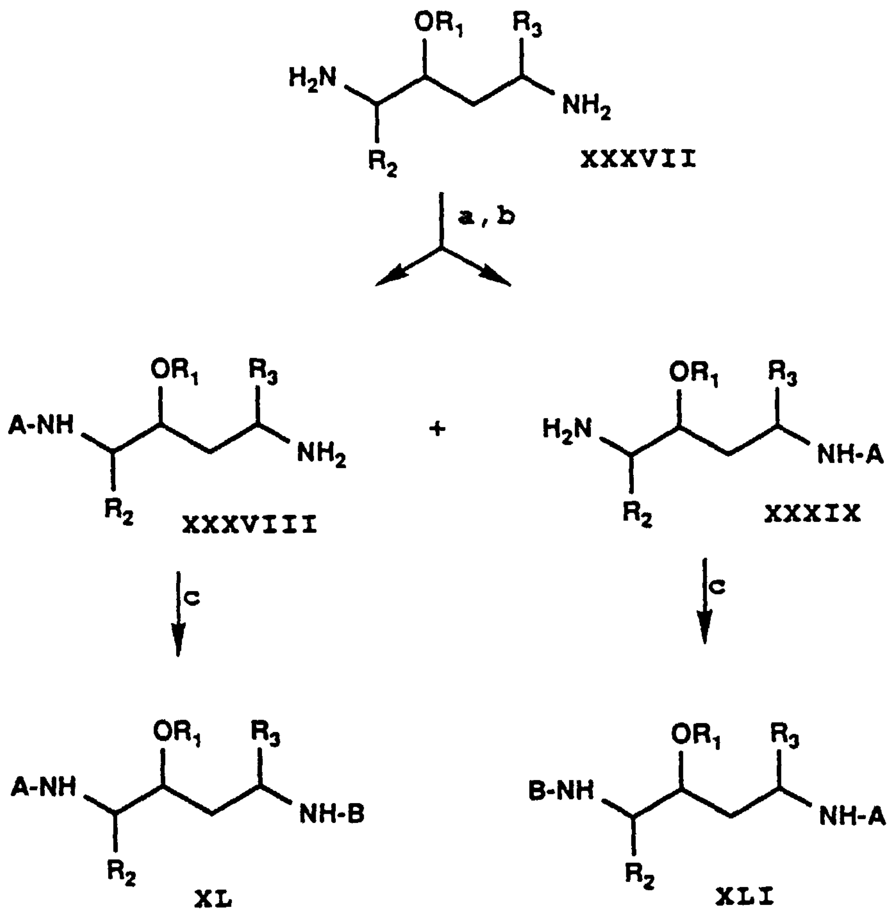


XXXVII

XXXVIII + XXXIX

XL XLI

a, A-OPNP, THF or $CH_2Cl_2$; b, silica gel chromatography; c, B-OPNP, THF or $CH_2Cl_2$.

The following examples will serve to further illustrate preparation of the novel compounds of the invention.

Example 1

A. Cbz-L-phenylalaninal.

A solution of 24.5 ml of anhydrous dimethyl sulfoxide in 870 ml of anhydrous dichloromethane was cooled under $N_2$ atmosphere to -60°C and treated over a period of 15 min with 131 ml of a 2 M solution of oxalyl chloride in dichloromethane in order that the internal temperature remained below -50°C. After addition, the solution was stirred at -60°C for 15 min and treated over a period of 20 min with a solution of 50 g (0.175 mol) of Cbz-L-phenylalaninol in 200 ml of dichloromethane. The resulting solution was stirred at -60°C for 1 h, then treated over a period of 15 min with 97 ml of triethylamine in order that the internal temperature remained below -50°C. After addition the solution was stirred at -60°C for 15 min, then, with the cooling bath in place, was treated rapidly (over a period of 1 min) with a solution of 163 g of citric acid in 550 ml of water. The resulting slurry was stirred vigorously for 10 min, allowed to warm, diluted to 1 liter with water, and separated. The organic layer was washed with 700 ml of water followed by a mixture of 550 ml of water and 150 ml of saturated aqueous $NaHCO_3$, dried over $MgSO_4$, and concentrated in vacuo at 20°C to give the crude desired compound as a light yellow solid.

B. (2S,3R,4R,5S)-2,5-Bis-(N-Cbz-amino)-3,4-dihydroxy-1,6-diphenylhexane and (2S,3S,4S,5S)-2,5-Bis-(N-Cbz-amino)-3,4-dihydroxy-1,6-diphenylhexane.

A suspension of 78.5 g of $VCl_3$ • (tetrahydrofuran)$_3$ and 16 g of zinc dust in 400 ml of dry dichloromethane was stirred under $N_2$ atmosphere for 1 h at 25°C. A solution of 0.175 mol of Cbz-L-phenylalaninal in 200 ml of dichloromethane was then added in one portion, and the resulting mixture was stirred at ambient temperature under $N_2$ atmosphere for 16 h. The resulting mixture was added to 500 ml of 1 M aqueous HCl, diluted with 500 ml of hot chloroform, and shaked vigorously for 2 min. The layers were separated, and the organic layer was washed with 1 M aqueous HCl and separated. Filtration of the organic phase provided the crude desired product as a solid residue. The residue was slurried in 1.25 liters of acetone, treated with 5 ml of concentrated $H_2SO_4$, and stirred for 16 h at ambient temperature. The resulting mixture was filtered, and the residue (residue A) was washed with 50 ml of acetone. The combined filtrate was concentrated to a volume of 250 ml, diluted with 1000 ml of dichloromethane, washed three times with water and once with saturated brine, dried over $MgSO_4$, and concentrated to give a viscous oil. The oil was taken up in 1000 ml of 1 M HCl in methanol (prepared from 71 ml of acetyl chloride and 1000 ml of methanol) and stirred at ambient temperature for 2 h. The resulting precipitate was filtered, washed with methanol, and air-dried on the filter to provide 26.7 g of the desired compound as a white solid. The filtrate was concentrated and filtered to give a second crop (8.3 g) of (2S,3R,4R,5S)-2,5-bis-(N-Cbz-amino)-3,4-dihydroxy-1,6-diphenylhexane. $^1H$ NMR ($d_6$-DMSO) $\delta$ 2.59 (dd, J = 13, 5 Hz, 2 H), 2.74 (dd, J = 13, 9 Hz, 2 H), 3.26 (br, 2 H), 4.19 (m, 2 H), 4.54 (m, 2 H), 4.92 (m, 4 H), 6.82 (d, J = 9 Hz, 2 H), 7.0-7.35 (m, 20 H). Mass spectrum: $(M + H)^+$ = 569.

Residue A (above, 2.65 g) was suspended in 75 ml of tetrahydrofuran and 75 ml of 1 M aqueous HCl and heated at reflux for 24 h. After concentration of the resulting solution in vacuo, the residue was taken up in 10% methanol in chloroform, washed two times with water, dried over $Na_2SO_4$, and concentrated in vacuo to provide (2S,3S,4S,5S)-2,5-bis-(N-Cbz-amino)-3,4-dihydroxy-1,6-diphenylhexane as a white solid. $^1H$ NMR ($d_6$-DMSO) $\delta$ 2.64 (m, 2 H), 3.04 (m, 2 H), 3.49 (m, 2 H), 3.78 (m, 2 H), 4.70 (d, J = 7 Hz, 2 H), 4.93 (AA', 4 H), 7.1-7.4 (m, 20 H). Mass spectrum: $(M + H)^+$ = 569.

C. (2S,3R,4S,5S)-3-Acetoxy-2,5-bis-(N-Cbz-amino)-3-bromo-1,6-diphenylhexane.

A suspension of 25 g (44 mmol) of (2S,3R,4R,5S)-2,5-bis-(N-Cbz-amino)-3,4-dihydroxy-1,6-diphenylhexane in 500 ml of 2:1 dichloromethane/hexane was treated with 23 g of $\alpha$-acetoxyisobutyryl bromide. The resulting mixture was stirred at ambient temperature until the reaction clarified, washed with two 200 ml portions of saturated aqueous $NaHCO_3$, dried over $MgSO_4$, and concentrated in vacuo to give 30.8 g of the crude desired compound. A portion was purified by silica gel chromatography using 9:1 dichloromethane:ethyl acetate to provide the pure desired compound as a white solid. $^1H$ NMR ($CDCl_3$) $\delta$ 2.21 (s, 3 H), 2.62 (dd, J = 13, 11 Hz, 1 H), 2.75 (d, J = 7 Hz, 2 H), 2.95 (br d, J = 15 Hz, 1 H), 4.03 (br t, J = 10 Hz, 1 h), 4.40 (br d, J = 10 Hz, 1 H), 4.6-5.0 (m, 6 H), 5.12 (br d, J = 13 Hz, 1 H), 5.33 (br d, J = 11 Hz, 1 H), 7.0-7.4 (m, 10 H). Mass spectrum: $(M + NH_4)^+$ = 690, 692.

D. (2S,3S,5S)-3-Acetoxy-2,5-bis-(N-Cbz-amino)-1,6-diphenylhexane.

A solution of 30.8 g (44 mmol) of the crude resultant compound of Example 1C in 600 ml of tetrahydrofuran was treated with 17.8 ml (66 mmol) of tri-n-butyltin hydride and 1.45 g (8.8 mmol) of 2,2'-azobis-[2-methylpropionitrile]. The resulting solution was heated at reflux under $N_2$ atmosphere for 1.5 h. After being allowed to cool, the solution was concentrated in vacuo, and the residue was taken up into acetonitrile and washed with four portions of hexane. The acetonitrile layer was dried over $MgSO_4$, filtered, and concentrated in vacuo to provide 32 g of the crude desired compound. Mass spectrum: $(M + NH_4)^+$ = 612.

E. (2S,3S,5S)-2,5-Diamino-1,6-diphenyl-3-hydroxyhexane.

A suspension of 32 g of the crude resultant compound of Example 1D and 55.5 g (176 mmol) of barium hydroxide octahydrate in 400 ml of 1,4-dioxane and 400 ml of water was heated at reflux for 4 h. The resulting mixture was filtered, and the residue was rinsed with dioxane. The combined filtrates were concentrated to a volume of approximately 200 ml and extracted with four 400 ml portions of chloroform. The combined organic layers were dried over $Na_2SO_4$, filtered, and concentrated in vacuo. The residue was purified by silica gel chromatography using first 2% isopropylamine in chloroform and then 2% isopropylamine/2% methanol in chloroform to provide 10.1 g (81%) of the pure desired compound as a white solid. $^1H$ NMR ($CDCl_3$) $\delta$ 1.54 (dt, J = 14, 10 Hz, 1 H), 1.67 (dt, J = 14, 3 Hz, 1 H), 2.50 (dd, J = 13, 8 Hz, 1 H), 2.58 (dd, J = 13, 8 Hz, 1 H), 2.8 (m, 2 H), 2.91 (dd, J = 13, 5 Hz, 1 H), 3.10 (m, 1 H), 3.72 (ddd, J = 11, 3, 2 Hz, 1 H), 7.1-7.4 (m, 10 H). Mass spectrum: $(M + H)^+$ = 285.

Example 2

A. α-Isocyanato-valine Methyl Ester.

A suspension of L-valine methyl ester hydrochloride (49 g, 0.29 mol) in toluene (700 ml) was heated to 100°C and phosgene gas was bubbled into the reaction mixture. After approximately 6 h, the mixture became homogeneous. The bubbling of phosgene was continued for 10 more min, then the solution was cooled with the bubbling of $N_2$ gas. The solvent was then evaporated and the residue chased with toluene two times. Evaporation of solvent gave 40.8 g (89%) of the crude desired compound.

B. N-((2-Pyridinyl)methoxycarbonyl)-valine Methyl Ester.

A solution of 0.78 g (5.0 mmol) of the resultant compound of Example 2A and 0.55 ml (5.7 mmol) of pyridine-2-methanol in 30 mL of toluene was heated at reflux under $N_2$ atmosphere for 4 h. The solvent was removed in vacuo, and the residue was purified by silica gel chromatography using 2% methanol in chloroform to give 0.72 g (54%) of the desired compound as an oil. $^1H$ NMR ($CDCl_3$) δ 0.91 (d, J = 7 Hz, 3 H), 0.98 (d, J = 7 Hz, 3 H), 2.19 (m, 1 H), 3.75 (s, 3 H), 4.32 (dd, J = 9, 5 Hz, 1 H), 5.24 (s, 2 H), 5.39 (br d, 1 H), 7.23 (ddd, J = 8, 4, 1 Hz, 1 H), 7.37 (d, J = 8 Hz, 1 H), 7.70 (td, J = 8, 2 Hz, 1 H), 8.60 (br d, 1 H). Mass spectrum: $(M + H)^+ = 267$.

C. N-((2-Pyridinyl)methoxycarbonyl)-valine.

Using the procedure of Example 3E but replacing the resultant compound of Example 3D with the resultant compound of Example 2B provided the desired compound.

D. N-((2-Pyridinyl)methoxycarbonyl)-valine p-Nitrophenyl Ester.

Using the procedure of Example 3F but replacing the resultant compound of Example 3E with the resultant compound of Example 2C provided the desired compound.

E. (2S,3S,5S)-2,5-Bis-(N-(N-((2-pyridinyl)methoxycarbonyl)- valinyl)-amino)-1,6-diphenyl-3-hydroxyhexan.

A solution of 0.13 g (0.46 mmol) of the resultant compound of Example 1E in 2 ml of dry dimethylformamide was treated with 0.5 g of the resultant compound of Example 2D. After being stirred at ambient temperature for 16 h, the solution was treated with saturated aqueous $NaHCO_3$, extracted with 5% methanol in chloroform, dried over $Na_2SO_4$, and concentrated in vacuo. The residue was purified by silica gel chromatography using a gradient of 2% - 3% - 5% methanol in chloroform to provide 161 mg (45%) of the pure desired compound, m.p. 220-222°C. Mass spectrum: $(M + H)^+ = 753$.

Anal. Calcd for $C_{42}H_{52}N_6O_7 \cdot 0.5H_2O$: C, 66.21; H, 7.01; N, 11.03. Found: C, 65.92; H, 6.90; N, 10.80.

Example 3

A. 2-(N-(t-Butyloxycarbonyl)aminomethyl)pyridine.

A solution of 21.2 g (97 mmol) of di-t-butyldicarbonate in 200 ml of dichloromethane was cooled to 0°C and treated in portions with 10 ml (97 mmol) of 2-(aminomethyl)pyridine. After being allowed to warm to ambient temperature and stirred overnight, the resulting solution was diluted with 100 ml of dichloromethane, washed with three 100 ml portions of water, dried over $Na_2SO_4$, and concentrated in vacuo to provide 19.8 g (98%) of the desired compound ($R_f$ 0.28, 5% methanol in chloroform). $^1H$ NMR ($CDCl_3$) δ 1.47 (s, 9 H), 4.45 (d, J = 6 Hz, 2 H), 5.56 (br, 1 H), 7.18 (m, 1 H), 7.28 (d, J = 8 Hz, 1 H), 7.66 (td, J = 7, 2 Hz, 1 H), 8.53 (m, 1 H). Mass spectrum: $(M + H)^+ = 209$.

B. 2-((N-(t-Butyloxycarbonyl)-N-methylamino)methyl)pyridine.

A solution of 19.8 g (95 mmol) of the resultant compound of Example 3A in anhydrous tetrahydrofuran was cooled under $N_2$ atmosphere to 0°C and treated with 4.95 g (124 mmol) of sodium hydride (60% dispersion in oil). The solution was stirred for 15 min, treated dropwise with 7.1 ml (114 mmol) of methyl iodide, stirred at ambient temperature for 2 h, and quenched cautiously with water. The resulting mixture was partitioned between ether and water, dried over $Na_2SO_4$, and concentrated in vacuo. Chromatography on silica gel provided 14.9 g (70%) of the desired compound as

a colorless oil. $^1H$ NMR ($CDCl_3$) δ 1.43, 1.49 (two s, 9 H), 2.89, 2.94 (two s, 3 H), 4.54, 4.57 (two s, 2 H), 7.2 (m, 2 H), 7.67 (td, j = 8, 2 Hz, 1 H), 8.55 (d, J = 4 Hz, 1 H). Mass spectrum: $(M + H)^+$ = 223.

C. 2-(N-Methylamino)methyl)pyridine Dihydrochloride.

The resultant compound of Example 3B (10 g) was treated with 200 ml of 6 M aqueous HCl and heated at reflux for 10 min. After being allowed to cool, the solution was concentrated in vacuo. The residue was treated twice with 50 ml of dioxane and concentrated in vacuo to provide the crude desired compound as a light brown solid.

D. N-((N-Methyl-N-((2-pyridinyl)methyl)amino)carbonyl)- valine Methyl Ester.

A mixture of 1.61 g (7.2 mmol) of the resultant compound of Example 3C and 1.14 g (7.2 mmol) of the resultant compound of Example 2A in 40 ml of dichloromethane was treated with 2 ml (18 mmol) of 4-methylmorpholine. After being stirred for 2 h, the solution was partitioned between dichloromethane and water, dried over $Na_2SO_4$, and concentrated. Chromatography on silica gel using 2% methanol in chloroform provided 1.94 g (96%) of the desired compound ($R_f$ 0.32, 5% methanol in chloroform) as a colorless oil. $^1H$ NMR ($CDCl_3$) δ 0.93 (d, J = 7 Hz, 3 H), 0.97 (d, J = 7 Hz, 3 H), 2.16 (m, 1 H), 3.03 (s, 3 H), 3.72 (s, 3 H), 4.43 (dd, J = 8, 5 Hz, 1 H), 4.55 (s, 2 H), 6.15 (br, 1 H), 7.22 (dd, J = 8, 6 Hz, 1 H), 7.28 (d, J = 6 Hz, 1 H), 7.69 (br t, 1 H), 8.55 (d, J = 5 Hz, 1 H). Mass spectrum: $(M + H)^+$ = 280.

E. N-((N-Methyl-N-((2-pyridinyl)-methyl)-amino)-carbonyl)-valine.

A solution of 4.47 g (16 mmol) of the resultant compound of Example 3D in 65 ml of dioxane was treated with 65 ml of 0.5 M aqueous lithium hydroxide. After being stirred at ambient temperature for 1 h, the resulting solution was concentrated in vacuo to a small volume (ca. 5 ml), neutralized to pH 5 with 1 M aqueous HCl, and extracted with three 100 ml portions of ethyl acetate. The combined organic layers were dried over $Na_2SO_4$ and concentrated in vacuo to provide 3.61 g (85%) of the desired compound as an oil.

F. N-((N-Methyl-N-((2-pyridinyl)-methyl)-amino)-carbonyl)-valine p-Nitrophenyl Ester.

A solution of 3.61 g (13.6 mmol) of the resultant compound of Example 3E and 2.3 g (16 mmol) of p-nitrophenol in 60 ml of anhydrous tetrahydrofuran was treated with 3.09 g (15 mmol) of dicyclohexyl carbodiimide and stirred under $N_2$ atmosphere at ambient temperature for 4 h. The resulting mixture was filtered and the residue was rinsed with fresh tetrahydrofuran. The combined filtrates were concentrated in vacuo to provide the crude desired compound as a yellow oil.

G. (2S,3S,5S)-2,5-Bis-(N-(N-((N-methyl-N-((2-pyridinyl)-methyl)-amino)-carbonyl)-valinyl)-amino)-1,6-diphenyl-3-hydroxyhexane.

A solution of 1.26 g (4.44 mmol) of the resultant compound of Example 1E in 20 ml of 1:1 tetrahydrofuran: dimethylformamide was treated with 11 mmol of the resultant compound of Example 3F. After being stirred at ambient temperature under $N_2$ atmosphere for 16 h, the resulting solution was diluted with 600 ml of ethyl acetate, washed with five 200 ml portions of aqueous $NaHCO_3$, dried over $Na_2SO_4$, and concentrated in vacuo. Purification of the residue on silica gel using first 2% methanol in chloroform then 5% methanol in chloroform provided 2.95 g (86%) of the pure desired compound as a white solid, m.p. 134-137°C. Mass spectrum: $(M + H)^+$ = 779.

Anal. Calcd for $C_{44}H_{58}N_8O_5 \cdot 1.5H_2O$: C, 65.57; H, 7.63; N, 13.90. Found: C, 65.74; H, 7.24; N, 13.83.

Example 4 (reference)

A. (2S,3R,4R,5S)-2,5-Diamino-3,4-dihydroxy-1,6- diphenylhexane.

Using the procedure of Example 1E with (2S,3R,4R,5S)-2,5-bis-(N-Cbz-amino)-3,4-dihydroxy-1,6-diphenylhexane provided the crude desired compound mixed with benzyl alcohol in 92% yield. Purification of a sample was achieved by silica gel chromatography using 2% isopropylamine in chloroform. $^1H$ NMR ($CDCl_3$) δ 2.71 (dd, J = 13, 9 Hz, 2 H), 2.92 (dd, J = 13, 5 Hz, 2 H), 3.03 (dd, J = 9, 5 Hz, 2 H), 3.68 (s, 2 H), 7.15-7.35 (m, 10 H). Mass spectrum: $(M + H)^+$ = 301.

B. (2S,3R,4R,5S)-2,5-Bis-(N-(Cbz-valinyl)amino)-3,4- dihydroxy-1,6-diphenylhexane.

A mixture of 2.5 g of the crude resultant compound of Example 4A and 6 g of Cbz-valine p-nitrophenyl ester in 80 ml of tetrahydrofuran was stirred at ambient temperature for 16 h. The resulting mixture was treated with 20 ml of 3 M aqueous NaOH, stirred for 3 h, and concentrated in vacuo to a volume of 20 ml. The mixture was filtered, and the residue was washed sequentially with aqueous NaOH (until the residue was white), water, and diethyl ether. The residue was then taken up into 10% methanol in chloroform, dried over $Na_2SO_4$, and concentrated in vacuo to provide 2.77 g (75%) of the desired compound, m.p. 231-232°C. Mass spectrum: $(M + H)^+$ = 767.

Anal. Calcd for $C_{44}H_{54}N_4O_8 \cdot 0.25H_2O$: C, 68.51; H, 7.12; N, 7.26. Found: C, 68.48; H, 7.11; N, 7.12.

C. (2S,3R,4R,5S)-2,5-Bis-(N-(valinyl)amino)-3,4-dihydroxy- 1,6-diphenylhexane.

A mixture of 2.21 g of the resultant compound of Example 4B and 0.55 g of 10% palladium on carbon in 150 ml of methanol was shaken under 4 atmospheres of hydrogen for 4 h. The resulting mixture was filtered through Celite and concentrated in vacuo to provide the desired compound ($R_f$ 0.07, 10% methanol in chloroform) as a white solid, m.p. 205-207°C. Mass spectrum: $(M + H)^+$ = 499.

Anal. Calcd for $C_{28}H_{42}N_4O_4 \cdot 0.75H_2O$: C, 65.66; H, 8.56; N, 10.94. Found: C, 65.47; H, 7.93; N, 10.59.

D. trans-(2S,3R,4R,5S)-2,5-Bis-(N-(N-(trans-3-(3-pyridinyl)-2-propenoyl)-valinyl)amino)-3,4-dihydroxy-1,6-diphenylhexane.

Using the procedure of Example 5I but replacing the resultant compound of Example 5H with the resultant compound of Example 4C provided the desired compound, m.p. >260°C. Mass spectrum: $(M + H)^+$ = 761.

Example 5 (reference)

A. 4-(t-Butyloxycarbonylamino)-3-hydroxy-5-phenyl-1-pentene.

A solution of 10.25 g (36.7 mmol) of N-(t-butyloxycarbonyl)phenylalanine methyl ester in 60 ml of toluene was cooled to -78°C under inert atmosphere and treated dropwise over a period of 45 min with 35 ml (52.5 mmol) of diisobutylaluminum hydride in toluene. The resulting solution was stirred for 5 min, treated with 200 ml (200 mmol) of vinylmagnesium bromide, and allowed to warm to 0°C for 16 h. The solution was subsequently quenched cautiously with methanol, treated with aqueous Rochelle salts, stirred for a few min, and filtered. The residue was digested several times with ethyl acetate and filtered; and the combined filtrates were washed with saturated brine, dried over $MgSO_4$, and concentrated. Silica gel chromatography using 20% ethyl acetate in hexane gave 5.46 g (54%) of the pure desired compound as a mixture of diastereomers.

B. 2-(t-Butyloxycarbonylamino)-1,5-diphenylpent-3-ene.

A solution of 15.1 g (54.5 mmol) of the resultant compound of Example 5A and 38 ml (220 mmol) of diisopropylethylamine in 450 ml of dry dichloromethane was cooled under $N_2$ atmosphere in an acetone/ice bath and treated dropwise with 8.5 ml (110 mmol) of methanesulfonyl chloride. The solution was stirred for 7 min after addition was complete, then was quenched with 400 ml of 10% citric acid. The bath was removed, and the mixture was extracted with 800 ml of ether. The organic layer was washed sequentially with 500 ml of water and 300 ml of saturated brine, dried over $MgSO_4$, and concentrated in vacuo to give the crude mesylate as an off-white solid. To a flame-dried 3-neck 1000 mL flask equipped with an internal low-temperature thermometer was added 1.45 g (16 mmol) of anhydrous cuprous cyanide. The flask was then charged with 500 ml of anhydrous tetrahydrofuran. The suspension was cooled under $N_2$ altmosphere in a dry ice/acetone bath. A solution of phenylmagnesium bromide (55 ml, 165 mmol) in ether (3M) was added via syringe. The bath was removed, and the resulting beige suspension was warmed with stirring by use of a water bath. As the internal temperature reached -5°C, the solid began to dissolve, and the solution began to turn darker. By the time the internal temperature reached -1°C, the solution was homogenous, and was immediately recoded by placement of the flask in a dry ice/acetone bath. As the internal temperature reached -65°C, addition of a solution of the above crude mesylate in 75 ml of tetrahydrofuran was added via cannula. The resulting solution was stirred at *ca*. -70°C for 15 min. The bath was then removed, and the solution was immediately treated with 100 ml of saturated aqueous ammonium chloride followed by 300 ml of ether. As the mixture warmed, 100 ml of 1 N $NH_4OH$ was added, and the mixture was stirred under air atmosphere for several hours while the aqueous layer turned dark blue. The mixture was

then extracted with 500 ml of ether. The organic layer was washed with saturated brine and concentrated in vacuo without drying to give a yellow oil. The combined aqueous layers were extracted with 500 ml of additional ether, which was added to the above oil. The resulting solution was washed with saturated brine, dried over $MgSO_4$, and concentrated to a yellow oil. The oil was taken up in 100 ml of dichloromethane, treated with 50 g of silica gel, and concentrated in vacuo until the residue was a freely flowing solid. The solid was placed on top of a 60 mm column containing 300 g of silica gel and eluted sequentially with 1200 ml of hexane (to bring out biphenyl formed as a side product) followed by 5000 ml of 5% ethyl acetate in hexane. Combination of the pure fractions gave 11.95 g (65%) of the desired compound. $^1H$ NMR ($CDCl_3$, major isomer) δ 1.40 (s, 9 H), 2.7-2.9 (m, 2 H), 3.32 (d, J = 7 Hz, 2 H), 4.4 (br, 2 H), 5.43 (dd, J = 15, 6 Hz, 1 H), 5.64 (dt, J = 15, 7 Hz, 1 H), 7.0-7.3 (m, 10 H).

C. 2-(t-Butyloxycarbonylamino)-1,5-diphenylpent-3-ene-3,4-oxide.

A solution of 11.71 g (34.75 mmol) of the resultant compound of Example 5B in 200 ml of dichloromethane was treated with 15 g (174 mmol) of solid sodium bicarbonate, cooled to 0°C, and treated with 24 g (69 mmol) of m-chloroperbenzoic acid (50%). The resulting suspension was sealed with a septum and stirred in a cold room (5°C) for three days. The resulting mixture, which contained much precipitate, was decanted into a 1000 ml flask. The white residue was broken up and washed out with 400 ml of 10% sodium thiosulfate solution and 300 ml of ether. The two-phase mixture was stirred for 2 hours, and the layers were separated. The organic layer was washed sequentially with 200 ml portions of 2 M NaOH, water, and saturated brine. The combined aqueous layers were extracted with 200 ml of ether, which was washed sequentially with 50 ml of water and 50 mL of aqueous brine, combined with the original organic phase, dried over $MgSO_4$, and concentrated in vacuo. The resulting oil was taken up in 100 ml of dichloromethane, treated with 50 g of silica gel, and concentrated in vacuo until the residue was a freely flowing solid. The solid was placed on top of a 60 mm column containing 300 g of silica gel and eluted sequentially with 1000 ml of 5% ethyl acetate in hexane followed by 3500 ml of 12% ethyl acetate in hexane. Concentration of the combined fractions gave 9.36 g (76%) of the desired compound (*ca*. 4:1 mixture of diastereomers) as an oil which solidified upon standing.

D. 4-Azido-2-(t-butyloxycarbonylamino)-1,5-diphenyl-3- hydroxypentane.

A solution of 9.12 g (25.84 mmol) of the resultant compound of Example 5C 7.0 g (140 mmol) of lithium azide, and 1.73 g (32 mmol) of ammonium chloride in 75 ml of dimethylformamide and 7.5 ml of water was heated in an oil bath at 70°C for 32 hours. After being allowed to cool, the resulting solution was treated with 1000 ml of 1:1 ether/hexane and 800 ml of water. The layers were separated, and the aqueous layer was extracted with 500 ml of additional 1:1 ether/hexane. The combined organic layers were washed sequentially with 400 ml of water and 200 ml of saturated brine, dried over $MgSO_4$, and concentrated in vacuo to a solid. The solid was taken up in 100 ml of dichloromethane, treated with 50 g of silica gel, and concentrated in vacuo until the residue was a freely flowing solid. The solid was placed on top of a 60 mm column containing 300 g of silica gel and eluted sequentially with 1000 ml of 10% ethyl acetate in hexane, 1000 ml of 15% ethyl acetate in hexane, and 2000 ml of 25% ethyl acetate in hexane. Concentration of the fractions gave 9.26 g (91%) of the desired compound as a *ca*. 4:1 mixture of diastereomers. $^1H$ NMR ($CDCl_3$, major isomer) δ 1.42 (s, 9 H), 2.78 (m, 1 H), 2.89 (m, 1 H), 3.13 (m, 1 H), 3.29 (m, 1 H), 3.41 (m, 1 H), 3.53 (m, 1 H), 3.80 (m, 1 H), 4.06 (m, 1 H), 4.83 (m, 1 H), 7.2-7.35 (m, 10 H). Mass spectrum $(M+H)^+$ = 338.

E. 4-Amino-2-(t-butyloxycarbonylamino)-1,5-diphenyl-3-hydroxypentane.

A rapidly stirring suspension of 1.8 g of 10% palladium on carbon in 50 ml of methanol was treated under inert atmosphere with 10 g (0.16 mol) of solid ammonium formate. After 10 min, a solution of 8.95 g (22.6 mmol) of the resultant compound of Example 5D in 80 ml of methanol was added. The resulting mixture was stirred for 2.5 h, filtered through Celite, and the catalyst was washed with 200 ml of 1:1 methanol:1 N ammonium hydroxide. The combined filtrates were concentrated in vacuo to a volume of 100 ml. The resulting mixture was treated with 1 N NaOH and extracted with two portions of chloroform. The combined organic layers were dried over sodium sulfate and concentrated. The residue was chromatographed on 300 g of silica gel using the following eluents: 500 ml of 2% methanol in chloroform, 500 ml of 5% methanol in chloroform, 1500 ml of 10% methanol in chloroform, and 1000 ml of 2% isopropylamine/10% methanol in chloroform. Concentration of the appropriate fractions provided 5.85 g (70%) of (2S,3S,4S)-4-amino-2-(t-butyloxycarbonylamino)-1,5-diphenyl-3-hydroxypentane ($R_f$ 0.38, 2.5% methanol/2% isopropylamine in chloroform) as a white solid, m.p. 134-135°C. $^1H$ NMR ($CDCl_3$) δ 1.48 (s, 9 H), 2.50 (dd, J = 13, 10 Hz, 1 H), 2.8-3.1 (m, 4 H), 3.41 (br d, J = 7 Hz, 1 H), 4.11 (br q, J = 8 Hz, 1 H), 4.83 (br d, J = 9 Hz, 1 H), 7.15-7.35 (m, 10 H). Mass spectrum $(M+H)^+$ = 370.

Anal. Calcd. for $C_{22}H_{30}N_2O_3 \cdot 0.15H_2O$: C, 70.81; H, 8.18; N, 7.51. Found: C, 70.89; H, 8.15; N, 7.43.

Also isolated in the chromatography was 1.22 g (15%) of (2S,3R,4R)-4-amino-2-(t-butyloxycarbonylamino)-1,5-diphenyl-3-hydroxypentane.

F. (2S,4S)-2.4-Diamino-1,5-diphenyl-3-hydroxypentane.

The resultant compound of Example 5E (18 mg, 0.049 mmol) was treated with 1 ml of 4 M HCl in dioxane, stirred for 0.5 h at ambient temperature, and concentrated in vacuo. The residue was partitioned between chloroform and aqueous $NaHCO_3$, dried over $Na_2SO_4$ and concentrated to provide the desired compound ($R_f$ 0.12, 10% methanol in chloroform) as a white solid, m.p. 106-107°C. $^1H$ NMR ($CDCl_3$) δ 2.51 (dd, J = 13, 10 Hz, 1 H), 2.67 (dd, J = 13, 9 Hz, 1 H), 2.85-3.0 (m, 2 H), 3.19 (m, 1 H), 3.38 (m, 2 H), 7.15-7.35 (m, 10 H). Mass spectrum: $(M + H)^+$ = 271.

G. (2S,4S)-2,4-Bis-(N-(Cbz-valinyl)-amino)-1,5-diphenyl-3-hydroxypentane.

A solution of 0.65 g (2.4 mmol) of the resultant compound of Example 5F, 2.68 g (7.2 mmol) of N-Cbz-valine p-nitrophenyl ester and 1.34 ml (9.6 mmol) of triethylamine in 6 ml of tetrahydrofuran was heated at reflux under $N_2$ atmosphere for 16 h. The resulting suspension was cooled, diluted with 30 ml of tetrahydrofuran, treated with 10 ml of 3 M aqueous NaOH, and stirred at ambient temperature for 3 h. The mixture was diluted with 250 ml of chloroform, washed with four 100 ml portions of 0.5 M aqueous NaOH, dried over $MgSO_4$, and concentrated in vacuo. Silica gel chromatography of the residue using 5% methanol in dichloromethane provided 1.70 g (96%) of the desired compound as a white solid, m.p. 198-200°C. Mass spectrum $(M+H)^+$ = 737.

Anal. Calcd. for $C_{43}H_{52}N_4O_7 \cdot 0.5H_2O$: C, 69.24; H, 7.16; N, 7.51. Found: C, 69.40; H, 7.29; N, 7.47.

H. (2S,4S)-2,4-Bis-(N-(valinyl)-amino)-1,5-diphenyl-3-hydroxypentane.

A mixture of 1.65 g (2.24 mmol) of the resultant compound of Example 5G and 165 mg of 10% palladium on carbon in 80 ml of methanol was stirred rapidly under an $H_2$ atmosphere for 16 h. The resulting solution was filtered through Celite and concentrated in vacuo to provide 1.04 g (99%) of the desired compound as a white solid, m.p. 131-132°C.

I. (2S,4S)-2,4-Bis-(N-(N-(trans-3-(3-pyridinyl)-2-propenoyl)-valinyl)amino)-1,5-diphenyl-3-hydroxypentane.

A mixture of 100 mg (0.213 mmol) of the resultant compound of Example 5H, 95.5 mg (0.64 mmol) of trans-3-(3-pyridyl)acrylic acid, and 86.5 mg (0.64 mmol) of 1-hydroxybenzotriazole monohydrate in 2 ml of dry dimethylformamide was cooled under $N_2$ atmosphere to 0°C and treated with 122.7 mg (0.64 mmol) of ethyl-(dimethylaminopropyl)carbodiimide. The resulting solution was stirred at 0°C for 0.5 h, then at ambient temperature for 16 h. The resulting mixture was concentrated in vacuo, and the residue was treated with saturated aqueous $NaHCO_3$ and extracted with five 10 ml portions of 10% methanol in dichloromethane. The combined organic layers were dried over $Na_2SO_4$ and concentrated. Silica gel chromatography of the residue using 10% methanol in dichloromethane provided 132 mg (85%) of the desired compound as a white solid, m.p. 271-273°C (dec). Mass spectrum: $(M + H)^+$ = 731.

Example 6

A. N-((4-Phenylpiperazin-1-yl)carbonyl)-valine Methyl Ester.

A solution of 2.016 g (12.8 mmol) of the resultant compound of Example 2A in 50 ml of dichloromethane was treated with 1.96 ml (12.6 mmol) of 1-phenylpiperazine. After being stirred at ambient temperature for 1 h, the solution was diluted with dichloromethane, washed with water, dried over $Na_2SO_4$, and concentrated in vacuo. Purification by silica gel chromatography using 25% ethyl acetate in chloroform provided the desired compound. $^1H$ NMR ($CDCl_3$) δ 0.93 (d, J = 7 Hz, 3 H), 0.97 (d, J = 7 Hz, 3H), 2.15 (m, 1 H), 3.20 (dd, J = 6, 5 Hz, 4 H), 3,58 (m, 4 H), 3.74 (s, 3 H), 4.48 (dd, J = 8, 5 Hz, 1 H), 5.00 (br d, J = 8 Hz, 1 H), 6.90 (t, J = 7 Hz, 1 H), 6.93 (d, J = 7 Hz, 1 H), 7.29 (m, 2 H). Mass spectrum: $(M + H)^+$ = 320.

B. N-((4-Phenylpiperazin-1-yl)carbonyl)-valine.

Using the procedure of Example 3E with the resultant compound of Example 6A provided the desired compound as a foam.

C. N-((4-Phenylpiperazin-1-yl)carbonyl)-valine p-Nitrophenyl Ester.

Using the procedure of Example 3F with the resultant compound of Example 6B provided the crude desired compound.

Example 7 (reference)

A. (2S,3R,4R,5S)-2,5-Bis-(N-Cbz-amino)-3,4-bis-(mesyloxy)- 1,6-diphenylhexane.

A slurry of 1.50 g (2.64 mmol) of (2S,3R,4R,5S)-2,5-bis-(N-Cbz-amino)-3,4-dihydroxy-1,6-diphenylhexane in 50 ml of anhydrous dichloromethane was cooled to 0°C and treated sequentially with 0.43 ml of methanesulfonyl chloride, 64 mg of 4-dimethylaminopyridine and 1.1 ml of triethylamine. The resulting mixture was stirred for 15 h with the temperature being allowed to slowly climb to ambient temperature. After treatment with aqueous $NH_4Cl$, the separated organic layer was washed with aqueous $NaHCO_3$, dried over $MgSO_4$, and concentrated in vacuo to provide 1.70 g (90%) of the desired compound, m.p. 153-155°C. $^1H$ NMR ($CDCl_3$) $\delta$ 2.73 (m, 2 H), 2.92 (m, 2 H), 3.09 (s, 6 H), 4.61 (m, 2 H), 4.83-5.06 (m, 8 H), 7.12-7.37 (m, 20 H).

B. (4S,5S,4'S,5'S)-4,4'-Dibenzyl-5,5'-bis-(oxazolidin-2-one).

A solution of 0.2 g of the resultant compound of Example 7A in 15 ml of dimethylformamide was heated at 120°C under $N_2$ atmosphere for 18 h. After removal of the solvent, the residue was recrystallized from ethyl acetate/hexane to provide 46 mg of the desired compound.

C. (2S,3S,4S,5S)-2,5-Diamino-3,4-dihydroxy-1,6- diphenylhexane.

Using the procedure of Example 1E but replacing the resultant compound of Example 1D with either (2S,3S,4S,5S)-2,5-bis-(N-Cbz-amino)-3,4-dihydroxy-1,6-diphenylhexane or with the resultant compound of Example 7B provided the desired compound. $^1H$ NMR ($CDCl_3$) $\delta$ 2.63 (dd, J = 14, 11 Hz, 2 H), 2.85 (dd, J = 14, 4 Hz, 2 H), 3.60 (dt, J = 11, 4 Hz, 2 H), 3.92 (d, J = 3 Hz, 2 H), 7.2-7.4 (m, 10 H). Mass spectrum: $(M + H)^+$ = 301.

D. (2S,3S,4S,5S)-2,5-Bis-(N-(N-((4-phenylpiperazin-1-yl)carbonyl)-valinyl)amino)-3,4-dihydroxy-1,6-diphenylhexane.

A solution of 105 mg (0.35 mmol) of the resultant compound of Example 7C in 3 ml of dimethylformamide was treated with 0.5 g of the resultant compound of Example 6C. After being stirred at ambient temperature under $N_2$ atmosphere for 16 h, the resulting mixture was diluted with ethyl acetate and washed with five portions of 0.1 M aqueous $K_2CO_3$. The solid product, which was not soluble in ethyl acetate, was collected by filtration, washed on the filter with water, digested on the filter two times with ether and filtered to provide the desired compound as a white solid, m.p. 165-166°C.

Example 8 trans-Ethyl 3-(2-Pyridinyl)acrylate.

A solution of 0.43 g (10.7 mmol) of sodium hydride (60% oil dispersion) in anhydrous tetrahydrofuran was cooled under $N_2$ atmosphere to 0°C and treated dropwise with 2.1 ml (10.5 mmol) of triethylphosphonoacetate. After being stirred for 10 min, the solution was treated with 1.0 ml of pyridine-2-carboxaldehyde, heated at reflux for 2 h, cooled, partitioned between ether and aqueous ammonium chloride, washed sequentially with water and saturated brine, dried over $MgSO_4$, and concentrated. Silica gel chromatography of the residue using 30% ethyl acetate in hexane provided 1.54 g (83%) of the desired compound as an oil. $^1H$ NMR ($CDCl_3$) $\delta$ 1.34 (t, J = 7 Hz, 3 H), 4.28 (q, J = 7 Hz, 2 H), 6.92 (d, J = 15 Hz, 1 H), 7.27 (ddd, J = 8, 5, 2 Hz, 1 H), 7.43 (dt, J = 8, 1 Hz, 1 H), 7.69 (d, J = 15 Hz, 1 H), 7.71 (td, J = 8, 2 Hz, 1 H), 8.66 (dm, 1 H).

Example 9 (reference)

A. Thiazole-2-carboxaldehyde

A solution of 5 g (60 mmol) of thiazole in 20 ml of anhydrous ether was cooled under $N_2$ atmosphere to -78°C and treated over a period of 20 min with a solution of 26 ml of n-butyllithium (2.5 M in hexane) diluted with 10 ml of ether. After addition, the solution was stirred for 30 min and treated with a solution of 6.0 ml (60 mmol) of N-formylmorpholine in 10 ml of anhydrous ether over a period of 10 min. The resulting solution was allowed to warm to ambient temperature

over a period of 4 h, after which it was quenched at 0°C with 4 N aqueous HCl. The mixture was diluted with 4 N HCl, after which the aqueous layer was washed with ether, neutralized to pH 8 with aqueous NaOH and aqueous $NaHCO_3$, extracted with four 50 ml portions of ether, dried over $MgSO_4$, and concentrated in vacuo. The crude product thus obtained (5.02 g, 76%) as a brown solid was of sufficient purity for the next step.

B. trans-Methyl 3-(thiazol-2-yl)-2-propenoate.

Using the procedure of Example 8 but replacing triethylphosphonoacetate with trimethylphosphonoacetate and replacing pyridine-2-carboxaldehyde with the resultant compound of Example 9A provided, after silica gel chromatography using 4:1 hexane:ethyl acetate, a 40% yield of the desired compound as a yellow crystalline solid, m.p.75-75.5°C. $^1H$ NMR ($CDCl_3$) δ 3.83 (s, 3 H), 6.73 (d, J = 15 Hz, 1 H), 7.45 (d, J = 3 Hz, 1 H), 7.80 (d, J = 15 Hz, 1 H), 7.93 (d, J = 3 Hz, 1 H).

C. trans-3-(Thiazol-2-yl)-2-propenoic Acid.

A solution of 1.46 g (8.6 mmol) of the resultant compound of Example 9B in 10 ml of 1,4-dioxane and 5 ml of water was treated with 0.73 g (17 mmol) of lithium hydroxide monohydrate and stirred at ambient temperature for 16 h. The resulting solution was concentrated in vacuo to a volume of 5 ml and acidified to pH 2 with 4 N HCl. The precipitate thus obtained was filtered and dried in vacuo to provide 1.2 g (90%) of the desired compound as an off-white solid, m.p. 185.5-187°C. $^1H$ NMR ($d_6$-DMSO) δ 6.67 (d, J = 15 Hz, 1 H), 7.70 (d, J = 15 Hz, 1 H), 7.95 (d, J = 3 Hz, 1 H), 8.01 (d, J = 3 Hz, 1 H), 12.81 (br s, 1 H). Mass spectrum: $(M + H)^+$ = 156.

D. (2S,3R,4R,5S)-2,5-Bis-(N-(N-(trans-3-(thiazol-2-yl)-2-propenoyl)-valinyl)-amino)-3,4-dihydroxy-1,6-diphenylhexane.

Using the procedure of Example 5I but replacing trans-3-(3-pyridyl)acrylic acid with the resultant compound of Example 9C and replacing the resultant compound of Example 6H with the resultant compound of Example 4C provided the desired compound, m.p. > 260°C. Mass spectrum: $(M + H)^+$ = 773.

Example 10

A. (2S,3S,4R,5S)-3-Acetoxy-2,5-bis-(N-Cbz-amino)-3-bromo-1,6- diphenylhexane.

Using the procedure of Example 1C but replacing (2S,3R,4R,5S)-2,5-bis-(N-Cbz-amino)-3,4-dihydroxy-1,6-diphenylhexane with (2S,3S,4S,5S)-2,5-bis-(N-Cbz-amino)-3,4-dihydroxy-1,6-diphenylhexane provided the desired compound in 11% yield along with (4S,5R,1'S,2'S)-5-(1-acetoxy-2-(N-Cbz-amino)-3-phenylpropyl)-4-benzyl-oxazolidin-2-one in 35% yield. (2S,3S,4R,5S)-3-Acetoxy-2,5-bis-(N-Cbz-amino)-3-bromo-1,6-diphenylhexane: $^1H$ NMR ($CDCl_3$) δ 2.05 (s, 3 H), 2.57 (dd, J = 13, 8 Hz, 1 H), 2.74 (m, 2 H), 2.92 (dd, J = 14, 7 Hz, 1 H), 3.82 (d, J = 9 Hz, 1 H), 4.32 (br q, 1 H), 4.64 (m, 1 H), 4.9-5.1 (m, 6 H), 5.33 (br d, 1 H), 7.0-7.4 (m, 20 H). Mass spectrum: $(M + H)^+$ = 673, 675.

B. (2S,3R,5S)-3-Acetoxy-2,5-bis-(N-Cbz-amino)-1,6- diphenylhexane.

Using the procedure of Example 1D but replacing the resultant compound of Example 1C with the resultant compound of Example 10A provided the desired compound. Mass spectrum: $(M + NH_4)^+$ = 612.

C. (2S,3R,5S)-2,5-Diamino-1,6-diphenyl-3-hydroxyhexane.

Using the procedure of Example 1E but replacing the resultant compound of Example 1D with the resultant compound of Example 10B provided, after silica gel chromatography using first 2% isopropylamine in chloroform followed by 2% methanol and 2% isopropylamine in chloroform, the desired compound contaminated with Sn salts. $^1H$ NMR ($CDCl_3$) δ 1.85 (m, 1 H), 2.43 (dd, J = 13, 10 Hz, 1 H), 2.66 (dd, J = 14, 9 Hz, 1 H), 2.86 (dd, J = 14, 4 Hz, 1 H), 3.0-3.1 (m, 2 H), 3.49 (m, 1 H), 3.89 (m, 1 H), 7.2-7.4 (m, 10 H). Mass spectrum: $(M + H)^+$ = 285.

D. (2S,3R,5S)-2,5-Bis-(N-(N-((N-methyl-N-((2-pyridinyl)-methyl)-amino)-carbonyl)-valinyl)-amino)-1,6-diphenyl-3-hydroxyhexane.

Using the procedure of Example 3G but replacing the resultant compound of Example 1E with the resultant compound of Example 15C provided, after silica gel chromatography using 1.5% methanol in chloroform followed by 2% methanol in chloroform, the desired compound, m.p. 92-96°C, in 65% yield. Mass spectrum: $(M + H)^+$ = 779.

Example 11

A. α-Isocyanato-isoleucine Methyl Ester.

Using the procedure of Example 2A but replacing L-valine methyl ester hydrochloride with L-isoleucine methyl ester hydrochloride provided the desired compound as an oil.

B. N-((N-Methyl-N-((2-pyridinyl)methyl)amino)carbonyl)-isoleucine Methyl Ester.

Using the procedure of Example 3D but replacing the resultant compound of Example 2A with the resultant compound of Example 11A provided the desired compound. $^1H$ NMR ($CDCl_3$) δ 0.92 (t, J = 7 Hz, 3 H), 0.94 (d, J = 7 Hz, 3 H), 1.21 (m, 1 H), 1.46 (m, 1 H), 1.90 (m, 1 H), 3.02 (s, 3 H), 3.71 (s, 3 H), 4.46 (dd, J = 8, 5 Hz, 1 H), 4.53 (s, 2 H), 6.15 (br, 1 H), 7.22 (dd, J = 7, 5 Hz, 1 H), 7.27 (d, J = 7 Hz, 1 H), 7.69 (td, J = 7, 2 Hz, 1 H), 8.55 (br d, 1 H).

C. N-((N-Methyl-N-((2-pyridinyl)methyl)amino)carbonyl)-isoleucine p-Nitrophenyl Ester.

Using the procedures of Example 3E and Example 3F with the resultant compound of Example 11B provided the crude desired compound.

D. (2S,3S,5S)-2,5-Bis-(N-(N-((N-methyl-N-((2-pyridinyl)-methyl)amino)carbonyl)isoleucinyl)amino)-1,6-diphenyl-3-hydroxyhexane.

Using the procedure of Example 3G but replacing the resultant compound of Example 3F with the resultant compound of Example 11C provided, after silica gel chromatography using 2% methanol in chloroform, the desired compound in 68% yield. The pure compound melted at 143-145°C, resolidified, and melted again at 173-174°C. Mass spectrum: $(M + H)^+$ = 807.

Example 12

(2S,3S,5S)-2,5-Bis-(N-Cbz-amino)-1,6-diphenyl-3- hydroxyhexane.

A solution of 200 mg (0.34 mmol) of the resultant compound of Example 1D in 4 ml of methanol was treated with 2 ml of concentrated aqueous ammonium hydroxide. The resulting solution was stirred at ambient temperature for 6 h, and at 50°C for 45 min. An additional 1 ml of concentrated aqueous ammonium hydroxide was added and heating was continued for 1 h. The resulting solution was diluted with 50 ml of dichloromethane, washed sequentially with water and saturated brine, dried over $MgSO_4$, and concentrated in vacuo. Silica gel chromatography of the residue using 25% ethyl acetate in hexane followed by 33% ethyl acetate in hexane provided 161 mg (84%) of the desired compound. $^1H$ NMR ($CDCl_3$) δ 1.63 (m, 2 H), 2.73 (m, 2 H), 2.85 (m, 2 H), 3.05 (br, 1 H), 3.64 (m, 1 H), 3.77 (br q, 1 H), 3.93 (br q, 1 H), 4.78 (br d, 1 H), 5.05 (m, 4 H), 7.0-7.4 (m, 20 H). Mass spectrum: $(M + H)^+$ = S53.

Anal. Calcd for $C_{34}H_{36}N_2O_5$: C, 73.89; H, 6.57; N, 5.07. Found: C, 73.81; H, 6.61; N, 5.04.

Example 13 (reference)

A. trans-(2S,5S)-2,5-Bis-(N-(benzyloxycarbonyl)amino)-1,6- diphenyl-3-hexene.

A solution of 4.64 g of the resultant compound of Example 10A in 48 ml of acetic acid was treated with 1.33 g of zinc dust and stirred at ambient temperature for 3 days. The resulting solution was concentrated in vacuo, taken up in ethyl acetate, washed with saturated aqueous $NaHCO_3$, dried over $MgSO_4$, and concentrated in vacuo to provide 3.27 g (89%) of the desired compound.

B. trans-(2S,5S)-2,5-Diamino-1,6-diphenyl-3-hexene.

A solution of 3.27 g of the resultant compound of Example 13A in 75 ml of 30% HBr in acetic acid was allowed to stand at ambient temperature for 16 h. The resulting solution was concentrated in vacuo, and the residue was washed with hexane to remove benzyl bromide. The solid was then taken up in 1 N NaOH, extracted with three 100 ml. portions of dichloromethane, dried over $Na_2SO_4$, and concentrated. Silica gel chromatography using first 2% isopropylamine in chloroform, then 2% methanol/2% isopropylamine in chloroform provided 1.35 g (83%) of the desired

compound.

C. trans-(2S,5S)-2,5-Bis-(N-(N-((N-methyl-N-((2-pyridinyl)methyl)amino)carbonyl)valinyl)amino)-1,6-diphenyl-3-hexane.

Using the procedure of Example 3G but replacing the resultant compound of Example 1E with the resultant compound of Example 13B provided, after silica gel chromatography using first 1.5% methanol in chloroform then 3% methanol in chloroform, 86 mg (75%) of the desired compound as a white solid. Mass spectrum: $(M + H)^+ = 761$.

Example 14

(2S,3S,5S)-3-Acetoxy-2,5-bis-(N-(N-((2-pyridinyl)methoxycarbonyl)-valinyl)-amino)-1,6-diphenylhexane.

A suspension of 59 mg (0.078 mmol) of the resultant compound of Example 2E in 1 ml of dichloromethane was treated sequentially with 0.017 ml (0.16 mmol) of 4-methylmorpholine, 0.007 ml (0.12 mmol) of acetic anhydride, and 5 mg of 4-dimethylaminopyridine. The resulting mixture was stirred at ambient temperature for 1 h, treated with 10 ml of aqueous $NaHCO_3$, stirred for 30 min, extracted with two 20 ml portions of dichloromethane, dried over $Na_2SO_4$, and concentrated in vacuo. Silica gel chromatography of the residue using 3% methanol in chloroform provided 51.6 mg (83%) of the desired compound.

Example 15

(2S,3S,5S)-2,5-Bis-(N-Boc-amino)-1,6-diphenyl-3- hydroxyhexane.

A solution of 1.0 g (4.1 mmol) of the resultant compound of Example 1E and 1.98 g (9.1 mmol) of di-t-butyldicarbonate in 40 ml of dichloromethane was stirred at ambient temperature for 1 h. The solvent was removed in vacuo, and the residue was chromatographed on silica gel using 25% ethyl acetate in hexane followed by 33% ethyl acetate in hexane to provide 1.32 g (72%) of the desired compound. $^1H$ NMR ($CDCl_3$) δ 1.39 (s, 18 H), 1.62 (t, J = 6 Hz, 2 H), 2.74 (m, 2 H), 2.85 (m, 2 H), 3.65 (m, 2 H), 3.86 (br q, 1 H), 4.54 (br, 1 H), 4.80 (br d, 1 H), 7.05-7.3 (m, 10 H). Mass spectrum: $(M + H)^+ = 485$.

Anal. Calcd for $C_{28}H_{40}N_2O_5$: C, 69.39; H, 8.32; N, 5.78. Found: C, 69.21; H, 8.38; N, 5.73.

Example 16

(2S,3S,5S)-2,5-Bis-(N-(t-butylacetyl)-amino)-1,6-diphenyl-3- hydroxyhexane.

A solution of 150 mg (0.53 mmol) of the resultant compound of Example 1E and 0.18 ml (1.3 mmol) of triethylamine in 6 ml of dichloromethane was cooled under $N_2$ atmosphere to -40°C and treated with 0.15 ml (1.1 mmol) of t-butylacetyl chloride. The resulting solution was stirred at -40°C for 30 min, diluted with 50 ml of dichloromethane, washed sequentially with water and saturated brine, dried over $MgSO_4$, and concentrated in vacuo. Silica gel chromatography of residue using first 25% then 33% ethyl acetate in hexane provided 216 mg (85%) of the desired compound. $^1H$ NMR ($CDCl_3$) δ 0.89 (s, 9 H), 0.95 (s, 9 H), 1.67 (m, 2 H), 1.93 (s, 2 H), 1.97 (s, 2 H), 2.76 (AA', 2 H), 2.88 (d, J = 7 Hz, 2 H), 3.61 (br t, 1 H), 3.97 (br q, 1 H), 4.08 (m, 1 H), 4.62 (br, 1 H), 5.55 (br d, J = 7 Hz, 1 H), 5.77 (br d, J = 9 Hz, 1 H), 7.05-7.3 (m, 10 H). Mass spectrum: $(M + H)^+ = 481$.

Anal. Calcd for $C_{30}H_{44}N_2O_3$: C, 74.96; H, 9.23; N, 5.83. Found: C, 74.41; H, 9.21; N, 5.73.

Example 17

(2S,3S,5S)-2,5-Bis-(N-((4-pyridinyl)methoxycarbonyl)-amino)-1,6-diphenyl-3-hydroxyhexane.

A solution of 0.12 mmol of triphosgene in 2 ml of anhydrous tetrahydrofuran was cooled under $N_2$ atmosphere to - 78°C. A solution of 0.36 mmol of pyridine-4-methanol and 0.36 mmol of 4-methylmorpholine in 1 ml of tetrahydrofuran was added dropwise. The resulting solution was stirred at 78°C for 30 min, treated with a solution of 0.18 mmol of the resultant compound of Example 1E and 0.36 mmol of 4-methylmorpholine in 1 ml of tetrahydrofuran, and stirred at - 10°C for 2 h. The solvent was then removed in vacuo, and the residue was chromatographed on silica gel to provide the desired compound.

Example 18

A. 3-(N-(t-Butyloxycarbonyl)aminomethyl)pyridine.

Using the procedure of Example 3A but replacing 2-(aminomethyl)pyridine with 3-(aminomethyl)pyridine provided the desired compound in 97% yield. $^1$H NMR ($CDCl_3$) δ 1.47 (s, 9 H), 4.33 (br d, J = 6 Hz, 2 H), 4.95 (br, 1 H), 7.27 (br t, J = 6 Hz, 1 H), 7.63 (br d, J = 8 Hz, 1 H), 8.52 (m, 2 H).

B. 3-((N-(t-Butyloxycarbonyl)-N-methylamino)methyl)pyridine.

Using the procedure of Example 3B but replacing the resultant compound of Example 3A with the resultant compound of Example 18A provided the desired compound.

C. 3-(N-Methylamino)methyl)pyridine Dihydrochloride.

Using the procedure of Example 3C but replacing the resultant compound of Example 3B with the resultant compound of Example 18B provided the desired compound.

D. N-((N-Methyl-N-((3-pyridinyl)methyl)amino)carbonyl)- valine Methyl Ester.

Using the procedure of Example 3D but replacing the resultant compound of Example 3C with the resultant compound of Example 24C provided the desired compound. $^1$H NMR ($CDCl_3$) δ 0.90 (d, J = 7 Hz, 3 H), 0.96 (d, J = 7 Hz, 3 H), 2.16 (pd, J = 7, 5 Hz, 1 H), 2.93 (s, 3 H), 3.74 (s, 3 H), 4.49 (dd, J = 9, 5 Hz, 1 H), 4.54 (s, 2 H), 4.95 (br d, J = 9 Hz, 1 H), 7.28 (td, J = 6, 1 Hz, 1 H), 7.61 (ddd, J = 7, 3, 2 Hz, 1 H), 8.53 (m, 2 H). Mass spectrum: $(M + H)^+$ = 280.

E. N-((N-Methyl-N-((3-pyridinyl)-methyl)-amino)-carbonyl)-valine.

Using the procedure of Example 3E but replacing the resultant compound of Example 3D with the resultant compound of Example 18D provided the desired compound.

F. N-((N-Methyl-N-((3-pyridinyl)-methyl)-amino)-carbonyl)-valine p-Nitrophenyl Ester.

Using the procedure of Example 3F but replacing the resultant compound of Example 3E with the resultant compound of Example 18E provided the desired compound.

G. (2S,3S,5S)-2,5-Bis-(N-(N-((N-methyl-N-((3-pyridinyl)-methyl)amino)carbonyl)valinyl)amino)-1,6-diphenyl-3-hydroxy-hexane.

Using the procedure of Example 3G but replacing the resultant compound of Example 3F with the resultant compound of Example 18F provided, after silica gel chromatography using first 2% methanol in chloroform, then 7% methanol in chloroform, and finally 10% methanol in chloroform, the desired compound ($R_f$ 0.19, 10% methanol in chloroform) in 49% yield.

Example 19

A. N-((2-Pyridinyl)methoxycarbonyl)-isoleucine Methyl Ester.

Using the procedure of Example 2B but replacing the resultant compound of Example 2A with the resultant compound of Example 11A provided the desired compound.

B. N-((2-Pyridinyl)methoxycarbonyl)-isoleucine.

Using the procedure of Example 3E but replacing the resultant compound of Example 3D with the resultant compound of Example 19A provided the desired compound.

C. N-((2-Pyridinyl)methoxycarbonyl)-isoleucine p-Nitrophenyl Ester.

Using the procedure of Example 3F but replacing the resultant compound of Example 3E with the resultant

compound of Example 19B provided the desired compound.

D. (2S,3S,5S)-2,5-Bis-(N-(N-((2-pyridinyl)methoxycarbonyl)-isoleucinyl)-amino)-1,6-diphenyl-3-hydroxyhexane.

Using the procedure of Example 1E but replacing the resultant compound of Example 1D with the resultant compound of Example 19C provided, after trituration of the residue with 4:1 ethyl acetate:hexane and filtration, the desired compound. Mass spectrum: $(M + H)^+ = 791$.

Anal. Calcd for $C_{44}H_{56}N_6O_7 \cdot 2H_2O$: C, 64.69; H, 7.40; N, 10.29. Found: C, 64.78; H, 6.90; N, 10.32.

Example 20

(2S,3S,5S)-3-Acetoxy-2,5-bis-(N-(N-((N-methyl-N-((2-pyridinyl)-methyl)-amino)-carbonyl)-valinyl)-amino)-1,6- diphenylhexane.

Using the procedure of Example 14 but replacing the resultant compound of Example 2E with the resultant compound of Example 3G provided, after silica gel chromatography using 3% methanol in chloroform, the desired compound in 89% yield. Mass spectrum: $(M + H)^+ = 821$.

Example 21

(2S,3S,5S)-2,5-Bis-(N-Boc-amino)-1,6-dicyclohexyl-3-hydroxyhexane.

A mixture of 100 mg of the resultant compound of Example 15 and 100 mg of 5% rhodium on carbon in 3 ml of methanol was shaken under 4 atmospheres of $H_2$ for 1 day. The resulting mixture was filtered and concentrated in vacuo. Silica gel chromatography of the residue using 20% ethyl acetate in hexane provided 92 mg (90%) of the desired compound. $^1H$ NMR ($CDCl_3$) $\delta$ 0.75-1.90 (br envelope, 28 H), 1.44 (s, 18 H), 3.30 (br, 1 H), 3.63 (m, 2 H), 3.72 (m, 1 H), 4.41 (br, 1 H), 4.66 (br d, 1 H). Mass spectrum: $(M + H)^+ = 497$.

Anal. Calcd for $C_{28}H_{50}N_2O_5 \cdot 0.75H_2O$: C, 66.17; H, 10.21; N, 5.51. Found: C, 65.98; H, 10.42; N, 5.47.

Example 22

A. (2S,3R,4R,5S)-2,5-Bis-(N-Cbz-amino)-1,6-di-(4-benzyloxyphenyl)-3,4-dihydroxy-hexane.

Using sequentially the procedures of Examples 1A and 1B but replacing Cbz-(L)-phenylalaninol with Cbz-(L)-O-benzyltyrosinol provided a crude mixture which was purified by silica gel chromatography to give the desired compound.

B. (2S,3S,5S)-2,5-Diamino-1,6-(4-hydroxyphenyl)-3-hydroxyhexane.

Using sequentially the procedures of Examples 1C, 1D and 12 but replacing (2S,3R,4R,5S)-2,5-bis-(N-Cbz-amino)-3,4-dihydroxy-1,6-diphenylhexane with the resultant compound of Example 22A provided a compound which was treated with methanol and 10% palladium on carbon, shaken under 4 atmospheres of $H_2$ for 4 h, filtered, and concentrated in vacuo to provide the desired compound.

C. (2S,3S,5S)-2,5-Bis-(N-(N-((2-pyridinyl)methoxycarbonyl)- valinyl)-amino)-1,6-di-(4-hydroxyphenyl)-3-hydroxyhexane.

Using the procedure of Example 2E but replacing the resultant compound of Example 1E with the resultant compound of Example 22B provided the desired compound.

Example 23

A. (2S,3S,5S)-2,5-Bis-(N-(Cbz-threoninyl)amino)-1,6- diphenyl-3-hydroxyhexane.

Using the procedure of Example 5I but replacing the resultant compound of Example 5H with the resultant compound of Example 1E and replacing trans-3-(3-pyridyl)acrylic acid with Cbz-(L)-threonine provided, after silica gel

chromatography, the desired compound.

Example 24

(2S,3S,5S)-2,5-Bis-(N-(Cbz-valinyl)-amino)-1,6-diphenyl-3 hydroxyhexane.

Using the procedure of Example 5G but replacing the resultant compound of Example 5F with the resultant compound of Example 1E provided the desired compound.

Example 25

(2S,3S,5S)-2,5-Bis-(N-(valinyl)-amino)-1,6-diphenyl-3- hydroxyhexane.

Using the procedure of Example 5H but replacing the resultant compound of Example 5G with the resultant compound of Example 24 provided the desired compound.

Example 26

(2S,3S,5S)-2,5-Bis-(N-(N-((2-pyridinyl)methoxycarbonyl)-valinyl)-amino)-1,6-diphenyl-3-(trifluoroacetoxy)-hexane.

Using the procedure of Example 14 but replacing acetic anhydride with trifluoroacetic anhydride and quenching the reaction with pH 6 buffer gave a two-layer mixture. The organic layer was diluted with dichloromethane, separated, dried over $Na_2SO_4$, and concentrated in vacuo to provide the desired compound.
$^1$H NMR ($CDCl_3$) δ 0.69 (d, 3H), 0.72 (d, 3H), 0.81 (d, 3H), 0.85 (d, 3H), 1.63 (m, 1H), 1.94 (m, 1H), 2.08 (m, 2H), 2.66 (m, 2H), 2.81 (m, 2H), 3.81 (dd, 1H), 3.87 (dd, 1H), 4.53 (br, 1H), 5.01 (m, 2H), 5.22-5.28 (m, 6H), 5.92 (br, 1H), 6.04 (br d, 1H), 7.12-7.24 (m, 12H), 7.34 (br t, 2H), 7.72 (td, 2H), 8.60 (br d, 2H). Mass spectrum: $(M+H)^+$ = 849.

Example 27

(2S,3S,5S)-2,5-Bis-(N-(N-((N-methyl-N-((2-pyridinyl)-methyl)-amino)-carbonyl)-valinyl)-amino)-1,6-diphenyl-3-(trifluoroacetoxy)-hexane.

Using the procedure of Example 14 but replacing acetic anhydride with trifluoroacetic anhydride, replacing the resultant compound of Example 2E with the resultant compound of Example 3G, and quenching the reaction with pH 6 buffer gave a two-layer mixture. The organic layer was diluted with dichloromethane, separated, dried over $Na_2SO_4$, and concentrated in vacuo to provide the desired compound.

Example 28

A. ((3-Pyridinyl)methyl)-(4-nitrophenyl)carbonate.

A solution 20 g (0.1 mol) of (4-nitrophenyl)-chloroformate in 150 ml of dichloromethane was cooled to 0°C and treated sequentially with 8.0 ml (0.083 mol) of pyridine-3-methanol and 11 ml (0.1 mol) of 4-methylmorpholine. After addition, the solution was allowed to come to ambient temperature, stirred for 0.5 h, diluted with dichloromethane, washed sequentially with aqueous $NaHCO_3$ and water, dried over $Na_2SO_4$, and concentrated in vacuo. The residue was broken up, triturated with 3:1 hexane:ethyl acetate, and filtered. The resulting solid was dissolved in a minimum amount of boiling ethyl acetate/hexane, filtered hot to remove an insoluble dark oil, and allowed to cool. The desired crystalline product (18.65 g, 82%) was collected by filtration.

B. (2S,3S,5S)-5-Amino-2-(N-((3-pyridinyl)methoxy-carbonyl)amino)-1,6-diphenyl-3-hydroxyhexane and (2S,3S,5S)- 2-Amino-5-(N-((3-pyridinyl)methoxycarbonyl)-amino)-1,6-diphenyl-3-hydroxyhexane.

A solution of 1.5 g (5.28 mmol) of the resultant compound of Example 1E in 10 ml of tetrahydrofuran was treated dropwise over a 5 hour period with a solution of 1.6 g (5.8 mmol) of the resultant compound of Example 28A in 10 ml of tetrahydrofuran. After addition, the resulting solution was stirred at ambient temperature for 16 h and concentrated in vacuo. Silica gel chromatography using a gradient of 2-3.5% methanol in chloroform provided a mixture of the two desired compounds. Silica gel chromatography of the mixture using first 2% isopropylamine in dichloromethane followed by 2% isopropylamine/2% methanol in dichloromethane provided 0.38 g (16%) of (2S,3S,5S)-5-amino-2-(N-((3-

pyridinyl)methoxycarbonyl)amino)-1,6-diphenyl-3-hydroxyhexane and 0.87 g (36%) of (2S,3S,5S)-2-amino-5-(N-((3-pyridinyl)methoxycarbonyl)amino)-1,6-diphenyl-3-hydroxyhexane.

C. (2S,3S,5S)-2-(N-(N-((N-Methyl-N-((2-pyridinyl)-methyl)amino)carbonyl)valinyl)amino)-5-(N-((3-pyridinyl)-methoxycarbonyl)amino)-1,6-diphenyl-3-hydroxyhexane.

A solution of 1.2 g (2.86 mmol) of (2S,3S,5S)-2-amino-5-(N-((3-pyridinyl)methoxycarbonyl)amino)-1,6-diphenyl-3-hydroxyhexane in 20 ml of tetrahydrofuran was treated with 1.55 g (4.01 mmol) of the resultant compound of Example 3F. The resulting solution was stirred at ambient temperature for 96 h, treated with aqueous $NaHCO_3$, extracted with chloroform, dried over $Na_2SO_4$, and concentrated in vacuo. The residued was purified by silica gel chromatography using first 2% then 4% methanol in chloroform to provide 1.75 g (92%) of the desired compound($R_f$ 0.28, 10% methanol in chloroform) as a white solid, m.p. 69-71°C. Mass spectrum: $(M + 1)^+ = 667$.

Anal. Calcd for $C_{38}H_{46}N_6O_5 \cdot 0.5H_2O$: C, 67.54; H, 7.01; N, 12.44. Found: C, 67.54; H, 6.83; N, 12.33.

Example 29

(2S,3S,5S)-5-(N-(N-((N-Methyl-N-((2-pyridinyl)-methyl)amino)carbonyl)valinyl)amino)-2-(N-((3-pyridinyl)-methoxycarbonyl)amino)-1,6-diphenyl-3-hydroxyhexane.

A solution of 0.95 g (2.27 mmol) of (2S,3S,5S)-5-amino-2-(N-((3-pyridinyl)methoxycarbonyl)amino)-1,6-diphenyl-3-hydroxyhexane in 15 ml of tetrahydrofuran was treated with 1.22 g (3.17 mmol) of the resultant compound of Example 3F. The resulting solution was stirred at ambient temperature for 24 h, treated with aqueous $NaHCO_3$, extracted with chloroform, dried over $Na_2SO_4$, and concentrated in vacuo. The residued was purified by silica gel chromatography using first 2% then 4% methanol in chloroform to provide 1.46 g (94%) of the desired compound($R_f$ 0.26, 10% methanol in chloroform) as a white solid, m.p. 58-61°C. Mass spectrum: $(M + 1)^+ = 667$.

Anal. Calcd for $C_{38}H_{46}N_6O_5 \cdot 1.1H_2O$: C, 66.47; H, 7.08; N, 12.24. Found: C, 66.12; H, 6.68; N, 12.10.

Example 30

(2S,3S,5S)-2-(N-(N-((2-Pyridinyl)methoxycarbonyl)-valinyl)amino)-5-(N-((3-pyridinyl)methoxycarbonyl)amino)-1,6-diphenyl-3-hydroxyhexane.

Using the procedure of Example 28C but replacing the resultant compound of Example 3F with the resultant compound of Example 2D provided, after silica gel chromatography using a gradient of 2-5% methanol in chloroform, 104 mg (95%) of the desired compound ($R_f$ 0.30, 10% methanol in chloroform) as a white solid, m.p. 169-171°C. Mass spectrum: $(M + 1)^+ = 654$.

Anal. Calcd for $C_{37}H_{43}N_5O_6 \cdot 0.5H_2O$: C, 67.05; H, 6.69; N, 10.51. Found: C, 66.98; H, 6.53; N, 10.57.

Example 31

(2S,3S,5S)-5-(N-(N-((2-Pyridinyl)methoxycarbonyl)-valinyl)amino)-2-(N-((3-pyridinyl)methoxycarbonyl)amino)-1,6-diphenyl-3-hydroxyhexane.

Using the procedure of Example 29 but replacing the resultant compound of Example 3F with the resultant compound of Example 2D provided, after silica gel chromatography using a gradient of 2-5% methanol in chloroform, 102 mg (94%) of the desired compound ($R_f$ 0.30, 10% methanol in chloroform) as a white solid, m.p. 172-174°C. Mass spectrum: $(M + 1)^+ = 654$.

Anal. Calcd for $C_{37}H_{43}N_5O_6 \cdot 0.5H_2O$: C, 67.05; H, 6.69; N, 10.51. Found: C, 66.70; H, 6.41; N, 10.37.

Example 32

A. 2-(((N-Methyl)amino)methyl)thiazole.

A mixture of 2.0 g (17.7 mmol) of the resultant compound of Example 9A, 4.78 g (71 mmol) of methylamine

hydrochloride, 4.36 g (53 mmol) of sodium acetate and 1.67 g (27 mmol) of sodium cyanoborohydride in 50 ml of isopropyl alcohol was stirred at ambient temperature for 3 days. The resulting mixture was concentrated in vacuo, and the residue was taken up in ethyl acetate and extracted with saturated aqueous $NaHCO_3$. The aqueous layer was concentrated in vacuo to a small volume, saturated with NaCl, and extracted with 10% methanol in chloroform until no product remained in the aqueous layer by tlc. The combined organic layers were dried over $Na_2SO_4$ and concentrated in vacuo. Silica gel chromatography using first 5% then 10% methanol in chloroform provided 0.4 g (18%) of the desired compound.

B. N-((N-Methyl-N-((2-thiazolyl)methyl)amino)carbonyl)-valine Methyl Ester.

A solution of 0.4 g (3.1 mmol) of the resultant compound of Example 32A and 3.1 mmol of the resultant compound of Example 2A in 10 ml of dichloromethane was stirred at ambient temperature for 1.5 h. The resulting solution was concentrated in vacuo, and the residue was purified by silica gel chromatography using first 1% then 2% methanol in chloroform to provide 0.57 g (64%) of the pure desired compound ($R_f$ 0.61, 10% methanol in chloroform).

C. N-((N-Methyl-N-((2-thiazolyl)methyl)amino)carbonyl)-valine.

A solution of 0.57 g (2.0 mmol) of the resultant compound of Example 32B in 8 ml of dioxane was treated with 8 ml (4.0 mmol) of 0.5 M aqueous lithium hydroxide. After being stirred at ambient temperature for 1 h, the resulting solution was neutralized with 1N aqueous HCl, concentrated in vacuo to a small volume, saturated with NaCl, and extracted with two 100 ml portions of ethyl acetate. The combined organic layers were dried over $Na_2SO_4$ and concentrated in vacuo to provide the desired compound.

D. N-((N-Methyl-N-((2-thiazolyl)methyl)amino)carbonyl)- valine p-Nitrophenyl Ester.

A solution of 2.0 mmol of the resultant compound of Example 32C and 0.3 g (2.2 mmol) of 4-nitrophenol in 10 ml of tetrahydrofuran was treated with 0.43 g (2.2 mmol) of dicyclohexyl carbodiimide. After being stirred for 3 h at ambient temperature, the mixture was filtered and the residue was washed with 10 ml of fresh tetrahydrofuran. The combined filtrates were concentrated in vacuo to provide the crude desired compound ($R_f$ 0.11, 20% ethyl acetate in chloroform).

E. (2S,3S,5S)-2-(N-(N-C(N-Methyl-N-((2-thiazolyl)-methyl)amino)carbonyl)valinyl)amino)-5-(N-((3-pyridinyl)- methoxycarbonyl)amino)-1,6-diphenyl-3-hydroxyhexane.

Using the procedure of Example 28C but replacing the resultant compound of Example 3F with the resultant compound of Example 32D provided the desired compound.

Example 33

A. 4-(((N-Methyl)amino)methyl)thiazole.

Aqueous methylamine (100 ml, 40% by weight) was treated with 1.1 g (6.5 mmol) of 4-(chloromethyl)thiazole hydrochloride. The resulting solution was stirred at ambient temperature for 15 min, concentrated in vacuo, taken up in 5% methanol in chloroform, dried over $Na_2SO_4$, and concentrated in vacuo to provide 0.81 g (97%) of the crude desired compound.

B. N-((N-Methyl-N-((4-thiazolyl)methyl)amino)carbonyl)-valine p-Nitrophenyl Ester.

Using sequentially the procedures of Examples 32B, 32C, and 32D, but replacing the resultant compound of Example 32A with the resultant compound of Example 33A provided the desired compound ($R_f$ 0.17, 20% ethyl acetate in chloroform).

C. (2S,3S,5S)-2-(N-(N-((N-Methyl-N-((4-thiazolyl)- methyl)amino)carbonyl)valinyl)amino)-5-(N-((3-pyridinyl)-methoxycarbonyl)amino)-1,6-diphenyl-3-hydroxyhexane.

Using the procedure of Example 37C but replacing the resultant compound of Example 3F with the resultant compound of Example 33B provided, after silica gel chromatography using a gradient of 2-3-5% methanol in chloroform, a 49% of the desired compound ($R_f$ 0.21, 10% methanol in chloroform) as a white solid. Mass spectrum: $(M + 1)^+$

= 673. m.p. 71-74°C.

Anal. Calcd for $C_{36}H_{44}N_6O_5S \cdot 0.15CHCl_3$: C, 62.87; H, 6.42; N, 12.17. Found: C, 62.63; H, 6.19; N, 12.02.

Example 34

(2S,3S,5S)-5-(N-(N-((N-Methyl-N-((4-thiazolyl)-methyl)amino)carbonyl)valinyl)amino)-2-(N-((3-pyridinyl)-methoxycarbonyl)amino)-1,6-diphenyl-3-hydroxyhexane.

Using the procedure of Example 29 but replacing the resultant compound of Example 3F with the resultant compound of Example 33B provided, after silica gel chromatography using a gradient of 2-3-5% methanol in chloroform, a 43% of the desired compound ($R_f$ 0.23, 10% methanol in chloroform) as a white solid. Mass spectrum: $(M + 1)^+$ = 673. m.p. 69-73°C.

Anal. Calcd for $C_{36}H_{44}N_6O_5S \cdot 0.2CHCl_3$: C, 62.42; H, 6.37; N, 12.07. Found: C, 62.34; H, 6.11; N, 11.97.

Example 35

A. 2-Amino-4-(((N-Methyl)amino)methyl)thiazole.

Using the procedure of Example 33A but replacing 4-(chloromethyl)thiazole hydrochloride with 2-amino-4-(chloromethyl)thiazole dihydrochloride provided the crude desired compound.

B. N-((N-Methyl-N-((2-amino-4-thiazolyl)methyl)amino)carbonyl)valine Methyl Ester.

A solution of 4.26 g (27 mmol) of the resultant compound of Example 2A in 100 ml of dichloromethane was added to 27 mmol of the crude resultant compound of Example 35A followed by 3 ml (54 mmol) of 4-methylmorpholine. The resulting mixture was stirred for 16 h at ambient temperature, washed with saturated aqueous sodium bicarbonate, dried over $Na_2SO_4$, and concentrated in vacuo to give the crude desired compound.

C. N-((N-Methyl-N-((2-((((t-butyl)oxy)carbonyl)amino)-4-thiazolyl)methyl)amino)carbonyl)valine Methyl Ester.

A solution of 1.0 g (3.33 mmol) of the crude resultant compound of Example 35B in 40 ml of dichloromethane was treated sequentially with 0.87 g (4 mmol) of di-t-butyldicarbonate and 10 mg of 4-dimethylaminopyridine. The resulting solution was stirred at ambient temperature for 3 days, washed with 10% citric acid, dried over $Na_2SO_4$, and concentrated in vacuo. Silica gel chromatography of the residue using first 30% then 40% ethyl acetate in chloroform provided 0.65 g (49%) of the desired compound ($R_f$ 0.58, 10% methanol in chloroform) as a foam.

D. N-((N-Methyl-N-((2-((((t-butyl)oxy)carbonyl)amino)-4-thiazolyl)methyl)amino)carbonyl)valine Lithium Salt.

A solution of 0.62 g (1.55 mmol) of the resultant compound of Example 35C in 6.2 ml of dioxane was treated with 6.2 ml (3.1 mmol) of 0.5 M aqueous lithium hydroxide. After being stirred for 2 h at ambient temperature, the resulting solution was concentrated in vacuo to give the crude desired compound.

E. (2S,3S,5S)-2-(N-(N-((N-Methyl-N-((2-((((t-butyl)oxy)-carbonyl)amino)-4-thiazolyl)methyl)amino)carbonyl)-valinyl)amino)-5-(N-((3-pyridinyl)methoxycarbonyl)amino)-1,6-diphenyl-3-hydroxyhexane.

To a solution of 70 mg (0.17 mmol) of (2S,3S,5S)-2-amino-5-(N-((3-pyridinyl)methoxycarbonyl)-amino)-1,6-diphenyl-3-hydroxyhexane, 0.20 mmol of the resultant compound of Example 35D 34 mg (0.25 mmol) of 1-hydroxybenzotriazole monohydrate and 37 µL (0.34 mmol) of 4-methylmorpholine in 1 ml of tetrahydrofuran was added 48 mg (0.25 mmol) of ethyl(3-dimethylaminopropyl)-carbodiimide. The resulting solution was stirred for 16 h at ambient temperature, diluted with chloroform, washed with saturated aqueous $NaHCO_3$, dried over $Na_2SO_4$, and concentrated in vacuo. Silica gel chromatography using sequentially 1.5% and 3% methanol in chloroform provided 97.2 mg (72.5%) of the desired compound ($R_f$ 0.59, 10% methanol in chloroform) as a white solid, m.p. 95-98°C. Mass spectrum: $(M + 1)^+$ = 788.

Example 36

(2S,3S,5S)-5-(N-(N-((N-Methyl-N-((2-((((t-butyl)oxy)-carbonyl)amino)-4-thiazolyl)methyl)amino)carbonyl)-valinyl)amino)-2-(N-((3-pyridinyl)methoxycarbonyl)amino)-1,6-diphenyl-3-hydroxyhexane.

Using the procedure of Example 35E but replacing (2S,3S,5S)-2-amino-5-(N-((3-pyridinyl)methoxycarbonyl)-amino)-1,6-diphenyl-3-hydroxyhexane with (2S,3S,5S)-5-amino-2-(N-((3-pyridinyl)methoxycarbonyl)amino)-1,6-diphenyl-3-hydroxyhexane provided, after silica gel chromatography using a gradient of 1.5% - 2% - 3% methanol in chloroform, 96 mg (71.6%) of the desired compound ($R_f$ 0.60, 10% methanol in chloroform) as a white solid, m.p. 103-105°C. Mass spectrum: $(M + 1)^+$ = 788.

Anal. Calcd for $C_{41}H_{53}N_7O_7S \cdot 0.75H_2O$: C, 61.44; H, 6.85; N, 12.23. Found: C, 61.16; H, 6.64, N, 11.91.

Example 37 (reference)

A. (4S,5S,1'R,2'S)-5-(1-Acetoxy-2-(N-Cbz-amino)-3- phenylpropyl)-4-benzyl-oxazolidin-2-one.

A suspension of 5.02 g (8.80 mmol) of (2S,3R,4R,5S)-2,5-bis-(N-Cbz-amino)-3,4-dihydroxy-1,6-diphenylbexane in 400 ml of acetonitrile was treated dropwise with 3 ml (20 mmol) of α-acetoxyisobutyryl bromide. The resulting solution was stirred under $N_2$ atmosphere at ambient temperature for 2 h, filtered to remove traces of solid starting material, quenched cautiously with 100 ml of aqueous $NaHCO_3$, and concentrated in vacuo to a volume of 100 ml. The resulting mixture was extracted with two 100 ml portions of dichloromethane, dried over $Na_2SO_4$, and concentrated in vacuo. The residue was purified by silica gel chromatography using first 10% then 25% ethyl acetate in dichloromethane to provide 3.15 g (71%) of the desired compound as a white foam. $^1H$ NMR ($CDCl_3$) δ 2.09 (s, 3 H), 2.53 (br t, J = 12 Hz, 1 H), 2.72 (dd, J = 13, 3 Hz, 1 H), 2.83 (dd, J = 14, 8 Hz, 1 H), 2.95 (dd, J = 14, 7 Hz, 1 H), 3.95 (m, 1 H), 4.45 (m, 1 H), 4.8 (m, 2 H), 5.0-5.1 (m, 3 H), 5.29 (dd, J = 9, 3 Hz, 1 H), 7.0-7.4 (m, 10 H). Mass spectrum: $(M + NH_4)^+$ = 520.

B. (2S,3R,4R,5S)-2,5-Bis-(N-Cbz-amino)-1,6-diphenyl-2- hydroxy-3-mesyloxyhexane.

A slurry of 1.098 g (1.93 mmol) of (2S,3R,4R,5S)-2,5-bis-(N-Cbz-amino)-3,4-dihydroxy-1,6-diphenylhexane in 50 ml of anhydrous dichloromethane was treated sequentially with 0.313 ml of methanesulfonylchloride, 0.546 ml of triethylamine and 23 mg of 4-dimethylaminopyridine. After being stirred for 24 h at ambient temperature, the solution was washed sequentially with aqueous $NH_4Cl$ and aqueous $NaHCO_3$, dried over $MgSO_4$, and concentrated in vacuo. The residue was purified by silica gel chromatography using 5% ethyl acetate in dichloromethane to provide 560 mg (45%) of the desired compound, m.p. 68-71°C. $^1H$ NMR ($CDCl_3$) δ 2.7-3.0 (m, 4 H), 3.17 (s, 3 H), 3.69 (m, 1 H), 3.92 (m, 1 H), 4.19 (br s, 1 H), 4.45 (m, 1 H), 4.68 (m, 1 H), 4.87-5.09 (m, 6 H), 7.1-7.4 (m, 20 H).

C. (4S,5S,1'R,2'S)-4-Benzyl-5-(2-(N-Cbz-amino)-1-hydroxy-3-phenylpropyl)-oxazolidin-2-one.

A solution of 320 mg (0.49 mmol) of the resultant compound of Example 37B in 15 ml of acetonitrile was heated at reflux under $N_2$ atmosphere for 18 h. After being allowed to cool, the solvent was removed in vacuo and the residue was recrystallized from ethyl acetate/ hexane to provide 89 mg (39%) of the desired compound.

D. (2S,3R,4S,5S)-2,5-Diamino-3,4-dihydroxy-1,6- diphenylhexane.

Using the procedure of Example 1E but replacing the resultant compound of Example 1D with either the resultant compound of Example 37A or the resultant compound of Example 13C provided the desired compound mixed with benzyl alcohol. Purification of a small portion by silica gel chromatography using 5% methanol/2% isopropylamine in chloroform provided the pure desired compound., m.p. 115-119°C. $^1H$ NMR ($CDCl_3$) δ 2.46 (dd, J = 14, 9 Hz, 1 H), 2.61 (dd, J = 14, 11 Hz, 1 H), 3.02 (td, J = 9, 3 Hz, 1 H), 3.19 (dd, J = 14, 4 Hz, 1 H), 3.35-3.4 (m, 2 H), 3.51 (t, J = 9 Hz, 1 H), 3.76 (dd, J = 9, 3 Hz, 1 H), 7.2-7.4 (m, 10 H).

Example 38 (reference)

A. (2S,3R,4S,5S)-5-Amino-2-(N-(N-((N-methyl-N-((2- pyridinyl)methyl)amino)carbonyl)valinyl)amino)-3,4-dihydroxy-1,6-diphenylhexane.

A solution of 0.40 g (0.133 mmol) of the resultant compound of Example 37D and 0.57 g (0.147 mmol) of

the resultant compound of Example 3F in 10 ml of tetrahydrofuran was stirred at ambient temperature for 16 h. The resulting solution was diluted with 50 ml of chloroform, washed with several portions of 3N aqueous NaOH, dried over $Na_2SO_4$, and concentrated in vacuo. Silica gel chromatography of the residue using sequentially 3%, 5% and 10% methanol in chloroform provided 0.41 g (56%) of the desired compound ($R_f$ 0.15, 10% methanol in chloroform).

B. (2S,3R,4S,5S)-2-(N-(N-((N-Methyl-N-((2-pyridinyl)-methyl)amino)carbonyl)valinyl)amino)-5-(N-((3-pyridinyl)methoxycarbonyl)amino)-3,4-dihydroxy-1,6-diphenylhexane.

A solution of 70 mg (0.13 mmol) of the resultant compound of Example 38A and 42 mg (0.15 mmol) of the resultant compound of Example 28A in 1 ml of tetrahydrofuran was stirred at ambient temperature for 16 h. The resulting solution was concentrated in vacuo, and the residue was purified by silica gel chromatography using a gradient of 2%-3.5% methanol in chloroform to provide 72 mg (83%) of the desired compound ($R_f$ 0.33, 10% methanol in chloroform) as a white solid, m.p. 86-88°C. Mass spectrum: $(M + 1)^+ = 683$.

Anal. Calcd for $C_{38}H_{46}N_6O_6 \cdot 0.5H_2O$: C, 65.97; H, 6.85; N, 12.15. Found: C, 65.79; H, 6.53; N, 11.95.

Example 39

A. N-((3-Pyridinyl)methoxycarbonyl)valine p-Nitrophenyl Ester.

Using the procedures of Examples 2B, 2C and 2D but replacing pyridine-2-methanol with pyridine-3-methanol provided the desired compound.

B. (2S,3S,5S)-5-(N-(N-((3-Pyridinyl)methoxycarbonyl)-valinyl)amino)-2-(N-((3-pyridinyl)methoxycarbonyl)amino)-1,6-diphenyl-3-hydroxyhexane.

Using the procedure of Example 29 but replacing the resultant compound of Example 3F with the resultant compound of Example 39A provided, after silica gel chromatography using a gradient of 2-3.5% methanol in chloroform, 81 mg (87%) of the desired compound ($R_f$ 0.30, 10% methanol in chloroform) as a white solid. Mass spectrum: $(M + 1)^+ = 654$.

Example 40

(2S,3S,5S)-2-(N-(N-((3-Pyridinyl)methoxycarbonyl)-valinyl)amino)-5-(N-((3-pyridinyl)methoxycarbonyl)amino)-1,6-diphenyl-3-hydroxyhexane.

Using the procedure of Example 28C but replacing the resultant compound of Example 3F with the resultant compound of Example 39A provided, after silica gel chromatography using a gradient of 2-3.5% methanol in chloroform, 76 mg (81%) of the desired compound ($R_f$ 0.30, 10% methanol in chloroform) as a white solid. Mass spectrum: $(M + 1)^+ = 654$.

Example 41

A. N-((2-Thiazolyl)methoxycarbonyl)valine p-Nitrophenyl Ester.

Using the procedures of Examples 2B, 2C and 2D but replacing pyridine-2-methanol with 2-(hydroxymethyl)-thiazole (Dondoni, et. al., *Synthesis*, **1987**, 998; *Tetrahedron Lett*. **1983**, *24*, 2901) provided the desired compound.

B. (2S,3S,5S)-5-(N-(N-((2-Thiazolyl)methoxycarbonyl)-valinyl)amino)-2-(N-((3-pyridinyl)methoxycarbonyl)amino)-1,6-diphenyl-3-hydroxyhexane.

Using the procedure of Example 29 but replacing the resultant compound of Example 3F with the resultant compound of Example 41A provided, after silica gel chromatography using a gradient of 2-3.5% methanol in chloroform, 69 mg (86%) of the desired compound ($R_f$ 0.36, 10% methanol in chloroform) as a white solid. Mass spectrum: $(M + 1)^+ = 660$.

Example 42

(2S,3S,5S)-2-(N-(N-((2-Thiazolyl)methoxycarbonyl)valinyl)amino)-5-(N-((3-pyridinyl)methoxycarbonyl)amino)-1,6-diphenyl-3-hydroxyhexane.

Using the procedure of Example 28C but replacing the resultant compound of Example 3F with the resultant compound of Example 41A provided, after silica gel chromatography using a gradient of 2-3.5% methanol in chloroform, a 90% of the desired compound ($R_f$ 0.36, 10% methanol in chloroform) as a white solid. Mass spectrum: $(M + 1)^+$ = 660.

Example 43

A. 4-(Chloromethyl)-2-methylthiazole.

A mixture of 7.13 g (56 mmol) of 1,3-dichloroacetone, 3.83 g (51 mmol) of thioacetamide and 4.73 g (56 mmol) of $NaHCO_3$ in 40 ml of dichloroethane was stirred at ambient temperature for four days. The resulting mixture was filtered and the filter cake was washed with fresh dichloroethane. The combined filtrates were added slowly to a precooled (0°C) solution of 4.1 ml (56 mmol) of thionyl chloride in 30 ml of dichloroethane. The resulting mixture was heated at 70°C for 40 min, cooled, and filtered. The residue was washed with a small amount of dichloromethane and dried under vacuum at 50°C to provide 3.0 g of the crude desired compound.

B. 4-((N-Methyl)aminomethyl)-2-methylthiazole.

The resultant compound of 43A (1.0 g) was added slowly in portions to 100 ml of a rapidly stirred 40% aqueous solution of methylamine. After being stirred for 1 h, the solution was concentrated in vacuo, taken up in dichloromethane, dried over $Na_2SO_4$, and concentrated to provide the crude desired compound as a yellow oil.

C. N-((4-Nitrophenyloxy)carbonyl)valine Methyl Ester.

A solution of 1.36 g (6.8 mmol) of 4-(nitrophenyl) chloroformate in 50 ml of dichloromethane was cooled to 0°C and treated sequentially with 1.03 g (6.1 mmol) of valine methyl ester hydrochloride and 1.42 ml (13 mmol) of 4-methylmorpholine. The resulting solution was stirred at ambient temperature for 1 h, diluted with dichloromethane, washed with aqueous $NaHCO_3$, dried over $Na_2SO_4$, and concentrated to give the crude desired compound.

D. N-((N-Methyl-N-((2-methyl-4-thiazolyl)methyl)amino)-carbonyl)valine Methyl Ester.

A mixture of 5.4 mmol of the crude resultant compound of Example 43B and 6.1 mmol of the crude resultant compound of Example 43C was treated with 0.5 mmol of 4-dimethylpyridine in 40 ml of toluene and heated at reflux for 4 h. The resulting solution was concentrated in vacuo, taken up in dichloromethane, washed sequentially with aqueous $NaHCO_3$ and 10% citric acid, dried over $Na_2SO_4$, and concentrated in vacuo. Silica gel chromatography using first chloroform, then 2%, then 5% methanol in chloroform provided 1.1 g of the desired compound.

E. N-((N-Methyl-N-((2-methyl-4-thiazolyl)methyl)amino)- carbonyl)valine p-Nitrophenyl Ester.

Using the procedures of Examples 32C and 32D but replacing the resultant compound of Example 32B with the resultant compound of Example 43D provided the desired compound.

F. (2S,3S,5S)-2-(N-(N-((N-Methyl-N-((2-methyl-4-thiazolyl)methyl)amino)carbonyl)valinyl)amino)-5-(N-((3-pyridinyl)methoxycarbonyl)amino)-1,6-diphenyl-3-hydroxyhexane.

Using the procedure of Example 28C but replacing the resultant compound of Example 3F with the resultant compound of Example 43E provided, after silica gel chromatography using a gradient of 1.5-3-5% methanol in chloroform, a 72% of the desired compound ($R_f$ 0.28, 10% methanol in chloroform) as a white solid. Mass spectrum: $(M + 1)^+$ = 687. m.p. 66-69°C.

Example 44

A. (2S,3S,5S)-2-(N-(N-((N-Methyl-N-((2-((((t-butyl)oxy)-carbonyl)amino)-4-thiazolyl)methyl)amino)carbonyl)-valinyl)amino)-5-(N-(((t-butyl)oxy)carbonyl)amino)-1,6-diphenyl-3-hydroxyhexane.

Using the procedure of Example 35E but replacing (2S,3S,5S)-2-amino-5-(N-((3-pyridinyl)methoxycarbonyl)-amino)-1,6-diphenyl-3-hydroxyhexane with (2S,3S,5S)-2-amino-5-(N-(((t-butyl)oxy)carbonyl)amino)-1,6-diphenyl-3-hydroxyhexane provided 274 mg (93%) of the crude desired compound ($R_f$ 0.43, 10% methanol in chloroform).

B. (2S,3S,5S)-5-Amino-2-(N-(N-((N-methyl-N-((2-amino-4-thiazolyl)methyl)amino)carbonyl)valinyl)amino)-1,6-diphenyl- 3-hydroxyhexane.

Using the procedure of Example 45B but replacing the resultant compound of Example 45A with the resultant compound of Example 44A provided the desired compound.

C. (2S,3S,5S)-2-(N-(N-((N-Methyl-N-((2-amino-4-thiazolyl)methyl)amino)carbonyl)valinyl)amino)-5-(N-((3- pyridinyl)methoxycarbonyl)amino)-1,6-diphenyl-3-hydroxyhexane.

Using the procedure of Example 38B but replacing the resultant compound of Example 38A with the resultant compound of Example 44B provided, after silica gel chromatography using a gradient of 2% - 3% - 3.5% - 5% - 7% methanol in chloroform, 42 mg (50%) of the desired compound ($R_f$ 0.22, 10% methanol in chloroform). Mass spectrum: $(M + 1)^+ = 688$.

Example 45

A. (2S,3S,5S)-5-(N-(N-((N-Methyl-N-((2-((((t-butyl)oxy)-carbonyl)amino)-4-thiazolyl)methyl)amino)carbonyl)-valinyl)amino)-2-(N-(((t-butyl)oxy)carbonyl)amino)-1,6-diphenyl-3-hydroxyhexane.

Using the procedure of Example 35E but replacing (2S,3S,5S)-2-amino-5-(N-((3-pyridinyl)methoxycarbonyl)-amino)-1,6-diphenyl-3-hydroxyhexane with (2S,3S,5S)-5-amino-2-(N-(((t-butyl)oxy)carbonyl)amino)-1,6-diphenyl-3-hydroxyhexane provided, after silica gel chromatography, 283 mg (96%) of the desired compound ($R_f$ 0.43, 10% methanol in chloroform).

B. (2S,3S,5S)-2-Amino-5-(N-(N-((N-methyl-N-((2-amino-4-thiazolyl)methyl)amino)carbonyl)valinyl)amino)-1,6-diphenyl-3-hydroxyhexane.

A solution of 283 mg of the resultant compound of Example 45A in 10 ml of dichloromethane was treated with 5 ml of trifluoroacetic acid and stirred overnight at ambient temperature. The resulting solution was concentrated in vacuo, partitioned between saturated aqueous $NaHCO_3$ and chloroform, dried over $Na_2SO_4$, and concentrated to provide the desired ($R_f$ 0.49, 2% isopropylamine/5% methanol in chloroform).

C. (2S,3S,5S)-5-(N-(N-((N-Methyl-N-((2-amino-4-thiazolyl)methyl)amino)carbonyl)valinyl)amino)-2-(N-((3- pyridinyl)methoxycarbonyl)amino)-1,6-diphenyl-3- hydroxyhexane.

Using the procedure of Example 38B but replacing the resultant compound of Example 38A with the resultant compound of Example 45B provided, after silica gel chromatography using a gradient of 2% - 3.5% - 5% methanol in chloroform, 48 mg (58%) of the desired compound. Mass spectrum: $(M + 1)^+ = 688$.

Example 46

A. 2-(N-Ethylamino)methyl)pyridine.

Using the procedure of Example 54A but replacing quinoline-2-carboxaldehyde with pyridine-2-carboxaldehyde and replacing methylamine with ethylamine provided the crude desired compound.

B. N-((N-Ethyl-N-((2-pyridinyl)methyl)amino)carbonyl)-valine p-Nitrophenyl Ester.

Using sequentially the procedures of Examples 32B, 32C, and 32D, but replacing the resultant compound of

Example 32A with the resultant compound of Example 46A provided the desired compound.

C. (2S,3S,5S)-5-(N-(N-((N-Ethyl-N-((2-pyridinyl)-methyl)amino)carbonyl)valinyl)amino)-2-(N-((3-pyridinyl)-methoxycarbonyl)amino)-1,6-diphenyl-3-hydroxyhexane.

Using the procedure of Example 29 but replacing the resultant compound of Example 3F with the resultant compound of Example 46B provided, after silica gel chromatography using a gradient of 2-5% methanol in chloroform, a 88% of the desired compound ($R_f$ 0.28, 10% methanol in chloroform) as a white solid. Mass spectrum: $(M + 1)^+ = 861$.

Example 47

(2S,3S,5S)-2-(N-(N-((N-Ethyl-N-((2-pyridinyl)- methyl)amino)carbonyl)valinyl)amino)-5-(N-((3-pyridinyl)- methoxycarbonyl)amino)-1,6-diphenyl-3-hydroxyhexane.

Using the procedure of Example 28C but replacing the resultant compound of Example 3F with the resultant compound of Example 59B provided, after silica gel chromatography using a gradient of 2-5% methanol in chloroform, a 93% of the desired compound ($R_f$ 0.28, 10% methanol in chloroform) as a white solid. Mass spectrum: $(M + 1)^+ = 861$

Example 48

A. (2S,3S,5S)-5-Amino-2-(N-(((t-butyl)oxy)-carbonyl)amino)-1,6-diphenyl-3-hydroxyhexane and (2S,3S,5S)-2-Amino-5-(N- (((t-butyl)oxy)carbonyl)amino)-1,6-diphenyl-3-hydroxyhexane.

A solution of 1.5 g (5.3 mmol) of the resultant compound of Example 1E and 1.4 g (6.3 mmol) of di-t-butyldicarbonate in 50 ml of dichloromethane was stirred at ambient temperature for 16 h. The resulting solution was concentrated in vacuo, and the residue was purified by silica gel chromatography using first 5% then 10% methanol in chloroform to provide a mixture of the desired compounds. A second silica column using sequentially 0%, 0.5%, and 1% methanol in 2% isopropylamine/chloroform provided 0.65 g of (2S,3S,5S)-5-amino-2-(N-(((t-butyl)oxy)carbonyl)amino)-1,6-diphenyl-3-hydroxyhexane ($R_f$ 0.27) and 0.18 g of (2S,3S,5S)-2-amino-5-(N-(((t-butyl)oxy)carbonyl)amino)-1,6-diphenyl-3-hydroxyhexane ($R_f$ 0.23, 2% methanol/2% isopropylamine in chloroform) along with 0.15 g of a mixture or the two desired compounds.

B. (2S,3S,5S)-2-(N-(N-((N-Methyl-N-((2-pyridinyl)-methyl)amino)carbonyl)valinyl)amino)-5-(N-(((t-butyl)oxy)-carbonyl)amino)-1,6-diphenyl-3-hydroxyhexane.

Using the procedure of Example 28C but replacing (2S,3S,5S)-2-amino-5-(N-((3-pyridinyl)methoxycarbonyl)-amino)-1,6-diphenyl-3-hydroxyhexane with (2S,3S,5S)-2-amino-5-(N-(((t-butyl)oxy)carbonyl)amino)-1,6-diphenyl-3-hydroxyhexane provided, after silica gel chromatography using 2% methanol in chloroform, 66 mg (92%) of the desired compound ($R_f$ 0.60, 10% methanol in chloroform) as a white solid, m.p. 84-85°C. Mass spectrum: $(M + 1)^+ =$ 632.

Anal. Calcd for $C_{36}H_{49}N_5O_5 \cdot 0.5H_2O$: C, 67.48; H, 7.86; N, 10.93. Found: C, 67.40; H, 7.54; N, 10.90.

Example 49

(2S,3S,5S)-5-(N-(N-((N-Methyl-N-((2-pyridinyl)-methyl)amino)carbonyl)valinyl)amino)-2-(N-(((t-butyl)oxy)- carbonyl)amino)-1,6-diphenyl-3-hydroxybexane.

Using the procedure of Example 28C but replacing (2S,3S,5S)-2-amino-5-(N-((3-pyridinyl)methoxycarbonyl)-amino)-1,6-diphenyl-3-hydroxyhexane with (2S,3S,5S)-5-amino-2-(N-(((t-butyl)oxy)carbonyl)amino)-1,6-diphenyl-3-hydroxyhexane provided, after silica gel chromatography using 2% methanol in chloroform, 57 mg (80%) of the desired compound ($R_f$ 0.60, 10% methanol in chloroform). Mass spectrum: $(M + 1)^+ = 632$.

Example 50

(2S,3S,5S)-5-(N-(N-((N-Methyl-N-((2-methyl-4-thiazolyl)methyl)amino)carbonyl)valinyl)amino)-2-(N-((3-pyridinyl)methoxycarbonyl)amino)-1,6-diphenyl-3-hydroxyhexane.

Using the procedure of Example 29 but replacing the resultant compound of Example 3F with the resultant compound of Example 43E provided, after silica gel chromatography using a gradient of 1-3-5% methanol in chloroform, a 82% of the desired compound ($R_f$ 0.30, 10% methanol in chloroform) as a white solid. Mass spectrum: $(M + 1)^+$ = 687. m.p. 69-72°C.

Example 51

(2S,3S,5S)-5-(N-(((t-Butyl)oxy)carbonyl)amino)-2-(N-(N-((N-methyl-N-((2-amino-4-thiazolyl)methyl)amino)-carbonyl)valinyl)amino)-1,6-diphenyl-3-hydroxyhexane.

A solution of 0.12 mmol of the resultant compound of Example 44B in 1 ml of dichloromethane was treated with 0.14 mmol of di-t-butyldicarbonate. After being stirred for three days at ambient temperature, the solution was concentrated in vacuo and purified by silica gel chromatography using a gradient of 2% - 3.5% - 5% methanol in chloroform to provide 48 mg (58%) of the desired compound ($R_f$ 0.22, 10% methanol in chloroform). Mass spectrum: $(M + 1)^+$ = 653.

Example 52

(2S,3S,5S)-2-(N-(((t-Butyl)oxy)carbonyl)amino)-5-(N-(N-((N-methyl-N-((2-amino-4-thiazolyl)methyl)amino)-carbonyl)valinyl)amino)-1,6-diphenyl-3-hydroxyhexane.

Using the procedure of Example 51 but replacing the resultant compound of Example 44B with the resultant compound of Example 45B provided, after silica gel chromatography using first 2% then 3.5% methanol in chloroform, 42 mg (54%) of the desired compound.

Example 53

A. 2-(((N-Methyl)amino)methyl)benzimidazole.

Using the procedure of Example 33A but replacing 4-(chloromethyl)thiazole hydrochloride with 2-(chloromethyl)benzimidazole hydrochloride provided the crude desired compound in 30% yield after silica gel chromatography using 2% isopropylamine/5% methanol in chloroform.

B. N-((N-Methyl-N-((2-benzimidazolyl)methyl)amino)- carbonyl)valine Methyl Ester.

Using the procedure of Example 32B but replacing the resultant compound of Example 32A with the resultant compound of Example 53A provided, after silica gel chromatography using 4% methanol in chloroform, 1.74 g (87%) of the desired compound ($R_f$ 0.50, 4% methanol in chloroform).

C. N-((N-Methyl-N-((2-benzimidazolyl)methyl)amino)- carbonyl)valine.

Using the procedure of Example 32C but replacing the resultant compound of Example 32B with the resultant compound of Example 53B provided the desired compound.

D. N-((N-Methyl-N-((2-benzimidazolyl)methyl)amino)-carbonyl)valine p-Nitrophenyl Ester.

Using the procedure of Example 32D but replacing the resultant compound of Example 32C with the resultant compound of Example 53C provided the desired compound.

E. (2S,3S,5S)-5-(N-(N-((N-Methyl-N-((2-benzimidazolyl)-methyl)amino)carbonyl)valinyl)amino)-2-(N-((3-pyridinyl)-methoxycarbonyl)amino)-1,6-diphenyl-3-hydroxyhexane.

Using the procedure of Example 35E but replacing (2S,3S,5S)-2-amino-5-(N-((3-pyridinyl)methoxycarbonyl)-amino)-1,6-diphenyl-3-hydroxyhexane with (2S,3S,5S)-5-amino-2-(N-((3-pyridinyl)methoxycarbonyl)amino)-1,6-diphenyl-3-hydroxyhexane and replacing the resultant compound of Example 35D with the resultant compound of Example 69C provided, after silica gel chromatography using first 2.5% then 4.5% methanol in chloroform, 74.2 mg (62%) of the desired compound ($R_f$ 0.30, 10% methanol in chloroform) as a white solid, m.p. 97-100°C. Mass spectrum: $(M + 1)^+$ =706.

Anal. Calcd for $C_{40}H_{47}N_7O_5 \cdot 0.5H_2O$: C, 67.21; H, 6.77; N, 13.72. Found: C, 66.83; H, 6.70; N, 13.57.

Example 54

A. 2-(N-Methylamino)methyl)quinoline.

A mixture of 1.93 g of quinoline-2-carboxaldehyde and 0.19 g of 10 palladium on carbon in 15 ml of anhydrous methylamine and 45 ml of methanol was shaken under 4 atmospheres of hydrogen for 8 h. The resulting mixture was filtered through Celite and concentrated in vacuo to provide the crude desired compound.

B. N-((N-Methyl-N-((2-quinolinyl)methyl)amino)carbonyl)-valine.

Using sequentially the procedures of Examples 32B and 32C but replacing the resultant compound of Example 32A with the resultant compound of Example 54A provided the desired compound.

C. (2S,3S,5S)-5-(N-(N-((N-Methyl-N-((2-quinolinyl)-methyl)amino)carbonyl)valinyl)amino)-2-(N-((3-pyridinyl)-methoxycarbonyl)amino)-1,6-diphenyl-3-hydroxyhexane.

Using the procedure of Example 36 but replacing the resultant compound of Example 35D with the resultant compound of Example 54B provided, after silica gel chromatography using a gradient of 1-2.5% methanol in chloroform, 105 mg (60%) of the desired compound ($R_f$ 0.40, 10% methanol in chloroform) as a white solid. Mass spectrum: $(M + 1)^+$ = 717.

Example 55

(2S,3S,5S)-2-(N-(N-((N-Methyl-N-((2-quinolinyl)-methyl)amino)carbonyl)valinyl)amino)-5-(N-((3-pyridinyl)-methoxycarbonyl)amino)-1,6-diphenyl-3-hydroxyhexane.

Using the procedure of Example 35E but replacing the resultant compound of Example 35D with the resultant compound of Example 54B provided, after silica gel chromatography using a gradient of 1-2.5% methanol in chloroform, 100 mg (60%) of the desired compound ($R_f$ 0.36, 10% methanol in chloroform) as a white solid. Mass spectrum: $(M + 1)^+$ = 717.

Example 56

A. 1-(N-Methylamino)methyl)isoquinoline.

Using the procedure of Example 54A but replacing quinoline-2-carboxaldehyde with isoquinoline-1-carboxaldehyde (Minisci, et. al., J. Org. Chem., **1986**, *51*, 536) provided the crude desired compound.

B. N-((N-Methyl-N-((1-isoquinolinyl)methyl)amino)- carbonyl)valine p-Nitrophenyl Ester.

Using sequentially the procedures of Examples 32B, 32C, and 32D, but replacing the resultant compound of Example 32A with the resultant compound of Example 56A provided the desired compound.

C. (2S,3S,5S)-5-(N-(N-((N-Methyl-N-((1-isoquinolinyl)-methyl)amino)carbonyl)valinyl)amino)-2-(N-((3-pyridinyl)-methoxycarbonyl)amino)-1,6-diphenyl-3-hydroxyhexane.

Using the procedure of Example 29 but replacing the resultant compound of Example 3F with the resultant compound of Example 56C provided, after silica gel chromatography using a gradient of 2-3.5% methanol in chloroform, 98 mg (96%) of the desired compound ($R_f$ 0.41, 10% methanol in chloroform) as a white solid. Mass spectrum: $(M + 1)^+$ = 717.

Example 57

(2S,3S,5S)-2-(N-(N-((N-Methyl-N-((1-isoquinolinyl)-methyl)amino)carbonyl)valinyl)amino)-5-(N-((3-pyridinyl)-methoxycarbonyl)amino)-1,6-diphenyl-3-hydroxyhexane.

Using the procedure of Example 28C but replacing the resultant compound of Example 3F with the resultant compound of Example 56C provided, after silica gel chromatography using a gradient of 2-3.5% methanol in chloroform, 69 mg (67%) of the desired compound ($R_f$ 0.41, 10% methanol in chloroform) as a white solid. Mass spectrum: $(M + 1)^+ = 717$.

Example 58

(2S,3S,5S)-2-(N-(N-((N-Methyl-N-((2-benzimidazolyl)-methyl)amino)carbonyl)valinyl)amino)-5-(N-((3-pyridinyl)- methoxycarbonyl)amino)-1,6-diphenyl-3-hydroxyhexane.

Using the procedure of Example 35E but replacing the resultant compound of Example 35D with the resultant compound of Example 53C provided, after silica gel chromatography using first 2.5% then 4.5% methanol in chloroform, 74 mg (62%) of the desired compound ($R_f$ 0.27, 10% methanol in chloroform) as a off-white solid, m.p. 110-114°C. Mass spectrum: $(M + 1)^+ = 706$.

Example 59

A. ((2-Thiazolyl)methyl)-(4-nitrophenyl)carbonate.

A solution 2.3 g (11.5 mmol) of (4-nitrophenyl)-chloroformate in 20 ml of dichloromethane was cooled to 0°C and treated sequentially with a solution of 1.2 g (10.4 mmol) of 2-(hydroxymethyl)thiazole (Dondoni, et. al., *Synthesis*, **1987**, 998; *Tetrahedron Lett*. **1983**, *24*, 2901) in 5 ml of dichloromethane and 1.7 ml (15.7 mmol) of 4-methylmorpholine. After addition, the solution was allowed to come to ambient temperature, stirred for 0.5 h, and concentrated in vacuo. Silica gel chromatography of the residue using first chloroform then 1% methanol in chloroform provided 1.15 g (39%) of the desired compound ($R_f$ 0.73, 10% methanol in chloroform).

B. (2S,3S,5S)-2,5-Bis-(N-((2-thiazolyl)methoxycarbonyl)- amino)-1,6-diphenyl-3-hydroxyhexane.

A solution of 130 mg (0.46 mmol) of the resultant compound of Example 59A and 60 mg (0.21 mmol) of the resultant compound of Example 1E in 0.5 ml of tetrahydrofuran was stirred at ambient temperature for 16 h. The resulting solution was concentrated in vacuo, and the residue was purified by silica gel chromatography using first 2% then 4% methanol in chloroform to provide 99 mg (83%) of the desired compound ($R_f$ 0.73, 10% methanol in chloroform) as a white solid, m.p. 66-69°C. Mass spectrum: $(M + 1)^+ = 567$.

Anal. Calcd for $C_{28}H_{36}N_4O_5S_2 \cdot 0.5H_2O$: C, 58.42; H, 5.43; N, 9.73. Found: C, 58.23; H, 5.20; N, 9.61.

Example 60 (reference)

(2S,3R,4R,5S)-2,5-Bis-(N-((3-pyridinyl)methoxy-carbonyl)amino)-3,4-dihydroxy-1,6-diphenylhexane.

Using the procedure of Example 59B but replacing the resultant compound of Example 59A with the resultant compound of Example 28A and replacing the resultant compound of Example 1E with the resultant compound of Example 4A provided, after silica gel chromatography using first 2% then 3.5% methanol in chloroform, 280 mg of the desired compound ($R_f$ 0.25, 10% methanol in chloroform) as a white solid, m.p. 191-193°C. Mass spectrum: $(M + 1)^+ = 571$.

Anal. Calcd for $C_{32}H_{34}N_4O_6$: C, 67.35; H, 6.01; N, 9.82. Found: C, 67.11; H, 6.01; N, 9.64.

Example 61

A. ((4-Thiazolyl)methyl)-(4-nitrophenyl)carbonate.

Using the procedure of Example 59A but replacing 2-(hydroxymethyl)thiazole with 4-(hydroxymethyl)thiazole

(Kollonitsch, U.S. patent 3,299,083) provided, after silica gel chromatography using first chloroform then 1% methanol in chloroform, 380 mg (31%) of the desired compound ($R_f$ 0.70, 10% methanol in chloroform).

B. (2S,3S,5S)-2,5-Bis-(N-((4-thiazolyl)methoxycarbonyl)- amino)-1,6-diphenyl-3-hydroxyhexane.

Using the procedure of Example 59B but replacing the resultant compound of Example 59A with the resultant compound of Example 61A provided, after silica gel chromatography using first 2% then 4% methanol in chloroform, 98.6 mg (83%) of the desired compound ($R_f$ 0.43, 10% methanol in chloroform) as a white solid, m.p. 64-66°C. Mass spectrum: $(M + 1)^+ = 567$.

Anal. Calcd for $C_{28}H_{36}N_4O_5S_2 \cdot 0.5H_2O$: C, 58.42; H, 5.43; N, 9.73. Found: C, 58.45; H, 5.24; N, 9.61.

Example 62

A. ((2-Methyl-5-thiazolyl)methyl)-(4-nitrophenyl)carbonate.

Using the procedure of Example 59A but replacing 2-(hydroxymethyl)thiazole with 2-methyl-5-(hydroxymethyl)-thiazole (Mashraqui and Keehn, J. Am. Chem. Soc. **1982**, *104*, 4461) provided, after silica gel chromatography using 6% ethyl acetate in chloroform, 243 mg (65%) of the desired compound ($R_f$ 0.25, 10% methanol in chloroform).

B. (2S,3S,5S)-2,5-Bis-(N-((2-methyl-5-thiazolyl)-methoxycarbonyl)amino)-1,6-diphenyl-3-hydroxyhexane.

Using the procedure of Example 59B but replacing the resultant compound of Example 59A with the resultant compound of Example 62A provided, after silica gel chromatography using first 2% then 3% methanol in chloroform, 49 mg (29%) of the desired compound ($R_f$ 0.5, 10% methanol in chloroform). Mass spectrum: $(M + 1)^+ = 595$.

Example 63

A. 5-(Carbethoxy)thiazole.

According to the procedure of Mashraqui and Keehn (J. Am. Chem. Soc. **1982**, *104*, 4461), ethyl α-chloro-α-formylacetate was condensed with thioformamide and vacuum distilled to provide 5.65 g (33%) of the desired compound.

B. 5-(Hydroxymethyl)thiazole

According to the procedure of Mashraqui and Keehn (J. Am. Chem. Soc. **1982**, *104*, 4461), 5-(carbethoxy)thiazole was reduced with lithium aluminum hydride to provide the crude desired compound in 44% yield.

C. ((5-Thiazolyl)methyl)-(4-nitrophenyl)carbonate.

Using the procedure of Example 59A but replacing 2-(hydroxymethyl)thiazole with 5-(hydroxymethyl)thiazole and allowing the reaction to proceed at ambient temperature for 2 days provided, after silica gel chromatography using 6% ethyl acetate in chloroform, 1.1 g (71%) of the desired compound ($R_f$ 0.22, 6% ethyl acetate in chloroform). Mass spectrum: $(M + 1)^+ = 281$.

D. (2S,3S,5S)-2,5-Bis-(N-((5-thiazolyl)methoxycarbonyl)-amino)-1,6-diphenyl-3-hydroxyhexane.

Using the procedure of Example 59B but replacing the resultant compound of Example 59A with the resultant compound of Example 63C provided, after silica gel chromatography using first 2% then 3% methanol in chloroform, 145 mg (73%) of the desired compound ($R_f$ 0.56, 10% methanol in chloroform). Mass spectrum: $(M + 1)^+ = 567$.

Example 64 (reference)

(2S,3R,4S,5S)-5-Amino-2-(N-(((t-butyl)oxy)-carbonyl)amino)-3,4-dihydroxy-1,6-diphenylhexane.

A solution of 0.70 g (2.33 mmol) of the resultant compound of Example 37D and 0.61 g (2.8 mmol) of di-t-butyldicarbonate in 20 ml of dichloromethane was stirred at ambient temperature for 16 h. The resulting solution was

concentrated in vacuo, and the residue was purified by silica gel chromatography using first 5% then 10% methanol in chloroform to provide 0.67 g (72%) of the desired compound ($R_f$ 0.32, 10% methanol in chloroform) as a white solid.

Example 65 (reference)

A. N-((4-Methylpiperazin-1-yl)carbonyl)valine Methyl Ester.

Using the procedure of Example 32B but replacing the resultant compound of Example 32A with 1-methylpiperazine provided, after silica gel chromatography using first 5% then 7.5% methanol in chloroform, 1.40 g (100%) of the desired compound ($R_f$ 0.14, 5% methanol in chloroform). Mass spectrum: $(M + 1)^+$ = 258.

B. N-((4-Methylpiperazin-1-yl)carbonyl)valine.

Using the procedure of Example 32C but replacing the resultant compound of Example 32B with the resultant compound of Example 64A provided the desired compound.

C. (2S,3R,4S,5S)-5-(N-(N-((4-Methylpiperazin-1-yl)carbonyl)-valinyl)amino)-2-(N-(((t-butyl)oxy-carbonyl)amino)-3,4-dihydroxy-1,6-diphenylhexane.

To a solution of 70 mg (0.175 mmol) of the resultant compound of Example 64A 0.21 mmol of the resultant compound of Example 65B 35 mg (0.26 mmol) of 1-hydroxybenzotriazole monohydrate and 38 μL (0.35 mmol) of 4-methylmorpholine in 1 ml of tetrahydrofuran was added 50 mg (0.26 mmol) of ethyl-(3-dimethylaminopropyl)-carbodiimide. The resulting solution was stirred for 16 h at ambient temperature, diluted with 5% methanol in chloroform, washed with saturated aqueous $NaHCO_3$ and saturated brine, dried over $Na_2SO_4$, and concentrated in vacuo. Silica gel chromatography using sequentially 3%, 6% and 10% methanol in chloroform provided 82 mg (75%) of the desired compound ($R_f$ 0.18, 10% methanol in chloroform). Mass spectrum: $(M + H)^+$ = 626.

Anal. Calcd for $C_{34}H_{51}N_5O_6 \cdot 0.5H_2O$: C, 64.33; H, 8.26; N, 11.03. Found: C, 64.05; H, 8.07; N, 11.07.

Example 66 (reference)

(2S,3R,4R,5S)-2,5-Bis-(N-(N-((N-methyl-N-((4-thiazolyl)-methyl)amino)carbonyl)valinyl)amino-3,4-dihydroxy-1,6-diphenylhexane.

A solution of 75 mg (0.25 mmol) of the resultant compound of Example 33B and 0.75 mmol of the resultant compound of Example 4A in 1 ml of tetrahydrofuran was stirred at ambient temperature for 54 h. The resulting solution was concentrated in vacuo. Silica gel chromatography of the residue using first 2% then 5% methanol in chloroform, provided 182 mg (91%) of the desired compound ($R_f$ 0.33, 10% methanol in chloroform) as a white solid, m.p. 92-94°C. Mass spectrum: $(M + 1)^+$ = 807.

Anal. Calcd for $C_{40}H_{54}N_8O_6S_2 \cdot H_2O$: C, 58.23; H, 6.84; N, 13.58. Found: C, 57.87; H, 6.49; N, 13.40.

Example 67

(2S,3S,5S)-2,5-Bis-(N-(N-((N-methyl-N-((4-thiazolyl)-methyl)amino)carbonyl)valinyl)amino)-1,6-diphenyl-3-hydroxyhexane.

Using the procedure of Example 66 but replacing the resultant compound of Example 4A with the resultant compound of Example 1E provided, after silica gel chromatography using first 2% then 5% methanol in chloroform, 151 mg (77%) of the desired compound ($R_f$ 0.31, 10% methanol in chloroform) as a white solid, m.p. 154-156°C. Mass spectrum: $(M + 1)^+$ = 791.

Anal. Calcd for $C_{40}H_{54}N_8O_5S_2 \cdot 0.25H_2O$: C, 60.39; H, 6.90; N, 14.09. Found: 60.30; H, 6.74; N, 13.96.

Example 68 (reference)

(2S,3S,4S,5S)-2,5-Bis-(N-(N-((N-methyl-N-((2-((((t-butyl)oxy)carbonyl)amino)-4-thiazolyl)methyl)amino)-carbonyl)valinyl)amino)-3,4-dihydroxy-1,6-diphenylhexane.

To a solution of 70 mg (0.23 mmol) of the resultant compound of Example 7C 0.52 mmol of the resultant compound of Example 35D 94 mg (0.69 mmol) of 1-hydroxybenzotriazole monohydrate and 50 µL (0.46 mmol) of 4-methylmorpholine in 1 ml of dimethylformamide was added 130 mg (0.69 mmol) of ethyl-(3-dimethylaminopropyl)-carbodiimide. The resulting solution was stirred for 16 h at ambient temperature, diluted with ethyl acetate, washed with saturated aqueous $NaHCO_3$, dried over $Na_2SO_4$, and concentrated in vacuo. Silica gel chromatography using sequentially 2% and 5% methanol in chloroform provided 210 mg (88%) of the desired compound ($R_f$ 0.57, 10% methanol in chloroform) as a white solid, m.p. 143-145°C. Mass spectrum: $(M + 1)^+ = 1037$.

Example 69

(2S,3S,5S)-2,5-Bis-(N-(N-((N-methyl-N-((2-((((t-butyl)oxy)carbonyl)amino)-4-thiazolyl)methyl)amino)-carbonyl)valinyl)amino)-1,6-diphenyl-3-hydroxyhexane.

Using the procedure of Example 68 but replacing the resultant compound of Example 7C with the resultant compound of Example 1E provided, after silica gel chromatography using 2% methanol in chloroform, 102 mg (43%) of the desired compound ($R_f$ 0.57, 10% methanol in chloroform) as a white solid, m.p. 115-120°C. Mass spectrum: $(M + 1)^+ = 1021$.

Example 70

(2S,3S,5S)-2,5-Bis-(N-(N-((N-methyl-N-((2-amino-4-thiazolyl)methyl)amino)carbonyl)valinyl)amino)-1,6-diphenyl-3-hydroxyhexane.

Using the procedure of Example 45B but replacing the resultant compound of Example 45A with the resultant compound of Example 69 provided, after silica gel chromatography using first 5% then 10% methanol in chloroform, 52 mg (72%) of the desired compound ($R_f$ 0.18, 10% methanol in chloroform) as a white solid, m.p. 110-114°C. Mass spectrum: $(M + 1)^+ = 821$.

Anal. Calcd for $C_{40}H_{56}N_{10}O_6S_2 \cdot 0.5CH_3OH \cdot 0.25CHCl_3$: C, 56.47; H, 6.75; N, 16.16. Found: C, 56.85; H, 6.47; N, 15.45.

Example 71

(2S,3S,5S)-2,5-Bis-(N-(N-((-methyl-N-((2-benzimidazolyl)methyl)amino)carbonyl)valinyl)amino)-1,6-diphenyl-3-hydroxyhexane.

Using the procedure of Example 66 but replacing the resultant compound of Example 33B with the resultant compound of Example 53D and replacing the resultant compound of Example 4A with the resultant compound of Example 1E provided, after silica gel chromatography using 6% methanol in chloroform, 130 mg (61%) of the desired compound ($R_f$ 0.28, 10% methanol in chloroform) as a white solid, m.p. 150-152°C. Mass spectrum: $(M + 1)^+ = 857$.

Example 72

A. (2S,3S,5S)-5-Amino-2-(N-(N-((N-methyl-N-((2-pyridinyl)methyl)amino)carbonyl)valinyl)amino)-1,6-diphenyl-3-hydroxyhexane.

Using the procedure of Example 45B but replacing the resultant compound of Example 45A with the resultant compound of Example 48B provided the desired compound ($R_f$ 0.28, 2% isopropylamine/2% methanol in chloroform).

B. (2S,3S,5S)-5-(N-(N-((N-Methyl-N-((2-thiazolyl)methyl)-amino)carbonyl)valinyl)-2-(N-(N-((N-methyl-N-((2-pyridinyl)methyl)amino)carbonyl)valinyl)amino)-1,6-diphenyl-3-hydroxyhexane.

Using the procedure of Example 29 but replacing the resultant compound of Example 3F with the resultant compound of Example 32D and replacing (2S,3S,5S)-5-amino-2-(N-((3-pyridinyl)methoxycarbonyl)amino)-1,6-diphenyl-3-hydroxyhexane with the resultant compound of Example 72A provided, after silica gel chromatography using first 2% then 4% methanol in chloroform, 75 mg (73%) of the desired compound ($R_f$ 0.34, 10% methanol in chloroform) as a white solid, m.p. 158-160°C. Mass spectrum: $(M + 1)^+ = 785$.

Anal. Calcd for $C_{42}H_{56}N_8O_5S$: C, 64.26; H, 7.19; N, 14.27. Found: C, 63.89; H, 7.14; N, 14.08.

Example 73

A. (2S,3S,5S)-2-Amino-5-(N-(N-((N-methyl-N-((2-pyridinyl)methyl)amino)carbonyl)valinyl)amino)-1,6-diphenyl-3-hydroxyhexane.

Using the procedure of Example 45B but replacing the resultant compound of Example 45A with the resultant compound of Example 49 provided the desired compound ($R_f$ 0.20, 2% isopropylamine/2% methanol in chloroform).

B. (2S,3S,5S)-2-(N-(N-((N-Methyl-N-((2-thiazolyl)methyl)-amino)carbonyl)valinyl)-5-(N-(N-((N-methyl-N-((2-pyridinyl)methyl)amino)carbonyl)valinyl)amino)-1,6-diphenyl-3-hydroxyhexane.

Using the procedure of Example 29 but replacing the resultant compound of Example 3F with the resultant compound of Example 32D and replacing (2S,3S,5S)-5-amino-2-(N-((3-pyridinyl)methoxycarbonyl)amino)-1,6-diphenyl-3-hydroxyhexane with the resultant compound of Example 73A provided, after silica gel chromatography using first 2% then 4% methanol in chloroform, 81 mg (78%) of the desired compound ($R_f$ 0.33, 10% methanol in chloroform) as a white solid, m.p. 156-158°C. Mass spectrum: $(M + 1)^+ = 785$.

Anal. Calcd for $C_{42}H_{56}N_8O_5S$: C, 64.26; H, 7.19; 14.27. Found: C, 63.96; H, 6.99; N, 13.89.

Example 74

(2S,3S,5S)-2,5-Bis-(N-(N-((N-methyl-N-((3-pyridinyl)methyl)amino)carbonyl)valinyl)amino)-1,6-diphenyl-3-hydroxyhexane.

Using the procedure of Example 66 but replacing the resultant compound of Example 4A with the resultant compound of Example 1E and replacing the resultant compound of Example 33B with the resultant compound of Example 18F provided, after silica gel chromatography using first 2%, then 7%, then 10% methanol in chloroform, 132 mg (81%) of the desired compound ($R_f$ 0.18, 10% methanol in chloroform) as a off-white solid, m.p. 193-196°C. Mass spectrum: $(M + 1)^+ = 779$.

Anal. Calcd for $C_{44}H_{58}N_8O_5 \cdot H_2O$: C, 66.31; H, 7.59; N, 14.06. Found: C, 66.22; H, 7.51; N, 13.59.

Example 75

(2S,3S,5S)-2,5-Bis-(N-(N-((N-methyl-N-((2-thiazolyl)methyl)amino)carbonyl)valinyl)amino)-1,6-diphenyl-3-hydroxyhexane.

Using the procedure of Example 66 but replacing the resultant compound of Example 4A with the resultant compound of Example 1E and replacing the resultant compound of Example 33B with the resultant compound of Example 32D provided, after silica gel chromatography using sequentially 1.5%, 2% and 5% methanol in chloroform, 102 mg (64.6%) of the desired compound ($R_f$ 0.30, 10% methanol in chloroform) as a white solid, m.p. 195-197°C. Mass spectrum: $(M + 1)^+ = 791$.

Anal. Calcd for $C_{40}H_{54}N_8O_5S_2 \cdot 0.25H_2O$: C, 60.39; H, 6.90; N, 14.09. Found: 60.27; H, 6.79; N, 13.94.

Example 76

A. (2S,3S,5S)-2-Amino-5-(N-((5-thiazolyl)- methoxycarbonyl)amino)-1,6-diphenyl-3-hydroxyhexane and (2S,3S,5S)-5-Amino-2-(N-((5-thiazolyl)methoxycarbonyl)amino)-1,6-diphenyl-3-hydroxyhexane.

Using the procedure of Example 28B but replacing the resultant compound of Example 28A with the resultant compound of Example 63C provided, after silica gel chromatography using first 2% then 5% methanol in chloroform provided a mixture of the two desired compounds. Silica gel chromatography of the mixture using first 2% isopropylamine in chloroform followed by 2% isopropylamine/1% methanol in chloroform and finally 2% isopropylamine/2% methanol in chloroform provided 111 mg (16%) of (2S,3S,5S)-2-amino-5-(N-((5-thiazolyl)-methoxycarbonyl)amino)-1,6-diphenyl-3-hydroxyhexane and 185 mg (28%) of (2S,3S,5S)-2-amino-5-(N-((5-thiazolyl)-methoxycarbonyl)amino)-1,6-diphenyl-3-hydroxyhexane. Mass spectrum for each compound: $(M + 1)^+ = 426$.

B. (2S,3S,5S)-2-(N-(N-((2-Pyridinyl)methoxycarbonyl)-valinyl)amino)-5-(N-((5-thiazolyl)methoxycarbonyl)amino)-1,6-diphenyl-3-hydroxyhexane.

Using the procedure of Example 28C but replacing (2S,3S,5S)-2-amino-5-(N-((3-pyridinyl)methoxycarbonyl)-amino)-1,6-diphenyl-3-hydroxyhexane with (2S,3S,5S)-2-amino-5-(N-((5-thiazolyl)methoxycarbonyl)amino)-1,6-diphenyl-3-hydroxyhexane and replacing the resultant compound of Example 3F with the resultant compound of Example 2D provided, after silica gel chromatography using a gradient of 1% - 3% - 5% methanol in chloroform, 56 mg (72%) of the desired compound ($R_f$ 0.50, 10% methanol in chloroform) as a white solid, m.p. 176-177°C. Mass spectrum: $(M + 1)^+ = 660$.

Anal. Calcd for $C_{35}H_{41}N_5O_6S \cdot 1.5H_2O$: C, 61.21; H, 6.46; N, 10.20. Found: C, 61.08; H, 6.00; N, 10.39.

Example 77

(2S,3S,5S)-5-(N-(N-((2-Pyridinyl)methoxycarbonyl)-valinyl)amino)-2-(N-(5-thiazolyl)methoxycarbonyl)amino)-1.6-diphenyl-3-hydroxyhexane.

Using the procedure of Example 28C but replacing (2S,3S,5S)-2-amino-5-(N-((3-pyridinyl)methoxycarbonyl)-amino)-1,6-diphenyl-3-hydroxyhexane with (2S,3S,5S)-5-amino-2-(N-((5-thiazolyl)methoxycarbonyl)amino)-1,6-diphenyl-3-hydroxyhexane and replacing the resultant compound of Example 3F with the resultant compound of Example 2D provided, after silica gel chromatography using a gradient of 1% - 3% - 5% methanol in chloroform, 48 mg (62%) of the desired compound ($R_f$ 0.47, 10% methanol in chloroform) as a white solid, m.p. 166-168°C. Mass spectrum: $(M + 1)^+ = 660$.

Example 78

(2S,3S,5S)-2-(N-(N-((N-Methyl-N-((2-pyridinyl)methyl)-amino)carbonyl)isoleucinyl)amino)-5-(N-((5-thiazolyl)-methoxycarbonyl)amino)-1,6-diphenyl-3-hydroxyhexane.

Using the procedure of Example 28C but replacing (2S,3S,5S)-2-amino-5-(N-((3-pyridinyl)methoxycarbonyl)-amino)-1,6-diphenyl-3-hydroxyhexane with (2S,3S,5S)-2-amino-5-(N-((5-thiazolyl)methoxycarbonyl)amino)-1,6-diphenyl-3- hydroxyhexane and replacing the resultant compound of Example 3F with the resultant compound of Example 11C provided, after silica gel chromatography using first 2% then 5% methanol in chloroform, 51 mg (62%) of the desired compound ($R_f$ 0.43, 10% methanol in chloroform) as a white solid, m.p. 66-69°C. Mass spectrum: $(M + 1)^+ = 687$.

Example 79

(2S,3S,5S)-5-(N-(N-((N-Methyl-N-((2-pyridinyl)methyl)-amino)carbonyl)isoleucinyl)amino)-2-(N-((5-thiazolyl)-methoxycarbonyl)amino)-1,6-diphenyl-3-hydroxyhexane.

Using the procedure of Example 28C but replacing (2S,3S,5S)-2-amino-5-(N-((3-pyridinyl)methoxycarbonyl)-amino)-1,6-diphenyl-3-hydroxyhexane with (2S,3S,5S)-5-amino-2-(N-((5-thiazolyl)methoxycarbonyl)amino)-1,6-diphenyl-3-hydroxyhexane and replacing the resultant compound of Example 3F with the resultant compound of Example 11C provided, after silica gel chromatography using a gradient of 2% - 5% methanol in chloroform, 51 mg (62%) of

the desired compound ($R_f$ 0.47, 10% methanol in chloroform) as a white solid, m.p. 64-67°C. Mass spectrum: $(M + 1)^+$ = 687.

Example 80

(2S,3S,5S)-2-(N-(N-((N-Methyl-N-((2-amino-4-thiazolyl)- methyl)amino)carbonyl)valinyl)amino)-5-(N-((5-thiazolyl)-methoxycarbonyl)amino)-1,6-diphenyl-3-hydroxyhexane.

Using the procedure of Example 38B but replacing the resultant compound of Example 38A with the resultant compound of Example 44B and replacing the resultant compound of Example 28A with the resultant compound of Example 63C provided, after silica gel chromatography using a gradient of 2% - 3% - 5% methanol in chloroform, 46 mg (55%) of the desired compound ($R_f$ 0.24, 10% methanol in chloroform). Mass spectrum: $(M + 1)^+$ = 694.

Example 81

(2S,3S,5S)-2,5-Bis-(N-(N-((2-pyridinyl)methoxycarbonyl)-valinyl)amino)-1,6-diphenyl-3-(trichloroacetoxy)hexane.

Using the procedure of Example 26 but replacing trifluoroacetic anhydride with trichloroacetic anhydride provided, after silica gel chromatography using 5% methanol in dichloromethane, 102.8 mg (86%) of the desired compound as a white solid. $^1H$ NMR ($CDCl_3$) δ 0.71 (d, 3H), 0.74 (d, 3H), 0.82 (d, 3H), 0.85 (d, 3H), 1.69 (m, 1H), 1.93 (m, 1H), 2.07 (m, 2H), 2.72 (m, 2H), 2.86 (m, 2H), 3.81 (br t, 1H), 3.89 (br t, 1H), 4.53 (br, 1H), 4.93 (m, 2H), 5.16-5.25 (m, 6H), 5.92 (br, 1H), 6.03 (br d, 1H), 7.12-7.24 (m, 12H), 7.36 (br t, 2H), 7.73 (br t, 2H), 8.61 (br d, 2H). Mass spectrum: $(M+H)^+$ = 897.

Example 82 (reference)

(2S,3S,5S)-2,5-Bis-(N-(N-((2-pyridinyl)methoxycarbonyl)- valinyl)amino)-1,6-diphenyl-3-(propanoxy)hexane

Using the procedure of Example 26 but replacing trifluoroacetic anhydride with propanoic anhydride provided 106.1 mg (99%) of the desired compound as a colorless crystal. $^1H$ NMR ($CDCl_3$) δ 0.77 (d, 6H), 0.85 (two d, 6H), 1.15 (t, 3H), 1.56 (m, 1H), 1.67 (m, 1H), 2.03 (m, 2H), 2.32 (q, 2H), 2.73 (m, 4H), 3.85 (m, 2H), 4.28 (m, 1H), 4.56 (m, 1H), 4.89 (m, 1H), 5.23 (s, 4H), 5.35 (br, 2H), 6.00 (br, 2H), 7.06-7.36 (m, 14H), 7.72 (br t, 2H), 8.59 (br s, 2H). Mass spectrum: $(M+H)^+$ = 809.

Example 83

(2S,3S,5S)-2,5-Bis-(N-(N-((2-pyridinyl)methoxycarbonyl)-valinyl)amino)-1,6-diphenyl-3-(methoxyacetoxy)hexane.

A suspension of 100 mg (0.13 mmol) of the resultant compound of Example 2E and 24.3 mg (0.20 mmol) of 4-dimethylaminopyridine in 10 ml of dichloromethane was treated with 0.017 ml (0.20 mmol) of methoxyacetyl chloride. The resulting mixture was stirred at ambient temperature for 1 h and then quenched with pH 6 buffer. The aqueous layer was extracted with dichloromethane. The combined organic layers was dried over $MgSO_4$, and concentrated in vacuo to provide 107.0 mg (98%) of the desired compound as a white solid. $^1H$ NMR ($CDCl_3$) δ 0.74 (d, 3H), 0.77 (d, 3H), 0.83 (d, 3H), 0.86 (d, 3H), 1.66 (m, 2H), 2.04 (m, 2H), 2.73 (m, 4H), 3.45 (s, 3H), 3.86 (m, 2H), 4.01 (m, 2H), 4.32 (m, 1H), 4.63 (m, 1H), 4.98 (m, 1H), 5.22 (br s, 5H), 5.30 (br d, 1H), 5.92 (br d, 1H), 6.11 (br d, 1H), 7.07 (br d, 2H), 7.15-7.24 (m, 10H), 7.36 (br t, 2H), 7.71(tt, 2H), 8.59 (br t, 2H). Mass spectrum: $(M+H)^+$ = 825.

Example 84

(2S,3S,5S)-2,5-Bis-(N-(N-((2-pyridinyl)methoxycarbonyl)-valinyl)amino)-1,6-diphenyl-3-(formoxy)hexane.

Using the procedure of Example 26 but replacing trifluoroacetic anhydride with acetic formic anhydride provided 106.3 mg (100%) of the desired compound as a white solid, m.p. 206-207 °C. $^1H$ NMR ($CDCl_3$) δ 0.73 (two d, 6H), 0.84 (two d, 6H), 1.68 (m, 2H), 2.06 (m, 2H), 2.65-2.79 (m, 4H), 3.85 (m, 2H), 4.36 (br, 1H), 4.68 (br q, 1H), 5.00 (br t, 1H), 5.14-5.28 (m, 6H), 5.87 (br, 1H), 6.04 (br d, 1H), 7.07 (br d, 2H), 7.13-7.23 (m, 10H), 7.34 (br d, 2H), 7.69 (td, 2H), 8.04 (br, 1H), 8.58 (br t, 2H). Mass spectrum: $(M+H)^+$ = 781.

Anal. Calcd for $C_{43}H_{52}N_6O_8 \cdot 0.5H_2O$: C, 65.38; H, 6.76; N, 10.64; Found: C, 65.69; H, 6.75; N, 10.60.

Example 85

(2S,3S,5S)-2,5-Bis-(N-(N-((N-methyl-N-((2-pyridinyl)- methyl)amino)carbonyl)valinyl)amino)-1,6-diphenyl-3-((N,N-dimethylamino)acetoxy)hexane.

A. (2S,3S,5S)-2,5-Bis-(N-(N-((N-methyl-N-((2-pyridinyl)methyl)amino)carbonyl)valinyl)amino)-1,6-diphenyl-3-(bromoacetoxy)hexane.

A suspension of 100 mg (0.13 mmol) of the resultant compound of Example 3G and 23.5 mg (0.19 mmol) of 4-dimethylaminopyridine in 2 ml of dichloromethane was treated with 0.022 ml (0.26 mmol) of bromoacetyl bromide. The resulting mixture was stirred at ambient temperature for 5 h and then quenched with pH 6 buffer. The organic layer was diluted with dichloromethane, separated, dried over $Na_2SO_4$, and concentrated in vacuo to provide, after silica gel chromatography using 10% methanol in dichloro-methane, 97.8 mg (85%) of the desired compound as a white solid. Mass spectrum: $(M+H)^+$ = 899.

B. (2S,3S,5S)-2,5-Bis-(N-(N-((N-methyl-N-((2-pyridinyl)-methyl)amino)carbonyl)valinyl)amino)-1,6-diphenyl-3-((N,N-dimethylamino)acetoxy)hexane.

A solution of 115.5 mg (0.13 mmol) of the resultant compound of Example 85A in 5 ml of dichloromethane was treated with 0.020 ml (0.26 mmol) of dimethylamine (1.3 M in diethyl ether). After being stirred at ambient temperature for 0.5 h, the solution was diluted with dichloromethane, washed with water, dried over $Na_2SO_4$, and concentrated in vacuo. Silica gel chromatography of the residue using 5% methanol in dichloromethane provided 81.7 mg (73%) of the desired compound as a white solid, m.p. 109-111°C. $^1$H NMR ($CDCl_3$) δ 0.79-0.88 (four d, 12H), 1.12 (m, 1H), 1.19 (m, 1H), 2.13 (m, 2H), 2.37 (s, 6H), 2.61-2.84 (m, 4H), 2.98 (s, 6H), 3.13 (br s, 2H), 4.04 (m, 2H), 4.30 (m, 1H), 4.43-4.58 (m, 5H), 5.02(br t, 1H), 6.01 (br, 1H), 6.12 (br, 1H), 6.20 (br, 2H), 6.52 (br d, 1H), 7.06-7.27 (m, 14H), 7.69 (m, 2H), 8.53 (m, 2H). Mass spectrum: $(M+H)^+$ = 864.

Anal. Calcd for $C_{48}H_{65}N_9O_6$: C, 66.72; H, 7.58; N, 14.59; Found: C, 66.35; H, 7.55; N, 14.69.

Example 86

(2S,3S,5S)-2,5-Bis-(N-(N-((N-methyl-N-((2-pyridinyl)-methyl)amino)carbonyl)valinyl)amino)-1,6-diphenyl-3-(1-(2-propoxy)ethoxy)hexane.

A solution of 80 mg (0.10 mmol) of the resultant compound of Example 3G and 40 mg (0.16 mmol) of pyridium p-toluenesulfonate in 4 ml of acetonitrile was treated with 4 ml of isopropyl vinyl ether. The resulting mixture was stirred at ambient temperature under $N_2$ for 20 h and then quenched with pH 6 buffer. The aqueous layer was extracted with dichloromethane. The combined organic layers were dried over $Na_2SO_4$ and concentrated in vacuo. Silica gel chromatography of the residue using 10% methanol in dichloromethane provided 86.1 mg (97%) of the desired compound as a white solid. $^1$H NMR ($CDCl_3$) δ 0.73 (d, 3H), 0.77 (d, 3H), 0.86 (two d, 6H), 1.08 (m, 6H), 1.24 (dd, 3H), 1.61-1.75 (m, 3H), 2.12 (m, 1H), 2.21 (m, 1H), 2.64-2.92 (m, 4H), 2.96 (two d, 6H), 3.53-3.75 (m, 2H), 4.00-4.14 (m, 2H), 4.33-4.64 (m, 6H), 5.94-6.47 (m, 4H), 7.10-7.24 (m, 14H), 7.22 (td, 2H), 8.54 (m, 2H). Mass spectrum: $(M+H)^+$ = 864.

Anal. Calcd for $C_{49}H_{68}N_8O_6$: C, 68.03; H, 7.92; N, 12.95; Found: C, 67.67; H, 7.90; N, 12.95.

Example 87

(2S,3S,5S)-2,5-Bis-(N-(N-((N-methyl-N-((2-pyridinyl)-methyl)amino)carhonyl)valinyl)amino)-1,6-diphenyl-3-(((N-(2-hydroxyethyl)-N-methyl)amino)acetoxy)hexane.

A solution of 100 mg (0.11 mmol) of the resultant compound of Example 85A in 5 ml of dichloromethane was treated with 0.018 ml (0.22 mmol) of 2-(methylamino)-ethanol. After being stirred at ambient temperature for 1 h, the solution was diluted with dichloromethane, washed with water, dried over $Na_2SO_4$, and concentrated in vacuo. Silica gel chromatography of the residue using 10% methanol in dichloromethane provided 27.9 mg (28%) of the desired compound as a white solid. $^1$H NMR ($CDCl_3$) δ 0.79 (d, 6H), 0.84 (t, 6H), 1.73 (br, 2H), 2.08 (m, 1H), 2.17 (m, 1H), 2.48 (s, 3H), 2.64-2.84 (m, 6H), 2.96 (s, 3H), 3.00 (s, 3H), 3.43 (br, 1H), 3.72 (br, 2H), 4.04 (m, 2H), 4.38 (m, 1H), 4.42-4.57 (m, 6H), 5.07 (td, 1H), 6.09 (br, 2H), 6.97 (br d, 2H), 7.08-7.26 (m, 14H), 7.69 (td, 2H), 8.52 (m, 2H). Mass spectrum: $(M+H)^+$ = 894.

Anal. Calcd for $C_{49}H_{67}N_9O_7 \cdot CH_3OH$: C, 64.84; H, 7.73; N, 13.61; Found: C, 65.08; H, 7.52; N, 13.41.

Example 88

(2S,3S,5S)-2,5-Bis-(N-(N-((N-methyl-N-((2-pyridinyl)-methyl)amino)carbonyl)valinyl)amino)-1,6-diphenyl-3-(((N-(2-acetoxyethyl)-N-methyl)amino)acetoxy)hexane.

A solution of 20.6 mg (0.023 mmol) of the resultant compound of Example 87 and 5.6 mg (0.046 mmol) of 4-dimethylaminopyridine in 2 ml of dichloromethane was treated with 3.3 µl (0.035 mmol) of acetic anhydride. The resulting mixture was stirred at ambient temperature for 1.5 h and then quenched with pH 6 buffer. The organic layer was diluted with dichloromethane, separated, dried over $Na_2SO_4$, and concentrated in vacuo. Silica gel chromatography of the residue using 10% methanol in dichioromethane provided 19.3 mg (92%) of the desired compound as a white solid. $^1H$ NMR ($CDCl_3$) δ 0.80 (d, 6H), 0.86 (d, 6H), 1.60 (m, 1H), 1.71 (m, 1H), 2.08 (s, 3H), 2.13 (m, 2H), 2.45 (s, 3H), 2.49-2.79 (m, 4H), 2.84 (t, 2H), 2.97 (s, 6H), 3.29 (q, 2H), 4.04 (q, 2H), 4.17 (t, 2H), 4.31 (m, 1H), 4.48 (br t, 4H), 4.56 (m, 1H), 5.01 (br t, 1H), 6.01 (br, 1H), 6.12 (br, 1H), 6.21 (br d, 1H), 6.48 (br d, 1H), 7.07-7.25 (m, 14H), 7.68 (m, 2H), 8.53 (br t, 2H). Mass spectrum: $(M+H)^+$ = 936.

Anal. Calcd for $C_{51}H_{69}N_9O_8$: C, 65.43; H, 7.43; N, 13.47; Found: C, 65.18; H, 7.10; N, 13.42.

Example 89

(2S,3S,5S)-2,5-Bis-(N-(N-((N-methyl-N-((2-pyridinyl)-methyl)amino)carbonyl)valiny)amino)-1,6-diphenyl-3- (acetoxymethoxy)hexane.

A. (2S,3S,5S)-2,5-Bis-(N-(benzylidene)amino)-1,6-diphenyl-3- hydroxyhexane.

A solution of 150 mg (0.53 mmol) of the resultant compound of Example 1E in 6 ml of tetrahydrofuran was treated with 0.11 ml of benzaldehyde. After being stirred at ambient temperature under $N_2$ atmosphere for 3 h, concentrated in vacuo, and left on oil pump for 1 day to provide the crude desired compound. Mass spectrum: $(M+H)^+$ = 461.

B. (2S,3S,5S)-2,5-Bis-(N-(benzylidene)amino)-1,6-diphenyl-3-(2-methyl-2-propenoxy)hexane.

A solution of the crude resultant compound of Example 89A in 6 ml of tetrahydrofuran was treated with 0.58 ml of 1 M solution of sodium bis(trimethylsilyl)amide in tetrahydrofuran at 0°C under $N_2$ atmosphere. The resulting mixture was stirred for 40 min and then treated with 3-iodo-2-methylpropene. Stirring was continued at 0°C for 1 h and at ambient temperature for 5 h. Evaporating of the solvent provided the crude desired compound. $^1H$ NMR ($CDCl_3$) δ 1.71 (s, 3H), 2.07 (m, 1H), 2.33 (m, 1H), 2.82-3.11 (m, 4H), 3.52 (m, 2H), 3.68 (m, 1H), 3.94 (s, 2H), 4.80 (s, 1H), 4.92 (q, 1H), 7.02-7.37 (m, 16H), 7.54-7.58 (m, 4H), 7.72 (s, 1H), 7.88 (s, 1H). Mass spectrum: $(M+H)^+$ = 515.

C. (2S,3S,5S)-2,5-Diamino-1,6-diphenyl-3-(2-methyl-2-propenoxy)hexane.

A solution of the crude resultant compound of Example 89B in 6 ml of tetrahydrofuran was treated with 6 ml of 1 M aqueous HCl and stirred at ambient temperature for 1.5 h. The reaction mixture was extracted with three 10 ml portions of hexane. The aqueous layer was neutralized with sodium bicarbonate and then extracted with five 10 ml portions of dichloromethane. The combined organic layers was dried over $Na_2SQ_4$, and concentrated in vacuo. Silica gel chromatography of the residue using 2% isopropylainine and 5% methanol in dichloromethane provided 114.0 mg (64%, 3 steps) of the desired compound as a oil. Mass spectrum: $(M+H)^+$ = 339.

D. (2S,3S,5S)-2,5-Bis-(N-(N-((N-methyl-N-((2-pyridinyl)-methyl)amino)carbonyl)valinyl)amino)-1,6-diphenyl-3-(2-methyl-2-propenoxy)hexane.

Using the procedure of Example 5G but replacing the resultant compound of Example 5F with the resultant compound of Example 89C and replacing N-Cbz-valine p-nitrophenyl ester with the resultant compound of Example 3F provided 228.4 mg (93%) of the desired compound as a white solid. Mass spectrum: $(M+H)^+$ = 833.

E. (2S,3S,5S)-2,5-Bis-(N-(N-((N-methyl-N-((2-pyridinyl)-methyl)amino)carbonyl)valinyl)amino)-1,6-diphenyl-3-(acetoxymethoxy)hexane.

A stream of ozone was passed through a solution of 150 mg (0.18 mmol) of the resultant compound of Example 89D in 9 mL of 5:1 $CH_2Cl_2$/MeOH at -78°C until the faint blue color of ozone persisted. The mixture was then purged with $N_2$ for 10 min and then concentrated in vacuo. The residue and 11.1 mg (0.09 mmol) of 4-dimethylaminopyridine were dissolved in 6 ml of dichloromethane, 35.1 µl (0.27 mmol) of triethylamine and 20.4 µl (0.22 mmol) of acetic anhydride were added. The resulting mixture was stirred at 40 °C for 2 h and then quenched with pH 6 buffer. The organic layer was diluted with dichloromethane, separated, dried over $Na_2SO_4$, and concentrated in vacuo to provide, after silica gel chromatography using 10% 2-propanol in dichloromethane, 90.7 mg (59%) of the desired compound as a white solid. $^1H$ NMR ($CDCl_3$) δ 0.72 (d, 3H), 0.78 (d, 3H), 0.84 (d, 6H), 1.54 (m, 1H), 1.73 (m, 1H), 1.99 (s, 3H), 2.14 (m, 2H), 2.63-2.87 (m, 4H), 2.96 (s, 3H), 2.98 (s, 3H), 3.66 (dd, 1H), 4.00-4.11 (m, 2H), 4.33 (m, 1H), 4.40-4.53 (m, 4H), 4.62 (m, 1H), 5.18 (dd, 2H), 6.08 (br, 2H), 6.22 (br d, 1H), 6.28 (br d, 1H), 7.08-7.23 (m, 14H), 7.71 (m, 2H), 8.54 (m, 2H). Mass spectrum: $(M+H)^+$ = 851.

Anal. Calcd for $C_{47}H_{62}N_8O_7$ • 1i-PrOH: C, 65.91; H, 7.74; N, 12.30; Found: C, 66.14; H, 7.64; N, 12.21.

Example 90

(2S,3S,5S)-2,5-Bis-(N-((3-pyrazinyl)methoxycarbonyl)-amino)- 1,6-diphenyl-3-hydroxyhexane.

A. Phenyl ((2-pyrazinyl)methoxy)formate.

Using the procedure of Example 99 but replacing 2-(hydroxymethyl)pyridine with 2-(hydroxymethyl)pyrazine provided 188.9 mg (70%) of the desired compound as a yellow oil.

B. (2S,3S,5S)-2,5-Bis-(N-((3-pyrazinyl)methoxycarbonyl)- amino)-1,6-diphenyl-3-hydroxyhexane.

Using the procedure of Example 99 but replacing the resultant compound of Example 99A with the resultant compound of Example 90A provided 21.2 mg (36%) of the desired compound as a white solid. $^1H$ NMR ($CDCl_3$) δ 1.70 (t, 2H), 2.78 (d, 2H), 2.88 (d, 2H), 3.12 (d, 1H), 3.72 (br, 1H), 3.83 (m, 1H), 4.00 (m, 1H), 4.99 (br d, 1H), 5.22 (m, 5H), 7.09-7.27 (m, 10H), 8.54 (m, 6H). Mass spectrum: $(M+H)^+$ = 557.

Example 91

(2S,3S,5S)-2,5-Bis-(N-((5-pyrimidinyl)-methoxycarbonyl)amino)-1,6-diphenyl-3-hydroxyhexane.

A. p-Nitrophenyl ((5-pyrimidinyl)methoxy)formate.

Using the procedure of Example 98 but replacing 4-(hydroxymethyl)pyridine with 5-(hydroxymethyl)pyrimidine provided 433.4 mg (77.5%) of the desired compound as a white solid.

B. (2S,3S,5S)-2,5-Bis-(N-((3-pyrimidinyl)- methoxycarbonyl)amino)-1,6-diphenyl-3-hydroxyhexane.

Using the procedure of Example 98 but replacing the resultant compound of Example 98A with the resultant compound of Example 91A provided 45.8 mg (78%) of the desired compound as a white solid. $^1H$ NMR ($CDCl_3$) δ 1.66 (m, 2H), 2.76 (d, 2H), 2.85 (d, 2H), 2.88 (m, 1H), 3.67 (br, 1H), 3.81 (m, 1H), 3.95 (m, 1H), 4.87-5.14 (m, 6H), 7.04-7.28 (m, 10H), 8.70 (d, 4H), 9.19 (s, 2H). Mass spectrum: $(M+H)^+$ = 557.

Example 92

(2S,3S,5S)-2,5-Bis-(N-((3,5-dimethyl-4-isoxazolyl)-methoxycarbonyl)amino)-1,6-diphenyl-3-hydroxyhexane.

A. Phenyl ((3,5-dimethyl-4-isoxazolyl)methoxy)formate.

Using the procedure of Example 98 but replacing 4-(hydroxymethyl)pyridine with 3,5-dimethyl-4-(hydroxymethyl)isoxazole provided 0.79 g (69%) of the desired compound as a pale yellow oil.

B. (2S,3S,5S)-2,5-Bis-(N-((3,5-dimethyl-4-isoxazolyl)- methoxycarbonyl)amino)-1,6-diphenyl-3-hydroxyhexane.

Using the procedure of Example 98 but replacing the resultant compound of Example 98A with the resultant compound of Example 92A provided 57.1 mg (92%) of the desired compound as a white solid. $^1H$ NMR (DMSO-d6) δ 1.44 (br t, 2H), 2.11 (s, 6H), 2.28 (s, 3H), 2.30 (5, 3H), 2.64 (m, 4H), 3.53 (m, 1H), 3.82 (m, 2H), 4.65 (d, 1H), 4.72 (m, 4H), 6.80 (br d, 1H), 7.02 (br d, 1H), 7.06-7.21 (m, 10H). Mass spectrum: $(M+H)^+$ = 591.

Example 93

(2S,3S,5S)-2-(N-(N-((N-methyl-N-((2-pyridinyl)- methyl)amino)carbonyl)isoleucinyl)amino)-5-(N-((3-pyridinyl)methoxycarbonyl)amino)-1,6-diphenyl-3-hydroxyhexane.

A mixture of 40 mg (0.095 mmol) of (2S,3S,5S)-2-amino-5-(N-((3-pyridinyl)methoxycarbonyl)amino)-1,6-diphenyl-3-hydroxyhexane from Example 28B and 57.3 mg (0.14 mmol) of the resultant compound of Example 11C in 1 ml of dry tetrahydrofuran was stirred at ambient temperature for 16 h. The solvent was then removed in vacuo, and the residue was purified by silica gel chromatography using 5% methanol in dichloromethane provided 62.1 mg (96%) of the desired compound as a white foamy solid. $^1H$ NMR ($CDCl_3$) δ 0.82 (t, 3H), 0.88 (d, 3H), 1.03 (m, 1H), 1.23 (m, 1H), 1.65 (t, 2H), 1.97 (m, 1H), 2.72 (dd, 2H), 2.82 (dd, 2H), 2.94 (s,3H), 3.65 (br, 1H), 3.96 (br q, 1H), 4,09 (m, 2H), 4.44 (s, 2H), 5.03 (dd, 2H), 5.32 (br d, 1H), 6.49 (br d, 2H), 7.08-7.26 (m 14H), 7.59 (br d, 1H), 7.71 (td, 1H), 8.49 (dt, 1H), 8.54 (dd, 2H). Mass spectrum: $(M+H)^+$ = 681.

Anal. Calcd for $C_{39}H_{48}N_6O_5 \cdot 0.5H_2O$: C, 67.90; H, 7.16; N, 12.18; Found: C, 67.71; H, 7.03; N, 12.13.

Example 94

(2S,3S,5S)-2-(N-(N-((2-pyridinyl)methoxycarbonyl)-isoleucinyl)amino)-5-(N-((3-pyridinyl)methoxycarbonyl)-amino)-1,6-diphenyl-3-hydroxyhexane.

Using the procedure of Example 93 but replacing the resultant compound of Example 11c with the resultant compound of Example 19C provided 37.4 mg (77%) of the desired compound as a white solid. $^1H$ NMR ($CDCl_3$) δ 0.82 (t, 3H), 0.84 (d, 3H), 0.97 (m, 1H), 1.25 (m, 1H), 1.64 (m, 2H), 1.86 (m, 1H), 2.75 (br d, 2H), 2.84 (d, 2H), 3.68 (m, 1H), 3.96 (br t, 2H), 4.10 (m, 1H), 5.03 (dd, 2H), 5.13-5.32 (m, 4H), 6.28 (br d, 1H), 7.07 (d, 2H), 7.17-7.27 (m, 11H), 7.32 (d, 1H), 7.58 (dt, 1H), 7.70 (td, 1H), 8.56 (m, 3H). Mass spectrum: $(M+H)^+$ = 668.

Anal. Calcd for $C_{38}H_{45}N_5O_6 \cdot 0.75H_2O$: C, 66.99; H, 6.88; N, 10.28; Found: C, 66.87; H, 6.66; N, 10.18.

Example 95

(2S,3S,5S)-5-(N-(N-((N-methyl-N-((2-pyridinyl)-methyl)amino)carbonyl)isoleucinyl)amino)-2-(N-((3-pyridinyl)methoxycarbonyl)amino)-1,6-diphenyl-3-hydroxyhexane.

Using the procedure of Example 93 but replacing (2S,3S,5S)-2-amino-5-(N-((3-pyridinyl)methoxycarbonyl)amino)-1,6-diphenyl-3-hydroxyhexane with (2S,3S,5S)-5-amino-2-(N-((3-pyridinyl)methoxycarbonyl)amino)-1,6-diphenyl-3-hydroxyhexane from Example 28B provided 62.1 mg (96%) of the desired compound as a white foamy solid. $^1H$ NMR ($CDCl_3$) δ 0.82 (t, 3H), 0.86 (d, 3H), 1.02 (m, 1H), 1.18 (m, 1H), 1.62 (m, 2H), 2.03 (m, 1H), 2.73 (br t, 2H), 2.84 (dd, 2H), 2.99 (s, 3H), 3.75 (br q, 1H), 4.06 (dd, 1H), 4.19 (m, 2H), 4.43 (m, 2H), 5.04 (dd, 2H), 5.18 (br d, 2H), 6.48 (br d, 1H), 6.57 (br, 1H), 7.07-7.27 (m, 14H), 7.60 (dt, 1H), 7.73 (td, 1H), 8.47 (br d, 1H), 8.28 (br d, 2H). Mass spectrum: $(M+H)^+$ = 681.

Anal. Calcd for $C_{39}H_{48}N_6O_5 \cdot 0.5H_2O$: C, 67 90; H, 7.16; N, 12.18; Found: C, 67,54; H, 7.01; N, 12,10.

Example 96

(2S,3S,5S)-5-(N-(N-((2-pyridinyl)methoxycarbonyl)-isoleucinyl)amino)-2-(N-((3-pyridinyl)methoxycarbonyl)-amino)-1,6-diphenyl-3-hydroxyhexane.

Using the procedure of Example 95 but replacing the resultant compound of Example 11C with the resultant compound of Example 19C provided 25.9 mg (56%) of the desired compound as a white solid. $^1H$ NMR ($CDCl_3$) δ 0.80

(t, 3H), 0.84 (d, 3H), 1.22 (m, 2H), 1.63 (m, 2H), 1.92 (m, 1H), 2.75 (br t, 2H), 2.86 (br d, 2H), 3.67 (m, 1H), 3.80 (m, 1H), 3.94 (br t, 1H), 4.16 (m, 1H), 5.04 (dd, 2H), 5.13-5.30 (m, 4H), 6.40 (br, 1H), 7.09-7.34 (m 14H), 7.59 (br d, 1H), 7.62 (br d, 1H), 8.58 (m, 3H). Mass spectrum: $(M+H)^+$ = 668.

Anal. Calcd for $C_{38}H_{45}N_5O_6 \cdot 0.5H_2O$: C, 67 44; H, 6.85; N, 10.35; Found: C, 67.71; H, 6.72; N, 10.30.

Example 97

(2S,3S,5S)-2,5-Di{N-[3-pyridylmethyl)oxy-carbonyl]amino}-3- hydroxy-1,6-diphenyl hexane

To the compound resulting from Example 1E (2.205 g, 7.866 mmol) dissolved in anhydrous dimethylformamide (10 mL) was added ((3-pyridinyl)methyl)-(4-nitrophenyl)carbonate (6.385 g, 0.0233 mmol). After 5.5 hours, the solvent was removed under reduced pressure and the residue dissolved in methylene chloride, washed with sodium bicarbonate and brine, dried over magnesium sulfate and concentrated under reduced pressure. The residue obtained was chromatographed on silica gel eluting with a gradient of methanol in methylene chloride (2%,5%,10%) to afford the title compound (2.215 g, 52%). $^1H$ NMR (DMSO-$d_6$, 300 MHz) δ 1.50 (m, 2H), 2.50-2.75 (m, 4H), 3.55 (m, 1H), 3.87 (m, 2H), 4.98 (d, 4H), 6.95 (d, 1H), 7.00-7.27 (m, 12H), 7.33 (m, 2H), 7.60 (m, 2H), 8.50 (m, 3H). Anal calcd for $C_{32}H_{34}N_4O_5 \cdot 0.33\ H_2O$: C, 68.64; H, 5.97; N, 9.89. Found: C, 68.57; H, 6.19; N, 10.00. MS (DCI/$NH_3$) m/e 555 $(M+H)^+$.

Example 98

(2S,3S,5S)-2,5-Di{N-[(4-pyridylmethyl)oxy-carbonyl]amino}-3- hydroxy-1,6-diphenyl hexane

A.((4-pyridinyl)methyl)-(4-nitrophenyl)carbonate

To a solution of 4-pyridylcarbinol (169 mg, 1.0 mmol) and 4-methylmorpholine (NMM) (165 μL, 1.5 mmol) dissolved in methylene chloride (1.0 mL) and cooled in an ice bath was added (4-nitrophenyl)chloroformate (300 mg, 1.5 mmol). After 1.33 hours, additional methylene chloride (1 mL) was added. After 2.5 hours, the reaction mixture was treated with methylene chloride and water and filtered. The filtrate was washed with water, saturated sodium bicarbonate solution and brine, dried over magnesium sulfate and concentrated under reduced pressure. The residue obtained was chromatographed on silica gel eluting with methylene chloride and 1% going to 2% methanol in methylene chloride to afford the title compound (83 mg).

B. (2S,3S,5S)-2,5-Di{N-[4-pyridylmethyl)oxy-carbonyl]amino}- 3-hydroxy-1,6-diphenyl hexane

The compound resulting from Example 98A (213 mg, 0.777 mmol) and the compound resulting from Example 1E (70 mg, 0.246 mmol) were dissolved in dimethylformamide (0.8 mL) and stirred at room temperature for 3 days. The solvent was removed under reduced pressure and the residue obtained dissolved in chloroform, filtered, and the filtrate washed with saturated sodium bicarbonate solution and brine, dried over magnesium sulfate, and concentrated under reduced pressure. Chromatography on silica gel eluting with 5% methanol in methylene chloride afforded the title compound. $^1H$ NMR (DMSO-$d_6$, 300 MHz) δ 1.50 (m, 2H), 2.50-2.75 (m, 4H), 3.55 (m, 1H), 3.87 (m, 2H), 4.98 (d, 4H), 6.95 (d, 1H), 7.00-7.27 (m, 12H), 7.33 (m, 2H), 8.50 (m, 3H). MS (DCI/$NH_3$) m/e 555 $(M+H)^+$.

Example 99

(2S,3S,5S)-2,5-Di{N-[2-pyridylmethyl)oxycarbonyl]amino}-3- hydroxy-1,6-diphenyl hexane

A. (2-Pyridylmethyl)phenylcarbonate

To a solution of 2-pyridine carbinol (109 mg, 1.0 mmol) dissolved in methylene chloride (3 mL) and NMM (165 μL, 1.5 mmol) and cooled in an ice bath was added phenyl chloroformate (188 μL, 1.5 mmol) dissolved in methylene chloride (1.0 mL) dropwise. The reaction mixture was stirred at 0 °C for 1 hour, diluted with methylene chloride, washed with saturated sodium bicarbonate and brine, dried over magnesium sulfate, and concentrated under reduced pressure. The residue obtained was chromatographed on silica gel eluting with 40% ethyl acetate in hexane to afford the title compound (176 mg).

B. (2S,3S,5S)-2,5-Di{N-[2-pyridylmethyl)oxy-carbonyl]amino}-3- hydroxy-1,6-diphenyl hexane

The compound resulting from Example 1E (92.5 mg, 0.326 mmol) and the compound resulting from Example 99A (310 mg, 1.35 mmol) were dissolved in dimethylformamide (1 mL) and warmed at 60 °C for 6.5 hours, allowed to stand overnight at room temperature, and then heated at 80 °C for 4 hours. The reaction mixture was concentrated under reduced pressure and the residue obtained chromatographed on silica gel eluting with 5% methanol in methylene chloride to afford the title compound (104 mg). $^1$H NMR (DMSO-$d_6$, 300 MHz) δ 1.56 (m, 2H), 2.54-2.75 (m, 4H), 3.63 (m, 1H), 3.90 (m, 2H), 4.73 (m, 1H), 4.80-5.06 (m, 4H), 7.04-7.30 (m, 17H), 7.72 (m, 2H), 8.49 (m, 2H). MS (DCI/$NH_3$) m/e 555 $(M+H)^+$.

Example 100

(2S,3S,5S)-2,5-Di[N-{[1-(3-pyridyl)ethyl]oxy-carbonyl}amino]-3-hydroxy-1,6-diphenyl hexane

The compound resulting from Example 1E (70 mg, 0.246 mmol) and [1-(3-pyridyl)ethyl]-(4-nitrophenyl)carbonate (220 mg, 0.764 mmol) were dissolved in dimethylformamide (1.0 mL) and stirred at room temperature for 2.5 days. The reaction mixture was concentrated under reduced pressure and the residue obtained chromatographed on silica gel eluting with a gradient of methanol in methylene chloride (2%,5%,10%,20%) containing 0.5% ammonium hydroxide to afford the title compound (80.6 mg). $^1$H NMR (DMSO-$d_6$, 300 MHz) δ 1.30-1.56 (m, 8H), 2.36-2.78 (m, 4H), 3.37-3.93 (m, 5H), 5.57 (m, 2H), 6.78-7.56 (m, 16H), 7.72 (m, 1H), 8.40-8.60 (m, 4H). MS (DCI/$NH_3$) m/e 583 $(M+H)^+$.

Example 101

(2S,3S,5S)-2-[(tert-Butyloxycarbonyl)amino]-5-[N-{(3- pyridyl)methyloxy-carbonyl}amino]-3-hydroxy-1,6-diphenyl hexane

A. (2S,3S,5S)-2-Amino-5-[N-{(3-pyridyl)methyloxy- carbonyl}amino]-3-hydroxy-1,6-diphenyl hexane

The compound resulting from Example 1E (820 mg, 2.89 mmol) and (3-pyridylmethyl) phenyl carbonate (728 mg, 3.179 mmol) were dissolved in dimethylformamide and warmed at 50 °C for 15.5 hours. The solvent was removed under reduced pressure and the residue obtained chromatographed on silica gel eluting with a gradient of methanol in methylene chloride (2%,5%,10%) to afford a mixture of compounds (919 mg). This material was re-chromatographed on silica gel eluting with 2% methanol in methylene chloride containing 1% isopropylamine to afford the title compound (424 mg, 35%). Also isolated was the regio-isomer in which substitution occurred at the 2-amino group instead of the 4-amino group.

B. (2S,3S,5S)-2-[(tert-Butyloxycarbonyl)amino]-5-[N-{(3-pyridyl)methyloxycarbonyl}amino]-3-hydroxy-1,6-diphenyl hexane

To the product of Example 101A (92.5 mg, 0.215 mmol) dissolved in methylene chloride was added di-*t*-butyl-dicarbonate (90 mg). After 2 hours, additional di-t-butyl-dicarbonate (33 mg) was added. After an additional hour, the reaction mixture was concentrated under reduced pressure. The residue obtained was chromatographed on silica gel eluting with methanol in methylene chloride (2%,5%) to afford the title compound (194 mg, 79%). $^1$H NMR (DMSO-$d_6$, 300 MHz) δ 1.30 (s, 9H), 1.50 (m, 2H), 2.53-2.74 (m, 4H), 3.52 (m, 1H), 3.72-3.97 (m, 3H), 4.58 (d, 1H), 4.82-5.00 (m, 2H), 6.31 (bd, 1H), 7.10-7.27 (m, 15H), 7.34 (m, 1H), 7.58 (m, 1H), 8.50 (m, 2H). MS (DCI/$NH_3$) m/e 520 $(M+H)^+$.

Example 102

(2S,3S,5S)-2-[(Benzyloxycarbonyl)amino]-5-[N-{(3- pyridyl)methyloxycarbonyl}amino]-3-hydroxy-1,6-diphenyl hexane

To the product of Example 101A (76 mg, 0.1814 mmol) dissolved in methylene chloride (2 mL) was added N-[benzyloxycarbonyl)oxy]succinimide (68 mg, 0.272 mmol). The reaction mixture was stirred overnight at room temperature and then concentrated under reduced pressure. The residue obtained was chromatographed on silica gel eluting with methanol in methylene chloride (0%,2%,5%) to afford the title compound. $^1$H NMR (DMSO-$d_6$, 300 MHz) δ 1.51 (m, 2H), 2.54-2.75 (m, 4H), 3.57 (m, 1H), 3.87 (m, 2H), 4.68 (m, 1H), 4.72-4.90 (m, 1H), 4.96 (m, 4H), 6.90 (d, 1H), 7.00-7.38 (m, 18H), 7.60 (m, 1H), 8.50 (m, 2H). MS (DCI/$NH_3$) m/e 554 $(M+H)^+$.

Example 103

(2S,3S,5S)-5-[(*tert*-Butyloxycarbonyl)amino]-2-[N-{(3- pyridyl)methyloxycarbonyl}amino]-3-hydroxy-1,6-diphenyl hexane

The compound resulting from isolation of the regioisomer in Example 101A (80 mg, 0.191 mmol) was reacted by the procedure described in Example 101B to give crude material. Chromatography on silica gel eluting with methanol in methylene chloride (0%,2%,5%) afforded the title compound (87 mg, 88%). $^1H$ NMR (DMSO-$d_6$, 300 MHz) δ 1.30 (s, 9H), 1.46 (m, 2H), 2.53-2.78 (m, 4H), 3.56 (m, 1H), 3.86 (m, 2H), 4.63 (bd, 1H), 4.83-5.03 (m, 3H), 6.63 (bd, 1H), 6.90 (bd, 1H), 7.00-7.27 (m, 14H), 7.34 (m, 2H), 7.59 (m, 1H), 8.49 (m, 2H). MS (DCI/$NH_3$) m/e 520 $(M+H)^+$.

Example 104

(2S,3S,5S)-5-[(Benzyloxycarbonyl)amino]-2-[N-{(3- pyridyl)methyloxycarbonyl}amino]-3-hydroxy-1,6-diphenyl hexane

The compound resulting from isolation of the regioisomer in Example 101A (80 mg, 0.191 mmol) was reacted with N-[benzyloxycarbonyl)oxy]succinimide (71 mg, 0.286 mmol) by the procedure described in Example 102 to give, after column chromatography on silica gel, the title compound (89.3 mg, 85%). $^1H$ NMR (DMSO-$d_6$, 300 MHz) δ 1.50 (m, 2H), 2.53-2.74 (m, 6H), 3.57 (m, 1H), 3.87 (m, 2H), 4.68 (m, 1H), 4.87 (m, 5H), 6.94 (bd, 1H), 7.00-7.37 (m, 18H), 7.60 (m, 1H), 8.50 (m, 2H). MS (DCI/$NH_3$) m/e 554 $(M+H)^+$.

Example 105

(2S,3S,5S)-2,5-Di[N-{[(2-methylpyridin-5-yl)methyl]oxycarbonyl}amino]-3-hydroxy-1,6-diphenyl hexane

2-Methylpyridine-5-carbinol (246 mg, 2.0 mmol) was converted to the 4-nitrophenyl carbonate by the procedure described in Example 98A. The crude material was chromatographed on a silica gel column eluting with a gradient of ethyl acetate in hexane (50%,90%) to give the carbonate. The 300 MHz $^1H$ NMR spectrum was found to be consistent with the proposed structure.

The compound resulting from Example 1E (93 mg, 0.327 mmol) was reacted with the above carbonate (282 mg, 0.981 mmol) in dimethylformamide (0.60 mL) overnight at room temperature. The reaction mixture was concentrated under reduced pressure and the residue obtained chromatographed on silica gel eluting with a gradient of methanol in methylene chloride (2%,5%) to afford the title compound (103 mg, 54%). $^1H$ NMR (DMSO-$d_6$, 300 MHz) δ 1.47 (m, 2H), 2.43 (bd, 6H), 2.53-2.74 (m, 4H), 3.52-3.60 (m, 2H), 3.87 (m, 2H), 4.67 (bd, 1H), 4.68-4.83 (m, 1H), 4.91 (bd, 4H), 6.89 (bd, 1H), 7.00-7.38 (m, 14H), 7.49 (d of d, 2H), 8.35 (m, 2H). MS (DCI/$NH_3$) m/e 583 $(M+H)^+$.

Example 106

(2S,3S,5S-2,5-Di[N-{(2-(3-pyridyl)propan-2-yl]oxy- carbonyl}amino]-3-hydroxy-1,6-diphenyl hexane

3-(2-Hydroxypropan-2-yl)pyridine (57 mg, 0.416 mmol) was converted to the 4-nitrophenyl carbonate by the procedure described in Example 98A The crude residue was chromatographed on silica gel eluting with 50% ethyl acetate in hexane to afford the carbonate. The 300 MHz $^1H$ NMR spectrum was found to be consistent with the proposed structure.

The compound resulting from Example 1E (88 mg, 0.31 mmol) was reacted with the above carbonate (281 mg, 0.93 mmol) in dimethylformamaide (0.60 mL) overnight at room temperature. The reaction mixture was concentrated under reduced pressure and the residue obtained chromatographed on silica gel eluting with a gradient of methanol in methylene chloride (2%,5%,10%) to afford the title compound (109 mg). $^1H$ NMR (DMSO-$d_6$, 300 MHz) δ 1.34-1.69 (m, 8H), 2.43-2.85 (m, 4H), 3.49 (m, 2H), 3.71 (m, 3H), 4.57 (d, 1H), 6.72 (bd, 1H), 6.86-7.32 (m, 14H), 7.49 (m, 2H), 8.36 (m, 2H), 8.51 (m, 2H). MS (DCI/$NH_3$) m/e 611 $(M+H)^+$.

Example 107

2(S),5(S)-Bis-(N-(p-nitrophenoxycarbonyl)-amino)-1,6- diphenyl-3(S)-trimethylsiloxy-hexane.

To a solution of 200 mg of the resultant product from Example 1E in 5 ml of dichloromethane was added at 0°C 0.112 ml of TEA and 0.098 ml of trimethylsilyl chloride. After 30 minutes at 0°C, 0.215 ml of TEA and 0.3 gm of p-nitrophenylchloroformate was added. After 1 hour at 0°C, the solvent was removed in vacuo and the crude product purified by silica gel column chromotography provided 0.3 gm of desired product. $^1$H NMR ($CDCl_3$): δ 0.20 (s, 9H), 1.70 (m, 1H), 1.90 (m, 1H), 2.85 (m, 4H), 3.90 (m, 1H), 4.00 (m, 1H), 4.20 (m, 1H), 4.90 (d, 1H), 5.30 (d, 1H), 7.10-7.30 (m, 14H), 8.20 (m, 4H).

Example 108

2(S),5(S)-Bis-(N-(3-pyridylmethylamino-carbonyl)-amino)-1,6-diphenyl-3(S)-hydroxyhexane.

To a solution of 87 mg of the resultant compound from Example 107 in 1 ml of DMF was added 0.028 ml of 3-aminomethylpyridine. After 18 hours, the solvent was removed in vacuo and the residue was dissolved in 1 ml of methanol and 0.05 ml of chlorotrimethylsilane was added. After 0.5 hour, the solvent was removed in vacuo, neutralized with sodium bicarbonate solution and extraction with ethyl acetate (2 x 25 ml). The organic solution was dried and concentrated. Purification by silica gel column chromotography provided 35 mg of desired product. $^1$H NMR ($CD_3OD$): δ 1.60 (t, 2H), 2.60-2.80 (m, 4H), 3.70 (m, 1H), 4.00 (m, 1H), 4.10 (m, 1H), 4.25-4.35 (m, 4H), 7.10-7.25 (m, 10H), 7.35 (m, 2H), 7.60 (m, 2H), 8.40 (m, 4H). Mass spectrum: $(M+H)^+$ = 553.

Example 109

2(S),5(S)-Bis-(N-(N-methyl-N-3-pyridylmethylamino)carbonylamino)-1,6-diphenyl-3(S)-hydroxyhexane.

Using the procedure described in Example 108 but replacing 3-aminomethylpyridine with N-methyl-3-aminomethylpyridine provided the desired product in 50% yield. $^1$H NMR ($CDCl_3$) : δ 1.65 (m, 2H), 2.70 (s, 3H), 2.74 (s, 3H), 2.80-3.00 (m, 4H), 3.70 (m, 1H), 3.82 (m, 1H), 4.02 (m, 1H), 4.38-4.55 (m, 4H), 4.80 (d, 1H), 4.88 (d, 1H), 5.15 (d, 1H), 7.10-7.30 (m, 12H), 7.48 (m, 2H), 8.45 (m, 2H), 8.50 (m, 2H). Mass spectrum: $(M+H)^+$ = 581.

Example 110

(2S,3S,5S)-2,5-Di-(Boc-amino)-1,6-diphenyl-3-hydroxy-hexane

To a solution of the resultant compound of Example 1E (1.00 g, 4.1 mmol) dissolved in methylene chloride (40 mL) was added di-*t*-butyldicarbonate (1.98 g, 2.2 equiv). The reaction mixture was stirred at room temperature for 1 hour and then concentrated *in vacuo* at 40 °C. The residue obtained was chromatographed on silica gel eluting with 1:3 going to 1:2 ethyl acetate/hexane to afford the title compound (1.315 g, 72%). $^1$H NMR ($CDCl_3$, 300 MHz) δ 1.39 (s, 18H), 1.62 (m, 2H), 2.74 (d, 2H), 2.85 (t, 2H), 3.64 (m, 2H), 3.86 (d of d, 1H), 4.55 (bs, 1H), 4.80 (bd, 1H), 7.07-7.32 (m, 10H). Anal calcd for $C_{28}H_{40}N_2O_5$: C, 69.39; H, 8.32; N, 5.78. Found: C, 69.21; H, 8.38; N, 5.73. MS (DCI/$NH_3$) m/e 485 $(M+H)^+$, 502 $(M+H+NH_3)^+$.

Example 111

(2S,3S,5S)-1,6-Diphenyl-2,5-di-(phenyloxycarbonylamino)- 3-hydroxy-hexane

To a solution of the resultant compound of Example 1E (100 mg, 0.35 mmol) and anhydrous triethylamine (0.12 mL, 2.5 equiv) dissolved in anhydrous methylene chloride (4 mL) and cooled under nitrogen to -40 °C was added phenyl chloroformate (0.09 mL, 2 equiv). After stirring at -40 °C for 1 hour, the reaction mixture was diluted with methylene chloride (50 mL) and washed with saturated sodium bicarbonate (20 mL) and saturated sodium chloride (20 mL). The organic phase was dried over magnesium sulfate and concentrated under reduced pressure. The residue obtained was chromatographed on silica gel eluting with 1:4 going to 1:2 going to 1:1 ethyl acetace/hexane to afford the title compound (64 mg, 35%). $^1$H NMR ($CDCl_3$, 300 MHz) δ 1.80 (m, 2H), 2.86 (d, 2H), 2.94 (d of d, 2H), 3.02 (bd, 1H), 3.76 (bs, 1H), 3.86 (d of d, 1H), 4.03 (d of d, 1H), 5.13 (bd, 1H), 5.35 (bd, 1H), 7.00-7.40 (m, 20H). MS (DCI/$NH_3$) m/e 525 $(M+H)^+$, 542 $(M+H+NH_3)^+$.

Example 112

(2S,3S,5S)-2,5-Di-(isopropyloxycarbonylamino)-1,6-diphenyl-3- hydroxy-hexane

The resultant compound of Example 1E (100 mg, 0.35 mmol) was reacted with 1*M* isopropyl chloroformate in toluene (0.70 mL, 2.0 equiv) by the procedure described in Example 111. Column chromatography on silica gel eluting with 1:2 going to 1:1 ethyl acetate/hexane afforded the title compound (92 mg, 57%). $^1$H NMR ($CDCl_3$, 300 MHz) δ

1.17 (m, 12H), 1.63 (m, 2H), 2.75 (d, 2H), 2.86 (m, 2H), 3.31 (bs, 1H), 3.69 (m, 2H), 3.90 (m, 1H), 4.63 (m, 1H), 4.89 (m, 3H), 7.04-7.42 (m, 10H). Anal calcd for $C_{26}H_{36}N_2O_5$: C, 68.40; H, 7.95; N, 6.14. Found: C, 68.10; H, 7.99; N, 6.14. MS (DCI/$NH_3$) m/e 457 $(M+H)^+$, 474 $(M+H+NH_3)^+$.

Example 113

(2S,3S,5S)-2,5-Di-(3,3-dimethylacryloylamino)-1,6-diphenyl-3-hydroxy-hexane

The resultant compound of Example 1E (150 mg, 0.53 mmol) was reacted with 3,3-dimethylacryloyl chloride (0.12 mL, 2.0 equiv) by the procedure described in Example 111 except that pyridine (0.26 mL, 6 equiv) was used instead of triethylamine. Column chromatography on silica gel eluting with 1:2 going to 1:1 ethyl acetate/hexane afforded the title compound (182 mg, 77%). $^1H$ NMR ($CDCl_3$, 300 MHz) δ 1.65 (m, 3H), 1.82 (m, 6H), 2.10 (m, 6H), 2.87 (m, 2H), 2.90 (m, 2H), 3.62 (m, 1H), 3.96 (m, 1H), 4.10 (m, 1H), 4.62 (bs, 1H), 5.43 (m, 1H), 5.50 (m, 1H), 5.53 (bd, 1H), 5.72 (bd, 1H), 7.05-7.30 (m, 10H). Anal calcd for $C_{28}H_{36}N_2O_3$: C, 74.97; H, 8.09; N, 6.24. Found: C, 74.25; H, 8.43; N, 6.12. MS (DCI/$NH_3$) m/e 449 $(M+H)^+$, 466 $(M+H+NH_3)^+$.

Example 114

(2S,3S,5S)-2,5-Di-(isovalerylamino)-1,6-diphenyl-3-hydroxy-hexane

The resultant compound of Example 1E (150 mg, 0.53 mmol) was reacted with isovaleryl chloride (0.13 mL, 2.0 equiv) by the pyridine procedure described in Example 113. Column chromatography eluting with 1:2 going to 1:1 going to 2:1 ethyl acetate/hexane afforded the title compound (38 mg, 16%). $^1H$ NMR ($CDCl_3$, 300 MHz) δ 0.84 (m, 12H), 1.65 (m, 3H), 1.97 (m, 6H), 2.78 (m, 2H), 2.89 (d, 2H), 3.63 (bs, 1H), 3.99 (m, 1H), 4.10 (m, 1H), 4.52 (bs, 1H), 5.60 (d, 1H), 5.80 (d, 1H), 7.07-7.30 (m, 10H). MS (DCI/$NH_3$) m/e 453 $(M+H)^+$, 470 $(M+H+NH_3)^+$.

Example 115

(2S,3S,5S)-2,5-Di-(isobutyloxycarbonylamino)-1,6-diphenyl-3- hydroxy-hexane

The resultant compound of Example 1E (100 mg, 0.35 mmol) was reacted with isobutylchloroformate (0.09 mL, 2.0 equiv) by the procedure described in Example 111. Column chromatography on silica gel eluting with 1:2 ethyl acetate/hexane afforded the title compound (114 mg, 69%). $^1H$ NMR ($CDCl_3$, 300 MHz) δ 0.89 (m, 12H), 1.65 (m, 2H), 1.86 (m, 2H), 2.77 (bd, 2H), 2.87 (m, 2H), 3.30 (bs, 1H), 3.60-3.97 (m, 7H), 4.70 (m, 1H), 4.97 (bd, 1H), 7.07-7.32 (m, 10H). Anal calcd for $C_{28}H_{40}N_2O_5$: C, 69.39; H, 8.32; N, 5.78. Found: C, 69.20; H, 8.32; N, 5.75. MS (DCI/$NH_3$) m/e 485 $(M+H)^+$, 502 $(M+H+NH_3)^+$.

Example 116

(2S,3R,5S)-1,6-Diphenyl-3-hydroxy-2,5-di-(Boc-amino)-hexane

A. 1,6-Diphenyl-3-oxo-2,5-di-(Boc-amino)-hexane

To a solution of oxalyl choride (0.09 mL, 1.03 mmol) dissolved in anhydrous methylene chloride (3 mL) and cooled to -78 °C was added dimethyl sulfoxide (0.147 mL, 2.07 mmol) dropwise. The reaction mixture was stirred at -78 °C for 10 minutes and then a solution of the resultant compound of Example 110 (250 mg, 0.52 mmol) dissolved in anhydrous methylene chloride (5 mL) was added dropwise. After stirring at -78 °C for 1 hour, anhydrous triethylamine (0.57 mL, 4.12 mmol) was added, the cooling bath was removed, and the reaction mixture was stirred for 15 minutes. The reaction mixture was diluted with methylene chloride (50 mL) and washed with saturated sodium bicarbonate (20 mL) and saturated sodium chloride (20 mL), dried over magnesium sulfate, and concentrated *in vacuo*. The residue obtained was chromatographed on silica gel eluting with 1:6 going to 1:4 ethyl acetate/hexane to afford the title compound (235 mg, 94%). MS (DCI/$NH_3$) m/e 483 $(M+H)^+$, 500 $(M+H+NH_3)^+$.

B. (2S,3S,4R)-1,6-Diphenyl-3-hydroxy-2,5-di-(Boc-amino)-hexane

To the resultant compound of Example 116A(20 mg, 0.04 mmol) dissolved in methanol (0.4 mL) and methylene chloride (0.3 mL) and cooled to -78 °C was added sodium borohydride (1.6 mg, 1.0 equiv). The reaction mixture was allowed to slowly warm to -20 °C and maintained at that temperature for 18 hours. The reaction mixture was diluted

with methylene chloride (10 mL) and washed with saturated sodium chloride solution (3 mL), dried over magnesium sulfate, and concentrated *in vacuo*. The residue obtained was chromatographed on silica gel eluting with 1:4 going to 1:2 ethyl acetate/hexane to afford the title compound (14.7 mg, 73%). $^1$H NMR ($CDCl_3$, 300 MHz) δ 1.35 (s, 9H), 1.40 (s, 9H), 1.42-1.73 (m, 3H), 2.80 (m, 2H), 2.87 (bd, 2H), 3.56 (bs, 1H), 3.84 (bs, 1H), 4.15 (m, 1H), 4.46 (m, 1H), 4.59 (m, 1H), 7.12-7.32 (m, 10H). MS ($DCI/NH_3$) m/e 485 $(M+H)^+$.

Example 117

(2S,3S,5S)-1,6-Diphenyl-2,5-di[N-(furan-2-ylmethyloxycarbonyl)amino]-3-hydroxy hexane

A. (Furan-2-yl)(4-nitrophenyl)carbonate

To a solution of 2-furanmethanol (413 mg, 0.261 mmol) and N-methylmorpholine (468 μL, 4.261 mmol) dissolved in methylene chloride (3 mL) and cooled in an ice bath was added a solution of 4-nitrophenylchloroformate ( 859 mg, 4.261 mmol) dissolved in methylene chloride (3 mL). The mixture was stirred at 0 °C for 3.5 hours and then worked up to give a residue which was chromatographed on silica gel eluting with 10% ethyl acetate in hexane followed by 10% ethyl acetate in methylene chloride to afford the title compound (113 mg) after cyrstallization from ethyl acetate and hexane.

B. (2S,3S,5S)-1,6-Diphenyl-2,5-di[N-(furan-2- ylmethyloxycarbonyl)amino]-3-hydroxy hexane

To the compound resulting from Example 1E (75 mg, 0.264 mmol) dissolved in dimethylformamide (0.6 mL) was added the compound resulting from Example 117 (208 mg, 0.79 mmol). The mixture was stirred at room temperature overnight and then the solvent removed under reduced pressure. The crude product was chromatograhed on silica gel eluting with 5-10% ethyl acetate in methylene chloride to afford the title compound. $^1$H NMR (DMSO-$d_6$, 300 MMz) δ 1.46 (m, 2H), 2.53-2.79 (m, 5H), 3.57 (m, 1H), 3.89 (m, 3H), 4.64 (d, 1H), 4.79-4.95 (m, 5H), 6.41 (m, 4H), 6.89 (d, 1H), 7.08-7.29 (m, 13H), 7.63 (m, 2H). Anal calcd for $C_{30}H_{32}N_2O_7$: C, 67.67; H, 6.01; N, 5.26. Found: C, 67.25; H, 5.94; N, 5.22. MS ($DCI/NH_3$) m/e 533 $(M+H)^+$, 550 $(M+H+NH_3)^+$.

Example 118

(2S,3S,5S)-1,6-Diphenyl-2,5-di[N-(furan-3-ylmethyloxycarbonyl)amino]-3-hydroxy hexane

(Furan-3-yl)(4-nitrophenyl)carbonate was prepared in analogy to Example 117 starting from 3-furanmethanol instead of 2-furanmethanol. The compound resulting from Example 1E (70 mg, 0.249 mmol) was reacted with the above carbonate (144 mg, 0.548 mmol) by the procedure described in Example 117B to afford crude material. Column chromatography on silica gel eluting with a gradient (5%,10%,50%) of ethyl acetate in methylene chloride afforded the title compound. $^1$H NMR (DMSO-$d_6$, 300 MHz) δ 1.48 (m, 2H), 2.52-2.78 (m, 5H), 3.56 (m, 1H), 3.88 (m, 2H), 4.64 (d, 1H), 4.78 (d, 4H), 6.37 (s, 2H), 6.78 (d, 1H), 7.03 (d, 1H), 7.10-7.28 (m, 10H), 7.60 (m, 3H). Anal calcd for $C_{30}H_{32}N_2O_7$: C, 67.67; H, 6.01; N, 5.26. Found: C, 67.31; H, 5.99, N, 5.21. MS ($DCI/NH_3$) m/e 533 $(M+H)^+$, 550 $(M+H+NH_3)^+$.

Example 119

(2S,3S,5S)-1,6-Diphenyl-2,5-di[N-(5-bromopyridin-3- ylmethyloxycarbonyl)amino]-3-hydroxy hexane

A. (5-Bromo-pyridin-3-ylmethyl)(4-nitrophenyl)carbonate

5-Bromo-nicotinic acid (5,00 g, 24.7 mmol) was dissolved in methanol (50 mL) and saturated with hydrochloric acid gas. The reaction mixture was allowed to stand for 2.5 days and then filtered. The filtrate was concentrated under reduced pressure and methylene chloride was added. The solution was washed with saturated sodium bicarbonate solution. The aqueous wash was back-extracted with inethylene chloride (2x). The combined organic extracts were dried over magnesium sulfate and concentrated *in vacuo* to afford 5-bromo-nicotinic acid methyl ester (4.72 g).

To the above methyl ester (4.536 g, 21 mmol) dissolved in tetrahydrofuran (15 mL) and cooled in a dry ice/acetone bath was added 1*M* lithium aluminum hydride (21 mL, 21 mmol) diluted with tetrahydrofuran (10 mL). The reaction mixture was stirred for 40 minutes and then water (0.80 mL) followed by 15% sodium hydroxide (0.80 mL) and water (2.4 mL) were added. The mixture was stirred for 1 hour and filtered. The filtrate was dried over magnesium sulfate and concentrated under reduced pressure. The crude product was chromatographed on silica gel eluting with 2% methanol in methylene chloride to afford 5-bromo-3-pyridinemethanol (2.472 g).

The above compound (817 mg, 4.346 mmol) was reacted with 4-nitrophenylchloroformate (1.051 g, 5.21 mmol) by the procedure described in Example 117A to give the title compound (847 mg, 55%).

B. (2S,3S,5S)-1,6-Diphenyl-2,5-di[N-(5-bromopyridin-3- ylmethyloxycarbonyl)amino]-3-hydroxy hexane

The compound resulting from Example 1E (65 mg, 0.229 mmol) was reacted with the compound resulting from Example 119A (242 mg, 0.687 mmol) by the procedure described in Example 117B to give the title compound (114 mg, 70%). $^1$H NMR (DMSO-$d_6$, 300 MHz) δ 1.50 (m, 2H), 2.53-2.74 (m, 5H), 3.56 (m, 1H), 3.76 (m, 2H), 4.72 (d, 1H), 4.98 (d, 4H), 6.99-7.25 (m, 12H), 7.92 (m, 2H), 8.49 (m, 2H), 8.64 (dd, 2H). Anal calcd for $C_{32}H_{32}Br_2N_4O_5$: C, 53.93; H, 4.49; N, 7.86. Found: C, 54.46; H, 4.63; N, 7.93. MS (DCI/$NH_3$) m/e 711 $(M+H)^+$.

Example 120

(2S,3S,5S)-1,6-Diphenyl-2,5-di[N-(5-methylpyridin-3-ylmethyloxycarbonyl)amino]-3-hydroxy hexane

5-Methylpyridine-3-methanol was prepared in analogy to the procedure described in Example 119A. It was reacted with 4-nitrophenylchloroformate by the procedure described in Example 117A to give (5-methylpyridin-3-yl)(4-nitrophenyl)carbonate (474 mg).

The compound resulting from Example 1E (36.1 mg, 0.127 mmol) was reacted with the above carbonate (110 mg, 0.38 mmol) by the procedure described in Example 117B to give crude material. Column chromatography on silica gel eluting with a gradient (2%,5%) methanol in methylene chloride afforded the title compound (64 mg). $^1$H NMR (DMSO-$d_6$, 300 MHz) δ 1.48 (m, 2H), 2.25 (d, 6H), 2.52-2.73 (m, 5H), 3.55 (m, 1H), 3.88 (m, 2H), 4.68 (d, 1H), 4.93 (d, 4H), 6.92 (d, 1H), 7.08-7.26 (m, 12H), 7.46 (s, 2H), 8.32 (m, 3H). Anal calcd for $C_{34}H_{38}N_4O_5$: C, 70.10; H, 6.53; N, 9.62. Found: C, 70.29; H, 6.62; N, 9.60. MS (DCI/$NH_3$) m/e 583 $(M+H)^+$.

Example 121

(2S,3S,5S)-1,6-Diphenyl-2,5-di[N-(N-Boc-6-amino-pyridin-3- ylmethyloxycarbonyl)amino]-3-hydroxy hexane

6-Amino-nicotinic acid (5.00 g, 36.2 mmol) was esterified by the procedure described in Example 119A to give the methyl ester. To the methyl ester (2.013 g, 9.32 mmol) dissolved in acetonitrile (80 mL) was added di-t-butyl-dicarbonate (2.235 g, 10.25 mmol) followed by dimethylamino-pyridine (122 mg, 1.0 mmol). The reaction mixture was stirred at room temperature for 4 hours and then additional di-t-butyl-dicarbonate (450 mg) was added. The reaction mixture was stirred an additional hour at room temperature and then stored in the refrigerator overnight. The solvent was removed under reduced pressure and the crude material chromatographed on silica gel eluting with a gradient (5%,10%) of ethyl acetate in methylene chloride to afford the N-Boc-6-amino-nicotinic acid methyl ester (1.85 g, 79%).

The methyl ester (1.85 g, 7.34 mmol) was reduced with lithium aluminum hydride by the procedure described in Example 119A to give, after chromatography on silica gel eluting with a gradient (2%,5%) of methanol in methylene chloride, N-Boc-6-amino-pyridine-3-methanol (1.064 g). This compound (311 mg, 1.388 mmol) was reacted with 4-nitrophenylchloroformate (308 mg, 1.53 mmol) by the procedure described in Example 117A to give (N-Boc-6-amino-pyridin-3-ylmethyl)(4-nitrophenyl)carbonate (388 mg).

The compound resulting from Example 1E (87 mg, 0.301 mmol) was reacted with the above carbonate (340 mg, 0.904 mmol) by the procedure described in Example 117B to give crude material. Chromatography on silica gel eluting with a gradient (2%,5%) of methanol in methylene chloride afforded the title compound (133 mg). $^1$H NMR (DMSO-$d_6$, 300 MHz) δ 1.47 (s, 20H), 2.53-2.75 (m, 5H), 3.55 (m, 1H), 3.87 (m, 2H), 4.65 (d, 1H), 4.89 (d, 4H), 6.87 (d, 1H), 7.07-7.27 (m, 11H), 7.59 (dd, 2H), 7.75 (dd, 2H), 8.18 (m, 2H), 9.80 (d, 2H). Anal calcd for $C_{42}H_{52}N_6O_9 \cdot 0.33\ H_2O$: C, 63.80; H, 6.71; N, 10.63. Found: C, 63.94; H, 6.67; N, 10.57.MS (FAB) M/E 785 (M + 1)

Example 122

(2S,3S,5S)-1,6-Diphenyl-2-[N-(furan-3- ylmethyloxycarbonyl)amino]-5-[N-(pyridin-3- ylmethyloxycarbonyl)amino]-3-hydroxy hexane

3-Pyridinemethanol was reacted with 4-nitrophenylchloro-formate by the procedure described in Example 117A to give the (3-pyridylmethyl)(4-nitrophenyl)carbonate. This compound was reacted with the compound resulting from Example 1E by the procedure described in Example 117B to give a mixture of 2- and 5- substituted compounds which were separable by column chromatography. The (2S,3R,5S)-1,6-diphenyl-5-[N-(pyridin-3-ylmethyloxycarbonyl)amino]-3-hydroxy hexane compound (55 mg, 0.131 mmol) was reacted with (furan-3-yl)(4-nitrophenyl)carbonate,

prepared as described in Example 118, by the procedure described in Example 117B to give, after chromatography on silica gel eluting with a gradient (2%,5%) of methanol in methylene chloride, the title compound (63.1 mg). $^1$H NMR (DMSO-$d_6$, 300 MHz) δ 1.50 (m, 2H), 2.54-2.75 (m, 5H), 3.56 (m, 1H), 3.89 (m, 2H), 4.66 (d, 1H), 4.68-4.92 (m, 1H), 4.79 (s, 2H), 4.97 (s, 2H), 6.37 (m, 1H), 6.79 (d, 1H), 6.90-7.38 (m, 12H), 7.25 (dd, 1H), 7.6 (m, 3H), 8.50 (m, 2H). Anal calcd for $C_{31}H_{33}N_3O_6$ • 0.33 $H_2O$: C, 67.78; H, 6.19; N, 7.65. Found: C, 67.97; H, 6.15; N, 7.69. MS (DCI/$NH_3$) m/e 544 $(M+H)^+$.

Example 123

(2S,3S,5S)-1,6-Diphenyl-5-[N-(furan-3- ylmethyloxycarbonyl)amino]-2-[N-(pyridin-3- yimethyloxycarbonyl)amino]-3-hydroxy hexane

The other regio-isomer described in Example 122, (2S,3R,5S)-1,6-diphenyl-2-[N-(pyridin-3-ylmethyloxycarbonyl)amino]-3-hydroxy hexane, (31.7 mg, 0.0756 mmol) was reacted with (furan-3-yl)(4-nitrophenyl)carbonate (24 mg, 0.091 mmol), prepared as described in Example 118, by the procedure described in Example 117B to give, after column chromatography on silica gel eluting with a gradient (2%,5%) methanol in methylene chloride, the title compound (22.7 mg). $^1$H NMR (DMSO-$d_6$, 300 MHz) δ 1.48 (m, 2H), 2.53-2.76 (m, 5H), 3.57 (m, 1H), 3.87 (m, 2H), 4.68 (d, 1H), 4.77 (s, 2H), 4.99 (s, 2H), 6.37 (s, 1H), 6.95 (d, 1H), 7.03 (d, 1H), 7.08-7.27 (m, 10H), 7.33 (dd, 1H), 7.60 (m, 3H), 8.50 (m, 2H). MS (DCI/$NH_3$) m/e 544 $(M+H)^+$.

Example 124

(2S,3S,5S)-1,6-Diphenyl-2,5-[N-(thiophene-3-ylmethyloxycarbonyl)amino]-3-hydroxy hexane

3-Thiophenemethanol (285 mg, 2.5 mmol) was reacted with 4-nitrophenylchloroformate (554 mg, 2.75 mmol) by the procedure described in Example 117A to give (thiophen-3-ylmethyl)(4-nitrophenyl)carbonate (598 mg). This compound (170 mg, 0.61 mmol) was reacted with the compound resulting from Example 1E (57.8 mg, 0.203 mmol) by the procedure described in Example 117B to give, after column chromatography on silica gel eluting with a gradient (5%,10%,20%) of ethyl acetate in methylene chloride, the title compound. $^1$H NMR (DMSO-$d_6$, 300 MHz) δ 1.50 (m, 2H), 2.54-2.78 (m, 5H), 3.58 (m, 1H), 3.90 (m, 3H), 4.67 (d, 1H), 4.85 (m, 1H), 4.91 (m, 4H), 6.84 (d, 1H), 6.95-7.33 (m, 16H), 7.49 (m, 2H). Anal calcd for $C_{30}H_{32}N_2O_5S_2$: C, 63.83; H, 5.67; N, 4.96. Found: C, 63.74; H, 5.76; N, 4.97. MS (DCI/$NH_3$) m/e 565 $(M+H)^+$, 582 $(M+H+NH_3)^+$.

Example 125

(2S,3S,5S)-1,6-Diphenyl-2,5-[N-(tihiophene-3-ylmethyloxycarbonyl)amino]-3-hydroxy hexane

This compound was prepared in analogy to Example 124 starting from 2-thiophenemethanol instead of 3-thiophenemethanol. The crude product was chromatographed on silica gel eluting with a gradient (5%,10%) ethyl acetate in methylene chloride to give the title compound. $^1$H NMR (DMSO-$d_6$, 300 MHz) δ 1.48 (m, 2H), 2.53-2.77 (m, 4H), 3.56 (m, 1H), 3.90 (m, 2H), 4.63 (d, 1H), 5.00-5.17 (m, 5H), 6.86 (d, 1H), 6.94-7.28 (m, 16H), 7.50 (m, 2H). Anal calcd for $C_{30}H_{32}N_2O_5S_2$: C, 63.83; H, 5.67; N, 4.96. Found: C, 63.80; H, 5.74; N, 4.89. MS (DCI/$NH_3$) m/e 565 $(M+H)^+$, 582 $(M+H+NH_3)^+$.

Example 126

(2S,3S,5S)-1,6-Diphenyl-2,5-[N-(2-methyl-pyridin-3-ylmethyloxycarbonyl)amino]-3-hydroxy hexane

Methyl 2-methylnicotinate (2.00 g, 12.1 mmol) was reduced to 2-methyl-3-pyridinemethanol using lithium aluminum hydride by the procedure described in Example 119A. This compound was converted to (2-methyl-pyridin-3-ylmethyl)(4-nitrophenyl)carbonate by the procedure also described in Example 119A. This carbonate (170 mg, 0.59 mmol) was reacted with the compound resulting from Example 1E (56 mg, 0.0197 mmol) by the procedure described in Example 117B to give, after column chromatography on silica gel eluting with a gradient (2%,5%) of methanol in methylene chloride, the title compound (63.6 mg). $^1$H NMR (DMSO-$d_6$, 300 MHz) δ 1.51 (m, 2H), 2.40 (s, 6H), 2.54-2.75 (m, 5H), 3.60 (m, 1H), 3.88 (m, 2H), 4.72 (d, 1H), 4.95 (m, 4H), 7.00 (d, 1H), 7.03-7.28 (m, 16H), 7.47 (m, 2H), 8.35 (m, 2H). Anal calcd for $C_{34}H_{38}N_4O_5$ • 0.5 $H_2O$: C, 69.04; H, 6.60; N, 9.48. Found: C, 68.98; H, 6.48; N, 9.39. MS (DCI/$NH_3$) m/e 583 $(M+H)^+$.

Example 127

(2S,3S,5S)-1,6-Diphenyl-2,5-[N-(tetrahydrofuran-3- ylmethyloxycarbonyl)amino]-3-hydroxy hexane

3-Tetrahydrofuranmethanol (265 mg, 2.59 mmol) was reacted with 4-nitrophenylchloroformate (575 mg, 2.85 mmol) by the procedure described in Example 117A to give (tetrahydrofuran-3-ylmethyl)(4-nitrophenyl)carbonate (585 mg). This compound (155 mg, 0.581 mmol) was reacted with the compound resulting from Example 1E (55 mg, 0.194 mmol) to give, after column chromatography on silica gel eluting with a gradient (2%,5%) of methanol in methylene chloride, the tite compound (68.8 mg). $^1H$ NMR (DMSO-$d_6$, 300 MHz) $\delta$ 1.48 (m, 2H), 1.85 (m, 2H), 2.35 (m, 2H), 2.54-2.77 (m, 5H), 3.52-3.88 (m, 16H), 4.64 (m, 2H), 6.70 (d, 1H), 6.98 (d, 1H), 7.10-7.28 (m, 10H). Anal calcd for $C_{30}H_{40}N_2O_7 \cdot H_2O$: C, 64.52; H, 7.53; N, 5.02. Found: C, 64.87; H, 7.22; N, 5.00. MS (DCI/$NH_3$) m/e 541 $(M+H)^+$, 558 $(M+H+NH_3)^+$.

Example 128

(6-Methylpyridin-2-yl)methoxycarbonyl-Valine

To a solution of the isocyanate derived from Valine methyl ester (17.9 mmol) dissolved in toluene was added 6-methyl-pyridine-2-methanol (2.42 g, 1.1 equivalent). The solution was heated at reflux for 2 hours. After concentration *in vacuo* and purifiction by silica gel column chromatography, (6-methylpyridin-2-yl)methoxycarbonyl-Valine methyl ester (2.8 g) was obtained. Hydrolysis of the methyl ester using aqueous lithium hydroxide provide the title compound upon recrystallization from hot ethyl acetate.

Example 129

(2S,3S,5S)-2-(N-[(6-Methylpyridin-2-yl)methoxycarbonyl-Valyllamino)5-(N-[(pyridin-3-yl)methoxycarbonyl]amino)-1,6-diphenyl-3-hydroxyhexane

Coupling of the resultant compound from Example 128 with (2S,3S,5S)-2-amino-5-(N-[(pyridin-3-yl)methoxycarbonyl]amino)-1,6-diphenyl-3-hydroxy hexane using standard EDAC/HOBT methodology afforded the title compound in 79% yield. m.p. 175-176 °C. $^1H$ NMR (DMSO-$d_6$, 300 MHz) $\delta$ 0.73 (d, 3H), 0.78 (d, 3H), 1.50 (m, 2H), 1.90 (m, 1H), 2.45 (s, 3H), 2.60-2.70 (m, 4H), 3.55 (m, 1H), 3.85 (m, 2H), 4.95 (ABq, 2H), 5.05 (s, 2H), 7.10-7.20 (m, 12H), 7.35 (m, 2H), 7.49 (d, 1H), 7.55 (d, 1H), 7.70 (t, 1H), 8.50 (m, 2H). MS (DCI/$NH_3$) m/e 668 $(M+H)^+$.

Example 130

(2S,3S,5S)-2-(N-[(pyridin-3-yl)methoxycarbonyl]amino)-5-(N-[(6-methylpyridin-2-yl)methoxycarbonyl-valyllamino)-1,6-diphenyl-3-hydroxyhexane

Coupling of the resultant compounds from Example 128 with (2S,3S,5S)-2-(N-[(pyridin-3-yl-methoxycarbonyl]amino)-5-amino-1,6-diphenyl-3-hydroxyhexane using standard EDAC/HOBt methodology afforded the title compound in 70% yield. $^1H$ NMR (DMSO-$d_6$; 300 MHz): $\delta$ 0.75 (d, 3H), 0.78 (d, 3H), 2.45 (s, 3H), 4.60 (d, 1H), 4.95 (m, 2H), 5.05 (s, 2H), 7.05-7.70 (m, 15H), 8.50 (m, 2H). Mass spectrum: $(M+H)^+$ = 668.

Example 131

[(6-Methylpyridin-3-yl)methyl](4-nitrophenyl)carbonate

To a solution of 4-nitrophenylchloroformate (2.00 g) dissolved in methylene chloride (20 mL) and cooled to 0°C was added (6-methylpyridin-3-yl)methanol (1 equivalent) and triethylamine (1 equivalent). The solution was allowed to warm to room temperature and stirred for 0.5 hours, diluted with methylene chloride, washed with saturated sodium bicarbonate solution, dired over sodium sulfate and concentrated *in vacuo*. The residue obtained was column chromatographed on silica gel to afford the title compound (80%).

Example 132

(6-Methylpyridin-3-yl)methoxycarbonyl-Valine

To a solution of the resultant compound from Example 131 (2.00 g) dissolved in dimethylformamide (20 mL) was added Valine methyl ester hydrochloride (1 equivalent) and triethylamine (2 equivalents). After stirring at room temperature for 1 hour, the solvent was removed *in vacuo*. The residue obtained was chromatographed on silica gel eluting with 5% methanol in methylene chloride to afford the title compound methyl ester. Hydrolysis with lithium hydroxide in aqueous dioxane afforded the title compound.

Example 133

(2S,3S,5S)-2-(N-[(6-Methylpyridin-3-yl)methoxycarbonyl-valyllamino)-5-(N[(pyridin-3-yl)methoxycarbonyl]amino- 1,6-diphenyl-3-hydroxyhexane

Coupling of the resultant compound from Example 132 with (2S,3S,5S)-2-amino-5-(N-[(pyridin-3-yl)methoxycarbonyl]amino-1,6-diphenyl-3-hydroxyhexane using standard EDAC/HOBt methodology afforded the title compound. $^1$H NMT (DMSO-$d_6$; 300 MHz): δ 0.70 (d, 3H), 0.75 (d, 3H), 2.45 (s, 3H), 4.90 ( m, 3H), 5.03 (s, 2H) 7.10-7.65 (m, 16H), 8.45 (m, 2H). Mass spectrum: $(M+H)^+$ = 668.

Example 134

(2S,3S,5S)-2-(N-[(Pyridin-3-yl)methoxycarbonyl]amino-5-(N-[(6-methyl-pyridin-3-yl)methoxycarbonyl-Valyl]amino)-1,6-diphenyl-3-hydroxyhexane

Coupling of the resultant compound from Example 132 with (2S,3S,5S)-2-(N-[(pyridin-3-yl)methxoycarbonyl]amino-5-amino-1,6-diphenyl-3-hydroxyhexane using standard EDAC/HOBt methodology afforded the title compound. $^1$H NMR (DMSO-$d_6$; 300 MHz): δ 0.73 (d, 3H), 0.75 (d, 3H), 1.80 (m, 1H), 2.45 (s, 3H), 4.67 (d, 1H), 4.96 (m, 2H), 5.05 (s, 2H), 6.90 (br d, 1H), 7.05-7.70 (m, 16H), 8.47 (m, 3H). Mass spectrum: $(M+H)^+$ = 668.

Example 135

(2S,3S-5S)-2-(N-(N-((N-Methyl-N-((6-methyl-2-pyridinyl)methyl)amino)carhonyl)valinyl)amino)-5-(N-((3-pyridinyl)-methoxycarbonyl)amino)-1,6-diphenyl-3-hydroxyhexane

Coupling of (N-((N-methyl-N-((6-methyl-2-pyridinyl)methyl)amino)carbonyl)valine with (2S,3S,5S)-2-amino-5-(N-((3-pyridinyl)methoxycarbonyl)amino)-1,6-diphenyl-3-hydroxyhexane using standard EDAC/HOBt methodology provided the desired compound in 91% yield. $^1$H NMR (DMSO-$d_6$; 300 MHz) δ 0.72 (d, 3H), 0.77 (d, 3H), 1.92 (m, 1H), 2.43 (s, 3H), 2.88 (s, 3H), 4.43 (s, 2H), 4.92 (m, 2H), 6.37 (br d, 1H), 7.0-7.65 (m, 15H), 8.50 (m, 2H). Mass spectrum: $(M+H)^+$ = 681.

Example 136

(2S,3S,5S)-2-(N-((3-Pyridinyl)-methoxycarbonyl)amino-5-(N-(N-((N-Methyl-N-((6-methyl-2-pyridinyl)methyl)-amino)carbonyl)valinyl)amino)-1,6-diphenyl-3-hydroxyhexane

Coupling of (N-((N-methyl-N-((6-methyl-2-pyridinyl)methyl)amino)carbonyl)valine with (2S,3S,5S)-2-(N-((3-pyridinyl)methoxy-carbonyl)amino)-5-amino-1,6-diphenyl-3-hydroxyhexane using standard EDAC/HOBt method provided the desired compound in 88% yield. $^1$H NMR (DMSO-$d_6$; 300 MHz); δ 0.74 (d, 6H), 2.45 (s, 3H), 2.88 (s, 3H), 4.42 (br s, 2H), 4.60 (d, 1H), 4.95 (m, 2H), 6.27 (br d, 1H), 6.87 (br d, 1H), 7.10-7.70 (m, 15H), 8.50 (m, 2H). Mass spectrum: $(M+H)^+$ = 681.

Example 137

(2S,3S,5S)-2-(N-((5-Methyl-3-pyridinyl)-methoxycarbonyl)valinyl)amino)-5-(N-(3-pyridinyl)methoxycarbonyl)amino)-1,6-diphenyl-3-hydroxyhexane

Coupling of (5-methyl-3-pyridinyl)methoxycarbonyl)valine with (2S,3S,5S)-2-amino-5-(N((3-pyridinyl)methoxycarbonyl)amino)-1,6-diphenyl-3-hydroxyhexane using standard EDAC/HOBt method provided the desired compound in 82% yield. $^1$H NMR (DMSO-$d_6$; 300 MHz): δ 0.72 (d, 3H), 0.75 (d, 3H), 1.50 (m, 2H), 1.90 (m, 1H), 2.28 (s, 3H), 4.88 (br d, 1H), 4.92 (m, 2H), 5.04 (s, 2H), 7.10-7.58 (m, 15H), 8.35 (m, 2H), 8.45 (m, 2H). Mass spectrum: $(M+H)^+$ = 668.

Example 138

(2S,3S,5S)-2-(N((3-Pyridinyl)methoxycarbonyl)amino)-5-(N((5-methyl-3-pyridinyl)methoxycarbonyl)valinyl)-amino)-1,6-diphenyl-3-hydroxyhexane

Coupling of (5-methyl-3-pyridinyl)methoxycarbonyl-valine with (2S,3S,5S)-2-(N-((3-pyridinyl)methoxycarbonyl)amino)-5-amino-1,6-diphenyl-3-hydroxyhexane using standard EDAC/HOBt method provided the desired compound in 80% yield. $^1$H NMR (DMSO-$d_6$; 300 MHz): δ 0.73 (d, 3H), 0.77 (d, 3H), 1.45 (m, 2H), 1.80 (m, 1H), 2.29 (s, 3H), 4.63 (br d, 1H), 4.95 (m, 2H), 5.05 (s, 2H), 6.90 (br d, 1H), 7.10-7.60 (m, 15H), 7.70 (br d, 1H), 8.37 (m, 2H), 8.50 (m, 2H). Mass spectrum: $(M+H)^+$ = 668.

Example 139

(2S,3S,5S)-2-(N-(N-((N-Methyl-N((6-methyl-3-pyridinyl)methyl)amino)carbonyl)valinyl)amino)-5-(N-((3-pyridinyl)-methoxycarbonyl)amino)-1,6-diphenyl-3-hydroxyhexane

Coupling of (N-((N-Methyl-N-((6-methyl-3-pyridinyl)methyl)amino)carbonyl)valine with (2S,3S,5S)-2-amino-5-(N-((3-pyridinyl)methoxycarbonyl)amino)-1,6- diphenyl-3-hydroxyhexane using standard EDAC/HOBt methodology provided the desired compound in 85% yield. $^1$H NMR (DMSO-$d_6$; 300 MHz): δ 0.70 (d, 3H), 0.76 (d, 3H), 1.50 (m, 2H), 1.90 (m, 1H), 2.40 (s, 3H), 2.79 (s, 3H), 4.40 (m, 2H), 4.85 (br d, 1H), 4.92 (m, 2H), 6.0 (br d, 1H), 7.10-7.55 (m, 15H), 8.30 (d, 1H), 8.50 (m, 2H). Mass spectrum: $(M+H)^+$ = 681.

Example 140

(2S,3S,5S)-2-(N((3-Pyridinyl)methoxycarbonyl)amino)-5-(N-(N-((N-methyl-N-((6-methyl-3-pyridinyl)methyl)amino) carbonyl)valinyl)amino)-1,6-diphenyl-3-hydroxyhexane

Coupling of (N-((N-Methyl-N-((6-methyl-3-pyridinyl)methyl)amino)carbonyl)valine with (2S,3S,5S)-2-(N((3-pyridinyl)methoxy-carbonyl)amino)-5-amino-1,6-diphenyl-3-hydroxyhexane using standard EDAC/HOBt method provided the desired compound in 83% yield. $^1$H NMR (DMSO-$d_6$; 300 MHz): δ 0.72 (d, 3H), 0.75 (d, 3H), 1.48 (m, 2H), 1.88 (m, 1H), 2.40 (s, 3H), 2.78 (s, 3H), 4.42 (s, 2H), 4.70 (d, 1H), 4.96 (m, 2H), 5.85 (d, 1H), 6.90 (d, 1H), 7.10-7.58 (m, 16H), 7.70 (d, 1H), 8.30 (d, 1H), 8.50 (m, 2H). Mass spectrum: $(M+H)^+$ = 681.

Example 141

(2S,3S,5S)-2-(N-(N-((N-(6-methyl-2-pyridinyl)methyl)amino) carbonyl)valinyl)amino-5-(N-((3-pyridinyl)- methoxycarbonyl)amino)-1,6-diphenyl-3-hydroxyhexane

Coupling of (N-((6-methyl-2-pyridinyl)methyl)-amino)carbonyl)valine with (2S,3S,5S)-2-amino-5-(N-((3-pyridinyl)methoxycarbonyl)amino)-1,6-diphenyl-3- hydroxyhexane using standard EDAC/HOBt method provided the desired compound. $^1$H NMR (DMSO-$d_6$; 300 MHz): δ 0.74 (d, 3H), 0.80 (d, 3H, 1.50 (m, 2H), 1.90 (m, 1H), 2.45 (s, 3H), 4.25 (m, 2H), 4.83 (d, 1H), 4.92 (m, 2H), 6.20 (d, 1H), 6.65 (t, 1H), 7.05-7.60 (m, 15H), 8.50 (m, 2H). Mass spectrum: $(M+H)^+$ = 667.

Example 142

(2S,3S,5S)-2-(N-((3-Pyridinyl)-methoxycarbonyl)amino)-5-((N-((N-6-methyl-2-pyridinyl)methyl)amino)carbonyl)-valinyl)amino)-1,6-diphenyl-3-hydroxyhexane

Coupling of N-((N-((6-methyl-2-pyridinyl)methyl)amino)carbonyl)valine with (2S,3S,5S)-2-(N-((3-pyridinyl)methoxy-carbonyl)amino)-5-amino-1,6-diphenyl-3-hydroxyhexane provided the desired compound in 80% yield. $^1$H NMR (DMSO-$d_6$; 300 MHz): δ 0.72 (d, 3H), 0.78 (d, 3H), 1.45 (m, 2H), 1.80 (m, 1H), 2.44 (s, 3H), 4.25 (d, 2H), 4.63 (d, 1H), 4.95 (m, 2H), 6.15 (d, 1H), 6.65 (t, 1H), 6.88 (d, 1H), 7.05-7.60 (m, 15H), 7.27 (d, 1H), 8.50 (m, 2H). Mass spectrum: $(M+H)^+$ = 667.

Example 143

(2S,3S,5S)-2-(N-((5-Methyl-2- pyrazinyl)methoxycarbonyl)valinyl) amino)5-(N-(3-pyridinyl)methoxycarbonyl)amino)-1,6-diphenyl-3-hydroxyhexane.

Coupling of ((5-methyl-2-pyrazinyl)methoxycarbonyl)valine with (2S,3S,5S)-2-amino-5(N((3-pyridinyl)methoxycarbonyl)amino)-1,6-diphenyl-3-hydroxyhexane using standard EDAC/HOBt method provided the desired compound in 80% yield. $^1H$ NMR (DMSO-$d_6$; 300 MHz): 0.72 (d, 3H), 0.77 (d, 3H), 1.50 (m, 2H), 1.85 (m, 1H), 2.48 (s, 3H), 2.60-270 (m, 4H), 3.80 (m, 2H), 4.10 (m, 1H), 4.88 (br d, 1H), 4.92 (m, 2H), 5.10 (s, 2H), 7.10-7.30 (m, 14H), 7.45 (br d, 1H), 7.50 (br d, 1H), 8.50 (m, 4H). Mass spectrum: $(M+H)^+$ = 669.

Example 144

(2S,3S,5S)-2-(N((3-Pyridinyl)methoxycarbonyl)amino)-5-(N((5-methyl-2-pyrazinyl)methoxycarbonyl)valinyl)amino)-1,6- diphenyl-3-hydroxyhexane.

Coupling of ((5-methyl-2-pyrazinyl)methoxycarbonyl)valine with (2S,3S,5S)-2-(N-((3-pyridinyl)methoxycarbonyl)amino)-5-amino-1,6-diphenyl-3-hydroxyhexane using standard EDAC/HOBt method provided the desired compound in 80% yield. $^1H$ NMR (DMSO-$d_6$; 300 MHz): 0.70 (d, 3H), 0.73 (d, 3H), 1.40 (m, 2H), 1.80 (m, 1H), 2.45 (s, 3H), 2.60 (m, 4H), 3.70 (m, 2H), 4.05 (m, 1H), 4.60 (d, 1H), 4.90 (m, 2H), 5.08 (s, 2H), 6.85 (br d, H), 7.05-7.30 (m, 12H), 7.50 (br d, 1H), 7.70 (br d, 1H), 8.45 (m, 4H). Mass spectrum: $(M+H)^+$ = 669.

Example 145

(2S,3S,5S)-2-(N-((6-Methoxy-3- pyridinyl)methoxycarbonyl)valinyl) amino)5-(N-(3-pyridinyl)methoxycarbonyl)amino)-1,6-diphenyl- 3-hydroxyhexane.

Coupling of ((6-methoxy-3-pyridinyl)methoxycarbonyl)valine with (2S,3S,5S)-2-amino-5(N((3-pyridinyl)methoxycarbonyl)amino)-1,6-diphenyl-3-hydroxyhexane using standard EDAC/HOBt method provided the desired compound in 90% yield. $^1H$ NMR (DMSO-$d_6$; 300 MHz): 0.70 (d, 3H), 0.73 (d, 3H), 1.50 (m, 2H), 1.85 (m, 1H), 2.65 (m, 4H), 3.82 (s, 3H), 4.90-4.96 (m, 5H), 6.80 (m, 1H), 7.10-7.20 (m, 12H), 7.30 (m, 1H), 7.40 (m, 1H), 7.50 (m, 1H), 7.65 (m, 1H), 8.18 (m, 1H), 8.46 (m, 2H). Mass spectrum: $(M+H)^+$ = 684.

Example 146

(2S,3S,5S)-2-(N((3-pyridinyl)methoxycarbonyl)amino)-5-(N((6-methoxy-3-pyridinyl)methoxycarbonyl)valinyl)amino)-1,6-diphenyl-3-hydroxyhexane.

Coupling of ((5-methoxy-3-pyridinyl)methoxycarbonyl)valine with (2S,3S,5S)-2-(N-((3-pyridinyl)methoxycarbonyl)amino)-5-amino-1,6-diphenyl-3-hydroxyhexane using standard EDAC/HOBt method provided the desired compound in 79% yield. $^1H$ NMR (DMSO-$d_6$; 300 MHz): δ 0.73 (m, 6H), 1.45 (m, 2H), 1.80 (m, 1H), 2.60-2.70 (m, 4H), 3.83 (s, 3H), 4.10 (m, 1H), 4.62 (m, 1H), 4.97 (m, 4H), 6.80 (br d, 1H), 6.90 (br d, 1H), 7.00-7.35 (m, 12H), 7.57 (br d, 1H), 7.70 (m, 2H), 8.18 (m, 1H), 8.50 (m, 2H). Mass spectrum: $(M+H)^+$ = 684.

Example 147

(2S,3S,5S)-2-(N-(N-((N-Methyl-N-((6-methoxy-3- pyridinyl)methyl) amino)carbonyl)valinyl)amino)-5-(N-((3- pyridinyl)methoxycarbonyl) amino)-1,6-diphenyl-3-hydroxyhexane.

Coupling of (N-((N-methyl-N-((6-methoxy-3-pyridinyl)methyl)amino)carbonyl)valine with (2S,3S,5S)-2-amino-5-(N-((3-pyridinyl)methoxycarbonyl)amino)-1,6-diphenyl-3-hydroxyhexane using standard EDAC/HOBt method provided the desired compound in 83% yield. $^1H$ NMR (DMSO-$d_6$; 300 MHz): δ 0.71 (d, 3H), 0.77 (d, 3H), 1.5 (m, 2H), 1.95 (m, 1H), 2.65-2.73 (m, 4H), 2.77 (s, 3H), 3.80 (s, 3H), 3.93 (m, 1H), 4.15 (m, 1H), 4.38 (m, 2H), 4.86 (br d, 1H), 4.93 (m, 2H), 6.00 (br d, 1H), 6.77 (d, 1H), 7.05-7.20 (m, 10H), 7.36 (m, 1H), 7.48 (br d, 1H), 7.55 (m, 1H), 8.03 (d, 1H), 8.50 (m, 2H). Mass spectrum: $(M+H)^+$ = 697.

Example 148

(2S,3S,5S)-2-(N-((3-Pyridinyl)methoxycarbonyl)amino-5-(N-(N-((N-methyl-N-((6-methoxy-3- pyridinyl)methyl)amino)carbonyl)valinyl) amino)-1,6-diphenyl-3-hydroxyhexane.

Coupling of (N-((N-methyl-N-((6-methoxy-3-pyridinyl)methyl)amino)carbonyl)valine with (2S,3S,5S)-2-(N-((3-pyridinyl)methoxycarbonyl)amino)-5-amino-1,6-diphenyl-3-hydroxyhexane using standard EDAC/HOBt method provided the desired compound in 90% yield. $^1$H NMR (DMSO-$d_6$; 300 MHz): δ 0.73 (d, 3H), 0.76 (d, 3H), 1.48 (m, 2H), 1.90 (m, 1H), 2.60-2.70 (m, 4H), 2.77 (s, 3H), 3.60 (m, 1H), 3.81 (s, 3H), 3.90 (m, 1H), 4.13 (m, 1H), 4.38 (m, 2H), 4.62 (d, 1H), 4.95 (m, 2H), 5.80 (br d, 1H), 6.75 (d, 1H), 6.88 (br d, 1H), 7.07-7.21 (m, 10H), 7.32 (m, 1H), 7.55 (m, 2H), 7.70 (br d, 1H), 8.03 (d, 1H), 8.50 (m, 2H). Mass spectrum: $(M+H)^+$ = 697.

Example 149

(2S,3S,5S)-2-(N-(N-((N-Methyl-N-((5-methyl-3- pyridinyl)methyl) amino)carbonyl)valinyl)amino)-5-(N-((3- pyridinyl)methoxycarbonyl) amino)-1,6-diphenyl-3-hydroxyhexane.

Coupling of (N-((N-methyl-N-((5-methyl-3-pyridinyl)methyl)amino)carbonyl)valine with (2S,3S,5S)-2-amino-5-(N-((3-pyridinyl)methoxycarbonyl)amino)-1,6-diphenyl-3-hydroxyhexane using standard EDAC/HOBt method provided the desired compound in 82% yield. $^1$H NMR (DMSO-$d_6$; 300 MHz): 0.72 (d, 3H), 0.77 (d, 3H), 1.50 (m, 2H), 1.90 (m, 1H), 2.25 (s, 3H), 2.60-276 (m, 4H), 2.80 (s, 3H), 3.55 (m, 1H), 3.85-4.10 (m, 3H), 4.45 (m, 2H), 4.86 (d, 1H), 4.93 (m, 2H), 6.02 (d, 1H), 7.10-7.20 (m, 11H), 7.35 (m, 1H), 7.42 (m, 1H), 7.50 (m, 2H), 8.27 (m, 2H), 8.50 (m, 2H). Mass spectrum: $(M+H)^+$ = 681.

Example 150

(2S,3S,5S)-2-(N-((3-pyridinyl)methoxycarbonyl)amino-5-(N-(N- ((N-methyl-N-((5-methyl-3- pyridinyl)methyl)amino)carbonyl)valinyl) amino)-1,6-diphenyl-3-hydroxyhexane.

Coupling of (N-((N-methyl-N-((5-methyl-3-pyridinyl)methyl)amino)carbonyl)valine with (2S,3S,5S)-2-(N-((3-pyridinyl)methoxycarbonyl)amino)-5-amino-1,6-diphenyl-3-hydroxyhexane using standard EDAC/HOBt method provided the desired compound in 80% yield. $^1$H NMR (DMSO-$d_6$; 300 MHz): δ 0.72 (d, 3H), 0.75 (d, 3H), 1.45 (m, 2H), 1.88 (m, 1H), 2.24 (s, 3H), 2.55-2.70 (m, 4H), 2.80 (s, 3H), 3.57 (m, 1H), 3.80 (m, 1H), 3.90 (m, 1H), 4.10 (m, 1H), 4.45 (s, 2H), 4.68 (d, 1H), 4.96 (m, 2H), 5.96 (br d, 1H), 6.92 (br d, 1H), 7.10-7.20 (m, 11H), 7.30 (m, 1H), 7.42 (m, 1H), 7.55 (m, 1H), 7.70 (br d, 1H), 8.28 (m, 2H), 8.50 (m, 2H). Mass spectrum: $(M+H)^+$ = 681.

Example 151

(2S,5S)-Bis-(N-((6-Methoxy-3- pyridinyl)methoxycarbonyl)valinyl) amino)-1,6-diphenyl-3(S)-hydroxyhexane.

Coupling of ((6-methoxy-3-pyridinyl)methoxycarbonyl)valine with (2S,3S,5S)-2,5-diamino-1,6-diphenyl-3-hydroxyhexane using standard EDAC/HOBt method provided the desired product in 60% yield. $^1$H NMR (DMSO-$d_6$; 300 MHz): δ 0.70 (d, 6H), 1.45 (m, 2H), 1.80 (m, 2H), 2.60-2.70 (m, 4H), 3.82 (s, 6H), 4.05 (m, 2H), 4.90 (d, 2H), 4.98 (4H), 6.82 (d, 2H), 7.00-7.20 (m, 14H), 7.45 (br d, 1H), 7.70 (m, 3H), 8.20 (m, 2H). Mass spectrum: $(M+H)^+$ = 813.

Example 152

(2S,3S,5S)-2-(N-((2-pyrazinyl)methoxycarbonyl)valinyl)amino)-5-(N-(3-pyridinyl)methoxycarbonyl)amino)-1,6-diphenyl-3-hydroxyhexane.

Coupling of N-((2-piprazinyl)methoxycarbonyl)valine with (2S,3S,5S)-2-amino-5-(N-(3-pyridinyl)methoxycarbonyl)amino)-1,6-diphenyl-3-hydroxyhexane provided the desired compound in 95% yield. $^1$H NMR (DMSO-$d_6$; 300 MHz): δ0.73 (d, 3H), 0.80 (d, 3H), 1.50 (m, 2H), 1.90 (m, 1H), 2.60-2.70 (m, 4H), 3.56 (m, 1H), 3.80 (m, 2H), 4.10 (m, 1H), 4.90 (m, 3H), 5.15 (s, 2H), 7.10-7.20 (m, 12H), 7.35 (m, 2H), 7.50 (m, 2H), 8.50-8.70 (m, 5H). Mass spectrum: $(M+H)^+$=655.

Example 153

(2S,3S,5S)-2-(N-((3-pyridinyl)methoxycarbonyl)amino)-5-(N((2- pyrazinyl)methoxycarbonyl)valinyl)amino)-1,6-diphenyl-3-hydroxyhexane.

Coupling of N-((2-pyrazinyl)methoxycarbonyl)valine with (2S,3S,5S)-2-(N-((3-pyridinyl)methoxycarbonyl)amino-5-amino-1,6-diphenyl-3-hydroxyhexane using standard EDAC/HOBt method provided the desired compound in 60% yield. $^1$H NMR (DMSO-$d_6$; 300 MHz): δ0.77 (t, 6H), 1.46 (m, 2H), 1.85 (m, 1H), 2.60-2.70 (m, 4H), 3.58 (m, 1H), 3.77 (m, 2H), 4.12 (m, 1H), 4.64 (d, 1H), 4.96 (m, 2H), 5.17 (s, 2H), 6.90 (br d, 1H), 7.10-7.30 (m, 14H), 7.55 (m, 1H), 7.70 (br d, 1H), 8.50 (m, 2H), 8.60 (m, 2H), 8.68 (s, 1H). Mass spectrum: $(M+H)^+$=655.

Example 154

A. (2S,3S,5S)-5-Amino-2-(N-((5-pyrimidinyl)methoxy-carbonyl)amino)-1,6-diphenyl-3-hydroxyhexane and (2S,3S,5S)-2-Amino-5-(N-(5-pyrimidinyl)methoxycarbonyl)amino)-1,6- diphenyl-3-hydroxyhexane.

Using the procedure of Example 28B but replacing the resultant compound of Example 28A with the resultant compound of Example 91A provided 68.1 mg (13%) of (2S,3S,5S)-5-amino-2-(N-((5-pyrimidinyl)methoxycarbonyl)-amino)-1,6-diphenyl-3-hydroxyhexane and 148.1 mg (28%) of (2S,3S,5S)-2-amino-5-(N-((5-pyrimidinyl)methoxycarbonyl)-amino)-1,6-diphenyl-3-hydroxyhexane.

B. (2S,3S,5S)-2-(N-(N-((N-Methyl-N-((2-pyridinyl)-methyl)amino)carbonyl)valinyl)amino)-5-(N-((5-pyrimidinyl)-methoxycarbonyl)amino)-1,6-diphenyl-3-hydroxyhexane.

A mixture of 50 mg (0.119 mmol) of (2S,3S,5S)-2-amino-5-(N-((5-pyrimidinyl)methoxycarbonyl)amino)-1,6-diphenyl-3-hydroxyhexane and 68.9 mg (0.178 mmol) of the resultant compound of Example 3F in 1 ml of tetrahydrofuran was stirred at ambient temperature for 16 h. The solvent was then removed in vacuo, and the residue was purified by silica gel chromatography using 5% methanol in dichioromethane provided 63.5 mg (80%) of the desired compound as a white solid. $^1$H NMR ($CDCl_3$) δ 0.78 (d, 3H), 0.92 (d, 3H), 1.65 (m, 2H), 2.26 (m, 1H), 2.74 (m, 2H), 2.83 (m, 2H), 2.97 (s, 3H), 3.63 (m, 1H), 3.95 (m, 1H), 4,05 (m, 2H), 4.45 (s, 2H), 5.02 (dd, 2H), 5.33 (br d, 1H), 6.48 (br d, 1H), 6.56 (br, 1H), 7.07-7.24 (m, 12H), 7.72 (td, 1H), 8.51 (d, 1H), 8.67 (s, 2H), 9.18 (s, 1H). Mass spectrum: $(M+H)^+$ = 668.

Example 155

(2S,3S,5S)-2-(N-(N-((2-Pyridinyl)methoxycarbonyl)- valinyl)amino)-5-(N-((5-pyrimidinyl)methoxycarbonyl)amino)- 1,6-diphenyl-3-hydroxyhexane.

Using the procedure of Example 154B but replacing the resultant compound of Example 3F with the resultant compound of Example 2D provided 48.9 mg (63%) of the desired compound as a white solid. $^1$H NMR ($CDCl_3$) δ 0.76 (d, 3H), 0.89 (d, 3H), 1.64 (m, 2H), 2.13 (m, 1H), 2.75 (d, 2H), 2.85 (d, 2H), 3.68 (m, 1H), 3.93 (m, 2H), 4.08 (m, 1H), 4.96-5.33 (m, 6H), 6.34 (br, 1H), 7.04-7.22 (m, 11H), 7.33 (d, 1H), 7.70 (td, 1H), 8.57 (d, 1H), 8.68 (s, 2H), 9.18 (s, 1H). Mass spectrum: $(M+H)^+$ = 655.

Example 156

(2S,3S,5S)-5-(N-(N-((N-Methyl-N-((2-pyridinyl)- methyl)amino)carbonyl)valinyl)amino)-2-(N-((5-pyrimidinyl)- methoxycarbonyl)amino)-1,6-diphenyl-3-hydroxyhexane.

Using the procedure of Example 154B but replacing (2S,3S,5S)-2-amino-5-(N-((5-pyrimidinyl)methoxycarbonyl)-amino)-1,6-diphenyl-3-hydroxyhexane with (2S,3S,5S)-5-amino-2-(N-(((5-pyrimidinyl)methoxycarbonyl)-amino)-1,6-diphenyl-3-hydroxyhexane provided 60.4 mg (76%) of the desired compound as a white solid. $^1$H NMR ($CDCl_3$) δ 0.77 (d, 3H), 0.90 (d, 3H), 1.62 (m, 2H), 2.31 (m, 1H), 2.73 (m, 2H), 2.84 (m, 2H), 2.99 (s, 3H), 3.66 (m, 1H), 3.74 (m, 1H), 4.04 (m, 1H), 4.22 (m, 1H), 4.43 (dd, 2H), 5.03 (dd, 2H), 5.24 (br d, 1H), 6.52 (br d, 1H), 6.66 (br, 1H), 7.08-7.28 (m, 12H), 7.74 (td, 1H), 8.49 (dd, 1H), 8.67 (s, 2H), 9.18 (s, 1H). Mass spectrum: $(M+H)^+$ = 668.

Example 157

(2S,3S,5S)-5-(N-(N-((2-Pyridinyl)methoxycarbonyl)valinyl)-amino)-2-(N-((5-pyrimidinyl)methoxycarbonyl)amino)-1,6-diphenyl-3-hydroxyhexane.

Using the procedure of Example 154B but replacing (2S,3S,5S)-2-amino-5-(N-((5-pyrimidinyl)methoxycarbonyl)-amino)-1,6-diphenyl-3-hydroxyhexane with (2S,3S,5S)-5-amino-2-(N-((5-pyrimidinyl)methoxycarbonyl)amino)-1,6-diphenyl-3-hydroxyhexane and replacing the resultant compound of Example 3F with the resultant compound of Example 2D provided 14.6 mg (78%) of the desired compound as a white foamy solid. $^1H$ NMR ($CDCl_3$) δ 0.71 (d, 3H), 0.86 (d, 3H), 1.67 (m, 2H), 2.07 (m, 1H), 2.30 (m, 4H), 3.68 (m, 1H), 3.77 (m, 1H), 3.90 (m, 1H), 4.26 (m, 1H), 5.03 (dd, 2H), 5.19 (dd, 2H), 5.22 (br, 1H), 6.45 (br, 1H), 7.09-7.23 (m, 12H), 7.34 (d, 1H), 7.73 (td, 1H), 8.57 (dd, 1H), 8.67 (s, 2H), 9.18 (s, 1H). Mass spectrum: $(M+H)^+$ = 655.

Example 158

A. (2S,3S,5S)-5-Amino-2-(N-((3-furyl)methoxy-carbonyl)amino)-1,6-diphenyl-3-hydroxyhexane and (2S,3S,5S)-2-Amino-5-(N-((3-furyl)methoxycarbonyl)amino)-1,6-diphenyl-3-hydroxyhexane.

Using the procedure of Example 28B but replacing the resultant compound of Example 28A with 3-furylmethyl 4-nitrophenyl carbonate from Example 118 provided 69.0 mg (17%) of (2S,3S,5S)-5-amino-2-(N-((3-furyl)methoxycarbonyl)amino)-1,6-diphenyl-3-hydroxyhexane and 156.4 mg (36%) of (2S,3S,5S)-2-amino-5-(N-((3-furyl)methoxycarbonyl)amino)-1,6-diphenyl-3-hydroxyhexane.

B. (2S,3S,5S)-2-(N-(N-(2-(6-Methylpyridinyl)-methoxycarbonyl)valinyl)amino)-5-(N-((3-furyl)methoxy-carbonyl)amino)-1,6-diphenyl-3-hydroxyhexane.

Using the procedure of Example 5I but replacing the resultant compound of Exainple 5H with (2S,3S,5S)-2-amino-5-(N-((3-furyl)methoxycarbonyl)amino)-1,6-diphenyl-3-hydroxyhexane and replacing trans-3-(pyridinyl)acrylic acid with the resultant compound of Example 128 provided 92.9 mg (96%) of the desired compound as a white solid. $^1H$ NMR ($CDCl_3$) δ 0.73 (d, 3H), 0.87 (d, 3H), 1.64 (m, 2H), 2.13 (m, 1H), 2.50 (s, 3H), 2.76 (dd, 2H), 2.84 (d, 2H), 3.67 (m, 1H), 3.75 (m, 1H), 3.88 (m, 1H), 4.17 (m, 1H), 4.90 (s, 2H), 5.09 (br d, 1H), 5.15-5.22 (m, 3H), 6.31 (br d, 1H), 6.37 (s, 1H), 7.07-7.25 (m, 12H), 7.39 (m, 2H), 7.60 (t, 1H). Mass spectrum: $(M+H)^+$ = 657.

Example 159

A. Methyl 3-(Methoxymethoxy)-5-isoxazole Carboxylate.

A solution of 2.0 g (14.0 mmol) of methyl 3-hydroxy-5-isoxazole carboxylate and 29.2 ml (16.8 mmol) of N,N-diisopropylethylamine in 20 ml of tetrahydrofuran was treated with 1.27 ml of chloromethyl methyl ether. After being stirred at ambient temperature for 2 h, the solution was diluted with dichloromethane, washed with water, dried over $MgSO_4$, and concentrated in vacuo. Silica gel chromatography of the residue using 10% methanol in dichloromethane provided 129.6 mg (78%) of the desired compound as a white solid. $^1H$ NMR ($CDCl_3$) δ 3.57 (s, 3H), 3.96 (s, 3H), 5.37 (s, 2H), 6.64 (s, 1H).

B. 5-(Hydroxymethyl)-3-(methoxymethoxy)isoxazole.

A suspension of 0.40 g (10.7 mmol) of lithium aluminum hydride in 40 ml of tetrahydrofuran was treated with 2.0 g of the resultant compound of Example 159A in 40 ml of tetrahydrofuran. After being stirred at ambient temperature for 5 h, the solution was treated with 20 ml of saturated ammonium chloride solution, extracted with three 20 ml portions of dichloromethane. The combined organic layers were dried over $MgSO_4$, and concentrated in vacuo provided 1.25 g of the desired compound as a yellow oil. $^1H$ NMR ($CDCl_3$) δ 3.55 (s, 3H), 4.66 (s, 2H), 5.31 (s, 2H), 5.97 (s, 1H). Mass spectrum: $(M+H)^+$ = 160.

C. p-Nitrophenyl (5-(3-(Methoxymethoxy)isoxazolyl)- methoxy)formate.

Using the procedure of Example 28A but replacing 3-(hydroxymethyl)pyridine with the resultant compound of Example 323B provided 2.14 g (84%) of the desired compound as a pale yellow solid. $^1H$ NMR ($CDCl_3$) δ 3.57 (s, 3H), 5.28 (s, 2H), 5.35 (s, 2H), 6.17 (s, 1H), 7.42 (dt, 2H), 8.30 (dt, 2H). Mass spectrum: $(M+H)^+$ = 325.

D. (2S,3S,5S)-2,5-Bis-(N-(5-(3-(methoxymethoxy)-isoxazolyl)methoxycarbonyl)amino)-1,6-diphenyl-3-hydroxyhexane.

A mixture of 50 mg (0.176 mmol) of the resultant compound of Example 1E and 171.0 mg (0.527 mmol) of the resultant compound of Example 159C in 2 ml of tetrahydrofuran was stirred at ambient temperature for 20 h. The solvent was then removed in vacuo, and the residue was purified by silica gel chromatography using 5% methanol in dichloromethane provided 78.2 mg (69%) of the desired compound as a white solid. $^1$H NMR ($CDCl_3$) δ 1.64 (m, 2H), 2.76 (d, 2H), 2.85 (d, 2H), 3.54 (s, 6H), 3.66 (m, 1H), 3.81 (m, 1H), 3.96 (m, 1H), 4.95 (br, 2H), 4.99 (s, 4H), 5.19 (br d, 1H),5.30 (s, 4H), 5.91 (d, 2H), 7.07-7.30 (m, 10H). Mass spectrum: $(M+H)^+$ = 655.

Example 160

A. 4-(Hydroxymethyl)isoxazole.

0.50 g (2.57 mmol) of 3,3-dimethoxy-2-(dimethoxymethyl)-1-propanol was added dropwise to a solution of 0.18 g (2.57 mmol) of hydroxyamine hydrochloride in 2 ml of water and 0.2 ml of 1N aqueous HCl. The mixture was then refluxed for 1 h. After cooling, the resulting solution was neutralized with solid $NaHCO_3$, extracted with five 5 ml portions of dichloromethane. The combined organic layers were dried over $MgSO_4$, and concentrated in vacuo provided 1.25 g of the desired compound as a yellow oil. $^1$H NMR ($CDCl_3$) δ 4.67 (s, 2H), 8.33 (s, 1H), 8.42 (s, 1H).

B. p-Nitrophenyl (4-Isoxazolyl)methoxy)formate.

Using the procedure of Example 28A but replacing 3-(hydroxymethyl)pyridine with the resultant compound of Example 160A provided 134.0 mg (39%) of the desired compound as a pale yellow solid. $^1$H NMR ($CDCl_3$) δ 5.23 (s, 2H), 7.39 (dt, 2H), 8.29 (dt, 2H), 8.44 (s, 1H), 8.63 (s, 1H).

C. (2S,3S,5S)-2,5-Bis-(N-(4-isoxazolyl)methoxycarbonyl)- amino -1,6-diphenyl-3-hydroxyhexane.

A mixture of 50 mg (0.176 mmol) of the resultant compound of Example 1E and 116.0 mg (0.440 mmol) of the resultant compound of Example 160B in 1 ml of tetrahydrofuran was stirred at ambient temperature for 20 h. The solvent was then removed in vacuo, and the residue was purified by silica gel chromatography using 5% methanol in dichloromethane provided 68.0 mg (72%) of the desired compound as a white solid. $^1$H NMR ($CDCl_3$) δ 1.61 (m, 2H), 2.74 (d, 2H), 2.83 (d, 2H), 3.65 (m, 1H), 3.79 (m, 1H), 3.94 (m, 1H), 4.73 (br, 1H), 4.94 (dd, 4H), 4.98 (br, 1H), 7.05-7.25 (m, 10H), 8.26 (two s, 2H), 8.40 (two s, 2H). Mass spectrum: $(M+H)^+$ = 535.

Example 161

(2S,3S,5S)-2,5-Bis-(N-((3-pyridazinyl)methoxycarbonyl)- amino)-1,6-diphenyl-3-hydroxyhexane.

A. Phenyl ((3-Pyridazinyl)methoxy)formate.

Using the procedure of Example 99 but replacing 2-(hydroxymethyl)pyridine with 3-(hydroxymethyl)pyridazine provided 252.0 mg (60%) of the desired compound as a white solid. $^1$H NMR ($CDCl_3$) δ 5.63 (s, 2H), 7.20-7.29 (m, 3H), 7.37-7.44 (m, 2H), 7.56 (dd, 1H), 9.21 (dd, 1H). Mass spectrum: $(M+H)^+$ = 231.

B. (2S,3S,5S)-2,5-Bis-(N-((3-pyridazinyl)methoxy-carbonyl)amino)-1,6-diphenyl-3-hydroxyhexane.

A mixture of 50 mg (0.176 mmol) of the resultant compound of Example 1E and 122.0 mg (0.527 mmol) of the resultant compound of Example 161A in 1 ml of dimethylformamide was stirred at 50°C for 2 days. The solvent was then removed in vacuo, and the residue was purified by silica gel chromatography using 5% methanol in dichloromethane provided 48.2 mg (49%) of the desired compound as a white solid. $^1$H NMR ($CDCl_3$) δ 1.73 (m, 2H), 2.79 (d, 2H), 2.91 (d, 2H), 3.78 (br, 1H), 3.89 (dd, 1H), 4.04 (br, 1H), 5.33 (dd, 2H), 5.36 (dd, 2H), 5.49 (br, 1H), 5.64 (br d, 1H), 7.11-7.34 (m, 12H), 7.43 (m, 2H), 9.11 (d, 2H).

Example 162

A. 2-(Hydroxymethyl)quinoline.

A solution of 3.0 g of quinoline-2-carboxaldehyde in 100 ml of ethanol was treated with 750 mg of sodium

borohydride and stirred at ambient temperature for 15 min. The resulting solution was neutralized with 1N HCl, concentrated in vacuo, and extracted three times with ethyl acetate. The combined organic layers were dried over $Na_2SO_4$ and concentrated to provide 2.65 g (88%) of the crude desired compound.

B. N-((2-Quinolinyl)methoxycarbonyl)valine Methyl Ester.

Using the procedure of Example 2B but replacing pyridine-2-methanol with the resultant compound of Example 162A provided the desired compound ($R_f$ 0.55, 50% ethyl acetate in hexane) in 85% yield.

C. N-((2-Quinolinyl)methoxycarbonyl)valine.

Using the procedure of Example 3E but replacing the resultant compound of Example 3D with the resultant compound of Example 162B provided the desired compound.

D. (2S,3S,5S)-2-(N-(N-((2-Quinolinyl)methoxycarbonyl)valinyl)amino)-5-(N-((3-pyridinyl)methoxycarbonyl)amino)-1,6-diphenyl-3-hydroxyhexane.

Using the procedure of Example 65C but replacing the resultant compound of Example 65B with the resultant compound of Example 162C and replacing the resultant compound of Example 64 with (2S,3S,5S)-2-amino-5-(N-((3-pyridinyl)methoxycarbonyl)-amino)-1,6-diphenyl-3-hydroxyhexane provided, after silica gel chromatography using a gradient of 2-4% methanol in chloroform, 105 mg (60%) of the desired compound ($R_f$ 0.63, 10% methanol in chloroform) as a white solid, m.p. 159-163°C. Mass spectrum: $(M + 1)^+ = 704$.

Example 163

(2S,3S,5S)-5-(N-(N-((2-Quinolinyl)methoxycarbonyl)valinyl)amino)-2-(N-((3-pyridinyl)methoxycarbonyl)amino)-1,6-diphenyl-3-hydroxyhexane.

Using the procedure of Example 65C but replacing the resultant compound of Example 65B with the resultant compound of Example 162C and replacing the resultant compound of Example 64 with (2S,3S,5S)-5-amino-2-(N-((3-pyridinyl)methoxycarbonyl)-amino)-1,6-diphenyl-3-hydroxyhexane provided, after silica gel chromatography using a gradient of 2-4% methanol in chloroform, 101 mg (59%) of the desired compound ($R_f$ 0.61, 10% methanol in chloroform) as a white solid, m.p. 141-143°C. Mass spectrum: $(M + 1)^+ = 704$.

Example 164 (reference)

(2S, 3S, 5S)-2,5-Bis-(N-((3-methyloxetan-3- yl)methoxycarbonyl)amino)-1,6-diphenyl-3-hydroxyhexane

Example 164a

3-Phenyloxycarbonyloxymethyl-3-methyloxetane

A 0.552 mL (4.42 mmol) sample of phenoxycarbonylchloride in 2 mL of methylene chloride was added to a solution of 376 mg (3.68 mmnol) of 3-hydroxymethyl-3-methyloxetane and 60.7 mL (5.52 mmol) of 4-methylmorpholine in 2 mL of methylene chloride cooled in an ice bath. The mixture was stirred at 0°C for 3.5 hours. The mixture was diluted with methylene chloride, which was washed with water, dried and concentrated. The crude product was chromatographed on silica gel, eluting with 20% ethyl acetate in hexane. The solvent was removed and the product dried to afford 0.582 g of the title compound, which was taken directly to the next step.

Example 164B

(2S, 3S, 5S)-2,5-Bis-(N-((3-methyloxetan-3- yl)methoxycarbonyl)amino)-1,6-diphenyl-3-hydroxyhexane

A 180 mg sample of the compound from Example 164A above was added to 57 mg (0.203 mmol) of the product of Example 1E and then dissolved in DMF and heated at 50°C overnight. The solvent was removed and the crude product chromatographed on silica gel, eluting with 2% methanol in chloroform. The solvent was removed and the product dried to afford 52.9 mg of the title compound. MS M/Z ($DCI/NH_3$): 541 (M+H), 558 (M+18). Anal. calc. for $C_{30}H_{40}N_2O7 \cdot 0.5\ H_2O$: C, 65.57; H, 7.47; N, 5.10; found: C, 65.80; H, 7.41; N, 5.19. Proton NMR (DMSO): $\delta$ 1.18 (m,

6H), 1.51 ((t, 2H), 2.58-2.73 (4H), 3.58 (m, 1H), 3.84 (m, 2 H), 3.95 (m, 4H), 4.15 (m, 4H), 4.30 (m, 4H), 4.67 (d, 1H), 6.78 (d, 1H), 7.05 (d, 1H), 7.11-7.27 (10H).

Example 165 (reference)

(2S,3S,5S)-2,5-Bis-(N-((2,3-dihydrofuro[2,3-b]pyridin-3-yl)oxycarbonyl)amino)-1,6-diphenyl-3-hydroxyhexane

Example 165A

3-p-Nitrophenoxycarbonyloxy-2,3-dihydrofuro[2,3-b]pyridine

A 534 mg (2.65 mmol) sample of p-nitrophenoxycarbonylchloride in methylene chloride was added to a solution of 330 mg (2.41 mmol) of 3-hydroxy-2,3-dihydrofuro[2,3-b]pyridine (prepared as described by H.Sliwa, *Bull. Soc. Chim. Fr.* 1970 (2), 646-652) aand 0.291 mL of 4-methylmorpholine in 2 mL of methylene chloride cooled in an ice bath. The mixture was stirred at 0°C for 2.75 hours. The mixture was diluted with methylene chloride, which was washed with water, dried and concentrated. The crude product was chromatographed on silica gel, eluting with 5% ethyl acetate in methylene chloride. The solvent was removed and the product dried to afford 0.493 g of the title compound, which was taken directly to the next step.

Example 165B

(2S,3S,5S)-2,5-Bis-(N-((2,3-dihydrofuro[2,3-b]pyridin-3-yl)-oxycarbonyl)amino)-1,6-diphenyl-3-hydroxyhexane

A 164 mg (0,544 mmol) sample of the compound from Example 165A above was added to 51.5 mg (0.181 mmol) of the product of Example 1E in 0.5 mL of DMF and stirred for 7 hours. The solvent was removed and the crude product chromatographed on silica gel, eluting with 2% and 5% methanol in chloroform. The solvent was removed and the product dried to afford 62.8 mg of the title compound. MS M/Z (FAB): 611 (M+H). Anal. calc. for $C_{34}H_{34}N_4O_7 \cdot 2H_2O$: C, 63.15; H, 5.88; N, 9.18; found: C, 63.22; H, 5.41; N, 8.64. Proton NMR (DMSO): δ 1.50 (m, 2H), 2.50-2.75 (4H), 3.92 (bs, 2H), 4.18 (m, 1H), 4.36 (m, 1H), 4.58 (m, 2H), 4.68 (m, 2H), 6.0 (m, 2H), 6.94 (m, 3H), 7.1-7.3 (11H), 7.59 (m, 1H) 7.83 (d, 1H), 8.12 (m, 2H).

Example 166

(2S,3S,5S,1'S)-2,5-Bis-(N-(1-(3- pyridyl)ethoxycarhonyl)amino)-1,6-diphenyl-3-hydroxyhexane

Example 166A

(S)-3-(1-(p-Nitrophenoxycarhonyloxy)ethyl)pyridine

A 534 mg (2.65 mmol) sample of p-nitrophenoxycarbonylchloride in methylene chloride was added to a solution of 74 mg (0.606 mmol) of (S)-1-(3-pyridyl)ethanol and 0.080 mL of 4-methylmorpholine in 2 mL of methylene chloride cooled in an ice bath. The mixture was stirred at 0°C for 3.5 hours. The mixture was diluted with methylene chloride, which was washed with water, dried and concentrated. The crude product was chromatographed on silica gel, eluting with 50% and 90% ethyl acetate in hexane. The solvent was removed and the product dried to afford 0.078 g of the title compound, which was taken directly to the next step.

Example 166B

(2S,3S,5S,1'S)-2,5-Bis-(N-(1-(3-pyridyl)ethoxycarbonyl)amino)-1,6-diphenyl-3-hydroxyhexane

A 68 mg (0.236 mmol) sample of the compound from Example 166A above was added to 22 mg (0.079 mmol) of the product from Example 1E in 1.0 mL of DMF and stirred for 16 hours. The solvent was removed and the crude product chromatographed on silica gel, eluting with 2%,5% and 10% methanol in methylene chloride. The solvent was removed and the product dried to afford 25.0 mg of the title compound. MS M/Z ($DCI/NH_3$): 583 (M+H). Proton NMR (DMSO): δ 1.02-1.16 (1H), 1.43 (m, 6H), 1.54 (m, 2H), 2.58-2.70 (m, 4H), 3.45 (m, 1H), 3.60 (m, 1H), 3.75-3.90 (2H), 4.70 (d, 1H), 5.62 (m, 2H), 6.93 (d, 1H), 7.09 (d, 2H), 7.14-7.22 (9H), 7.30 (m, 1H), 7.53 (m, 1H), 8.47 (br, 4H).

Example 167

(2S,3S,5S)-2,5-Bis-(N-(isoxazol-5-ylmethoxycarbonyl)amino)- 1,6-diphenyl-3-hydroxyhexane

Example 167A

5-(p-Nitroohenoxycarbonyloxymethyl)isoxazole

A 631 mg (2.65 mmol) sample of p-nitrophenoxycarbonylchloride in 2 mL of methylene chloride was added to a solution of 310 mg (3.13 mmol) of 5-hydroxymethyisoxazole and 0.344 mL of 4-methylmorpholine in 2 mL of methylene chloride cooled in an ice bath. The solution was stirred for 4 hours. The mixture was diluted with methylene chloride, which was washed with water, dried and concentrated. The crude product was chromatographed on silica gel, eluting with 2% ethyl acetate in methylene chloride. The solvent was removed and the product dried to afford 0.473 g of the title compound, which was taken directly to the next step.

Example 167B

(2S,3S,5S)-2,5-Bis-(N-(isoxazol-5-ylmethoxycarhonyl)amino)- 1,6-diphenyl-3-hydroxyhexane

A 125.8 mg (0.476 mmol) sample of the compound from Example 167A above was added to 45 mg (0.159 mmol) of the product of Example 1E in 0.5 mL of DMF and stirred for 16 hours. The solvent was removed and the crude product chromatographed on silica gel, eluting with 20% ethyl acetate in methylene chloride. The solvent was removed and the product dried to afford 30.6 mg of the title compound. MS M/Z (DCI/$NH_3$): 552 (m+$H_2O$), 535 (M+H) . Proton NMR (DMSO): δ 1.5 (t, 2H), 2.65-2.77 (4H), 3.57 (m, 1H), 3.86 (br, 2H), 4.71 (d, 1H), 4.95-5.12 (4H), 6.32 (m, 2H), 7.07-7.34 (12H), 8.53 (d, 2H). Anal calc. for $C_{28}H_{30}N_4O_7$ • 1/3 $H_2O$: C, 62.22; H, 5.68; N, 10.37; found: C, 62.06; H, 5.63; N, 10.33.

Example 168

(2S,3S,5S)-2,5-Bis-(N-(2,5-dihydrofuran-3-yl-methoxycarbonyl)amino)-1,6-diphenyl-3-hydroxyhexane

Example 168A

3-((t-butyldimethylsilyloxy)methyl)-4-hydroxybut-2-eneoic acid, 1,4-lactone

A 2.960 g (196 mmol) sample of t-butyldimethylsilyl chloride was added to a solution of 1.86 g (163.7 mmol) of 3-hydroxymethyl-4-hydroxybut-2-eneoic acid, 1,4-lactone and 2.783 g (40.9 mmol) of imidazole in 6 mL of DMF. The mixture was stirred for 5.5 hours. The mixture was diluted with water and extracted with ethyl acetate. The solvent was removed and the residue was chromatographed on a silica gel column, eluting with 20% ethyl acetate in hexane. The solvent was removed and the product dried to afford 3.109 g of the title product.

Example 168B

2-((t-Butyldimethylsilyloxy)methyl)-but-2-ene-1,4-diol

A 2.210 g (9.69 mmol) sample of 3-((t-butyldimethylsilyloxy)methyl)-4-hydroxybut-2-eneoic acid, 1,4-lactone, from Example 168A was dissolved in 6 mL of methylene chloride, cooled in a dry ice bath, and 14.2 mL (21.3 mmol) of DIBAL was added. The mixture was stirred at - 78°C for 4 hours, allowed to warm to room temperature and stirred for 16 hours. The mixture was cooled to -78°C and quenched with 1.53 mL of methanol and 2.55 mL of water. The mixture was filtered, and the filtrate concentrated and chromatographed on silica gel, eluting with 50% and 90% ethyl acetate in hexane. The solvent was removed and the product dried to afford 1.055 g of the title product, which was taken directly to the next step.

Example 168C

3-t-(Butyldimethylsilyloxy)methyl-2,5-dihydrofuran

To a 0.792 g sample of the compound from Example 168B above in 5 mL of methylene chloride was added

a solution of 4.59 g (6.53 mmol) of Martin's Sulfurane (Aldrich) in methylene chloride. The solution was stirred for 3.25 hours, diluted, washed with 20% KOH and saturated brine, dried and concentrated. The residue was chromatographed on silica gel, eluting with 10% ethyl acetate in hexane. The solvent was removed and the product dried to afford 0.282 g of the title product, which was taken directly to the next step.

Example 168D

3-hydroxymethyl-2,5-dihydrofuran

To a 0.270 g (1.26 mmol) sample of the compound from Example 168C above dissolved in 2 mL of methanol was added 0.160 mL (1.26 mmol) of trimethylsilyl chloride The solution was sitted for 2.25 hours, the solvent was removed, and the residue chromatographed on a silica gel column, eluting with 50% and 90% ethyl acetate in hexane. The solvent was removed and the product dried to afford 65 mg of the title compound, which was taken directly to the next step.

Example 168E

3-p-nitrophenoxycarhonyloxymethyl-2,5-dihydrofuran

To 65 mg (0.65 mmol) of the compound from Example 168D in methylene chloride was added 0.079 mL of 4-methylmorpholine and 144 mg (0.715 mmol) of p-nitrophenoxycarbonylchloride. The mixture was stirred for 2 hours, diluted with solvent, washed with saturated brine, and the solvent removed. The residue was chromatographed on a silica gel column, eluting with 20% and 30% ethyl acetate in hexane. The solvent was removed and the product dried to afford 0.115 g of the title product.

Example 168F

(2S,3S,5S)-2,5-Bis-(N-(2,5-dihydrofuran-3-yl-methoxycarbonyl)amino)-1,6-diphenyl-3-hydroxyhexane

A 0.110 g (0.415 mmol) sample of the compound from Example 168D above and 47 mg (0.166 mmol) of the product of Example 1E were stirred in 0.80 mL of DMF for 16 hours. The solvent was removed, and the residue was chromatographed on a silica gel column, eluting with 20% and 50 % ethyl acetate in methylene chloride. The solvent was removed and the product dried to afford 34.8 mg of the title compound. MS M/Z ($DCI/NH_3$): 537 (M+H), 554 ($M+NH_4$). Proton NMR (DMSO): δ 1.48 (t, 2H), 2.62-2.72 (4H), 3.56 (m, 1H), 3.78-3.93 (2H), 4.36 (br, 4H), 4.47 (br, 8H), 4.68 (d, 1H), 5.67 (s, 2H), 6.82 (d, 1H), 7.05-7.25 (11H).

Fluorogenic Assay for Screening InhibitorS of HIV Protease

The inhibitory potencyof the compounds of the invention can be determined by the following method.

A compound of the invention is dissolved in DMSO and a small aliquot further diluted with DMSO to 100 times the final concentration desired for testing. The reaction is carried out in a 6 X 50 mm tube in a total volume of 300 microliters. The final concentrations of the components in the reaction buffer are: 125 mM sodium acetate, 1 M sodium chloride, 5 mM dithiothreitol, 0.5 mg/ml bovine serum albumin, 1.3 μM fluorogenic substrate, 2% (v/v) dimethylsulfoxide, pH 4.5. After addition of inhibitor, the reaction mixture is placed in the fluorometer cell holder and incubated at 30°C for several minutes. The reaction is initiated by the addition of a small aliquot of cold HIV protease. The fluorescence intensity (excitation 340 nM, emmision 490 nM) is recorded as a function of time. The reaction rate is determined for the first six to eight minutes. The observed rate is directly proportional to the moles of substrate cleaved per unit time. The percent inhibition is 100 X (1 - (rate in presence of inhibitor)/(rate in absence of inhibitor)) .

Fluorogenic substrate: Dabcyl-Ser-Gln-Asn-Tyr-Pro-Ile-Val-Gln-EDANS wherein DABCYL = 4-(4-dimethylaminophenyl)azobenzoic acid and EDANS = 5-((2-aminoethyl)amino)-naphthalene-1-sulfonic acid.

Table 1 shows the inhibitory potencies of compounds of the invention against HIV-1 protease.

TABLE 1

| Compound of Example | Percent Inhibition | Inhibitor Concentration (nanomolar) |
|---|---|---|
| 28C | 61 | 1 |

TABLE 1 (continued)

| Compound of Example | Percent Inhibition | Inhibitor Concentration (nanomolar) |
|---|---|---|
| 29 | 55 | 0.5 |
| 30 | 61 | 0.5 |
| 31 | 61 | 0.5 |
| 95 | 62 | 0.5 |
| 97 | 51 | 0.5 |
| 130 | 65 | 0.5 |
| 135 | 43 | 0.5 |
| 136 | 56 | 0.5 |
| 143 | 64 | 0.5 |
| 144 | 67 | 0.5 |
| 145 | 97 | 0.5 |
| 146 | 83 | 0.5 |
| 147 | 84 | 0.5 |
| 148 | 84 | 0.5 |
| 150 | 63 | 0.5 |
| 151 | 80 | 0.5 |
| 155 | 52 | 0.5 |
| 156 | 60 | 0.5 |
| 157 | 64 | 0.5 |
| 158B | 72 | 0.5 |
| 162 | 66 | 0.5 |
| 163 | 69 | 0.5 |
| 167B | 56 | 1.0 |

Antiviral Activity

The anti-HIV activity of the compounds of the invention can be determined in MT4 cells according to the procedure of Pauwels et. al. (*J. Virol. Methods* **1988**, *20*, 309) . The $IC_{50}$ is the concentration of compound that gives 50% inhibition of the cytopathic effect of HIV. The $LC_{50}$ is the concentration of compound at which 50% of the cells remain viable.

Table 2 shows the inhibitory potencies of compounds of the invention against HIV-$1_{3B}$ in MT4 cells.

Table 2

| Compound of Example | $IC_{50}$ (micromolar) | $LC_{50}$ (micromolar) |
|---|---|---|
| 28C | 0.84-1.44 | >100 |
| 29 | 0.55-0.61 | >100 |
| 30 | 0.13-0.25 | >100 |
| 31 | 0.23-0.55 | 64 |
| 95 | 0.38-0.55 | >100 |
| 97 | 0.14-0.23 | >100 |

Table 2 (continued)

| Compound of Example | $IC_{50}$ (micromolar) | $LC_{50}$ (micromolar) |
|---|---|---|
| 130 | 0.026-0.075 | >100 |
| 135 | 0.31-0.35 | 81 |
| 136 | 0.22-0.29 | 59 |
| 143 | 0.16-0.26 | >100 |
| 144 | 0.077-0.124 | 96 |
| 146 | 0.013-0.025 | 55 |
| 147 | 0.058-0.067 | 58 |
| 148 | 0.058-0.077 | 61 |
| 149 | 0.46-0.5 | >100 |
| 150 | 0.098-0.144 | >100 |
| 151 | 0.003-0.005 | >100 |
| 155 | 0.21-0.29 | >100 |
| 156 | 0.4-0.5 | >100 |
| 157 | 0.14-0.19 | >100 |
| 158B | 0.086-0.094 | >100 |
| 162 | 0.053-0.086 | 27 |
| 163 | 0.034-0.064 | 84 |
| 167B | 0.74-1.6 | 91 |

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate (isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, p-toluenesulfonate and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other salts include salts with alkali metals or alkaline earth metals, such as sodium, potassium, calcium or magnesium or with organic bases.

Preferred salts of the compounds of the invention include hydrochloride, methanesulfonate, sulfonate, phosphonate and isethionate.

The compounds of the present invention can also be used in the form of esters. Examples of such esters include a hydroxyl-substituted compound of formula I which has been acylated with a blocked or unblocked amino acid residue, a phosphate function, a hemisuccinate residue, an acyl residue of the formula R*C(O)- or R*C(S)- wherein R* is hydrogen,

alkyl, haloalkyl, alkoxy, thioalkoxy, alkoxyalkyl, thioalkoxyalkyl or haloalkoxy, or an acyl residue of the formula $R_a$-$C(R_b)(R_d)$-C(O)- or $R_a$-$C(R_b)(R_d)$-C(S)- wherein $R_b$ and $R_d$ are independently selected from hydrogen or alkyl and $R_a$ is -$N(R_e)(R_f)$, $OR_e$ or -$SR_e$ wherein $R_e$ and $R_f$ are independently selected from hydrogen, alkyl and haloalkyl, or an amino-acyl residue of the formula $R_{180}NH(CH_2)_2NHCH_2C(O)$- or $R_{180}NH(CH_2)_2OCH_2C(O)$- wherein $R_{180}$ is hydrogen, alkyl, arylalkyl, cycloalkylalkyl, alkanoyl, benzoyl or an $\alpha$-amino acyl group. The amino acid esters of particular interest are glycine and lysine; however, other amino acid residues can also be used, including those

wherein the amino acyl group is $-C(O)CH_2NR_{200}R_{201}$ wherein $R_{200}$ and $R_{201}$ are independently selected from hydrogen and alkyl or the group $-NR_{200}R_{201}$ forms a nitrogen containing heterocyclic ring. These esters serve as pro-drugs of the compounds of the present invention and serve to increase the solubility of these substances in the gastrointestinal tract. These esters also serve to increase solubility for intravenous administration of the compounds. Other prodrugs include a hydroxyl-substituted compound of formula I wherein the hydroxyl group is functionalized with a substituent of the formula $-CH(R_g)OC(O)R_{181}$ or $-CH(R_g)OC(S)R_{181}$ wherein $R_{181}$ is alkyl, haloalkyl, alkoxy, thioalkoxy or haloalkoxy and $R_g$ is hydrogen, alkyl, haloalkyl, alkoxycarbonyl, aminocarbonyl, alkylaminocarbonyl or dialkylaminocarbonyl. The prodrugs of this invention are metabolized in vivo to provide the hydroxyl-substituted compound of formula I. The preparation of the prodrug esters is carried out by reacting a hydroxyl-substituted compound of formula I with an activated amino acyl, phosphoryl, hemisuccinyl or acyl derivative as defined above. The resulting product is then deprotected to provide the desired pro-drug ester. Prodrugs of the invention can also be prepared by alkylation of the hydroxyl group with (haloalkyl)esters, transacetalization with bis-(alkanoyl)acetals or condensation of the hydroxyl group with an activated aldehyde followed by acylation of the intermediate hemiacetal.

The compounds of the invention are useful for inhibiting retroviral protease, in particular HIV protease, in vitro or in vivo. The compounds of the present invention are also useful for the treatment or prophylaxis of diseases caused by retroviruses, especially acquired immune deficiency syndrome or an HIV infection in a human or other mammal.

Total daily dose administered to a human or other mammal host in single or divided doses may be in amounts, for example, from 0.001 to 300 mg/kg body weight daily and more usually 0.1 to 10 mg. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, sublingually, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleagenous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-propanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

The compounds of the present invention can also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically aceptable and metabolizable lipid capabale of forming liposomes can be used. The present compositions in liposome form can contain, in addition to a compound of the present invention, stabilizers, preservatives, excipients, and the like. The preferred lipids are the phospholipids and phosphatidyl cholines (lecithins), both natureal and synthetic.

Methods to form liposomes are known in the art. See, for example, Prescott, Ed., Methods in Cell Biology, Volume XIV, Academic Press, New York, N.Y. (1976), p. 33 et seq.

While the compounds of the invention can be administered as the sole active pharmaceutical agent, they can also be used in combination with one or more immunomodulators, antiviral agents, other antiinfective agents or vaccines. Other antiviral agents to be administered in combination with a compound of the present invention include AL-721, beta interferon, polymannoacetate, reverse transcriptase inhibitors ( for example, ganciclovir, dideoxycytidine (DDC), dideoxyinosine (DDI), BCH-189, AzdU, carbovir, DDA, D4C, D4T, DP-AZT, FLT (fluorothymidine), BCH-189, 5-halo-3'-thia-dideoxycytidine, PMEA, zidovudine (AZT) and the like), non-nucleoside reverse transcriptase inhibitors (for example, R82193, L-697,661, BI-RG-587 (nevirapine), HEFT compounds, L,697, 639, R82150, U-87201E and the like), TAT inhibitors (for example, RO-24-7429 and the like), trisodium phosphonoformate, HPA-23, eflonithine, Peptide T, Reticulose (nucleophosphoprotein), ansamycin LM 427, trimetrexate, UA001, ribavirin, alpha interferon, oxetanocin, oxetanocin-G, cylobut-G, cyclobut-A, ara-M, BW882C87, foscarnet, BW256U87, BW348U87, BV ara-U, CMV triclonal antibodies, FIAC, HOE-602, HPMPC, MSL-109, TI-23, trifluridine, vidarabine, famciclovir, penciclovir, acyclovir, castanospermine, rCD4/CD4-IgG, CD4-PE40, butyl-DNJ, hypericin, oxamyristic acid, dextran sulfate and pentosan polysulfate. Immunomodulators that can be administered in combination with a compound of the present invention include bropirimine, Ampligen, anti-human alpha interferon antibody, colony stimulting factor, CL246,738, Imreg-1, Imreg-2, diethydithiocarbamate, interleukin-2, alpha-interferon, inosine pranobex, methionine enkephalin, muramyl-tripeptide, TP-5, erythropoietin, naltrexone, tumor necrosis facator, beta interferon, gamma interferon, interleukin-4, autologous CD8+ infusion, alpha interferon immunoglobulin, anti-Leu-3A, autovaccination, biostimulation, extracorporeal photophoresis, FK-565, FK-506, G-CSF, GM-CSF, hyperthermia, isopinosine, IVIG, passisve immunotherapy and polio vaccine hyperimmunization. Other antiinfective agents that can be administered in combination with a compound of the present invention include pentamidine isethionate. Any of a variety of HIV or AIDS vaccines (for example, gp120 (recombinant), Env 2-3 (gp120), HIVAC-le (gp120), gp160 (recombinant), VaxSyn HIV-1 (gp160), Immuno-Ag (gp160), HGP-30, HIV-Immunogen, p24 (recombinant), VaxSyn HIV-1 (p24) can be used in combination with a compound of the present invention.

Other agents that can be used in combination with the compounds of this invention are ansamycin LM 427, apurinic acid, ABPP, Al-721, carrisyn, AS-101, avarol, azimexon, colchicine, compound Q, CS-85, N-acetyl cysteine (2-oxothiazolidine-4-carboxylate), D-penicillamine, diphenylhydantoin, EL-10, erythropoieten, fusidic acid, glucan, HPA-23, human growth hormone, hydorxchloroquine, iscador, L-ofloxacin or other quinolone antibiotics, lentinan, lithium carbonate, MM-1, monolaurin, MTP-PE, naltrexone, neurotropin, ozone, PAI, panax ginseng, pentofylline, Peptide T, pine cone extract, polymannoacetate, reticulose, retrogen, ribavirin, ribozymes, RS-47, Sdc-28, silicotungstate, THA, thymic humoral factor, thymopentin, thymosin fraction 5, thymosin alpha one, thymostimulin, UA001, uridine, vitamin B12 and wobemugos.

Other agents that can be used in combination with the compounds of this invention are antifungals such as amphotericin B, clotrimazole, flucytosine, fluconazole, itraconazole, ketoconazole and nystatin and the like.

Other agents that can be used in combination with the compounds of this invention are anitbacterials such as amikacin sulfate, azithromycin, ciprofloxacin, temafloxacin, tosufloxacin, clarithromycin, clofazimine, ethambutol, isoniazid, pyrazinamide, rifabutin, rifampin, streptomycin and TLC G-65 and the like.

Other agents that can be used in combination with the compounds of this invention are anti-neoplastics such as alpha interferon, COMP (cyclophosphamide, vincristine, methotrexate and prednisone), etoposide, mBACOD (methotrexate, bleomycin, doxorubicin, cyclophosphamide, vincristine and dexamethasone), PRO-MACE/MOPP(prednisone, methotrexate (w/leucovin rescue), doxorubicin, cyclophosphamide, etoposide/mechiorethamine, vincristine, prednisone and procarbazine), vincristine, vinblastine, angioinhibins, pentosan polysulfate, platelet factor 4 and SP-PG and the like.

Other agents that can be used in combination with the compounds of this invention are drugs for treating neurological disease such as peptide T, ritalin, lithium, elavil, phenytoin, carbamazipine, mexitetine, heparin and cytosine arabinoside and the like.

Other agents that can be used in combination with the compounds of this invention are anti-protozoals such as albendazole, azithromycin, clarithromycin, clindamycin, corticosteroids, dapsone, DIMP, eflornithine, 566C80, fansidar, furazolidone, L,671,329, letrazuril, metronidazole, paromycin, pefloxacin, pentamidine, piritrexim, primaquine, pyrimethamine, somatostatin, spiramycin, sulfadiazine, trimethoprim, TMP/SMX, trimetrexate and WR 6026 and the like.

Among the preferred agents for treatment of HIV or AIDS in combination with the compounds of this invention are reverse transcriptase inhibitors.

It will be understood that agents which can be combined with the compounds of the present invention for the treatment or prophylaxis of AIDS or an HIV infection are not limited to those listed above, but include in principle any agents useful for the treatment or prophylaxis of AIDS or an HIV infection.

When administered as a combination, the therapeutic agents can be formulated as separate compositions

which are given at the same time or different times, or the therapeutic agents can be given as a single composition.

**Claims**

**Claims for the following Contracting States : AT, BE, DE, DK, FR, GB, LU, NL, SE**

1. A compound of the formula:

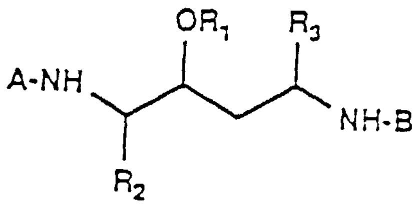

wherein $R_1$ is hydrogen and $R_2$ and $R_3$ are independently selected from arylalkyl, cycloalkylalkyl and (heterocyclic)alkyl;
A and B are independently selected from $R_6$-C(O)-(NH)-(CH($R_5$))-C(O)- and $R_6$-C(O)- wherein at each occurrence $R_5$ is independently selected from alkyl and at each occurrence $R_6$ is independently selected from $R_7$-NH-, $R_7$-N(alkyl)-, $R_7$-O- and $R_7$-S- wherein $R_7$ is heterocyclic or (heterocyclic)alkyl and $R_7$ on the heterocyclic ring may be optionally substituted with one or two substituents independently selected from halo, amino, (N-protected)amino, alkoxy, polyalkoxy and alkyl;
alkyl by itself or as part of another group is a straight or branched chain alkyl radical containing from one to six carbon atoms;
cycloalkyl as part of cycloalkylalkyl is an aliphatic ring having 3 to 7 carbon atoms;
aryl as part of arylalkyl is a $C_6$ monocyclic aryl group or a $C_9$ or $C_{10}$ bicyclic carbocyclic ring system having one or more aromatic rings;
heterocyclic by itself or as part of another group is selected from pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, pyrimidinyl, pyridazinyl, oxazolyl, oxazolinyl, oxazolidinyl, isoxazolyl, isoxazolinyl, isoxazolidinyl, morpholinyl, thiazolyl, thiazolidinyl, isothiazolyl, isothiazolidinyl, indolyl, quinolinyl, tetrahydroquinolyl, isoquinolinyl, benzimidazolyl, benzothiazolyl, benzoxazolyl, benzofuranyl, furyl, dihydrofuranyl, tetrahydrofuranyl, pyranyl, dihydropyranyl, tetrahydropyranyl, dioxanyl, dioxolanyl, thienyl and benzothienyl;
or a pharmaceutically acceptable salt, ester or prodrug thereof.

2. A compound according to Claim 1 of the formula:

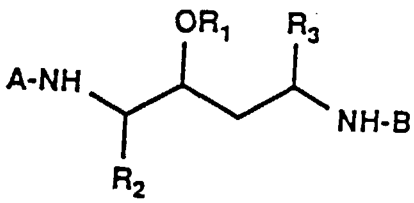

wherein $R_1$ is hydrogen, $R_2$ and $R_3$ are independently selected from arylalkyl; and A is $R_6$-C(O)-(NH)-(CH($R_{5a}$)-C(O)- wherein $R_{5a}$ is alkyl and $R_6$ is $R_7$-$R_9$- wherein $R_7$ is heterocyclic or (heterocyclic)alkyl optionally substituted in the heterocyclic ring and $R_9$ is -N($R_{7a}$)-, S or O wherein $R_{7a}$ is hydrogen or alkyl and B is -C(O)-$R_{6'}$ wherein $R_{6'}$ is $R_{7'}$-$R_{9'}$ wherein $R_{7'}$ is heterocyclic or (heterocyclic)alkyl optionally substituted in the heterocyclic ring and $R_{9'}$ is N($R_{7a'}$)-, S or O wherein $R_{7a'}$ is hydrogen or alkyl; or a pharmaceutically acceptable salt, ester or prodrug thereof.

**3.** A compound according to Claim 1 of the formula:

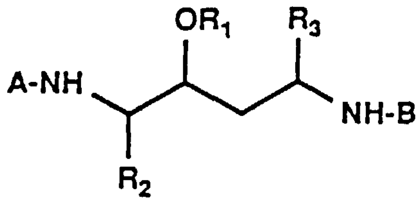

wherein $R_1$ is hydrogen, $R_2$ and $R_3$ are independently selected from arylalkyl; and A is -C(O)-$R_{6'}$ wherein $R_{6'}$ is $R_{7'}$-$R_{9'}$ wherein $R_{7'}$ is heterocyclic or (heterocyclic)alkyl optionally substituted in the heterocyclic ring and $R_{9'}$ is -N($R_{7a'}$)-, S or O wherein $R_{7a'}$ is hydrogen or alkyl and B is $R_6$-C(O)-(NH)-(CH($R_{5a}$))-C(O)- wherein $R_{5a}$ is alkyl and $R_6$ is $R_7$-$R_9$- wherein $R_7$ is heterocyclic or (heterocyclic)alkyl optionally substituted in the heterocyclic ring and $R_9$ is -N($R_{7a}$)-, S or O wherein $R_{7a}$ is hydrogen or alkyl; or a pharmaceutically acceptable salt, ester or prodrug thereof.

**4.** A compound according to Claim 1 of the formula:

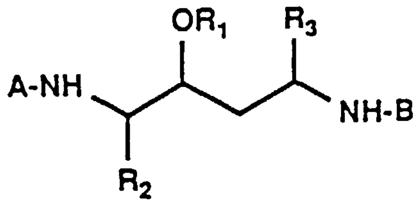

wherein $R_1$ is hydrogen, $R_2$ and $R_3$ are independently selected from arylalkyl; and A and B are independently -C(O)-$R_6$ wherein $R_6$ is $R_7$-$R_9$ wherein at each occurrence $R_7$ is independently selected from heterocyclic and (heterocyclic)alkyl optionally substituted in the heterocyclic ring and at each occurrence $R_9$ is independently selected from -N($R_{7a}$)-, S and O wherein $R_{7a}$ is hydrogen or alkyl; or a pharmaceutically acceptable salt, ester or prodrug thereof.

**5.** The compound according to Claim 1 which is (2S,3S,5S)-2-(N-(N-((2-Pyridinyl)methoxycarbonyl)-valinyl)amino)-5-(N-((3-pyridinyl)methoxycarbonyl)amino)-1,6-diphenyl-3-hydroxyhexane; or a pharmaceutically acceptable salt, ester or prodrug thereof.

**6.** The compound according to Claim 1 which is (2S,3S,5S)-2-(N-(N-(2-Pyridinyl)methoxycarbonyl)-valinyl)amino)-5-(N-((3-pyridinyl)methaxycarbonyl)amino)-1,6-diphenyl)-3-hydroxyhexane; or a pharmaceutically acceptable salt thereof.

**7.** A compound according to Claim 1 selected from the group consisting of:

(2S,3S,5S)-5-(N-(N-((N-Methyl-N-((2-pyridinyl)-methyl)amino)carbonyl)valinyl)amino)-2-(N-((3-pyridinyl)-methoxycarbonyl)amino)-1,6-diphenyl-3-hydroxyhexane;
(2S,3S,5S)-2-(N-((3-Pyridinyl)-methoxycarbonyl)amino-5-(N-(N-((N-Methyl-N-((6-methyl-2-pyridinyl)methyl)-amino)carbonyl)valinyl)amino)-1,6-diphenyl-3-hydroxyhexane; (2S,3S,5S)-2-(N-(N-((N-Methyl-N-((2-pyridinyl)-methyl)amino)carbonyl)valinyl)amino)-5-(N-((3-pyridinyl)-methoxycarbonyl)amino)-1,6-diphenyl-3-hydroxyhexane;
(2S,3S,5S)-5-(N-(N-((2-Pyridinyl)methoxycarbonyl)-valinyl)amino)-2-(N-((3-pyridinyl)methoxycarbonyl)amino)-1,6-diphenyl-3-hydroxyhexane;
(2S,3S,5S)-5-(N-(N-((N-methyl-N-((2-pyridinyl)-methyl)amino)carbonyl)isoleucinyl)amino)-2-(N-((3-pyridinyl)methoxycarbonyl)amino)-1,6-diphenyl-3-hydroxyhexane;
(2S,3S,5S)-2,5-Di(N-(3-pyridylmethyl)oxy-carbonyl)amino)-3-hydroxy-1,6-diphenylhexane;
(2S,3S-5S)-2-(N-(N-((N-Methyl-N-((6-methyl-2-pyridinyl)methyl)amino)carbonyl)valinyl)amino)-5-(N-((3-pyridinyl)-methoxycarbonyl)amino)-1,6-diphenyl-3-hydroxyhexane;
(2S,3S,5S)-2-(N-[(pyridin-3-yl)methoxycarbonyl]amino)-5-(N-[(6-methylpyridin-2-yl)methoxycarbonyl-valyllamino)-1,6-diphenyl-3-hydroxyhexane;
(2S,3S,5S)-5-(N-(N-((N-Methyl-N-((2-methyl-4-thiazolyl)methyl)amino)carbonyl)valinyl)amino)-2-(N-((3-pyridinyl)methoxycarbonyl)amino)-1,6-diphenyl-3-hydroxyhexane;
(2S,3S,5S)-2-(N-((3-Pyridinyl)methoxycarbonyl)amino-5-(N-(N-((N-methyl-N-((6-methoxy-3-pyridinyl)methyl)amino)carbonyl)valinyl) amino)-1,6-diphenyl-3-hydroxyhexane; and
(2S,3S,5S,1'S,2'S)-2-(N-(2-(N-((3-Pyridinyl)-methoxycarbonyl)amino-1-cyclopentyl)oxycarbonyl)amino)-5-(N-((3-pyridinyl)methoxycarbonyl)amino)-1,6-diphenyl-3-hydroxyhexane;
or a pharmaceutically acceptable salt, ester or prodrug thereof.

**8.** A compound of any one of Claims 1-7 for use as a therapeutic agent.

**9.** Use of a compound of any one of Claims 1-7 for manufacturing a medicament for inhibiting HIV protease in a human.

**10.** Use of a compound of any one of Claims 1-7 for manufacturing a medicament for treating an HIV infection in a human.

**11.** A pharmaceutical composition comprising a pharmaceutical carrier and a therapeutically effective amount of a compound of any one of Claims 1-7.

**12.** A process for the preparation of a compound of any one of Claims 1-7 comprising reacting a compound of the formula:

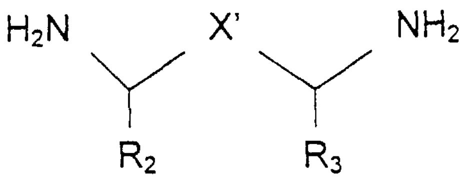

wherein X' is -CH(OH)$CH_2$ - and wherein $R_2$ and $R_3$ are as defined therein with A-OH or an active ester derivative thereof and with B-OH or an active ester derivative thereof wherein A and B are as defined therein.

**13.** A compound of the formula:

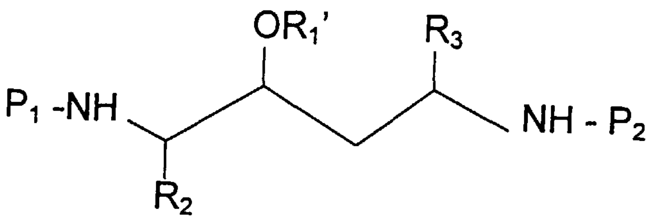

wherein $P_1$ and $P_2$ are independently selected from hydrogen and an N-protecting group; $R_1$' is hydrogen or an O-protecting group; and $R_2$ and $R_3$ are independently selected from arylalkyl, cycloalkylalkyl and (heterocyclic)alkyl;

alkyl as part of another group is a straight or branched chain alkyl radical containing from one to six carbon atoms;

cycloalkyl as part of cycloalkylalkyl is an aliphatic ring having 3 to 7 carbon atoms;

aryl as part of arylalkyl is a $C_6$ monocyclic aryl group or a $C_9$ or $C_{10}$ bicyclic carbocyclic ring system having one or more aromatic rings;

heterocyclic as part of (heterocyclic)alkyl is selected from pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, pyrimidinyl, pyridazinyl, oxazolyl, oxazolinyl, oxazolidinyl, isoxazolyl, isoxazolinyl, isoxazolidinyl, morpholinyl, thiazolyl, thiazolidinyl, isothiazolyl, isothiazolidinyl, indolyl, quinolinyl, tetrahydroquinolyl, isoquinolinyl, benzimidazolyl, benzothiazolyl, benzoxazolyl, benzofuranyl, furyl, dihydrofuranyl, tetrahydrofuranyl, pyranyl, dihydropyranyl, tetrahydropyranyl, dioxanyl, dioxolanyl, thienyl and benzothienyl;

or a salt thereof.

**14.** A compound according to Claim 13 of the formula:

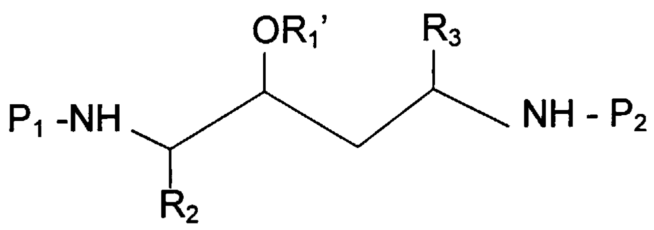

wherein $P_1$ and $P_2$ are independently selected from hydrogen and an N-protecting group; $R_1$' is hydrogen or an O-protecting group; and $R_2$ and $R_3$ are independently selected from arylalkyl; or a salt thereof.

**15.** A compound according to Claim 13 of the formula:

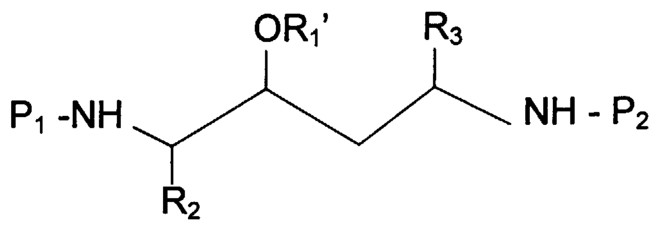

wherein $P_1$ and $P_2$ are independently selected from hydrogen and an N-protecting group; $R_1$' is hydrogen or an O-protecting group; and $R_2$ and $R_3$ are independently selected from arylalkyl; or a salt thereof; wherein the carbon atom bearing the $R_2$ substituent has the "S" configuration, the atom bearing the $OR_1$' substituent has the "S" configuration and the carbon atom bearing the $R_3$ substituent has the "S" configuration.

**16.** A compound according to Claim 13 of the formula:

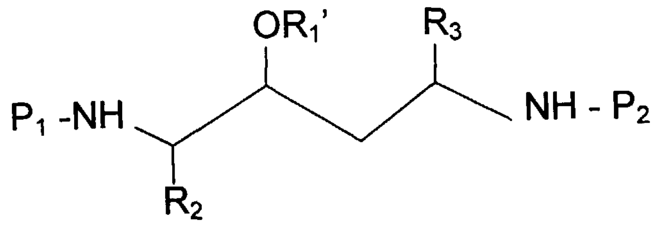

wherein $P_1$ and $P_2$ are independently selected from hydrogen and an N-protecting group; $R_1$' is hydrogen or an O-protecting group; and $R_2$ and $R_3$ are benzyl; or a salt thereof; wherein the carbon atom bearing the $R_2$ substituent has the "S" configuration, the atom bearing the $OR_1$' substituent has the "S" configuration and the carbon atom bearing the $R_3$ substituent has the "S" configuration.

**17.** The compound according to Claim 13 which is (2S,3S,5S)-2,5-Diamino-1,6-diphenyl-3-hydroxyhexane; or a salt thereof.

**18.** The compound according to Claim 16 which is (2S,3S,5S)-5-Amino-2-(N-((t-butyl)oxy)carbonyl)amino)-1,6-diphenyl-3-hydroxyhexane; or a salt thereof.

**19.** The compound according to Claim 16 which is (2S,3S,5S)-2-Amino-5-(N-((t-butyl)oxy)carbonyl)amino)-1,6-diphenyl-3-hydroxyhexane; or a salt thereof.

**Claims for the following Contracting State : GR**

**1.** A process for the preparation of a compound of the formula:

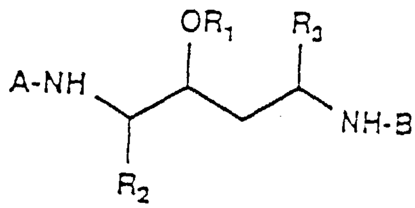

wherein $R_1$ is hydrogen and $R_2$ and $R_3$ are independently selected from arylalkyl, cycloalkylalkyl and (heterocyclic)alkyl;
A and B are independently selected from $R_6$-C(O)-(NH)-(OH($R_5$))-C(O)- and $R_6$-C(O)- wherein at each occurrence $R_5$ is independently selected from alkyl and at each occurrence $R_6$ is independently selected from $R_7$-NH-, $R_7$-N(alkyl)-, $R_7$-O- and $R_7$-S- wherein $R_7$ is heterocyclic or (heterocyclic)alkyl and $R_7$ on the heterocyclic ring may be optionally substituted with one or two substituents independently selected from halo, amino, (N-protected)amino, alkoxy, polyalkoxy and alkyl;
alkyl by itself or as part of another group is a straight or branched chain alkyl radical containing from one to six carbon atoms;
cycloalkyl as part of cycloalkylalkyl is an aliphatic ring having 3 to 7 carbon atoms;
aryl as part of arylalkyl is a $C_6$ monocyclic aryl group or a $C_9$ or $C_{10}$ bicyclic carbocyclic ring system having one or more aromatic rings;
heterocyclic by itself or as part of another group is selected from pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, pyrimidinyl, pyridazinyl, oxazolyl, oxazolinyl, oxazolidinyl, isoxazolyl, isoxazolinyl, isoxazolidinyl, morpholinyl, thiazolyl, thiazolidinyl, isothiazolyl, isothiazolidinyl, indolyl, quinolinyl, tetrahydroquinolyl, isoquinolinyl, benzimidazolyl,

benzothiazolyl, benzoxazolyl, benzofuranyl, furyl, dihydrofuranyl, tetrahydrofuranyl, pyranyl, dihydropyranyl, tetrahydropyranyl, dioxanyl, dioxolanyl, thienyl and benzothienyl;
or a pharmaceutically acceptable salt, ester or prodrug thereof,
said process comprising reacting a compound of the formula:

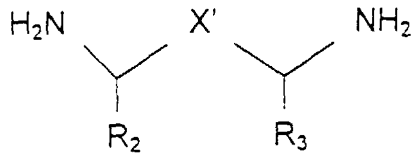

wherein X' is -CH(OH)$CH_2$- and wherein $R_2$ and $R_3$ are as defined above with A-OH or an active ester derivative thereof and with B-OH or an active ester derivative thereof wherein A and B are as defined above.

**2.** A process according to Claim 1 for the preparation of a compound of the formula:

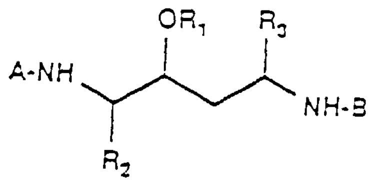

wherein $R_1$ is hydrogen, $R_2$ and $R_3$ are independently selected from arylalkyl; and A is $R_6$-C(O)-(NH)-(CH($R_{5a}$))-C(O)- wherein $R_{5a}$ is alkyl and $R_6$ is $R_7$-$R_9$- wherein $R_7$ is heterocyclic or (heterocyclic)alkyl optionally substituted in the heterocyclic ring and $R_9$ is -N($R_{7a}$)-, S or O wherein $R_{7a}$ is hydrogen or alkyl and B is -C(O)-$R_{6'}$ wherein $R_{6'}$ is $R_{7'}$-$R_{9'}$ wherein $R_{7'}$ is heterocyclic or (heterocyclic)alkyl optionally substituted in the heterocyclic ring and $R_{9'}$ is N($R_{7a'}$)-, S or O wherein $R_{7a'}$ is hydrogen or alkyl; or a pharmaceutically acceptable salt, ester or prodrug thereof.

**3.** A process according to Claim 1 for the preparation of a compound of the formula:

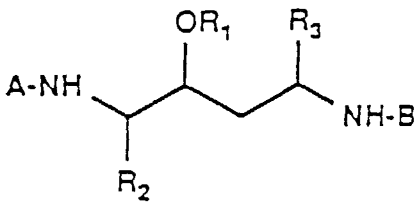

wherein $R_1$ is hydrogen, $R_2$ and $R_3$ are independently selected from arylalkyl; and A is -C(O)-$R_{6'}$ wherein $R_{6'}$ is $R_{7'}$-$R_{9'}$ wherein $R_{7'}$ is heterocyclic or (heterocyclic)alkyl optionally substituted in the heterocyclic ring and $R_{9'}$ is -N($R_{7a'}$)-, S or O wherein $R_{7a'}$ is hydrogen or alkyl and B is $R_6$-C(O)-(NH)-(CH($R_{5a}$)-C(O)- wherein $R_{5a}$ is alkyl and $R_6$ is $R_7$-$R_9$- wherein $R_7$ is heterocyclic or (heterocyclic)alkyl optionally substituted in the heterocyclic ring and $R_9$ is -N($R_{7a}$)-, S or O wherein $R_{7a}$ is hydrogen or alkyl; or a pharmaceutically acceptable salt, ester or prodrug thereof.

4. A process according to Claim 1 for the preparation of a compound of the formula:

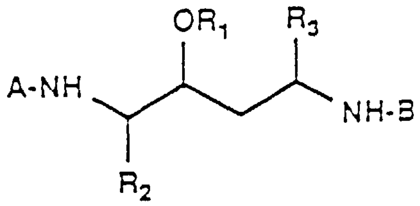

wherein $R_1$ is hydrogen, $R_2$ and $R_3$ are independently selected from arylalkyl; and A and B are independently -C(O)-$R_6$ wherein $R_6$ is $R_7$-$R_9$ wherein at each occurrence $R_7$ is independently selected from heterocyclic and (heterocyclic)alkyl optionally substituted in the heterocyclic ring and at each occurrence $R_9$ is independently selected from -N($R_{7a}$)-, S and O wherein $R_{7a}$ is hydrogen or alkyl; or a pharmaceutically acceptable salt, ester or prodrug thereof.

5. The process according to Claim 1 for the preparation of a compound which is (2S,3S,5S)-2-(N-(N-((2-Pyridinyl)methoxycarbonyl)-valinyl)amino)-5-(N-((3-pyridinyl)methoxycarbonyl)amino)-1,5-diphenyl-3-hydroxyhexane; or a pharmaceutically acceptable salt, ester or prodrug thereof.

6. The process according to Claim 1 for the preparation of a compound which is (2S,3S,5S)-2-(N-(N-((2-Pyridinyl)methoxycarbonyl)-valinyl)amino)-5-(N-((3-pyridinyl)methoxycarbonyl)amino)-1,6-diphenyl-3-hydroxyhexane; or a pharmaceutically acceptable salt thereof.

7. A process according to Claim 1 for the preparation of a compound selected from the group consisting of:

(2S,3S,5S)-5-(N-(N-((N-Methyl-N-((2-pyridinyl)-methyl)amino)carbonyl)valinyl)amino)-2-(N-((3-pyridinyl) - methoxycarbonyl)amino)-1,6-diphenyl-3-hydroxyhexane;
(2S,3S,5S)-2-(N-((3-Pyridinyl)-methoxycarbonyl)amino-5-(N-(N-((N-Methyl-N-((6-methyl-2-pyridinyl)methyl)-amino)carbonyl)valinyl)amino)-1,6-diphenyl-3-hydroxyhexane; (2S,3S,5S)-2-(N-(N-((N-Methyl-N-((2-pyridinyl)-methyl)amino)carbonyl)valinyl)amino)-5-(N-((3-pyridinyl)-methoxycarbonyl)amino)-1,6-diphenyl-3-hydroxyhexane;
(2S,3S,5S)-5-(N-(N-((2-Pyridinyl)methoxycarbonyl)-valinyl)amino)-2-(N-((3-pyridinyl)methoxycarbonyl)amino)-1,6-diphenyl-3-hydroxyhexane;
(2S,3S,5S)-5-(N-(N-((N-methyl-N-((2-pyridinyl)-methyl)amino)carbonyl)isoleucinyl)amino)-2-(N-((3-pyridinyl)methoxycarbonyl)amino)-1,6-diphenyl-3-hydroxyhexane;
(2S,3S,5S)-2,5-Di(N-(3-pyridylmethyl)oxy-carbonyl)amino)-3-hydroxy-1,6-diphenylhexane;
(2S,3S-5S)-2-(N-(N-((N-Methyl-N-((6-methyl-2-pyridinyl)methyl)amino)carbonyl)valinyl)amino)-5-(N-((3-pyridinyl)-methoxycarbonyl)amino)-1,6-diphenyl-3-hydroxyhexane;
(2S,3S,5S)-2-(N-[(pyridin-3-yl)methoxycarbonyl]amino)-5-(N-[(6-methylpyridin-2-yl)methoxycarbonyl-valyllamino)-1,6-diphenyl-3-hydroxyhexane;
(2S,3S,5S)-5-(N-(N-((N-Methyl-N-((2-methyl-4-thiazolyl)methyl)amino)carbonyl)valinyl)amino)-2-(N-((3-pyridinyl)methoxycarbonyl)amino)-1,6-diphenyl-3-hydroxyhexane;
(2S,3S,5S)-2-(N-((3-Pyridinyl)methoxycarbonyl)amino-5-(N-(N-((N-methyl-N-((6-methoxy-3-pyridinyl)methyl)amino)carbonyl)valinyl) amino)-1,6-diphenyl-3-hydroxyhexane; and
(2S,3S,5S,1'S,2'S)-2-(N-(2-(N-((3-Pyridinyl)-methoxycarbonyl)amino-1-cyclopentyl)oxycarbonyl)amino)-5-(N-((3-pyridinyl)methoxycarbonyl)amino)-1,6-diphenyl-3-hydroxyhexane;
or a pharmaceutically acceptable salt, ester or prodrug thereof.

8. A compound of the formula;

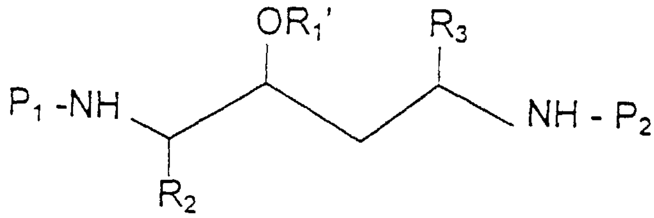

wherein $P_1$ and $P_2$ are independently selected from hydrogen and an N-protecting group; $R_1$' is hydrogen or an O-protecting group; and $R_2$ and $R_3$ are independently selected from arylalkyl, cycloalkylalkyl and (heterocyclic)alkyl;
alkyl as part of another group is a straight or branched chain alkyl radical containing from one to six carbon atoms;
cycloalkyl as part of cycloalkylalkyl is an aliphatic ring having 3 to 7 carbon atoms;
aryl as part of arylalkyl is a $C_6$ monocyclic aryl group or a $C_9$ or $C_{10}$ bicyclic carbocyclic ring system having one or more aromatic rings;
heterocyclic as part of (heterocyclic)alkyl is selected from pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, pyrimidinyl, pyridazinyl, oxazolyl, oxazolinyl, oxazolidinyl, isoxazolyl, isoxazolinyl, isoxazolidinyl, morpholinyl, thiazolyl, thiazolidinyl, isothiazolyl, isothiazolidinyl, indolyl, quinolinyl, tetrahydroquinolyl, isoquinolinyl, benzimidazolyl, benzothiazolyl, benzoxazolyl, benzofuranyl, furyl, dihydrofuraflyl, tetrahydrofuranyl, pyranyl, dihydropyranyl, tetrahydropyranyl, dioxanyl, dioxolanyl, thienyl and benzothienyl;
or a salt thereof.

**9.** A compound according to Claim 8 of the formula:

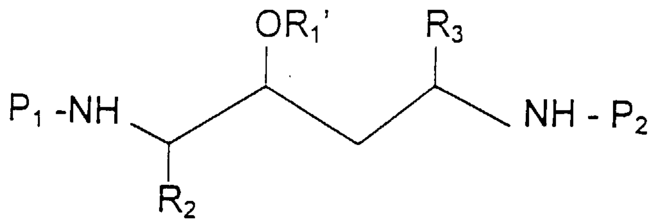

wherein $P_1$ and $P_2$ are independently selected from hydrogen and an N-protecting group; $R_1$' is hydrogen or an O-protecting group; and $R_2$ and $R_3$ are independently selected from arylalkyl; or a salt thereof.

**10.** A compound according to Claim 8 of the formula:

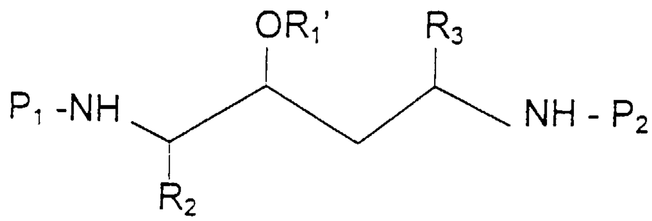

wherein $P_1$ and $P_2$ are independently selected from hydrogen and an N-protecting group; $R_1$' is hydrogen or an O-protecting group; and $R_2$ and $R_3$ are independently selected from arylalkyl; or a salt thereof; wherein the carbon atom bearing the $R_2$ substituent has the "S" configuration, the atom bearing the $OR_1$' substituent has the "S" configuration and the carbon atom bearing the $R_3$ substituent has the "S" configuration.

**11.** A compound according to Claim 8 of the formula:

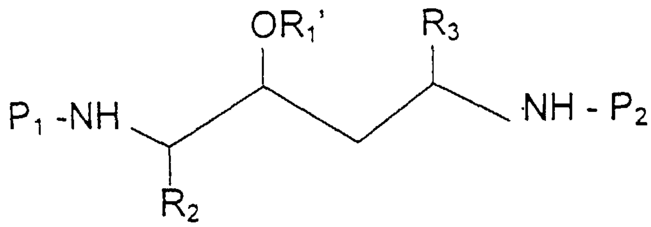

wherein $P_1$ and $P_2$ are independently selected from hydrogen and an N-protecting group; $R_1$' is hydrogen or an O-protecting group; and $R_2$ and $R_3$ are benzyl; or a salt thereof; wherein the carbon atom bearing the $R_2$ substituent has the "S" configuration, the atom bearing the $OR_1$' substituent has the "S" configuration and the carbon atom bearing the $R_3$ substituent has the "S" configuration.

**12.** The compound according to Claim 8 which is (2S,3S,5S)-2,5-Diamino-1,6-diphenyl-3-hydroxyhexane; or a salt thereof.

**13.** The compound according to Claim 11 which is (2S,3S,5S)-5-Amino-2-(N-((t-butyl)oxy)carbonyl)amino)-1,6-diphenyl-3-hydroxyhexane; or a salt thereof.

**14.** The compound according to Claim 11 which is (2S,3S,5S)-2-Amino-5-(N-((t-butyl) oxy)carbonyl)amino)-1,6-diphenyl-3-hydroxyhexane; or a salt thereof.

**15.** Use of a compound of the formula:

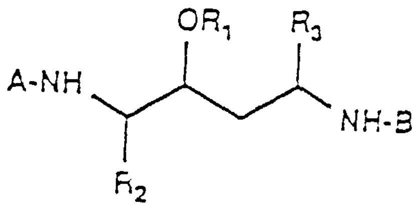

wherein $R_1$ is hydrogen and $R_2$ and $R_3$ are independently selected from arylalkyl, cycloalkylalkyl and (heterocyclic)alkyl;
A and B are independently selected from $R_6$-C(O)-(NH)-(CH($R_5$))-C(O)- and $R_6$-C(O)- wherein at each occurrence $R_5$ is independently selected from alkyl and at each occurrence $R_6$ is independently selected from $R_7$-NH-, $R_7$-N(alkyl)-, $R_7$-O- and $R_7$-S- wherein $R_7$ is heterocyclic or (heterocyclic)alkyl and $R_7$ on the heterocyclic ring may be optionally substituted with one or two substituents independently selected from halo, amino, (N-protected)amino, alkoxy, polyalkoxy and alkyl;
alkyl by itself or as part of another group is a straight or branched chain alkyl radical containing from one to six carbon atoms;
cycloalkyl as part of cycloalkylalkyl is an aliphatic ring having 3 to 7 carbon atoms;
aryl as part of arylalkyl is a $C_6$ monocyclic aryl group or a $C_9$ or $C_{10}$ bicyclic carbocyclic ring system having one or more aromatic rings;
heterocyclic by itself or as part of another group is selected from pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, pyrimidinyl, pyridazinyl, oxazolyl, oxazolinyl, oxazolidinyl, isoxazolyl, isoxazolinyl, isoxazolidinyl, morpholinyl, thiazolyl, thiazolidinyl, isothiazolyl, isothiazolidinyl, indolyl, quinolinyl, tetrahydroquinolyl, isoquinolinyl, benzimidazolyl, benzothiazolyl, benzoxazolyl, benzofuranyl, furyl, dihydrofuranyl, tetrahydrofuranyl, pyranyl, dihydropyranyl, tetrahydropyranyl, dioxanyl, dioxolanyl, thienyl and benzothienyl;
or a pharmaceutically acceptable salt, ester or prodrug thereof,
for manufacturing a medicament for inhibiting HIV protease in a human.

**16.** Use of a compound of the formula:

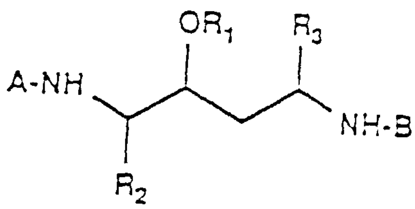

wherein $R_1$ is hydrogen and $R_2$ and $R_3$ are independently selected from arylalkyl, cycloalkylalkyl and (heterocyclic)alkyl;
A and B are independently selected from $R_6$-C(O)-(NH)-(OH($R_5$))-C(O)- and $R_6$-C(O)- wherein at each occurrence $R_5$ is independently selected from alkyl and at each occurrence $R_6$ is independently selected from $R_7$-NH-, $R_7$-N(alkyl)-, $R_7$-O- and $R_7$-S- wherein $R_7$ is heterocyclic or (heterocyclic)alkyl and $R_7$ on the heterocyclic ring may be optionally substituted with one or two substituents independently selected from halo, amino, (N-protected)amino, alkoxy, polyalkoxy and alkyl;
alkyl by itself or as part of another group is a straight or branched chain alkyl radical containing from one to six carbon atoms;
cycloalkyl as part of cycloalkylalkyl is an aliphatic ring having 3 to 7 carbon atoms;
aryl as part of arylalkyl is a $C_6$ monocyclic aryl group or a $C_9$ or $C_{10}$ bicyclic carbocyclic ring system having one or more aromatic rings;

heterocyclic by itself or as part of another group is selected from pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, pyrimidinyl, pyridazinyl, oxazolyl, oxazolinyl, oxazolidinyl, isoxazolyl, isoxazolinyl, isoxazolidinyl, morpholinyl, thiazolyl, thiazolidinyl, isothiazolyl, isothiazolidinyl, indolyl, quinolinyl, tetrahydroquinolyl, isoquinolinyl, benzimidazolyl, benzothiazolyl, benzoxazolyl, benzofuranyl, furyl, dihydrofuranyl, tetrahydrofuranyl, pyranyl, dihydropyranyl, tetrahydropyranyl, dioxanyl, dioxolanyl, thienyl and benzothienyl;
or a pharmaceutically acceptable salt, ester or prodrug thereof for manufacturing a medicament for treating an HIV infection in a human.

**17.** The use according to Claim 15 or 16 of a compound of the formula:

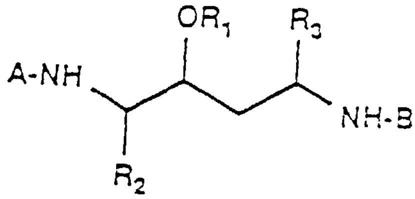

wherein $R_1$ is hydrogen, $R_2$ and $R_3$ are independently selected from arylalkyl; and A is $R_6$-C(O)-(NH)-(OH($R_{5a}$))-C(O)- wherein $R_{5a}$ is alkyl and $R_6$ is $R_7$-$R_9$- wherein $R_7$ is heterocyclic or (heterocyclic)alkyl optionally substituted in the heterocyclic ring and $R_9$ is -N($R_{7a}$)-, S or O wherein $R_{7a}$ is hydrogen or alkyl and B is -C(O)-$R_{6'}$ wherein $R_{6'}$ is $R_{7'}$-$R_{9'}$ wherein $R_{7'}$ is heterocyclic or (heterocyclic)alkyl optionally substituted in the heterocyclic ring and $R_{9'}$ is N($R_{7a'}$)-, S or O wherein $R_{7a'}$ is hydrogen or alkyl; or a pharmaceutically acceptable salt, ester or prodrug thereof.

**18.** The use according to Claim 15 or 16 of a compound of the formula:

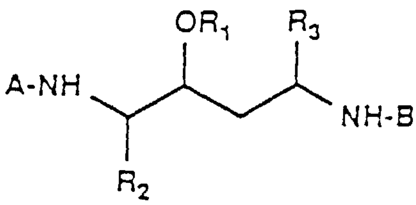

wherein $R_1$ is hydrogen, $R_2$ and $R_3$ are independently selected from arylalkyl; and A is -C(O)-$R_{6'}$ wherein $R_{6'}$ is $R_{7'}$-$R_{9'}$ wherein $R_{7'}$ is heterocyclic or (heterocyclic) alkyl optionally substituted in the heterocyclic ring and $R_{9'}$ is -N($R_{7a'}$)-, S or O wherein $R_{7a'}$ is hydrogen or alkyl and B is $R_6$-C(O)-(NH)-(CH($R_{5a}$))-C(O)- wherein $R_{5a}$ is alkyl and $R_6$ is $R_7$-$R_9$- wherein $R_7$ is heterocyclic or (heterocyclic)alkyl optionally substituted in the heterocyclic ring and $R_9$ is -N($R_{7a}$)-, S or O wherein $R_{7a}$ is hydrogen or alkyl; or a pharmaceutically acceptable salt, ester or prodrug thereof.

**19.** The use according to Claim 15 or 16 of a compound of the formula:

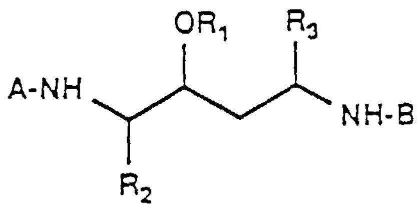

wherein $R_1$ is hydrogen, $R_2$ and $R_3$ are independently selected from arylalkyl; and A and B are independently $-C(O)-R_6$ wherein $R_6$ is $R_7$-$R_9$ wherein at each occurrence $R_7$ is independently selected from heterocyclic and (heterocyclic) alkyl optionally substituted in the heterocyclic ring and at each occurrence $R_9$ is independently selected from $-N(R_{7a})-$, S and O wherein $R_{7a}$ is hydrogen or alkyl; or a pharmaceutically acceptable salt, ester or prodrug thereof.

**20.** The use according to Claim 15 or 16 of a compound which is (2S,3S,5S)-2-(N-(N-((2-Pyridinyl)methoxycarbonyl)-valinyl)amino)-5-(N-((3-pyridinyl)methoxycarbonyl)amino)-1,6-diphenyl-3-hydroxyhexane; or a pharmaceutically acceptable salt, ester or prodrug thereof.

**21.** The use according to Claim 15 or 16 of a compound which is (2S,3S,5S)-2-(N-(N-((2-Pyridinyl)methoxycarbonyl)-valinyl)amino)-5-(N-((3-pyridinyl)methoxycarbonyl)amino)-1,6-diphenyl-3-hydroxyhexane; or a pharmaceutically acceptable salt thereof.

**22.** The use according to Claim 15 or 16 of a compound selected from the group consisting of:

(2S,3S,5S)-5-(N-(N-((N-Methyl-N-((2-pyridinyl)-methyl)amino)carbonyl)valinyl)amino)-2-(N-((3-pyridinyl)-methoxycarbonyl)amino)-1,6-dephenyl-3-hydroxyhexane;
(2S,3S,5S)-2-(N-((3-Pyridinyl)-methoxycarbonyl)amino-5-(N-(N-((N-Methyl-N-((6-methyl-2-pyridinyl)methyl)-amino)carbonyl)valinyl)amino)-1,6-diphenyl-3-hydroxyhexans; (2S,3S,5S)-2-(N-(N-((N-Methyl-N-((2-pyridinyl)-methyl)amino)carbonyl)valinyl)amino)-5-(N-((3-pyridinyl)-methoxycarbonyl)amino)-1,6-diphenyl-3-hydroxy-hexane;
(2S,3S,5S)-5-(N-(N-((2-Pyridinyl)methoxycarbonyl)-valinyl)amino)-2-(N-((3-pyridinyl)methoxycarbonyl)amino)-1,6-diphenyl-3-hydroxyhexane;
(2S,3S,5S)-5-(N-(N-((N-methyl-N-((2-pyridinyl)-methyl)amino)carbonyl)isoleucinyl)amino)-2-(N-((3-pyridinyl)methoxycarbonyl)amino)-1,6-diphenyl-3-hydroxyhexane;
(2S,3S,5S)-2,5-Di(N-(3-pyridylmethyl)oxy-carbonyl)amino)-3-hydroxy-1,6-diphenylhexane;
(2S,3S-5S)-2-(N-(N-((N-Methyl-N-((6-methyl-2-pyridinyl)methyl)amino)carbonyl)valinyl)amino)-5-(N-((3-pyridinyl)-methoxycarbonyl)amino)-1,6-diphenyl-3-hydroxyhexane;
(2S,3S,5S)-2-(N-[(pyridin-3-yl)methoxycarbonyl]amino)-5-(N-[(6-methylpyridin-2-yl)methoxycarbonyl-valyllamino)-1,6-diphenyl-3-hydroxyhexane;
(2S,3S,5S)-5-(N-(N-((N-Methyl-N-((2-methyl-4-thiazolyl)methyl)amino) carbonyl)valinyl)amino)-2-(N-((3-pyridinyl)methoxycarbonyl)amino)-1,5-diphenyl-3-hydroxyhexane;
(2S,3S,5S)-2-(N-((3-Pyridinyl)methoxycarbonyl)amino-5-(N-(N-((N-methyl-N-((6-methoxy-3-pyridinyl)methyl)amino)carbonyl)valinyl) amino)-1,6-diphenyl-3-hydroxyhaxane; and
(2S,3S,5S,1'S,2'S)-2-(N-(2-(N-((3-Pridinyl)-methoxycarbonyl)amino-1-cyclopentyl)oxycarbonyl)amino)-5-(N-((3-pyridinyl)methoxycarbonyl)amino)-1,6-diphenyl-3-hydroxyhexane;
or a pharmaceutically acceptable salt, ester or prodrug thereof.

**Patentansprüche**

**Patentansprüche für folgende Vertragsstaaten : AT, BE, DE, DK, FR, GB, LU, NL, SE**

**1.** Eine Verbindung der Formel :

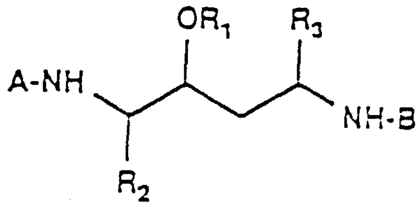

worin $R_1$ Wasserstoff ist und $R_2$ und $R_3$ unabhängig voneinander gewählt sind aus Arylalkyl, Cycloalkylalkyl und (heterozyklisch) Alkyl;
A und B sind unabhängig voneinander gewählt aus $R_6$ -C(O)-(NH)-(CH($R_5$))-C(O)- und $R_6$-C(O)- worin $R_5$, jedesmal wenn es vorkommt, unabhängig gewählt ist aus Alkyl und $R_6$, jedesmal wenn es vorkommt, unabhängig gewählt ist aus $R_7$-NH-, $R_7$-N(Alkyl)-, $R_7$-O- und $R_7$-S-, worin $R_7$ heterozyklisch ist oder (heterozyklisch)Alkyl und $R_7$ am heterozyklischen Ring könnte wahlweise substituiert sein mit einem oder zwei Substituenten unabhängig voneinander gewählt aus Halo, Amino, (N-geschützt)Amino, Alkoxy, Polyalkoxy und Alkyl;
Alkyl selbst oder als Teil einer anderen Verbindung ist ein gerade- oder verzweigtkettiges Alkylradikal, das von eins bis sechs Kohlenstoffatome enthält;
Cycloalkyl als Teil von Cycloalkylalkyl ist ein aliphatischer Ring, der 3 bis 7 Kohlenstoffatome enthält;
Aryl als Teil von Arylalkyl ist eine $C_6$ monozyklische Arylgruppe oder ein $C_9$ oder $C_{10}$ bizyklisches carbozyklisches Ringsystem, das einen oder mehrere aromatische Ringe enthält;
der Heterozyklus selbst oder als Teil einer anderen Gruppe ist gewählt aus Pyrrolyl, Pyrrolinyl, Pyrrolidinyl, Pyrazolyl, Pyrazolinyl, Pyrazolidinyl, Imidazolyl, Imidazolinyl, Imidazolidinyl, Pyridyl, Piperidinyl, Pyrazinyl, Piperazinyl, Pyrimidinyl, Pyridazinyl, Oxazolyl, Oxazolinyl, Oxazolidinyl, Isoxazolyl, Isoxazolinyl, Isoxazolidinyl, Morpholinyl, Thiazolyl, Thiazolidinyl, Isothiazolyl, Isothiazolidinyl, Indolyl, Chinolinyl, Tetrahydrochinolyl, Isochinolinyl, Benzimidazolyl, Benzothiazolyl, Benzoxazolyl, Benzofuranyl, Furyl, Dihydrofuranyl, Tetrahydrofuranyl, Pyranyl, Dihydropyranyl, Tetrahydropyranyl, Dioxanyl, Dioxolanyl, Thienyl und Benzothienyl;
oder einem pharmazeutisch verträglichen Salz, Ester oder Prodrug davon.

**2.** Eine Verbindung nach Anspruch 1 mit der Formel:

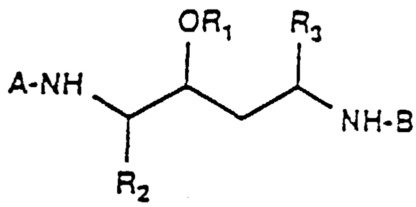

worin R1 Wasserstoff ist, $R_2$ und $R_3$ sind unabhängig voneinander gewählt aus Arylalkyl; und A ist $R_6$-C(O)-(NH)-(CH($R_{5a}$))-C(O)-worin $R_{5a}$ Alkyl ist und $R_6$ ist $R_7$-$R_9$- worin $R_7$ heterozyklisch ist oder (heterozyklisch)Alkyl, das im heterozyklischen Ring wahlweise substituiert ist und $R_9$ ist -N($R_{7a}$)-, S oder O, worin $R_{7a}$ Wasserstoff oder Alkyl ist und B ist -C(O)-$R_{6'}$, worin $R_{6'}$ $R_{7'}$-$R_{9'}$ ist, worin $R_{7'}$ heterozyklisch oder (heterozyklisch)Alkyl ist, wahlweise substituiert in dem heterozyklischen Ring und $R_{9'}$ ist N($R_{7a'}$)-, S oder O, worin $R_{7a'}$ Wasserstoff oder Alkyl ist; oder ein pharmazeutisch verträgliches Salz, Ester oder Prodrug davon.

**3.** Eine Verbindung nach Anspruch 1 mit der Formel:

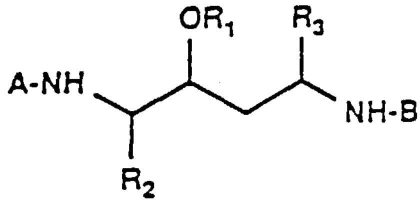

worin $R_1$ Wasserstoff ist, $R_2$ und $R_3$ sind unabhängig voneinander gewählt aus Arylalkyl; und A ist -C(O)-$R_{6'}$, worin $R_{6'}$ ist $R_{7'}$-$R_{9'}$, worin $R_{7'}$ heterozyklisch oder (heterozyklisch)Alkyl ist, wahlweise substituiert im heterozyklischen Ring und $R_{9'}$ ist -N($R_{7a'}$)-, S oder O, worin $R_{7a'}$ Wasserstoff oder Alkyl ist und B ist $R_6$-C(O)-(NH)-(CH($R_{5a}$)-C(O)-, worin $R_{5a}$ Alkyl ist und $R_6$ ist $R_7$-$R_9$-, worin $R_7$ heterozyklisch oder (heterozyklisch)Alkyl ist, wahlweise substituiert in dem heterozyklischen Ring und $R_9$ ist -N($R_{7a}$)-, S oder O, worin $R_{7a}$ Wasserstoff oder Alkyl ist; oder ein pharmazeutisch verträgliches Salz, Ester oder Prodrug davon.

**4.** Eine Verbindung nach Anspruch 1 mit der Formel:

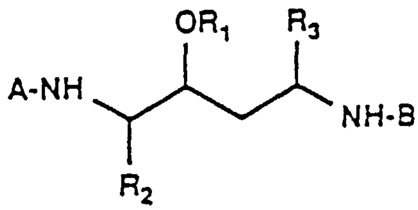

worin $R_1$ Wasserstoff ist, $R_2$ und $R_3$ sind unabhängig voneinander gewählt aus Arylalkyl; und A und B sind unabhängig voneinander -C(O)-$R_6$, worin $R_6$ $R_7$-$R_9$ ist, worin $R_7$, jedesmal wenn es vorkommt, unabhängig gewählt ist aus heterozyklisch und (heterozyklisch)Alkyl, wahlweise substituiert im heterozyklischen Ring und $R_9$, jedesmal wenn es vorkommt, ist unabhängig gewählt aus -N($R_{7a}$)-, S und O, worin $R_{7a}$ Wasserstoff ist oder Alkyl; oder ein pharmazeutisch verträgliches Salz, Ester oder Prodrug davon.

**5.** Die Verbindung nach Anspruch 1, die (2S,3S,5S)-2-(N-(N-((2-Pyridinyl)methoxycarbonyl)-valinyl)amino)-5-(N-((3-pyridinyl)methoxycarbonyl)amino)-1,6-diphenyl-3-hydroxyhexan ist; oder ein pharmazeutisch verträgliches Salz, Ester oder Prodrug davon.

**6.** Die Verbindung nach Anspruch 1, die (2S,3S,5S)-2-(N-(N-((2-Pyridinyl)methoxycarbonyl)-valinyl)amino)-5-(N-((3-pyridinyl)methoxycarbonyl)amino)-1,6-diphenyl-3-hydroxyhexan ist, oder ein pharmazeutisch verträgliches Salz davon.

**7.** Eine verbindung nach Anspruch 1, die aus der Gruppe gewählt ist bestehend aus:

(2S,3S,5S)-5-(N-(N-((N-Methyl-N-((2-pyridinyl)-methyl)amino)carbonyl)valinyl)amino)-2-(N-((3-pyridinyl)-methoxycarbonyl)amino-1,6-diphenyl-3-hydroxyhexan;
(2S,3S,5S)-2-(N-((3-Pyridinyl)-methoxycarbonyl)amino-5-(N-(N-((N-Methyl-N-((6-methyl-2-pyridinyl)methyl)-amino)carbonyl)valinyl)amino)-1,6-diphenyl-3-hydroxyhexan;
(2S,3S,5S)-2-(N-(N-((N-Methyl-N-((2-pyridinyl)-methyl)amino)carbonyl)valinyl)amino)-5-(N-((3-pyridinyl)-methoxycarbonyl)amino)-1,6-diphenyl-3-hydroxyhexan;
(2S,3S,5S)-5-(N-(N-((2-Pyridinyl)methoxycarbonyl)-valinyl)amino)-2-(N-((3-pyridinyl)methoxycarbonyl)amino)-1,6-diphenyl-3-hydroxyhexan;

(2S,3S,5S)-5-(N-(N-((N-Methyl-N-((2-pyridinyl)-methyl)amino)carbonyl)isoleucinyl)amino)-2-(N-((3-pyridinyl)methoxycarbonyl)amino)-1,6-diphenyl-3-hydroxyhexan;
(2S,3S,5S)-2,5-Di{N-(3-Pyridylmethyl)oxy-carbonyl)amino}-3-hydroxy-1,6-diphenylhexan;
(2S,3S-5S)-2-(N-(N-((N-Methyl-N-((6-methyl-2-pyridinyl)methyl)amino)carbonyl)valinyl)amino)-5-(N-((3-pyridinyl)-methoxycarbonyl)amino)-1,6-diphenyl-3-hydroxyhexan;
(2S,3S,5S)-2-(N-[(Pyridin-3-yl)methoxycarbonyl]amino)-5-(N-[(6-methylpyridin-2-yl)methoxycarbonyl-valyllamino)-1,6-diphenyl-3-hydroxyhexan;
(2S,3S,5S)-5-(N-(N-((N-Methyl-N-((2-methyl-4-thiazolyl)methyl)amino)carbonyl)valinyl)amino)-2-(N-((3-pyridinyl)methoxycarbonyl)amino)-1,6-diphenyl-3-hydroxyhexan;
(2S,3S,5S)-2-(N-((3-Pyridinyl)methoxycarbonyl)amino-5-(N-(N-((N-methyl-N-((6-methoxy-3-pyridinyl)methyl)amino)carbonyl)valinyl)amino)-1,6-diphenyl-3-hydroxyhexan; und
(2S,3S,5S,1'S,2'S)-2-(N-(2-(N-((3-Pyridinyl)-methoxycarbonyl)amino-1-cyclepentyl)oxycarbonyl)amine)-5-(N-((3-pyridinyl)methoxycarbonyl)amino)-1,6-diphenyl-3-hydroxyhexan;
oder ein pharmazeutisch verträgliches Salz, Ester oder Prodrug davon.

**8.** Verbindung nach irgendeinem der Ansprüche 1-7 zur Verwendung als Heilmittel.

**9.** Verwendung einer Verbindung nach irgendeinem der Ansprüche 1-7 für die Herstellung eines Medikamentes zur Hemmung der HIV Protease in einem Menschen.

**10.** Verwendung einer Verbindung nach irgendeinem der Ansprüche 1-7 für die Herstellung eines Medikamentes zur Behandlung einer HIV Infektion in einem Menschen.

**11.** Eine pharmazeutische Zusammensetzung, die einen pharmazeutischen Träger und eine therapeutisch wirksame Menge einer Verbindung nach irgendeinem der Ansprüche 1-7 enthält.

**12.** Ein Verfahren zur Herstellung einer Verbindung nach irgendeinem der Ansprüche 1-7, das das Reagieren einer Verbindung der Formel beinhaltet:

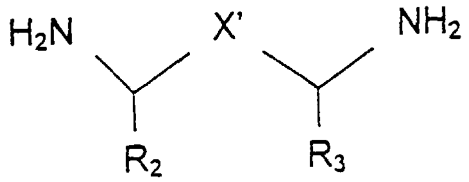

worin X' gleich $-CH(OH)CH_2-$ ist und worin $R_2$ und $R_3$ wie darin definiert sind, mit A-OH oder einem aktiven Esterderivat davon und mit B-OH oder einem aktiven Esterderivat davon, worin A und B wie darin definiert sind.

**13.** Eine Verbindung mit der Formel:

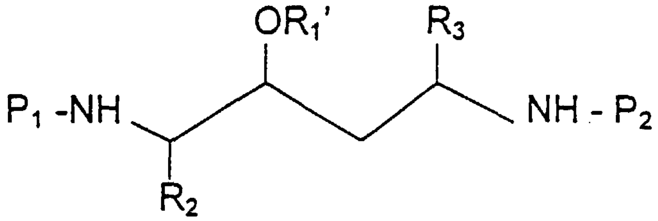

worin $P_1$ und $P_2$ unabhängig voneinander gewählt sind aus Wasserstoff und einer N-Schutzgruppe; $R_1$' ist Wasserstoff oder ein O-Schutzgruppe; und $R_2$ und $R_3$ sind unabhängig voneinander gewählt aus Arylalkyl, Cycloalkylalkyl und (heterozyklisch)Alkyl;

Alkyl als Teil einer anderen Gruppe ist ein gerade- oder verzweigtkettiges Alkylradikal, das von eins bis sechs Kohlenstoffatome enthält.

Cycloalkyl als Teil von Cycloalkylalkyl ist ein aliphatischer Ring, der 3 bis 7 Kohlenstoffatome enthält;

Aryl als Teil von Arylalkyl ist eine $C_6$ monozyklische Arylgruppe oder ein $C_9$ oder $C_{10}$ bizyklisches carbozyklisches Ringsystem, das einen oder mehrere aromatische Ringe enthält;

Heterozyklisch als Teil von (heterozyklisch)Alkyl ist gewählt aus Pyrrolyl, Pyrrolinyl, Pyrrolidinyl, Pyrazolyl, Pyrazolinyl, Pyrazolidinyl, Imidazolyl, Imidazolinyl, Imidazolidinyl, Pyridyl, Piperidinyl, Pyrazinyl, Piperazinyl, Pyrimidinyl, Pyridazinyl, Oxazolyl, Oxazolinyl, Oxazolidinyl, Isoxazolyl, Isoxazolinyl, Isoxazolidinyl, Morpholinyl, Thiazolyl, Thiazolidinyl, Isothiazolyl, Isothiazolidinyl, Indolyl, Chinolinyl, Tetrahydrochinolyl, Isochinolinyl, Benzimidazolyl, Benzothiazolyl, Benzoxazolyl, Benzofuranyl, Furyl, Dihydrofuranyl, Tetrahydrofuranyl, Pyranyl, Dihydropyranyl, Tetrahydropyranyl, Dioxanyl, Dioxolanyl, Thienyl und Benzothienyl;

oder ein Salz davon.

**14.** Eine Verbindung nach Anspruch 13 mit der Formel:

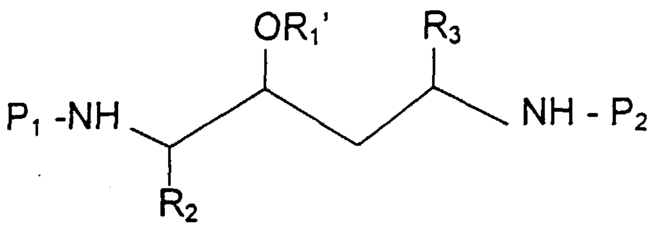

worin $P_1$ und $P_2$ unabhängig voneinander gewählt sind aus Wasserstoff und einer N-Schutzgruppe; $R_1$' ist Wasserstoff oder eine O-Schutzgruppe; und $R_2$ und $R_3$ sind unabhängig voneiander gewählt aus Arylalkyl; oder einem Salz davon.

**15.** Eine Verbindung nach Anspruch 13 mit der Formel:

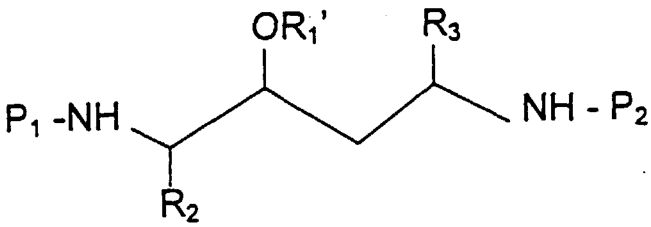

worin $P_1$ und $P_2$ unabhängig voneinander gewählt sind aus Wasserstoff und einer N-Schutzgruppe; $R_1$' ist Wasserstoff oder ein O-Schutzgruppe; und $R_2$ und $R_3$ sind unabhängig voneinander gewählt aus Arylalkyl; oder einem Salz davon; worin das Kohlenstoffatom, das den $R_2$ Substituenten trägt, die "S" Konfiguration hat, das Atom, das den $OR_1$' Substituenten trägt, hat die "S" Konfiguration und das Kohlenstoffatom, das den $R_3$ Substituenten trägt, hat die "S" Konfiguration.

**16.** Eine Verbindung nach Anspruch 13 mit der Formel:

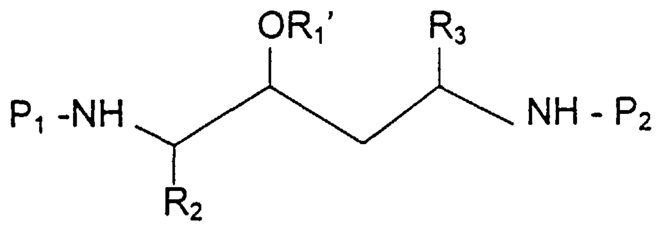

worin $P_1$ und $P_2$ unabhängig voneinander gewählt sind aus Wasserstoff und einer N-Schutzgruppe; $R_1$' ist Wasserstoff oder eine O-Schutzgruppe; und $R_2$ und $R_3$ sind Benzyl; oder ein Salz davon; worin das Kohlenstoffatom, das den $R_2$ Substituenten trägt, die "S" Konfiguration hat, das Atom, das den $OR_1$' Substituenten trägt, hat die "S" Konfiguration und das Kohlenstoffatom, das den $R_3$ Stubstiutenten trägt, hat die "S" Konfiguration.

**17.** Die Verbindung nach Anspruch 13, die (2S,3S,5S)-2,5-Diamino-1,6-diphenyl-3-hydroxyhexan ist; oder ein Salz davon.

**18.** Die Verbindung nach Anspruch 16, die (2S,3S,5S)-5-Amino-2-(N-((t-butyl)oxy)carbonyl)amino)-1,6-diphenyl-3-hydroxyhexan ist; oder ein Salz davon.

**19.** Die Verbindung nach Anspruch 16, die (2S,3S,5S)-2-Amino-5-(N-((t-butyl)oxy)carbonyl)amino)-1,6-diphenyl-3-hydroxyhexan ist; oder ein Salz davon.

**Patentansprüche für folgenden Vertragsstaat : GR**

**1.** Verfahren zur Herstellung einer Verbindung mit der Formel:

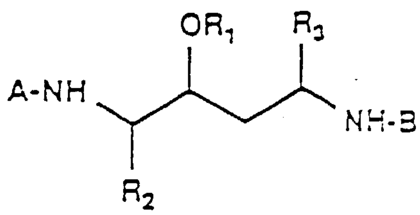

worin $R_1$ Wasserstoff ist und $R_2$ und $R_3$ sind unabhängig voneinander gewählt aus Arylalkyl, Cycloalkylalkyl und (heterozyklisch)Alkyl;
A und B sind unabhängig voneinander gewählt aus $R_6$-C(O)-(NH)-(CH($R_5$))-C(O)- und $R_6$-C(O)-, worin $R_5$, jedesmal wenn es vorkommt, unabhängig gewählt ist aus Alkyl, und $R_6$, jedesmal wenn es vorkommt, unabhängig gewählt ist aus $R_7$-NH-, $R_7$-N(Alkyl)-, $R_7$-O- und $R_7$-S-, worin $R_7$ heterozyklisch oder (heterozyklisch)Alkyl ist und $R_7$ am heterozyklischen Ring kann wahlweise substituiert sein mit einem oder zwei Substituenten, die unabhängig voneinander gewählt sind aus Halo, Amino, (N-geschützt)Amino, Alkoxy, Polyalkoxy und Alkyl;
Alkyl selbst oder als Teil einer anderen Gruppe ist ein gerade- oder verzweigtkettiges Alkybradikal, das von eins bis sechs Kohlenstoffatome enthält;
Cycloalkyl als Teil von Cycloalkylalkyl ist ein aliphatischer Ring, der 3 bis 7 Kohlenstoffatome enthält;
Aryl als Teil von Arylalkyl ist eine $C_6$ monozyklische Arylgruppe oder ein $C_9$ oder $C_{10}$ bizyklisches carbozyklisches Ringsystem, das einen oder mehrere aromatische Ringe enthält;
Heterozyklisch selbst oder als Teil einer anderen Gruppe ist gewählt aus Pyrrolyl, Pyrrolinyl, Pyrrolidinyl, Pyrazolyl, Pyrazolinyl, Pyrazolidinyl, Imidazolyl, Imidazolinyl, Imidazolidinyl, Pyridyl, Piperidinyl, Pyrazinyl, Piperazinyl, Pyrimidinyl, Pyridazinyl, Oxazolyl, Oxazolinyl, Oxazolidinyl, Isoxazolyl, Isoxazolinyl, Isoxazolidinyl, Morpholinyl, Thiazolyl, Thiazolidinyl, Isothiazolyl, Isothiazolidinyl, Indolyl, Chinolinyl, Tetrahydrochinolyl, Isochinolinyl, Benzimidazolyl, Benzothiazolyl, Benzoxazolyl, Benzofuranyl, Furyl, Dihydrofuranyl, Tetrahydrofuranyl, Pyranyl, Dihydropyranyl, Tetrahydropyranyl, Dioxanyl, Dioxolanyl, Thienyl und Benzothienyl;
oder einem pharmazeutisch verträglichen Salz, Ester oder Prodrug davon,
wobei das Verfahren das Reagieren einer Verbindung der folgenden Formel beinhaltet:

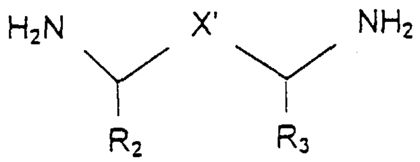

worin X' $-CH(OH)CH_2-$ ist und worin $R_2$ und $R_3$ wie oben definiert sind mit A-OH oder einem aktiven Esterderivat davon und mit B-OH oder einem aktiven Esterderivat davon, worin A und B wie oben definiert sind.

**2.** Verfahren nach Anspruch 1 zur Herstellung einer Verbindung der Formel:

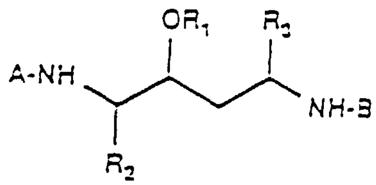

worin $R_1$ Wasserstoff ist, $R_2$ und $R_3$ sind unabhängig voneinander gewählt aus Arylalkyl; und A ist $R_6$-C(O)-(NH)-(CH($R_{5a}$))-C(O)-, worin $R_{5a}$ Alkyl ist und $R_6$ ist $R_7$-$R_9$-, worin $R_7$ heterozyklisch oder (heterozyklisch)Alkyl ist, wahlweise substituiert im heterozyklischen Ring, und $R_9$ ist -N($R_{7a}$)-, S oder O, worin $R_{7a}$ Wasserstoff ist oder Alkyl und B ist -C(O)-$R_{6'}$, worin $R_{6'}$ $R_{-7'}$-$R_{9'}$ ist, worin $R_{7'}$ heterozyklisch oder (heterozyklisch)Alkyl ist, wahlweise substituiert in dem heterozyklischen Ring, und $R_{9'}$ ist N($R_{7a'}$)-, S oder O, worin $R_{7a'}$ Wasserstoff oder Alkyl ist; oder ein pharmazeutisch verträgliches Salz, Ester oder Prodrug davon.

**3.** Ein Verfahren nach Anspruch 1 zur Herstellung einer Verbindung der Formel:

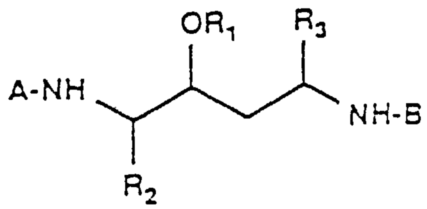

worin $R_1$ Wasserstoff ist, $R_2$ und $R_3$ sind unabhängig voneinander gewählt aus Arylalkyl; und A ist -C(O)-$R_{6'}$, worin $R_{6'}$ $R_{7'}$-$R_{9'}$ ist, worin $R_{7'}$ heterozyklisch oder (heterozyklisch)Alkyl ist, wahlweise substituiert im heterozyklischen Ring, und $R_{9'}$ ist -N($R_{7a'}$)- S oder O, worin $R_{7a'}$ Wasserstoff oder Alkyl ist und B $R_6$-C(O)-(NH)-

$(CH(R_{5a}))$-C(O)- ist, worin $R_{5a}$ Alkyl ist und $R_6$ $R_7$-$R_9$-ist, worin $R_7$ heterozyklisch oder (heterozyklisch)Alkyl ist, wahlweise substituiert im heterozyklischen Ring, und $R_9$ ist -N($R_{7a}$)-, S oder O, worin $R_{7a}$ Wasserstoff oder Alkyl ist; oder ein pharmazeutisch verträgliches Salz, Ester oder Prodrug davon.

**4.** Ein Verfahren nach Anspruch 1 zur Herstellung einer Verbindung der Formel:

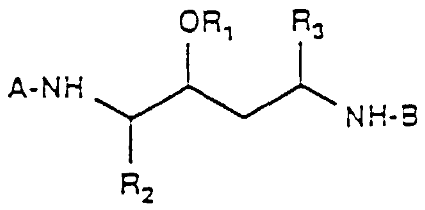

worin $R_1$ Wasserstoff ist, $R_2$ und $R_3$ sind unabhängig voneinander gewählt aus Arylalkyl; und A und B sind unabhängig voneinander -C(O)-$R_6$ worin $R_6$ $R_7$-$R_9$ ist, worin $R_7$, jedesmal wenn es vorkommt, unabhängig gewählt ist von heterozyklisch und (heterozyklisch)Alkyl, wahlweise substituiert im heterozyklischen Ring und $R_9$, jedesmal wenn es vorkommt, ist unabhängig gewählt aus -N($R_{7a}$)-, S und O, worin $R_{7a}$ Wasserstoff ist oder Alkyl; oder ein pharmazeutisch verträgliches Salz, Ester oder Prodrug davon.

**5.** Das Verfahren nach Anspruch 1 zur Herstellung einer Verbindung, die (2S,3S,5S)-2-(N-(N-((2-Pyridinyl)methoxycarbonyl)-valinyl)amino)-5-(N-((3-pyridinyl)methoxycarbonyl)amino)-1,6-diphenyl-3-hydroxyhexan ist; oder ein pharmazeutisch verträgliches Salz, Ester oder Prodrug davon.

**6.** Das Verfahren nach Anspruch 1 zur Herstellung einer Verbindung, die (2S,3S,5S)-2-(N-(N-((2-Pyridinyl)methoxycarbonyl)-valinyl)amino)-5-(N-((3-pyridinyl)methoxycarbonyl)amino)-1,6-diphenyl-3-hydroxyhexan ist; oder ein pharmazeutisch verträgliches Salz davon.

**7.** Ein Verfahren nach Anspruch 1 zur Herstellung einer Verbindung, die aus der Gruppe gewählt ist bestehend aus:

(2S,3S,5S)-5-(N-(N-((N-Methyl-N-((2-pyridinyl)-methyl)amino)carbonyl)valinyl)amino)-2-(N-((3-pyridinyl)-methoxycarbonyl)amino-1,6-diphenyl-3-hydroxyhexan;
(2S,3S,5S)-2-(N-((3-Pyridinyl)-methoxycarbonyl)amino-5-(N-(N-((N-Methyl-N-((6-methyl-2-pyridinyl)methyl)-amino)carbonyl)valinyl)amino)-1,6-diphenyl-3-hydroxyhexan;
(2S,3S,5S)-2-(N-(N-((N-Methyl-N-((2-pyridinyl)-methyl)amino)carbonyl)valinyl)amino)-5-(N-((3-pyridinyl)-methoxycarbonyl)amino)-1,6-diphenyl-3-hydroxyhexan;
(2S,3S,5S)-5-(N-(N-((2-Pyridinyl)methoxycarbonyl)-valinyl)amino)-2-(N-((3-pyridinyl)methoxycarbonyl)amino)-1,6-diphenyl-3-hydroxyhexan;
(2S,3S,5S)-5-(N-(N-((N-Methyl-N-((2-pyridinyl)-methyl)amino)carbonyl)isoleucinyl)amino)-2-(N-((3-pyridinyl)methoxycarbonyl)amino)-1,6-diphenyl-3-hydroxyhexan;
(2S,3S,5S)-2,5-Di{N-(3-Pyridylmethyl)oxy-carbonyl)amino}-3-hydroxy-1,6-diphenylhexan;
(2S,3S-5S)-2-(N-(N-((N-Methyl-N-((6-methyl-2-pyridinyl)methyl)amino)carbonyl)valinyl)amino)-5-(N-((3-pyridinyl)-methoxycarbonyl)amino)-1,6-diphenyl-3-hydroxyhexan;
(2S,3S,5S)-2-(N-[(Pyridin-3-yl)methoxycarbonyl]amino)-5-(N-[(6-methylpyridin-2-yl)methoxycarbonyl-valyllamino)-1,6-diphenyl-3-hydroxyhexan;
(2S,3S,5S)-5-(N-(N-((N-Methyl-N-((2-methyl-4-thiazolyl)methyl)amino)carbonyl)valinyl)amino)-2-(N-((3-pyridinyl)methoxycarbonyl)amino)-1,6-diphenyl-3-hydroxyhexan;
(2S,3S,5S)-2-(N-((3-Pyridinyl)methoxycarbonyl)amino-5-(N-(N-((N-methyl-N-((6-methoxy-3-pyridinyl)methyl)amino)carbonyl)valinyl)amino)-1,5-diphenyl-3-hydroxyhexan; und
(2S,3S,5S,1'S,2'S)-2-(N-(2-(N-((3-Pyridinyl)-methoxycarbonyl)amino-1-cyclopentyl)oxycarbonyl)amino)-5-(N-((3-pyridinyl)methoxycarbonyl)amino)-1,6-diphenyl-3-hydroxyhexan;
oder ein pharmazeutisch verträgliches Salz, Ester oder Prodrug davon.

**8.** Verbindung der Formel:

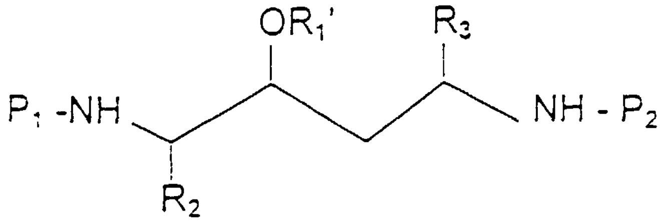

worin $P_1$ und $P_2$ unabhängig voneinander gewählt sind aus Wasserstoff und einer N-Schutzruppe; $R_1$' ist Wasserstoff oder eine O- Schutzgruppe; und $R_2$ und $R_3$ sind unabhängig gewählt aus Arylalkyl, Cyclalkylalkyl und (heterozyklisch)Alkyl;
Alkyl als Teil einer anderen Gruppe ist ein gerade- oder verzweigtkettiges Alkylradikal, das von eins bis sechs Kohlenstoffatome enthält;
Cycloalkyl als Teil von Cycloalkylalkyl ist ein aliphatischer Ring, der 3 bis 7 Kohlenstoffatome enthält;
Aryl als Teil von Arylalkyl ist eine $C_6$ monozyklische Arylgruppe oder ein $C_9$ oder $C_{10}$ bizyklisches carbozyklisches Ringsystem, das ein oder mehrere aromatische Ringe enthält;
heterozyklisch als Teil von (heterozyklisch)Alkyl ist gewählt aus Pyrrolyl, Pyrrolinyl, Pyrrolidinyl, Pyrazolyl, Pyrazolinyl, Pyrazolidinyl, Imidazolyl, Imidazolinyl, Imidazolidinyl, Pyridyl, Piperidinyl, Pyrazinyl, Piperazinyl, Pyrimidinyl, Pyridazinyl, Oxazolyl, Oxazolinyl, Oxazolidinyl, Isoxazolyl, Isoxazolinyl, Isoxazolidinyl, Morpholinyl, Thiazolyl, Thiazolidinyl, Isothiazolyl, Isothiazolidinyl, Indolyl, Chinolinyl, Tetrahydrochinolyl, Isochinolinyl, Benzimidazolyl, Benzothiazolyl, Benzoxazolyl, Benzofuranyl, Furyl, Dihydrofuranyl, Tetrahydrofuranyl, Pyranyl, Dihydropyranly, Tetrahydropyranyl, Dioxanyl, Dioxolanyl, Thienyl und Benzothienyl;
oder einem Salz davon.

**9.** Eine Verbindung nach Anspruch 8 mit der Formel:

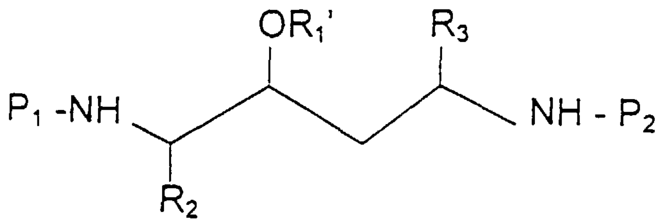

worin $P_1$ und $P_2$ unabhängig voneinander gewählt sind aus Wassertoff und einer N-Schutzgruppe. $R_1$' ist Wasserstoff oder eine O-Schutzgruppe; und $R_2$ und $R_3$ sind unabhängig gewählt aus Arylalkyl; oder einem Salz davon.

**10.** Eine Verbindung nach Anspruch 8 mit der Formel:

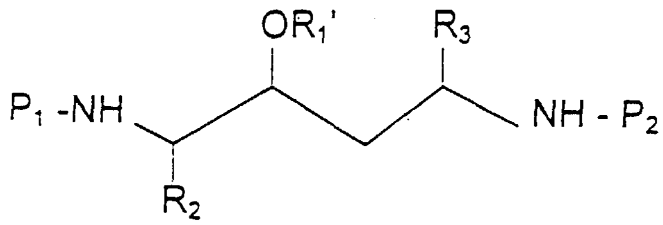

worin $P_1$ und $P_2$ unabhängig voneinander gewählt sind aus Wasserstoff und einer N-Schutzgruppe; $R_1$' ist Wasserstoff oder ein O-Schutzgruppe; und $R_2$ und $R_3$ sind unabhängig gewählt aus Arylalkyl; oder einem Salz davon; worin das Kohlenstoffatom, das den $R_2$ Substituenten trägt, die "S" Konfiguration hat, das Atom, das den $OR_1$' Substituenten trägt, hat die "S" Konfiguration und das Kohlenstoffatom, das den $R_3$ Substituenten trägt, hat die "S" Konfiguration.

**11.** Eine Verbindung nach Anspruch 8 mit der Formel:

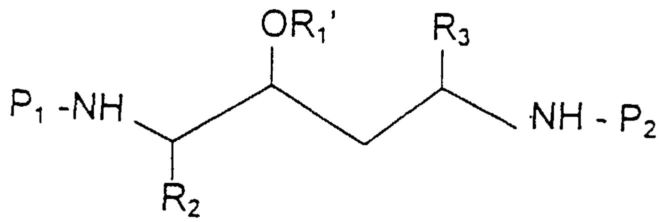

worin $P_1$ und $P_2$ unabhängig voneinander gewählt sind aus Wasserstoff und einer N-Schutzgruppe; $R_1$' ist Wasserstoff oder eine O-Schutzgruppe; und $R_2$ und $R_3$ sind Benzyl; oder ein Salz davon; worin das Kohlenstoffatom, das den $R_2$ Substituenten trägt, die "S" Konfiguration hat, das Atom, das den $OR_1$' Substituenten trägt, hat die "S" Konfiguration und das Kohlenstoffatom, das den $R_3$ Substituenten trägt, hat die "S" Konfiguration.

**12.** Die Verbindung nach Anspruch 8, die (2S,3S,5S)-2,5-Diamino-1,6-diphenyl-3-hydrOxyhexan ist; oder ein Salz davon.

**13.** Die Verbindung nach Anspruch 11, die (2S,3S,5S)-5-Amino-2-(N-((t-butyl)oxy)carbonyl)amino)-1,6-diphenyl-3-hydroxyhexan ist; oder ein Salz davon.

**14.** Die Verbindung nach Anspruch 11, die (2S,3S,5S)-2-Amino-5-(N-((t-butyl)oxy)carbonyl)amino)-1,6-diphenyl-3-hydroxyhexan ist; oder ein Salz davon.

**15.** Verwendung einer Verbindung mit der Formel:

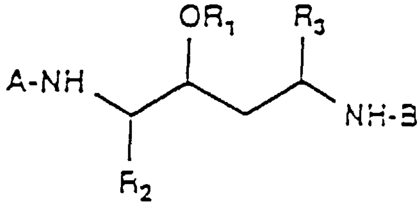

worin $R_1$ Wasserstoff ist und $R_2$ und $R_3$ unabhängig voneinander gewählt sind aus Arylalkyl, Cycloalkylalkyl und (heterozyklisch)Alkyl;
A und B sind unabhängig gewählt aus $R_6$-C(O)-(NH)-(CH($R_5$))-C(O)-und $R_6$-C(O)-, worin $R_5$, jedesmal wenn es vorkommt, unabhängig gewählt ist aus Alkyl, und $R_6$, jedesmal wenn es vorkommt, gewählt ist aus $R_7$-NH-, $R_7$-N(Alkyl)-, $R_7$-O- und $R_7$-S-, worin $R_7$ heterozyklisch oder (heterozyklisch)Alkyl ist und $R_7$ am heterozyklischen Ring wahlweise substituiert sein kann mit einem oder mehreren Substituenten, die unabhängig voneinander gewählt sind aus Halo, Amino, (N-geschützt)Amino, Alkoxy, Polyalkoxy und Alkyl;
Alkyl selbst oder als Teil einer anderen Gruppe ist ein gerade- oder verzweigtkettiges Alkylradikal, das von eins bis sechs Kohlenstoffatome enthält;
Cycloalkyl als Teil von Cycloalkylalkyl ist ein aliphatischer Ring, der 3 bis 7 Kohlenstoffatome hat;
Aryl als Teil von Arylalkyl ist eine $C_6$ monozyklische Arylgruppe oder ein $C_9$ oder $C_{10}$ bizyklisches carbozyklisches Ringsystem, das ein oder mehrere aromatische Ringe enthält;
heterozyklisch selbst oder als Teil einer anderen Gruppe ist gewählt aus Pyrrolyl, pyrrolinyl, Pyrrolidinyl, pyrazolyl, Pyrazolinyl, Pyrazolidinyl, Imidazolyl, Imidazolinyl, Imidazolidinyl, Pyridyl, Piperidinyl, Pyrazinyl, Piperazinyl, Pyrimidinyl, Pyridazinyl, Oxazolyl, Oxazolinyl, Oxazolidinyl, Isoxazolyl, Isoxazolinyl, Isoxazolidinyl, Morpholinyl, Thiazolyl, Thiazolidinyl, Isothiazolyl, Isothiazolidinyl, Indolyl, Chinolinyl, Tetrahydrochinolyl, Isochinolinyl, Benzimidazolyl, Benzothiazolyl, Benzoxazolyl, Benzofuranyl, Furyl, Dihydrofuranyl, Tetrahydrofuranyl, Pyranyl, Dihydropyranyl, Tetrahydropyranyl, Dioxanyl, Dioxolanyl, Thienyl und Benzothienyl;
oder ein pharmazeutisch verträgliches Salz, Ester oder Prodrug davon, zur Herstellung eine Medikamentes zur Hemmung der HIV Protease in einem Menschen.

**16.** Verwendung einer Verbindung mit der Formel:

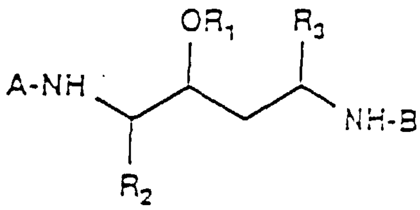

worin $R_1$ Wasserstoff ist und $R_2$ und $R_3$ sind unabhängig voneinander gewählt aus Arylalkyl, Cycloalkylalkyl und (heterozyklisch)Alkyl;
A und B sind unabhängig gewählt aus $R_6$-C(O)-(NH)-(CH($R_5$))-C(O)-und $R_6$-C(O)-, worin $R_5$, jedesmal wenn es vorkommt, unabhängig gewählt ist aus Alkyl, und $R_6$, jedesmal wenn es vorkommt, unabhängig gewählt ist aus $R_7$-NH-, $R_7$-N(Alkyl)-, $R_7$-O- und $R_7$-S-, worin $R_7$ heterozyklisch oder (heterozyklisch)Alkyl ist und $R_7$ am heterozyklischen Ring wahlweise substituiert sein kann mit einem oder zwei Substituenten, die unabhängig voneinander gewählt sind aus Halo, Amino, (N-geschützt)Amino, Alkoxy, Polyalkoxy und Alkyl;
Alkyl selbst oder als Teil einer anderen Gruppe ist ein gerade- oder verzweigtkettiges Alkylradikal, das von eins bis sechs Kohlenstoffatome enthält;

Cycloalkyl als Teil von Cycloalkylalkyl ist ein aliphatischer Ring, der 3 bis 7 Kohlenstoffatome hat;
Aryl als Teil von Arylalkyl ist eine $C_6$ monozyklische Arylgruppe oder ein $C_9$ oder $C_{10}$ bizyklisches carbozyklisches Ringsystem, das ein oder mehrere aromatische Ringe enthält;
heterozyklisch selbst oder als Teil einer anderen Gruppe ist gewählt aus Pyrrolyl, Pyrrolinyl, Pyrrolidinyl, Pyrazolyl, Pyrazolinyl, Pyrazolidinyl, Imidazolyl, Imidazolinyl, Imidazolidinyl, Pyridyl, Piperidinyl, Pyrazinyl, Piperazinyl, Pyrimidinyl, Pyridazinyl, Oxazolyl, Oxazolinyl, Oxazolidinyl, Isoxazolyl, Isoxazolinyl, Isoxazolidinyl, Morpholinyl, Thiazolyl, Thiazolidinyl, Isothiazolyl, Isothiazolidinyl, Indolyl, Chinolinyl, Tetrahydrochinolyl, Isochinolinyl, Benzimidazolyl, Benzothiazolyl, Benzoxazolyl, Benzofuranyl, Furyl, Dihydrofuranyl, Tetrahydrofuranyl, Pyranyl, Dihydropyranyl, Tetrahydropyranyl, Dioxanyl, Dioxolanyl, Thienyl und Benzothienyl;
oder einem pharmazeutisch verträglichen Salz, Ester oder Prodrug davon, zur Herstellung eines Medikamentes zur Behandlung der HIV Infektion in einem Menschen.

**17.** Die Verwendung nach Anspruch 15 oder 16 von einer Verbindung mit der Formel:

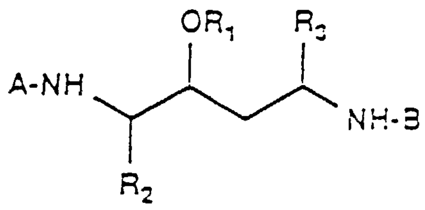

worin $R_1$ Wasserstoff ist, $R_2$ und $R_3$ sind unabhängig voneinander gewählt aus Arylalkyl; und A ist $R_6$-C(O)-(NH)-(CH($R_{5a}$))-C(O)-, worin $R_{5a}$ Alkyl ist und $R_6$ $R_7$-$R_9$- ist, worin $R_7$ heterozyklisch oder (hererozyklisch)Alkyl ist, wahlweise substituiert im heterozyklischen Ring, und $R_9$ ist -N($R_{7a}$)-, S oder O, worin $R_{7a}$ Wasserstoff oder Alkyl ist und B ist -C(O)-$R_{6'}$, worin $R_{6'}$ $R_{7'}$-$R_{9'}$ ist, worin $R_{7'}$ heterozyklisch oder (heterozyklisch)Alkyl ist, wahlweise substituiert im heterozyklischen Ring, und $R_{9'}$ ist N($R_{7a'}$)-, S oder O, worin $R_{7a'}$ Wasserstoff oder Alkyl ist, oder ein pharmazeutisch verträgliches Salz, Ester oder Prodrug davon.

**18.** Die Verwendung nach Anspruch 15 oder 16 von einer Verbindung mit der Formel:

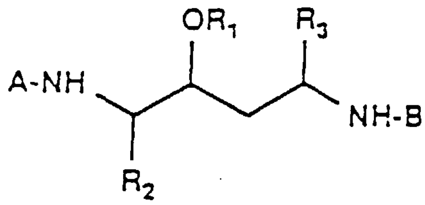

worin $R_1$ Wasserstoff ist, $R_2$ und $R_3$ sind unabhängig voneinander gewählt aus Arylalkyl; und A ist -C(O)-$R_{6'}$ worin $R_{6'}$ $R_{7'}$-$R_{9'}$ ist, worin $R_{7'}$ heterozyklisch oder (hererozyklisch)Alkyl ist, wahlweise substituiert im heterozyklischen Ring, und $R_{9'}$ ist -N($R_{7a'}$)-, S oder O, worin $R_{7a'}$ Wasserstoff ist oder Alkyl und B ist $R_6$-C(O)-(NH)-(CH($R_{5a}$))-C(O)- worin $R_{5a}$ Alkyl ist und $R_6$ $R_7$-$R_9$-ist, worin $R_7$ heterozyklisch oder (heterozyklisch)Alkyl ist, wahlweise substituiert im heterozyklischen Ring, und $R_9$ ist -N($R_{7a}$)-, S oder O, worin $R_{7a}$ Wasserstoff oder Alkyl ist; oder ein pharmazeutisch verträgliches Salz, Ester oder Prodrug davon.

**19.** Die Verwendung nach Anspruch 15 oder 16 von einer Verbindung mit der Formel:

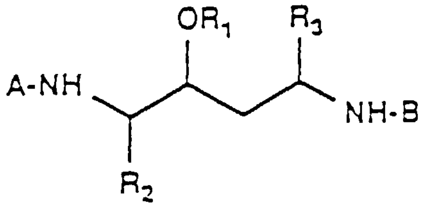

worin $R_1$ Wasserstoff ist, $R_2$ und $R_3$ sind unabhängig voneinander gewählt aus Arylalkyl; und A und B sind unabhängig voneinander -C(O)-$R_6$, worin $R_6$ $R_7$-$R_9$ ist, worin $R_7$, jedesmal wenn es vorkommt, unabhängig gewählt ist aus heterozyklisch und (heterozyklisch)Alkyl, wahlweise substituiert im heterozyklischen Ring, und $R_9$, jedesmal wenn es vorkommt, ist unabhängig gewählt aus -N($R_{7a}$)-, S und O, worin $R_{7a}$ Wasserstoff oder Alkyl ist; oder ein pharmezeutisch verträgliches Salz, Ester oder Prodrug davon.

**20.** Die Verwendung nach Anspruch 15 oder 16 von einer Verbindung, die (2S,3S,5S)-2-(N-(N-((2-Pyridinyl)methoxycarbonyl)-valinyl)amino)-5-(N-((3-pyridinyl)methoxycarbonyl)amino)-1,6-diphenyl-3-hydroxyhexan ist; oder ein pharmazeutisch verträgliches Salz, Ester oder Prodrug davon.

**21.** Die Verwendung nach Anspruch 15 oder 16 von einer Verbindung, die (2S,3S,5S)-2-(N-(N-((2-Pyridinyl)methoxycarbonyl)-valinyl)amino)-5-(N-((3-pyridinyl)methoxycarbonyl)amino)-1,6-diphenyl-3-hydroxyhexan ist; oder ein pharmezeutisch verträgliches Salz davon.

**22.** Die Verwendung nach Anspruch 15 oder 16 von einer Verbindung, die aus der Gruppe gewählt ist bestehend aus:

(2S,3S,5S)-5-(N-(N-((N-Methyl-N-((2-pyridinyl)-methyl)amino)carbonyl)valinyl)amino)-2-(N-((3-pyridinyl)-methoxycarbonyl)amino-1,6-diphenyl-3-hydroxyhexan;
(2S,3S,5S)-2-(N-((3-Pyridinyl)-methoxycarbonyl)amino-5-(N-(N-((N-Methyl-N-((6-methyl-2-pyridinyl)methyl)-amino)carbonyl)valinyl)amino)-1,6-diphenyl-3-hydroxyhexan;
(2S,3S,5S)-2-(N-(N-((N-Methyl-N-((2-pyridinyl)-methyl)amino)carbonyl)valinyl)amino)-5-(N-((3-pyridinyl)-methoxycarbonyl)amino)-1,6-diphenyl-3-hydroxyhexan;
(2S,3S,5S)-5-(N-(N-((2-Pyridinyl)methoxycarbonyl)-valinyl)amino)-2-(N-((3-pyridinyl)methoxycarbonyl)amino)-1,6-diphenyl-3-hydroxyhexan;
(2S,3S,5S)-5-(N-(N-((N-Methyl-N-((2-pyridinyl)-methyl)amino)carbonyl)isoleucinyl)amino)-2-(N-((3-pyridinyl)methoxycarbonyl)amino)-1,6-diphenyl-3-hydroxyhexan;
(2S,3S,5S)-2,5-Di{N-(3-Pyridylmethyl)oxy-carbonyl)amino}-3-hydroxy-1,6-diphenylhexan;
(2S,3S-5S)-2-(N-(N-((N-Methyl-N-((6-methyl-2-pyridinyl)methyl)amino)carbonyl)valinyl)amino)-5-(N-((3- pyridinyl)-methoxycarbonyl)amino)-1,6-diphenyl-3-hydroxyhexan;
(2S,3S,5S)-2-(N-[(Pyridin-3-yl)methoxycarbonyl]amino)-5-(N-[(6-methylpyridin-2-yl)methoxycarbonyl-valyllamino)-1,6-diphenyl-3-hydroxyhexan;
(2S,3S,5S)-5-(N-(N-((N-Methyl-N-((2-methyl-4-thiazolyl)mnethyl)amino)carbonyl)valinyl)amino)-2-(N-((3-pyridinyl)methoxycarbonyl)amino)-1,6-diphenyl-3-hydroxyhexan;
(2S,3S,5S)-2-(N-((3-Pyridinyl)methoxycarbonyl)arnino-5-(N-(N-((N-methyl-N-((6-methoxy-3-pyridinyl)methyl)amino)carbonyl)valinyl)amino)-1,6-diphenyl-3-hydroxyhexan; und
(2S,3S,5S,1'S,2'S)-2-(N-(2-(N-((3-Pyridinyl)-methoxycarbonyl)amino-1-cyclopentyl)oxycarbonyl)amino)-5-(N-((3-pyridinyl)methoxycarbonyl)amino)-1,6-diphenyl-3-hydroxyhexan;
oder ein pharmazeutisch verträgliches Salz, Ester oder Prodrug davon.

**Revendications**

**Revendications pour les Etats contractants suivants : AT, BE, DE, DK, FR, GB, LU, NL, SE**

**1.** Composé de formule:

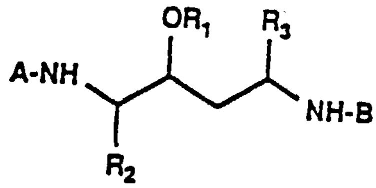

dans laquelle

$R_1$ est un atome d'hydrogène et $R_2$ et $R_3$ sont choisis indépendamment parmi les groupes arylalkyle, cycloalkylalkyle et (hétérocyclyl)alkyle;
A et B sont choisis indépendamment parmi $R_6$-C(O)-(NH)-(CH($R_5$))-C(O)- et $R_6$-C(O)-, dans lesquels $R_5$ est choisi indépendamment à chaque fois parmi les groupes aikyle et $R_6$ est choisi indépendamment à chaque fois parmi les groupes $R_7$-NH-, $R_7$-N(alkyle)-, $R_7$-O- et $R_7$-S- dans lesquels $R_7$ est un groupe hétérocyclyle ou (hétérocyclyl)alkyle et $R_7$ peut être éventuellement substitué sur le noyau hétérocyclique par un ou deux substituants choisis indépendamment parmi les groupes halogéno, ammo, amino N-protégé, alcoxy, polyalcoxy et alkyle;
le groupe alkyle en lui-même ou en tant qu'élément d'un autre groupe est un radical alkyle linéaire ou ramifié contenant de 1 à 6 atomes de carbone;
le groupe cycloalkyle en tant qu'élément du groupe cycloalkylalkyle est un cycle aliphatique de 3 à 7 atomes de carbone;
le groupe aryle en tant qu'élément du groupe arylalkyle est un groupe aryle monocyclique en $C_6$ ou un système cyclique carbocyclique bicyclique en $C_9$ ou $C_{10}$ ayant un ou plusieurs cycles aromatiques;
le groupe hétérocyclyle en lui-même ou en tant qu'élément d'un autre groupe est choisi parmi les groupes pyrrolyle, pyrrolinyle, pyrrolidinyle, pyrazolyle, pyrazolinyle, pyrazolidinyle, imidazolyle, imidazolinyle, imidazolidinyle, pyridyle, pipéridinyle, pyrazinyle, pipérazinyle, pyrimidinyle, pyridazinyle, oxazolyle, oxazolinyle, oxazolidinyle, isoxazolyle, isoxazolinyle, isoxazolidinyle, morpholinyle, thiazolyle, thiazolidinyle, isothiazolyle, isothiazolidinyle, indolyle, quinolinyle, tétrahydroquinolyle, isoquinolinyle, benzimidazolyle, benzothiazolyle, benzoxazolyle, benzofuranyle, furyle, dihydrofuranyle, tétrahydrofuranyle, pyranyle, dihydropyranyle, tétrahydropyranyle, dioxanyle, dioxolanyle, thiényle and benzothiényle;
ou un de ses sels, esters ou promédicaments pharmaceutiquement acceptables.

2. Composé selon la revendication 1, de formule:

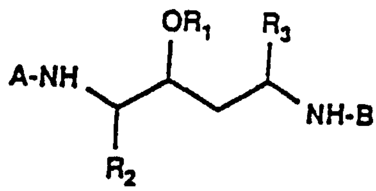

dans laquelle $R_1$ est un atome d'hydrogène, $R_2$ et $R_3$ sont choisis indépendamment parmi les groupes arylaikyle; et A est $R_6$-C(O)-(NH)-(CH($R_{5a}$))-C(O)- où $R_{5a}$ est un groupe alkyle et $R_6$ est $R_7$-$R_9$- où $R_7$ est un groupe hétérocyclyle ou (hétérocyclyl)alkyle éventuellement substitué sur le noyau hétérocyclique, et $R_9$ est -N($R_{7a}$)-, S ou O, où $R_{7a}$ est un atome d'hydrogène ou un groupe alkyle, et B est -C(O)-$R_{6'}$ où $R_{6'}$ est $R_{7'}$-$R_{9'}$ où $R_{7'}$ est un groupe hétérocyclyle ou (hétérocyclyl)alkyle éventuellement substitué sur le noyau hétérocyclique et $R_{9'}$ est -N($R_{7a'}$)-, S ou O, où $R_{7a'}$ est un atome d'hydrogène ou un groupe alkyle; ou un de ses sels, esters ou promédicaments pharmaceutiquement acceptables.

3. Composé selon la revendication 1, de formule:

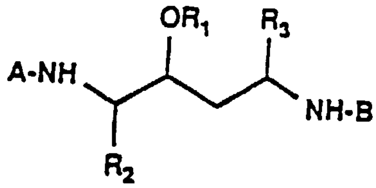

dans laquelle $R_1$ est un atome d'hydrogène, $R_2$ et $R_3$ sont choisis indépendamment parmi les groupes arylalkyle; et A est -C(O)-$R_{6'}$ où $R_{6'}$ est $R_{7''}$-$R_{9'}$ où $R_{7'}$ est un groupe hétérocyclyle ou (hétérocyclyl)alkyle éventuellement substitué sur le noyau hétérocyclique et $R_{9'}$ est -N($R_{7a'}$)-, S ou O, où $R_{7a'}$ est un atome d'hydrogène ou un groupe alkyle; et B est $R_6$-C(O)-(NH)-(CH($R_{5a}$))-C(O)- où $R_{5a}$ est un groupe alkyle et $R_6$ est $R_7$-$R_9$- où $R_7$ est un groupe hétérocyclyle ou (hétérocyclyl)alkyle éventuellement substitué sur le noyau hétérocyclique, et $R_9$ est -N($R_{7a}$)-, S ou O, où $R_{7a}$ est un atome d'hydrogène ou un groupe alkyle, ou un de ses sels, esters ou promédicaments pharmaceutiquement acceptables.

**4.** Composé selon la revendication 1, de formule:

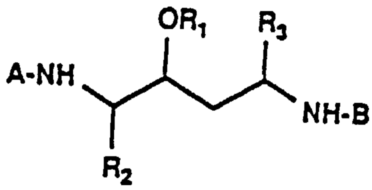

dans laquelle $R_1$ est un atome d'hydrogène, $R_2$ et $R_3$ sont choisis indépendamment parmi les groupes arylalkyle; et A et B sont indépendamment -C(O)-$R_6$ où $R_6$ est $R_7$-$R_9$ où $R_7$ est à chaque fois choisi indépendamment parmi les groupes hétérocyclyle et (hétérocyclyl)alkyle éventuellement substitués sur le noyau hétérocyclique et $R_9$ est à chaque fois choisi indépendamment parmi -N($R_{7a}$)-, S et O, où $R_{7a}$ est un atome d'hydrogène ou un groupe alkyle; ou un de ses sels, esters ou promédicaments pharmaceutiquement acceptables.

**5.** Composé selon la revendication 1, qui est le (2S,3S,5S)-2-(N-(N-((2-pyridinyl)méthoxycarbonyl)valinyl)amino)-5-(N-((3-pyridinyl)méthoxycarbonyl)-amino)-1,6-diphényl-3-hydroxyhexane; ou un de ses sels, esters ou promédicaments pharmaceutiquement acceptables.

**6.** Composé selon la revendication 1, qui est le (2S,3S,5S)-2-(N-(N-((2-pyridinyl)méthoxycarbonyl)valinyl)amino)-5-(N-((3-pyridinyl)méthoxycarbonyl)-amino)-1,6-diphényl-3-hydroxyhexane; ou un de ses sels pharmaceutiquement acceptables.

**7.** Composé selon la revendication 1, choisi dans le groupe constitué par:

le (2S,3S,5S)-5-(N-(N-((N-méthyl-N-((2-pyridinyl)méthyl)amino)carbonyl)valinyl)-amino)-2-(N-((3-pyridinyl)méthoxycarbonyl)amino)-1,6-diphényl-3-hydroxyhexane;
le (2S,3S,5S)-2-(N-((3-pyridinyl)méthoxycarbonyl)amino-5-(N-(N-((N-méthyl-N-((6-méthyl-2-pyridinyl)méthyl)amino)carbonyl)valinyl)amino)-1,6-diphényl-3-hydroxyhexane;
le (2S,3S,5S)-2-(N-(N-((N-méthyl-N-((2-pyridinyl)méthyl)amino)carbonyl)valinyl)-amino)-S-(N-((3-pyridinyl)méthoxycarbonyl)amino)-1,6-diphényl-3-hydroxyhexane;
le (2S,3S,5S)-5-(N-(N-((2-pyridinyl)méthoxycarbonyl)valinyl)amino)-2-(N-((3-pyridinyl)méthoxycarbonyl)amino)-1,6-diphényl-3-hydroxyhexane;
le (2S,3S,5S)-5-(N-(N-((N-méthyl-N-((2-pyridinyl)méthyl)amino)carbonyl)-isoleucinyl)amino)-2-(N-((3-pyridinyl)méthoxycarbonyl)amino)-1,6-diphényl-3-hydroxyhexane;
le (2S,3S,5S)-2,5-di(N-(3-pyridylméthyl)oxycarbonyl)amino)-3-hydroxy-1,6-diphénylhexane;
le (2S,3S,5S)-2-(N-(N-((N-méthyl-N-((6-méthyl-2-pyridinyl)méthyl)amino)-carbonyl)valinyl)amino)-5-(N-((3-pyridinyl)méthoxycarbonyl)amino)-1,6-diphényl- 3-hydroxyhexane;
le (2S,3S,5S)-2-(N-[(pyridin-3-yl)méthoxycarbonyl]amino)-5-(N-[(6-méthylpyridin-2-yl)méthoxycarbonyl)valyl]amino)-1,6-diphényl-3-hydroxyhexane;
le (2S,3S,5S)-5-(N-(N-((N-méthyl-N-((2-méthyl-4-thiazolyl)méthyl)amino)-carbonyl)valinyl)amino)-2-(N-((3-pyridinyl)méthoxycarbonyl)amino)-1,6-diphényl-3-hydroxyhexane;
le (2S,3S,5S)-2-(N-((3-pyridinyl)méthoxycarbonyl)amino-5-(N-(N-((N-méthyl-N-((6-méthoxy-3-pyridinyl)méthyl)amino)carbonyl)valinyl)amino)-1,6-diphényl-3-hydroxyhexane; et
le (2S,3S,5S,1'S,2'S)-2-(N-(2-(N-((3-pyridinyl)méthoxycarbonyl)amino-1-cyclopentyl)oxycarbonyl)amino)-5-(N-((3-pyridinyl)méthoxycarbonyl)amino)-1,6-diphényl-3-hydroxyhexane;
ou un de ses sels, esters ou promédicaments pharmaceutiquement acceptables.

**8.** Composé selon l'une quelconque des revendications 1 à 7, à utiliser comme agent thérapeutique.

**9.** Utilisation d'un composé selon l'une quelconque des revendications 1 à 7 pour la production d'un médicament destiné à inhiber la protéase de VIH chez l'homme.

**10.** Utilisation d'un composé selon l'une quelconque des revendications 1 à 7 pour la production d'un médicament destiné au traitement d'une infection à VIH chez l'homme.

**11.** Composition pharmaceutique comprenant un support pharmaceutique et une quantité thérapeutiquement efficace d'un composé selon l'une quelconque des revendications 1 à 7.

**12.** Procédé de préparation d'un composé selon l'une quelconque des revendications 1 à 7, comprenant la réaction d'un composé de formule:

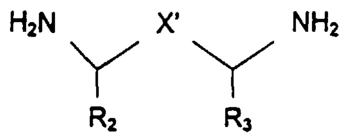

dans laquelle X' est $-CH(OH)CH_2-$ et $R_2$ et $R_3$ ont la définition indiquée précédemment, avec A-OH ou un de ses dérivés esters actifs et avec B-OH ou un de ses dérivés esters actifs, A et B ayant la signification indiquée précédemment.

**13.** Composé de formule:

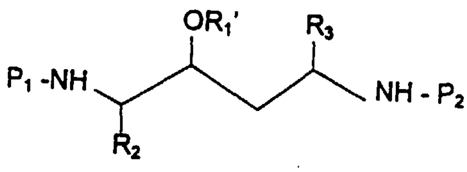

dans laquelle

$P_1$ et $P_2$ sont choisis indépendamment parmi un atome d'hydrogène et un groupe protecteur de N; $R_1$' est un atome d'hydrogène ou un groupe protecteur de O; et $R_2$ et $R_3$ sont choisis indépendamment parmi les groupes arylalkyle, cycloalkylalkyle et (hétérocyclyl)alkyle;
le groupe alkyle en tant qu'élément d'un autre groupe est un radical alkyle linéaire ou ramifié contenant de 1 à 6 atomes de carbone;
le groupe cycloalkyle en tant qu'élément du groupe cycloalkylalkyle est un cycle aliphatique de 3 à 7 atomes de carbone;
le groupe aryle en tant qu'élément du groupe arylalkyle est un groupe aryle monocyclique en $C_6$ ou un système cyclique carbocyclique bicyclique en $C_9$ ou $C_{10}$ ayant un ou plusieurs cycles aromatiques;
le groupe hétérocyclyle en tant qu'élément du groupe (hétérocyclyl)alkyle est choisi parmi les groupes pyrrolyle, pyrrolinyle, pyrrolidinyle, pyrazolyle, pyrazolinyle, pyrazolidinyle, imidazolyle, imidazolinyle, imidazolidinyle,

pyridyle, pipéridinyle, pyrazinyle, pipérazinyle, pyrimidinyle, pyridazinyle, oxazolyle, oxazolinyle, oxazolidinyle, isoxazolyle, isoxazolinyle, isoxazolidinyle, morpholinyle, thiazolyle, thiazolidinyle, isothiazolyle, isothiazolidinyle, indolyle, quinolinyle, tétrahydroquinolyle, isoquinolinyle, benzimidazolyle, benzothiazolyle, benzoxazolyle, benzofuranyle, furyle, dihydrofuranyle, tétrahydrofuranyle, pyranyle, dihydropyranyle, tétrahydropyranyle, dioxanyle, dioxolanyle, thiényle and benzothiényle;
ou un de ses sels.

**14.** Composé selon la revendication 13, de formule:

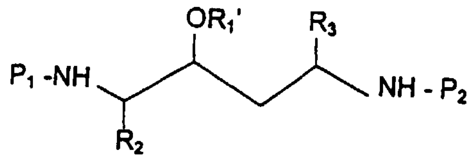

dans laquelle $P_1$ et $P_2$ sont choisis indépendamment parmi un atome d'hydrogène et un groupe protecteur de N; $R_1$' est un atome d'hydrogène ou un groupe protecteur de O; et $R_2$ et $R_3$ sont choisis indépendamment parmi les groupes arylalkyle; ou un de ses sels.

**15.** Composé selon la revendication 13, de formule:

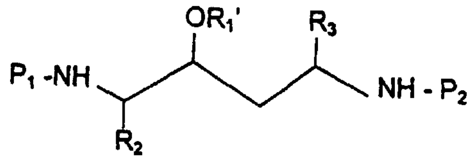

dans laquelle $P_1$ et $P_2$ sont choisis indépendamment parmi un atome d'hydrogène et un groupe protecteur de N; $R_1$' est un atome d'hydrogène ou un groupe protecteur de O; et $R_2$ et $R_3$ sont choisis indépendamment parmi les groupes arylalkyle; ou un de ses sels; où l'atome de carbone portant le substituant $R_2$ a la configuration "S", l'atome portant le substituant $OR_1$' a la configuration "S" et l'atome de carbone portant le substituant $R_3$ a la configuration "S".

**16.** Composé selon la revendication 13, de formule:

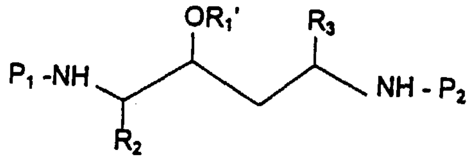

dans laquelle $P_1$ et $P_2$ sont choisis indépendamment parmi un atome d'hydrogène et un groupe protecteur de N; $R_1$' est un atome d'hydrogène ou un groupe protecteur de O; et $R_2$ et $R_3$ sont des groupes benzyle; ou un de ses sels; où l'atome de carbone portant le substituant $R_2$ a la configuration "S", l'atome portant le substituant $OR_1$' a la configuration "S" et l'atome de carbone portant le substituant $R_3$ a la configuration "S".

**17.** Composé selon la revendication 13, qui est le (2S,3S,5S)-2,5-diamino-1,6-diphényl-3-hydroxyhexane; ou un de ses sels.

**18.** Composé selon la revendication 16, qui est le (2S,3S,5S)-5-amino-2-(N-((t-butyl)oxy)carbonyl)amino)-1,6-diphényl-3-hydroxyhexane; ou un de ses sels.

**19.** Composé selon la revendication 16, qui est le (2S,3S,5S)-2-amino-5-(N-((t-butyl)oxy)carbonyl)amino)-1,6-diphényl-3-hydroxyhexane; ou un de ses sels.

**Revendications pour l'Etat contractant suivant : GR**

**1.** Procédé de préparation d'un composé de formule:

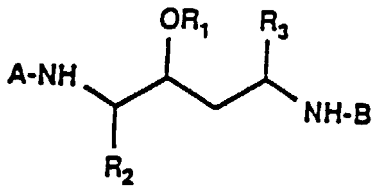

dans laquelle

$R_1$ est un atome d'hydrogène et $R_2$ et $R_3$ sont choisis indépendamment parmi les groupes arylalkyle, cycloalkylalkyle et (hétérocyclyl)alkyle;
A et B sont choisis indépendamment parmi $R_6$-C(O)-(NH)-(CH($R_5$))-C(O)- et $R_6$-C(O)-, dans lesquels $R_5$ est choisi indépendamment à chaque fois parmi les groupes alkyle et $R_6$ est choisi indépendamment à chaque fois parmi les groupes $R_7$-NH-, $R_7$-N(alkyle), $R_7$-O- et $R_7$-S- dans lesquels $R_7$ est un groupe hétérocyclyle ou (hétérocyclyl)alkyle et $R_7$ peut être éventuellement substitué sur le noyau hétérocyclique par un ou deux substituants choisis indépendamment parmi les groupes halogéno, amino, amino N-protégé, alcoxy, polyalcoxy et alkyle;
le groupe alkyle en lui-même ou en tant qu'élément d'un autre groupe est un radical alkyle linéaire ou ramifié contenant de 1 à 6 atomes de carbone;
le groupe cycloalkyle en tant qu'élément du groupe cycloalkylalkyle est un cycle aliphatique de 3 à 7 atomes de carbone;
le groupe aryle en tant qu'élément du groupe arylalkyle est un groupe aryle monocyclique en $C_6$ ou un système cyclique carbocyclique bicyclique en $C_9$ ou $C_{10}$ ayant un ou plusieurs cycles aromatiques;
le groupe hétérocyclyle en lui-même ou en tant qu'élément d'un autre groupe est choisi parmi les groupes pyrrolyle, pyrrolinyle, pyrrolidinyle, pyrazolyle, pyrazolinyle, pyrazolidinyle, imidazolyle, imidazolinyle, imidazolidinyle, pyridyle, pipéridinyle, pyrazinyle, pipérazinyle, pyrimidinyle, pyridazinyle, oxazolyle, oxazolinyle, oxazolidinyle, isoxazolyle, isoxazolinyle, isoxazolidinyle, morpholinyle, thiazolyle, thiazolidinyle, isothiazolyle, isothiazolidinyle, indolyle, quinolinyle, tétrahydroquinolyle, isoquinolinyle, benzimidazolyle, benzothiazolyle, benzoxazolyle, benzofuranyle, furyle, dihydrofuranyle, tétrahydrofuranyle, pyranyle, dihydropyranyle, tétrahydropyranyle, dioxanyle, dioxolanyle, thiényle and benzothiényle;
ou d'un de ses sels, esters ou promédicaments pharmaceutiquement acceptables,

ce procédé comprenant la réaction d'un composé de formule:

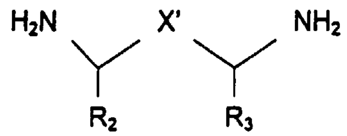

dans laquelle X' est -CH(OH)$CH_2$- et $R_2$ et $R_3$ ont la définition indiquée précédemment, avec A-OH ou un de ses dérivés esters actifs et avec B-OH ou un de ses dérivés esters actifs, A et B ayant la signification indiquée précédemment.

**2.** Procédé selon la revendication 1 pour la préparation d'un composé de formule:

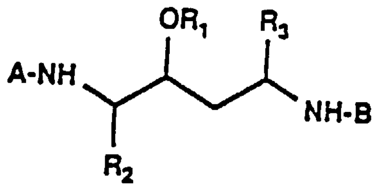

dans laquelle $R_1$ est un atome d'hydrogène, $R_2$ et $R_3$ sont choisis indépendamment parmi les groupes arylalkyle; et A est $R_6$-C(O)-(NH)-(CH($R_{5a}$))-C(O)- où $R_{5a}$ est un groupe alkyle et $R_6$ est $R_7$-$R_9$- où $R_7$ est un groupe hétérocyclyle ou (hétérocyclyl)alkyle éventuellement substitué sur le noyau hétérocyclique, et $R_9$ est -N($R_{7a}$)-, S ou O, où $R_{7a}$ est un atome d'hydrogène ou un groupe alkyle, et B est -C(O)-$R_{6'}$ où $R_{6'}$ est $R_{7'}$-$R_{9'}$ où $R_{7'}$ est un groupe hétérocyclyle ou (hétérocyclyl)alkyle éventuellement substitué sur le noyau hétérocyclique et $R_{9'}$ est -N($R_{7a'}$)-, S ou O, où $R_{7a'}$ est un atome d'hydrogène ou un groupe alkyle; ou d'un de ses sels, esters ou promédicaments pharmaceutiquement acceptables.

**3.** Procédé selon la revendication 1, pour la préparation d'un composé de formule:

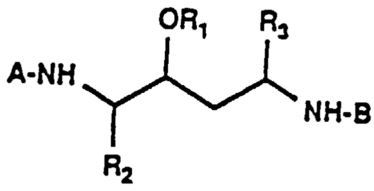

dans laquelle $R_1$ est un atome d'hydrogène, $R_2$ et $R_3$ sont choisis indépendamment parmi les groupes arylaikyle; et A est -C(O)-$R_{6'}$ où $R_{6'}$ est $R_{7'}$-$R_{9'}$ où $R_{7'}$ est un groupe hétérocyclyle ou (hétérocyclyl)alkyle éventuellement substitué sur le noyau hétérocyclique et $R_{9'}$ est -N($R_{7a'}$)-, S ou O, où $R_{7a'}$ est un atome d'hydrogène ou un groupe alkyle; et B est $R_6$-C(O)-(NH)-(CH($R_{5a}$))-C(O)- où $R_{5a}$ est un groupe alkyle et $R_6$ est $R_7$-$R_9$- où $R_7$ est un groupe hétérocyclyle ou (hétérocyclyl)alkyle éventuellement substitué sur le noyau hétérocyclique, et $R_9$ est -N($R_{7a}$)-, S ou O, où $R_{7a}$ est un atome d'hydrogène ou un groupe alkyle, ou d'un de ses sels, esters ou promédicaments pharmaceutiquement acceptables.

**4.** Procédé selon la revendication 1 pour la préparation d'un composé de formule:

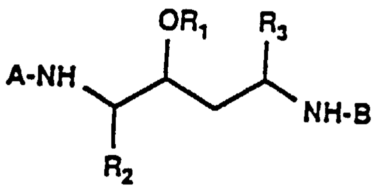

dans laquelle $R_1$ est un atome d'hydrogène, $R_2$ et $R_3$ sont choisis indépendamment parmi les groupes arylalkyle; et A et B sont indépendamment -C(O)-$R_6$ où $R_6$ est $R_7$-$R_9$ où $R_7$ est à chaque fois choisi indépendamment parmi les groupes hétérocyclyle et (hétérocyclyl)alkyle éventuellement substitués sur le noyau hétérocyclique et $R_9$ est à chaque fois choisi indépendamment parmi -N($R_{7a}$)-, S et O, où $R_{7a}$ est un atome d'hydrogène ou un groupe alkyle; ou d'un de ses sels, esters ou promédicaments pharmaceutiquement acceptables.

**5.** Procédé selon la revendication 1 pour la préparation d'un composé qui est le (2S,3S,5S)-2-(N-(N-((2-pyridinyl)méthoxycarbonyl)valinyl)amino)-5-(N-((3-pyridinyl)méthoxycarbonyl)amino)-1,6-diphényl-3-hydroxyhexane; ou d'un de ses sels, esters ou promédicaments pharmaceutiquement acceptables.

6. Procédé selon la revendication 1 pour la préparation d'un composé qui est le (2S,3S,5S)-2-(N-(N-((2-pyridinyl)méthoxycarbonyl)valinyl)amino)-5-(N-((3-pyridinyl)méthoxycarbonyl)amino)-1,6-diphényl-3-hydroxyhexane; ou d'un de ses sels pharmaceutiquement acceptables.

7. Procédé selon la revendication 1 pour la préparation d'un composé choisi dans le groupe constitué par:

le (2S,3S,5S)-5-(N-(N-((N-méthyl-N-((2-pyridinyl)méthyl)amino)carbonyl)valinyl)amino)-2-(N-((3-pyridinyl)méthoxycarbonyl)amino)-1,6-diphényl-3-hydroxyhexane;
le (2S,3S,5S)-2-(N-((3-pyridinyl)méthoxycarbonyl)amino-5-(N-(N-((N-méthyl-N-((6-méthyl-2-pyridinyl)méthyl)amino)carbonyl)valinyl)amino)-1,6-diphényl-3-hydroxyhexane;
le (2S,3S,5S)-2-(N-(N-((N-méthyl-N-((2-pyridinyl)méthyl)amino)carbonyl)valinyl)amino)-S-(N-((3-pyridinyl)méthoxycarbonyl)amino)-1,6-diphényl-3-hydroxyhexane;
le (2S,3S,5S)-5-(N-(N-((2-pyridinyl)méthoxycarbonyl)valinyl)amino)-2-(N-((3-pyridinyl)méthoxycarbonyl)amino)-1,6-diphényl-3-hydroxyhexane;
le (2S,3S,5S)-5-(N-(N-((N-méthyl-N-((2-pyridinyl)méthyl)amino)carbonyl)isoleucinyl)amino)-2-(N-((3-pyridinyl)méthoxycarbonyl)amino)-1,6-diphényl-3-hydroxyhexane;
le (2S,3S,5S)-2,5-di(N-(3-pyridylméthyl)oxycarbonyl)amino)-3-hydroxy-1,6-diphénylhexane;
le (2S,3S,5S)-2-(N-(N-((N-méthyl-N-((6-méthyl-2-pyridinyl)méthyl)amino)carbonyl)valinyl)amino)-5-(N-((3-pyridinyl)méthoxycarbonyl)amino)-1,6-diphényl-3-hydroxyhexane;
le (2S,3S,5S)-2-(N-[(pyridin-3-yl)méthoxycarbonyl]amino)-5-(N-[(6-méthylpyridin-2-yl)méthoxycarbonyl)valyl]amino)-1,6-diphényl-3-hydroxyhexane;
le (2S,3S,5S)-5-(N-(N-((N-méthyl-N-((2-méthyl-4-thiazolyl)méthyl)amino)carbonyl)valinyl)amino)-2-(N-((3-pyridinyl)méthoxycarbonyl)amino)-1,6-diphényl-3-hydroxyhexane;
le (2S,3S,5S)-2-(N-((3-pyridinyl)méthoxycarbonyl)amino-5-(N-(N-((N-méthyl-N-((6-méthoxy-3-pyridinyl)méthyl)amino)carbonyl)valinyl)amino)-1,6-diphényl-3-hydroxyhexane; et
le (2S,3S,5S,1'S,2'S)-2-(N-(2-(N-((3-pyridinyl)méthoxycarbonyl)amino-1-cyclopentyl)oxycarbonyl)amino)-5-(N-((3-pyridinyl)méthoxycarbonyl)amino)-1,6-diphényl-3-hydroxyhexane;
ou d'un de ses sels, esters ou promédicaments pharmaceutiquement acceptables.

8. Composé de formule:

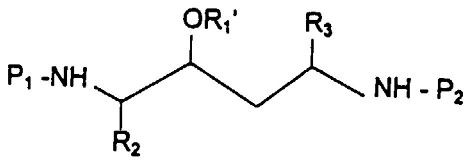

dans laquelle

$P_1$ et $P_2$ sont choisis indépendamment parmi un atome d'hydrogène et un groupe protecteur de N; $R_1$' est un atome d'hydrogène ou un groupe protecteur de O; et $R_2$ et $R_3$ sont choisis indépendamment parmi les groupes arylalkyle, cycloalkylalkyle et (hétérocyclyl)alkyle;
le groupe alkyle en tant qu'élément d'un autre groupe est un radical alkyle linéaire ou ramifié contenant de 1 à 6 atomes de carbone;
le groupe cycloalkyle en tant qu'élément du groupe cycloalkylalkyle est un cycle aliphatique de 3 à 7 atomes de carbone;
le groupe aryle en tant qu'élément du groupe arylalkyle est un groupe aryle monocyclique en $C_6$ ou un système cyclique carbocyclique bicyclique en $C_9$ ou $C_{10}$ ayant un ou plusieurs cycles aromatiques;
le groupe hétérocyclyle en tant qu'élément du groupe (hétérocyclyl)alkyle est choisi parmi les groupes pyrrolyle, pyrrolinyle, pyrrolidinyle, pyrazolyle, pyrazolinyle, pyrazolidinyle, imidazolyle, imidazolinyle, imidazolidinyle, pyridyle, pipéridinyle, pyrazinyle, pipérazinyle, pyrimidinyle, pyridazinyle, oxazolyle, oxazolinyle, oxazolidinyle, isoxazolyle, isoxazolinyle, isoxazolidinyle, morpholinyle, thiazolyle, thiazolidinyle, isothiazolyle, isothiazolidinyle, indolyle, quinolinyle, tétrahydroquinolyle, isoquinolinyle, benzimidazolyle, benzothiazolyle, benzoxazolyle, benzofuranyle, furyle, dihydrofuranyle, tétrahydrofuranyle, pyranyle, dihydropyranyle, tétrahydropyranyle, dioxanyle, dioxolanyle, thiényle and benzothiényle;
ou un de ses sels.

9. Composé selon la revendication 8, de formule:

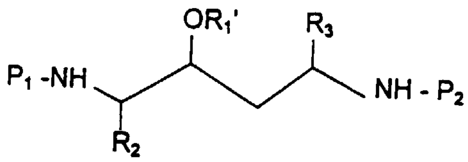

dans laquelle $P_1$ et $P_2$ sont choisis indépendamment parmi un atome d'hydrogène et un groupe protecteur de N; $R_1$' est un atome d'hydrogène ou un groupe protecteur de O; et $R_2$ et $R_3$ sont choisis indépendamment parmi les groupes arylalkyle; ou un de ses sels.

10. Composé selon la revendication 8, de formule:

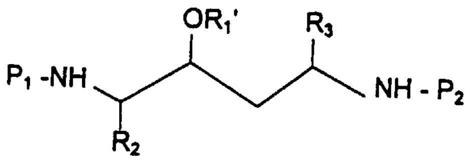

dans laquelle $P_1$ et $P_2$ sont choisis indépendamment parmi un atome d'hydrogène et un groupe protecteur de N; $R_1$' est un atome d'hydrogène ou un groupe protecteur de O; et $R_2$ et $R_3$ sont choisis indépendamment panni les groupes arylalkyle; ou un de ses sels; où l'atome de carbone portant le substituant $R_2$ a la configuration "S", l'atome portant le substituant $OR_1$' a la configuration "S" et l'atome de carbone portant le substituant $R_3$ a la configuration "S".

11. Composé selon la revendication 8, de formule:

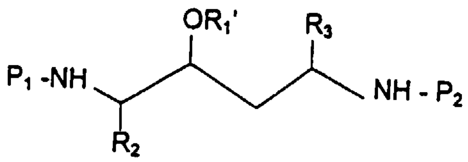

dans laquelle $P_1$ et $P_2$ sont choisis indépendamment parmi un atome d'hydrogène et un groupe protecteur de N; $R_1$' est un atome d'hydrogène ou un groupe protecteur de O; et $R_2$ et $R_3$ sont des groupes benzyle; ou un de ses sels; où l'atome de carbone portant le substituant $R_2$ a la configuration "S", l'atome portant le substituant $OR_1$' a la configuration "S" et l'atome de carbone portant le substituant $R_3$ a la configuration "S".

12. Composé selon la revendication 8, qui est le (2S,3S,5S)-2,5-diamino-1,6-diphényl-3-hydroxyhexane; ou un de ses sels.

13. Composé selon la revendication 11, qui est le (2S,3S,5S)-5-amino-2-(N-((t-butyl)oxy)carbonyl)amino)-1,6-diphényl-3-hydroxyhexane; ou un de ses sels.

14. Composé selon la revendication 11, qui est le (2S,3S,5S)-2-amino-5-(N-((t-butyl)oxy)carbonyl)amino)-1,6-diphényl-3-hydroxyhexane; ou un de ses sels.

15. Utilisation d'un composé de formule:

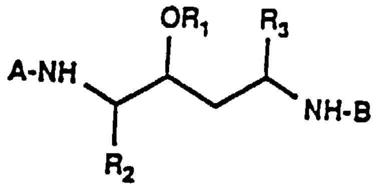

dans laquelle

$R_1$ est un atome d'hydrogène et $R_2$ et $R_3$ sont choisis indépendamment parmi les groupes arylalkyle, cycloalkylalkyle et (hétérocyclyl)alkyle;
A et B sont choisis indépendamment parmi $R_6$-C(O)-(NH)-(CH($R_5$))-C(O)- et $R_6$-C(O)-, dans lesquels $R_5$ est choisi indépendamment à chaque fois parmi les groupes alkyle et $R_6$ est choisi indépendamment à chaque fois parmi les groupes $R_7$-NH-, $R_7$-N(alkyle)-, $R_7$-O- et $R_7$-S- dans lesquels $R_7$ est un groupe hétérocyclyle ou (hétérocyclyl)alkyle et $R_7$ peut être éventuellement substitué sur le noyau hétérocyclique par un ou deux substituants choisis indépendamment parmi les groupes halogéno, amino, amino N-protégé, alcoxy, polyalcoxy et alkyle;
le groupe alkyle en lui-même ou en tant qu'élément d'un autre groupe est un radical alkyle linéaire ou ramifié contenant de 1 à 6 atomes de carbone;
le groupe cycloalkyle en tant qu'élément du groupe cycloalkylalkyle est un cycle aliphatique de 3 à 7 atomes de carbone;
le groupe aryle en tant qu'élément du groupe arylalkyle est un groupe aryle monocyclique en $C_6$ ou un système cyclique carbocyclique bicyclique en $C_9$ ou $C_{10}$ ayant un ou plusieurs cycles aromatiques;
le groupe hétérocyclyle en lui-même ou en tant qu'élément d'un autre groupe est choisi parmi les groupes pyrrolyle, pyrrolinyle, pyrrolidinyle, pyrazolyle, pyrazolinyle, pyrazolidinyle, imidazolyle, imidazolinyle, imidazolidinyle, pyridyle, pipéridinyle, pyrazinyle, pipérazinyle, pyrimidinyle, pyridazinyle, oxazolyle, oxazolinyle, oxazolidinyle, isoxazolyle, isoxazolinyle, isoxazolidinyle, morpholinyle, thiazolyle, thiazolidinyle, isothiazolyle, isothiazolidinyle, indolyle, quinolinyle, tétrahydroquinolyle, isoquinolinyle, benzimidazolyle, benzothiazolyle, benzoxazolyle, benzofuranyle, fuiyle, dihydrofuranyle, tétrahydrofuranyle, pyranyle, dihydropyranyle, tétrahydropyranyle, dioxanyle, dioxolanyle, thiényle and benzothiényle;
ou d'un de ses sels, esters ou promédicaments pharmaceutiquement acceptables, pour la production d'un médicament destiné à inhiber la protéase de VIH chez l'homme.

**16.** Utilisation d'un composé de formule:

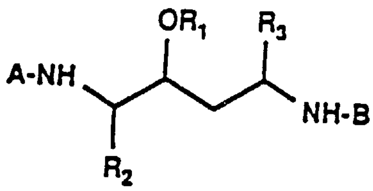

dans laquelle

$R_1$ est un atome d'hydrogène et $R_2$ et $R_3$ sont choisis indépendamment parmi les groupes arylalkyle, cycloalkylalkyle et (hétérocyclyl)alkyle;
A et B sont choisis indépendamment parmi $R_6$-C(O)-(NH)-(CH($R_5$))-C(O)- et $R_6$-C(O)-, dans lesquels $R_5$ est choisi indépendamment à chaque fois parmi les groupes alkyle et $R_6$ est choisi indépendamment à chaque fois parmi les groupes $R_7$-NH-, $R_7$-N(alkyle)-, $R_7$-O- et $R_7$-S- dans lesquels $R_7$ est un groupe hétérocyclyle ou (hétérocyclyl)alkyle et $R_7$ peut être éventuellement substitué sur le noyau hétérocyclique par un ou deux substituants choisis indépendamment parmi les groupes halogéno, amino, amino N-protégé, alcoxy, polyalcoxy et alkyle;
le groupe alkyle en lui-même ou en tant qu'élément d'un autre groupe est un radical alkyle linéaire ou ramifié

contenant de 1 à 6 atomes de carbone;

le groupe cycloalkyle en tant qu'élément du groupe cycloalkylalkyle est un cycle aliphatique de 3 à 7 atomes de carbone;

le groupe aryle en tant qu'élément du groupe arylalkyle est un groupe aryle monocyclique en $C_6$ ou un système cyclique carbocyclique bicyclique en $C_9$ ou $C_{10}$ ayant un ou plusieurs cycles aromatiques;

le groupe hétérocyclyle en lui-même ou en tant qu'élément d'un autre groupe est choisi parmi les groupes pyrrolyle, pyrrolinyle, pyrrolidinyle, pyrazolyle, pyrazolinyle, pyrazolidinyle, imidazolyle, imidazolinyle, imidazolidinyle, pyridyle, pipéridinyle, pyrazinyle, pipérazinyle, pyrimidinyle, pyridazinyle, oxazolyle, oxazolinyle, oxazolidinyle, isoxazolyle, isoxazolinyle, isoxazolidinyle, morpholinyle, thiazolyle, thiazolidinyle, isothiazolyle, isothiazolidinyle, indolyle, quinolinyle, tétrahydroquinolyle, isoquinolinyle, benzimidazolyle, benzothiazolyle, benzoxazolyle, benzofuranyle, furyle, dihydrofuranyle, tétrahydrofuranyle, pyranyle, dihydropyranyle, tétrahydropyranyle, dioxanyle, dioxolanyle, thiényle and benzothiényle;

ou d'un de ses sels, esters ou promédicaments pharmaceutiquement acceptables, pour la production d'un médicament destiné au traitement d'une infection à VIH chez l'homme.

**17.** Utilisation selon la revendication 15 ou 16 d'un composé de formule:

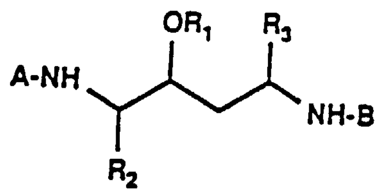

dans laquelle $R_1$ est un atome d'hydrogène, $R_2$ et $R_3$ sont choisis indépendamment parmi les groupes arylalkyle; et A est $R_6$-C(O)-(NH)-(CH($R_{5a}$))-C(O)- où $R_{5a}$ est un groupe alkyle et $R_6$ est $R_7$-$R_9$- où $R_7$ est un groupe hétérocyclyle ou (hétérocyclyl)alkyle éventuellement substitué sur le noyau hétérocyclique, et $R_9$ est -N($R_{7a}$)-, S ou O, où $R_{7a}$ est un atome d'hydrogène ou un groupe alkyle, et B est -C(O)-$R_{6'}$ où $R_{6'}$ est $R_{7'}$-$R_{9'}$ où $R_{7'}$ est un groupe hétérocyclyle ou (hétérocyclyl)alkyle éventuellement substitué sur le noyau hétérocyclique et $R_{9'}$ est -N($R_{7a'}$)-, S ou O, où $R_{7a'}$ est un atome d'hydrogène ou un groupe alkyle; ou d'un de ses sels, esters ou promédicaments pharmaceutiquement acceptables.

**18.** Utilisation selon la revendication 15 ou 16 d'un composé de formule:

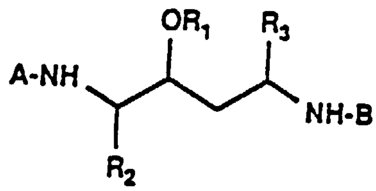

dans laquelle $R_1$ est un atome d'hydrogène, $R_2$ et $R_3$ sont choisis indépendamment parmi les groupes arylalkyle; et A est -C(O)-$R_{6'}$ où $R_{6'}$ est $R_{7'}$-$R_{9'}$ où $R_{7'}$ est un groupe hétérocyclyle ou (hétérocyclyl)alkyle éventuellement substitué sur le noyau hétérocyclique et $R_{9'}$ est -N($R_{7a'}$)-, S ou O, où $R_{7a'}$ est un atome d'hydrogène ou un groupe alkyle; et B est $R_6$-C(O)-(NH)-(CH($R_{5a}$))-C(O)- où $R_{5a}$ est un groupe alkyle et $R_6$ est $R_7$-$R_9$- où $R_7$ est un groupe hétérocyclyle ou (hétérocyclyl)alkyle éventuellement substitué sur le noyau hétérocyclique, et $R_9$ est -N($R_{7a}$)-, S ou O, où $R_{7a}$ est un atome d'hydrogène ou un groupe alkyle, ou d'un de ses sels, esters ou promédicaments pharmaceutiquement acceptables.

**19.** Utilisation selon la revendication 15 ou 16 d'un composé de formule:

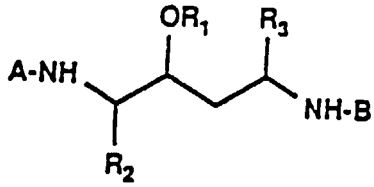

dans laquelle $R_1$ est un atome d'hydrogène, $R_2$ et $R_3$ sont choisis indépendamment parmi les groupes arylalkyle; et A et B sont indépendamment -C(O)-$R_6$ où $R_6$ est $R_7$-$R_9$ où $R_7$ est à chaque fois choisi indépendamment parmi les groupes hétérocyclyle et (hétérocyclyl)alkyle éventuellement substitués sur le noyau hétérocyclique et $R_9$ est à chaque fois choisi indépendamment parmi -N($R_{7a}$)-, S et O, où $R_{7a}$ est un atome d'hydrogène ou un groupe alkyle; ou d'un de ses sels, esters ou promédicaments pharmaceutiquement acceptables.

**20.** Utilisation selon la revendication 15 ou 16 d'un composé qui est le (2S,3S,5S)-2-(N-(N-((2-pyridinyl)méthoxycarbonyl)valinyl)amino)-5-(N-((3-pyridinyl)méthoxycarbonyl)amino)-1,6-diphényl-3-hydroxyhexane; ou d'un de ses sels, esters ou promédicaments pharmaceutiquement acceptables.

**21.** Utilisation selon la revendication 15 ou 16 d'un composé qui est le (2S,3S,5S)-2-(N-(N-((2-pyridinyl)méthoxycarbonyl)valinyl)amino)-5-(N-((3-pyridinyl)méthoxycarbonyl)amino)-1,6-diphényl-3-hydroxyhexane; ou d'un de ses sels pharmaceutiquement acceptables.

**22.** Utilisation selon la revendication 15 ou 16 d'un composé choisi dans le groupe constitué par:

le (2S,3S,5S)-5-(N-(N-((N-méthyl-N-((2-pyridinyl)méthyl)amino)carbonyl)valinyl)amino)-2-(N-((3-pyridinyl)méthoxycarbonyl)amino)1,6-diphényl-3-hydroxyhexane;
le (2S,3S,5S)-2-(N-((3-pyridinyl)méthoxycarbonyl)amino-5-(N-(N-((N-méthyl-N-((6-méthyl-2-pyridinyl)méthyl)amino)carbonyl)valinyl)amino)-1,6-diphényl-3-hydroxyhexane;
le (2S,3S,5S)-2-(N-(N-((N-méthyl-N-((2-pyridinyl)méthyl)amino)carbonyl)valinyl)amino)-S-(N-((3-pyridinyl)méthoxycarbonyl)amino)-1,6-diphényl-3-hydroxyhexane;
le (2S,3S,5S)-5-(N-(N-((2-pyridinyl)méthoxycarbonyl)valinyl)amino)-2-(N-((3-pyridinyl)méthoxycarbonyl)amino)-1,6-diphényl-3-hydroxyhexane;
le (2S,3S,5S)-5-(N-(N-((N-méthyl-N-((2-pyridinyl)méthyl)amino)carbonyl)isoleucinyl)amino)-2-(N-((3-pyridinyl)méthoxycarbonyl)amino)-1,6-diphényl-3-hydroxyhexane;
le (2S,3S,5S)-2,5-di(N-(3-pyridylméthyl)oxycarbonyl)amino)-3-hydroxy-1,6-diphénylhexane;
le (2S,3S,5S)-2-(N-(N-((N-méthyl-N-((6-méthyl-2-pyridinyl)méthyl)amino)carbonyl)valinyl)amino)-5-(N-((3-pyridinyl)méthoxycarbonyl)amino)-1,6-diphényl-3-hydroxyhexane;
le (2S,3S,5S)-2-(N-[(pyridin-3-yl)méthoxycarbonyl]amino)-5-(N-[(6-méthylpyridin-2-yl)méthoxycarbonyl)valyl]amino)-1,6-diphényl-3-hydroxyhexane;
le (2S,3S,5S)-5-(N-(N-((N-méthyl-N-((2-méthyl-4-thiazolyl)méthyl)amino)carbonyl)valinyl)amino)-2-(N-((3-pyridinyl)méthoxycarbonyl)amino)-1,6-diphényl-3-hydroxyhexane;
le (2S,3S,5S)-2-(N-((3-pyridinyl)méthoxycarbonyl)amino-5-(N-(N-((N-méthyl-N-((6-méthoxy-3-pyridinyl)méthyl)amino)carbonyl)valinyl)amino)-1,6-diphényl-3-hydroxyhexane; et
le (2S,3S,5S,1'S,2'S)-2-(N-(2-(N-((3-pyridinyl)méthoxycarbonyl)amino-1-cyclopentyl)oxycarbonyl)amino)-5-(N-((3-pyridinyl)méthoxycarbonyl)amino)-1,6-diphényl-3-hydroxyhexane;
ou d'un de ses sels, esters ou promédicaments pharmaceutiquement acceptables.